(19) Europäisches Patentamt / European Patent Office / Office européen des brevets

(11) EP 3 685 857 A1

(12) EUROPEAN PATENT APPLICATION

(43) Date of publication:
**29.07.2020 Bulletin 2020/31**

(21) Application number: **20158832.4**

(22) Date of filing: **26.01.2015**

(51) Int Cl.:
***A61K 39/395*** *(2006.01)* ***G01N 33/573*** *(2006.01)*
***G01N 33/53*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.01.2014 US 201461931878 P**
**09.05.2014 US 201461990932 P**

(62) Document number(s) of the earlier application(s) in accordance with Art. 76 EPC:
**15740660.4 / 3 099 323**

(71) Applicant: **MedImmune, LLC**
**Gaithersburg, MD 20878 (US)**

(72) Inventors:
- **STREICHER, Katie**
**Gaithersburg, MD 20878 (US)**
- **YAO, Yihong**
**Gaithersburg, MD 20878 (US)**
- **RANADE, Koustubh**
**Gaithersburg, MD 20878 (US)**
- **LIANG, Meina**
**Gaithersburg, MD 20878 (US)**
- **VAINSHTEIN, Inna**
**Gaithersburg, MD 20878 (US)**
- **PIPER, Edward**
**Cambridge, Cambridgeshire CB21 6GH (GB)**
- **MAY, Richard**
**Cambridge, Cambridgeshire CB21 6GH (GB)**
- **NORDENMARK, Lars**
**S-431 83 Molndal (SE)**

(74) Representative: **Mewburn Ellis LLP**
**Aurora Building**
**Counterslip**
**Bristol BS1 6BX (GB)**

Remarks:
This application was filed on 21.02.2020 as a divisional application to the application mentioned under INID code 62.

# (54) DIPEPTIDYL PEPTIDASE-4 (DPP4/CD26) AS A PERIPHERAL BIOMARKER OF IL-13 ACTIVATION IN ASTHMATIC LUNG

(57) The present disclosure relates to the use of DPP4, protein or gene expression levels, as a biomarker for IL-13 mediated diseases or disorders, e.g., asthma, IPF, COPD or atopic dermatitis. Levels of the DPP4 biomarker above or below a predetermined DPP4 threshold level can be used (i) to determine a patient's eligibility for a certain treatment with a IL-13 antagonist, (ii) to determine whether a certain treatment of an IL-13 mediated condition or disorder with a specific IL-13 antagonist should commence, be suspended, or be modified, (iii) to diagnose whether an IL-13 mediated condition or disorder is treatable or not treatable with a specific IL-13 antagonist, (iv) to prognosticate the outcome of treating an IL-13 mediated condition or disorder with a specific IL-13 antagonist. The disclosure further provides assay kits for the detection of DPP4, as well as computer implemented diagnostic methods.



Printed by Jouve, 75001 PARIS (FR)

## Description

REFERENCE TO SEQUENCE LISTING SUBMITTED ELECTRONICALLY

The content of the electronically submitted sequence listing in ASCII text file (Name: IL13-400WO_Sequence_Listing_ascii.txt; Size: 174,694 bytes; and Date of Creation: January 15, 2015) filed with the application is incorporated herein by reference in its entirety.

BACKGROUND

Bronchial asthma is a common persistent inflammatory disease of the lung characterized by airways hyper-responsiveness, mucus overproduction, fibrosis, and raised serum IgE levels. Airways hyper-responsiveness (AHR) is the exaggerated constriction of the airways to non-specific stimuli such as cold air. Both AHR and mucus overproduction are thought to be responsible for the variable airway obstruction that leads to the shortness of breath characteristic of asthma attacks (exacerbations) and which is responsible for the mortality associated with this disease.

Current British Thoracic Society (BTS) and Global Initiative for Asthma (GINA) guidelines suggest a stepwise approach to the treatment of asthma (Society, B. T., Thorax, 2003. 58 Suppl 1:1-94; GINA, Global Strategy for Asthma Management and Prevention. 2002, National Institute of Health). Mild to moderate asthma can usually be controlled by the use of inhaled corticosteroids, in combination with beta-agonists or leukotriene inhibitors. However, due to the documented side effects of corticosteroids, patients tend not to comply with the treatment regime, which reduces the effectiveness of treatment (Milgrom, H. et al. Ann Allergy Asthma Immunol, 2002. 88:429-31; Fish, L. and C. L. Lung, Ann Allergy Asthma Immunol, 2001. 86:24-30; Bender, B. G. J. Allergy Clin. Immunol, 2002. 109:S554-9). Asthma presents significant heterogeneity in response to various treatments, thereby highlighting the need to develop more effective therapies for this disease or identify biomarkers that predict response to specific therapies.

Atopic dermatitis is a common chronic inflammatory skin disease that is often associated with other atopic disorders such as allergic rhinitis and asthma (Bieber, New England Journal of Medicine, 2008, 358: 1483-1494). Upregulation of IL-13 mRNA has been observed in subacute and chronic lesions of atopic dermatitis (Tazawa et al., Arch. Dermatol. Res., 2004, 295:459-464; Purwar et al,, J. Invest. Derm., 2006, 126, 1043-1051; Oh et al., J Immunol., 2011, 186:7232-42).

Chronic Obstructive Pulmonary Disease (COPD) includes patient populations with varying degrees of chronic bronchitis, small airway disease and emphysema and is characterized by progressive irreversible lung function decline that responds poorly to current asthma based therapy. The underlying causes of COPD remain poorly understood. Zheng et al (J Clin Invest, 106: 1081-93, 2000) have demonstrated that overexpression of IL-13 in the mouse lung caused emphysema, elevated mucus production and inflammation, reflecting aspects of human COPD. Furthermore, AHR, an IL-13 dependent response in murine models of allergic inflammation, has been shown to be predictive of lung function decline in smokers (Tashkin et al., Am J Respir Crit Care Med, 153(6 Pt 1): 1802-1 1, 1996). A link has also been established between an IL-13 promoter polymorphism and susceptibility to develop COPD (Van Der Pouw Kraan et al., Genes Immun. 3: 436-9, 2002). The signs are therefore that IL-4/IL-13 pathway, and in particular IL-13, plays an important role in the pathogenesis of COPD. *See, e.g.,* Chen et al. PLoS One 8:e68222, 2013; Dente et al., Respiration 84:98-100, 2012; Grubek-Jaworska et al., Respiration 84:101-107, 2012; Walsh, Curr. Opin. Investig. Drugs 11:1305-12, 2010, all of which are herein incorporated by reference in their entireties.

Interleukin (IL)-13 is a 114 amino acid cytokine with an unmodified molecular mass of approximately 12 kDa (McKenzie, A. N., et al. J Immunol, 1993. 150:5436-44; Minty, A., et al. Nature, 1993. 362:248-50). IL-13 levels have been shown to correlate with disease severity in asthmatics and rodent models of allergic inflammation (*see* U.S. Pat. Appl. Publ. No. 2012-0052060, published March 1, 2012, and incorporated herein by reference in its entirety). IL-13 may also play a role in the pathogenesis of inflammatory bowel disease, and has been associated with fibrotic conditions, such as idiopathic pulmonary fibrosis (IPF). Anti-IL-13 antibodies are currently been developed as therapies for treatment of patients with moderate to severe asthma. However, only a subset of asthma patients appear to have IL-13 driven disease. Thus, there is a need to identify such patients and predict the outcome of the treatment with IL-13 antagonists such as anti-IL-13 antibodies using a simple biomarker or combination of biomarkers.

BRIEF SUMMARY

The present disclosure provides a method of treating a patient having an interleukin-13 (IL-13)-mediated disease or disorder, comprising administering an IL-13 antagonist to the patient if the level of DPP4 (dipeptidyl peptidase-4) in a sample taken from the patient is above a predetermined DPP4 threshold level, or is above the DPP4 level in one or more control samples. Also provided is a method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents (i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ 0.14 × $10^9$/L), (iv) FEV1 reversibility to a short-acting β2 agonist ≥ 12%, (v) wall area % (WA%) of subsegmental airways above about 68% as measured via CT scan of the lungs, or (vi) combinations thereof.

In addition, the present disclosure provides a method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents high periostin (≥ median serum periostin or about 23 ng/mL). Also provided is a method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents a high eosinophil cell count (blood eosinophil count ≥ 300 cells/μL). Also provided is a method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents with high Th2 (high Th2 defined as IgE > 100 IU/mL and blood eosinophils ≥ 0.14 × $10^9$/L). Also provided is a method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents with FEV1 reversibility to a short-acting β2 agonist ≥ 12%. Also provided is a method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents with one or more of: i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ 0.14 × $10^9$/L), (iv) FEV1 reversibility to a short-acting β2 agonist ≥ 12% and (v) wall area % (WA%) of subsegmental airways above about 68% as measured via CT scan of the lungs.

In addition, the present disclosure provides a method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents a level of at least one additional biomarker in a sample taken from the patient which is above a predetermined biomarker threshold level, or is above the biomarker level in one or more control samples, wherein said additional biomarker is selection from the group consisting of POSTN (SEQ ID NO:8), CSTI (SEQ ID NO:9), CCL26 (SEQ ID NO:10), CLCAI (SEQ ID NO:11), CST2 (SEQ ID NO:12), PRR4 (SEQ ID NO:13), SERPINB2 (SEQ ID NO:14), CEACAM5 (SEQ ID NO:15), iNOS (SEQ ID NO:16), SERPINB4 (SEQ ID NO:17), CST4 (SEQ ID NO:18), PRB4 (SEQ ID NO:19), TPSDI (SEQ ID NO:20), TPSGI (SEQ ID NO: 21), MFSD2 (SEQ ID NO:22), CPA3 (SEQ ID NO:23), GPR105 (SEQ ID NO:24), CDH26 (SEQ ID NO:25), GSN (SEQ ID NO:26), C2ORF32 (SEQ ID NO:27), TRACH2000196 (TMEM71) (SEQ ID NO:28), DNAJC12 (SEQ ID NO:29), RGS13 (SEQ ID NO: 30), SLC18A2 (SEQ ID NO: 31), SERPINBI0 (SEQ ID NO:32), SH3RF2 (SEQ ID NO:33), FCERIB (SEQ ID NO:34), RUNX2 (SEQ ID NO:35), PTGSI (SEQ ID NO:36), ALOX15 (SEQ ID NO:37), and combinations thereof.

In some aspects of the methods disclosed herein, a sample is obtained from the patient and submitted for measurement of the level of DPP4 in the sample. In other aspects, the patient's DPP4 level is measured in an immunoassay. In some aspects, the immunoassay employs one or more anti-DPP4 antibodies or antigen binding fragments thereof which recognize human DDP4.

Also provided is a method of treating a patient having an IL-13-mediated disease or disorder comprising (a) submitting a sample taken from the patient for measurement of the DPP4 level in the sample, wherein the patient's DPP4 level is measured in an immunoassay employing one or more anti-DPP4 antibodies or antigen binding fragments thereof which recognize human DPP4; and, (b) administering an IL-13 antagonist to the patient if the patient's DPP4 level in the sample is above a predetermined DPP4 threshold level, or is above the DPP4 level in one or more control samples. Also provided is a method of treating a patient having an IL-13-mediated disease or disorder comprising (a) measuring the DPP4 level in a sample obtained from a patient having an IL-13-mediated disease or disorder, wherein the patient's DPP4 level in the sample is measured in an immunoassay employing one or more anti-DPP4 antibodies or antigen binding fragments thereof which recognize human DPP4; (b) determining whether the patient's DPP4 level in the sample is above a predetermined DPP4 threshold level, or is above the DPP4 level in one or more control samples; and, (c) advising a healthcare provider to administer an IL-13 antagonist to the patient if the patient's DPP4 level is above a predetermined DPP4 threshold level, or is above the DPP4 level in one or more control samples.

In some aspects of the above disclosed methods, the IL-13-mediated disease or disorder is a pulmonary disease or disorder, an inflammatory bowel disease or disorder, or a chronic inflammatory skin disease or disorder. In other aspects the pulmonary disease or disorder is asthma or allergic rhinitis. In some aspects the chronic inflammatory skin disease or disorder is atopic dermatitis. In some aspects the pulmonary disease or disorder is COPD. In some aspects, COPD is stable COPD or acute exacerbation of COPD (AECOPD).

The present disclosure also provides a method of treating a patient diagnosed with a pulmonary disease or disorder comprising administering an IL-13 antagonist to the patient if the DPP4 level in a sample taken from the patient is above a predetermined DPP4 threshold level, or is above the DPP4 level in one or more control samples. In some aspects, the patient's DPP4 level is measured in an immunoassay employing one or more anti-DPP4 antibodies or antigen binding fragments thereof which recognize human DPP4.

Also provided is a method of treating a patient diagnosed with a pulmonary disease or disorder, an inflammatory bowel disease or disorder, or a chronic inflammatory skin disease or disorder comprising (a) submitting a sample taken from the patient for measurement of the DPP4 level in the sample, wherein the patient's DPP4 level is measured in an immunoassay employing one or more anti-DPP4 antibodies or antigen binding fragments thereof which recognize human DPP4; and (b) administering an IL-13 antagonist to a patient if the patient's DPP4 level in the sample is above a predetermined DPP4 threshold level, or is above the DPP4 level in one or more control samples.

Also provided is a method of determining whether to treat a patient diagnosed with a pulmonary disease or disorder, an inflammatory bowel disease or disorder, or a chronic inflammatory skin disease or disorder with an IL-13 antagonist therapeutic regimen comprising (a) measuring, or instructing a clinical laboratory to measure the DPP4 level in a sample obtained from a patient diagnosed with a pulmonary disease or disorder, an inflammatory bowel disease or disorder, or a chronic inflammatory skin disease or disorder, wherein the patient's DPP4 level is measured in an immunoassay employing one or more anti-DPP4 antibodies or antigen binding fragments thereof which recognize human DPP4; and (b) treating, or instructing a healthcare provider to treat the patient with an IL-13 antagonist therapeutic regimen if the patient's DPP4 level in the sample is above a predetermined DPP4 threshold level, or is above the DPP4 level in one or more control samples.

Also provided is a method of selecting a patient diagnosed with a pulmonary disease or disorder, an inflammatory bowel disease or disorder, or a chronic inflammatory skin disease or disorder as a candidate for treatment with an IL-13 antagonist therapeutic regimen comprising (a) measuring, or instructing a clinical laboratory to measure the DPP4 level in a sample obtained from a patient diagnosed with a pulmonary disease or disorder, an inflammatory bowel disease or disorder, or a chronic inflammatory skin disease or disorder, wherein the patient's DPP4 level is measured in an immunoassay employing one or more anti-DPP4 antibodies or antigen binding fragments thereof which recognize human DPP4; and (b) treating, or instructing a healthcare provider to treat the patient with an IL-13 antagonist therapeutic regimen if the patient's DPP4 level in the sample is above a predetermined DPP4 threshold level, or is above the DPP4 level in one or more control samples. In some aspects, the pulmonary disease or disorder is asthma, IPF, COPD (stable COPD or AECOPD), chronic rhinosinusitis, or allergic rhinitis. In some aspects, the asthma is allergic asthma, atopic asthma, corticosteroid naive asthma, chronic asthma, corticosteroid resistant asthma, corticosteroid refractory asthma, asthma due to smoking, or asthma uncontrolled on corticosteroids. In some aspects, the chronic inflammatory skin disease or disorder is atopic dermatitis.

In some aspects of the above disclosed methods, the IL-13 antagonist comprises one or more of an anti-IL-13 antibody or antigen-binding fragment thereof, an IL-13 mutein, an IL-4 mutein, an anti-IL-13Rα1 antibody or antigen-binding fragment thereof, or an anti-IL-4Rα antibody or antigen-binding fragment thereof. In some aspects, the patient has been treated with one or more additional medications, either before, during, or after administration of an IL-13 antagonist. In some aspects, the one or more additional medications comprises a steroid. In other aspects, the one or more additional medications further comprises a bronchodilator. In some aspects, the steroid is fluticasone or budesonide. In some aspects, the bronchodilator is salbutamol or salmeterol. In other aspects, the one or more additional medications are administered by inhalation, by oral administration, by injection, or by a combination thereof. In some aspects, inhalation administration is conducted using a metered dose inhaler (MDI) or a dry powder inhaler (DPI). In some aspects, the steroid is administered at a high dose.

In some aspects of the methods disclosed herein, the IL-13 antagonist is an anti-IL-13 antibody, or antigen-binding fragment thereof. In some aspects, the antibody or fragment thereof binds to the same IL-13 epitope as tralokinumab or competitively inhibits binding of tralokinumab to IL-13, or both. In other aspects, the antibody or fragment thereof comprises tralokinumab or an antigen-binding fragment thereof. In some aspects, the antibody or fragment thereof consists of tralokinumab or an antigen-binding fragment thereof. In some aspects, the antibody or fragment thereof binds to the same IL-13 epitope as lebrikizumab or competitively inhibits binding of lebrikizumab to IL-13, or both. In some aspects, the antibody or fragment thereof comprises lebrikizumab or an antigen-binding fragment thereof. In other aspects, the antibody or fragment thereof consists of lebrikizumab or an antigen-binding fragment thereof.

In some aspects of the methods disclosed herein, the sample taken from the patient comprises one or more of whole blood, serum, plasma, skin, saliva, sputum, bronchoalveolar lavage fluid, lung epithelial cells, urine, or nasal polyps. In some aspects, the sample taken from the patient is blood serum. In some aspects, the methods disclosed herein further comprise determining, submitting a sample taken from the patient for determination, or instructing a clinical laboratory to determine (i) the level of the patient's IgE levels, (ii) the patient's eosinophil count, (iii) the patient's Fraction of Exhaled Nitric Oxide ($FE_{NO}$), (iv) the patient's Eosinophil/Lymphocyte and Eosinophil/Neutrophil (ELEN) index, (v) the patient's EOS index, (vi) wall area % (WA%) of subsegmental airways above about 68% as measured via CT scan of the lungs, or (vii) a combination of two or more thereof. In some aspects, the methods disclosed herein further comprises determining, submitting a sample taken from the patient for determination, or instructing a clinical laboratory to determine the expression level or activity of isoforms 1, 2, 3, or 4 of human periostin, a patient's blood eosinophil cell count, the level of the patient's IgE levels, pre- or post-bronchodilator FEV1 reversibility, or combinations thereof.

In some aspects, the methods disclosed herein further comprise determining, submitting a sample taken from the patient for determination, or instructing a clinical laboratory to determine the expression level or activity of sCTLA-3 (soluble CTLA-3; also known as Cytotoxic T-Lymphocyte-Associated serine Esterase 3, granzyme A, or granzyme 1; Uniprot: P12544), sCD28 (soluble CD28; also known as cluster of differentiation 28 or Tp44; Uniprot: P10747), CCL5 (chemokine C-C motif ligand 5; also known as RANTES; Uniprot: P13501), CCL11 (C-C motif chemokine 11; also known as eosinophil chemotactic protein or eotaxin-1; Uniprot: P51671), CCL22 (C-C motif chemokine 22; Uniprot: O00626), or combinations thereof.

In some aspects, the methods disclosed herein further comprise determining, submitting a sample taken from the patient for determination, or instructing a clinical laboratory to determine the expression level or activity of POSTN (SEQ ID NO:8), CSTI (SEQ ID NO:9), CCL26 (SEQ ID NO:10), CLCAl (SEQ ID NO:11), CST2 (SEQ ID NO:12), PRR4 (SEQ ID NO:13), SERPINB2 (SEQ ID NO:14), CEACAM5 (SEQ ID NO:15), iNOS (SEQ ID NO:16), SERPINB4 (SEQ ID NO:17), CST4 (SEQ ID NO:18), PRB4 (SEQ ID NO:19), TPSDl (SEQ ID NO:20), TPSGl (SEQ ID NO: 21), MFSD2 (SEQ ID NO:22), CPA3 (SEQ ID NO:23), GPR105 (SEQ ID NO:24), CDH26 (SEQ ID NO:25), GSN (SEQ ID NO:26), C2ORF32 (SEQ ID NO:27), TRACH2000196 (TMEM71) (SEQ ID NO:28), DNAJC12 (SEQ ID NO:29), RGS13 (SEQ ID NO: 30), SLC18A2 (SEQ ID NO: 31), SERPINBl0 (SEQ ID NO:32), SH3RF2 (SEQ ID NO:33), FCERlB (SEQ ID NO:34), RUNX2 (SEQ ID NO:35), PTGSl (SEQ ID NO:36), ALOX15 (SEQ ID NO:37), and combinations thereof.

In some aspects, the IL-13 antagonist is administered at a fixed dose. In specific aspects, tralokinumab is administered at a fixed dose of about 300 mg/dose. In some aspects, the IL-13 antagonist is administered in two or more doses. In other aspects, the IL-13 antagonist is administered week, biweekly or monthly. In certain aspects, the IL-13 antagonist is administered biweekly.

In some aspects, the IL-13 antagonist is administered intravenously, intramuscularly, subcutaneously, or a combination thereof. In other aspects, the predetermined DPP4 threshold level is at least about 250 ng/ml, at least about 275 ng/ml, at least about 300 ng/ml, at least about 325 ng/ml at least about 350 ng/mL, at least about 375 ng/mL, at least about 400 ng/mL, at least about 425 ng/mL, at least about 450 ng/mL, at least about 475 ng/mL, at least about 500 ng/mL, at least about 525 ng/mL, at least 550 ng/mL, at least about 575 ng/mL, or at least about 600 ng/mL, as measured in serum using an ELISA. In some aspects, the ELISA is a QUANTIKINE® assay. In some aspects, the predetermined DPP4 threshold level is about 365 ng/mL.

In some aspects, the one or more control samples are obtained from normal healthy individuals; patients with a non-IL-13-mediated subset of asthma; asthma patients naive for corticosteroid treatment; asthma patients treated with corticosteroids; a pre-determined standard amount of isolated DPP4; or a combination thereof. In some aspects, the one or more control samples comprise one or more of whole blood, serum, plasma, saliva, sputum, bronchoalveolar lavage fluid, lung epithelial cells, urine, or a combination thereof.

In some aspects of the methods disclosed herein, administration of the IL-13 antagonist results in (a) AER (Acute Exacerbation Rate) reduction; (b) $FEV_1$ (Forced Expiratory Volume in one second) increase; (c) improved ACQ-6 (Asthma Control Questionnaire, 6-item version) results; (d) improved AQLQ (Asthma Quality of Life Questionnaire) results; or, (e) a combination thereof. In some aspects, the AER reduction is at least 5%, at least 10%, at least 15%, at least 20%, at least 25%, at least 30%, at least 35%, at least 40%, or at least 45% compared to the AER observed in a population of patients treated with a placebo. In other aspects, the mean AER reduction is about 28% compared to the mean AER observed in a population of patients treated with a placebo. In some aspects, the $FEV_1$ increase is at least 3%, at least 5%, at least 7%, at least 9%, at least 11%, at least 13%, at least 15%, at least 17%, or at least 19% compared to the $FEV_1$ observed in a population of patients treated with a placebo. In other aspects, the mean $FEV_1$ increase is about 10% compared to the mean $FEV_1$ observed in a population of patients treated with a placebo.

## BRIEF DESCRIPTION OF THE DRAWINGS/FIGURES

FIG. 1 presents a scheme used to identify genes that are regulated by IL-13 in the lung. Protein levels in normal and asthmatic sera of the candidate genes identified in the screen were determined. Normal human bronchial epithelial cells or human bronchial epithelial cells from the EPIAIRWAY™ model were stimulated with IL-13 for 24 hours, harvested and lysed, and the resulting transcriptional alterations were analyzed by whole genome array (WGA) and TAQMAN® PCR.

FIG. 2 shows genes up-regulated by IL-13 stimulation of normal human bronchial epithelial cells from the EPIAIRWAY™ model. Shown are log2 fold change (fc) values for each gene following IL-13 stimulation. A number of genes including CCL26/Eotaxin-3, dipeptidyl peptidase-4 (DPP4), fetuin B (FETUB) and periostin (POSTN) were found to be up-regulated by IL-13 treatment. Total RNA was prepared from normal bronchial epithelial cells grown in a multilayered, highly differentiated, air-liquid interface model (EPIAIRWAY™, Mattek Corp.) either unstimulated or stimulated with 100ng/mL IL-13 for 24 hours. RNA was reverse transcribed to cDNA and assayed by whole genome microarray.

FIG. 3 shows genes up-regulated by IL-13 stimulation of normal human bronchial epithelial cells. Shown are log2 fold change (fc) values for each gene following IL-13 stimulation. A number of genes including CCL26/Eotaxin-3, dipeptidyl peptidase-4 (DPP4), fetuin B (FETUB) and periostin (POSTN) were found to be up-regulated by IL-13 treatment. Total RNA was prepared from normal bronchial epithelial cells grown in a monolayer, either unstimulated or stimulated with 100ng/mL IL-13 for 24 hours. RNA was reverse transcribed to cDNA and assayed by whole genome microarray.

FIG. 4 shows TAQMAN® qPCR confirmation of microarray data following IL-13 stimulation presented in FIG. 2 and FIG. 3. Shown are mean ± SD linear fold change values comparing gene expression in unstimulated cells/tissues with gene expression following IL-13 stimulation. Four genes shown to be elevated by IL-13 stimulation (DPP4, POSTN, CCL26 and FETUB) were analyzed by TAQMAN® qPCR (Fluidigm). Results corresponding to unstimulated (Unstim) samples as well as dipeptidyl peptidase-4 (DPP4), periostin (POSTN), CCL26/Eotaxin-3 (CCL26), and fetuin B (FETUB) are shown. Total RNA was isolated from either bronchial epithelium (2 donors; left column for each sample), normal EPIAIRWAY™ tissue (3 donors; middle column for each sample), or asthma EPIAIRWAY™ tissue (4 donors; right column for each sample) following no stimulation or stimulation with IL-13 for 24 hours. Samples were reverse-transcribed to cDNA and pre-amplified.

FIG. 5 shows that DPP4 protein levels were elevated in serum from severe asthma patients. Serum from healthy volunteers (n = 38) and asthma patients identified as either moderate (n = 13) or severe (n = 12) were obtained from commercial sources. DPP4 levels (ng/mL) were measured using the QUANTIKINE® ELISA from R&D Systems. Median DPP4 values in each population are indicated with black bars. A trend to increased DPP4 was observed in asthma patients with moderate disease, with a statistically significant increase (* p value <0.05) in DPP4 expression in asthma patients with severe disease.

FIG. 6 shows that DPP4 protein levels were lower in serum from asthma patients taking oral and inhaled steroids. Serum from asthma patients identified as taking various medications (No medication, n = 8; ADVAIR® only, n = 18; Albuterol and inhaled steroids, n = 27; or oral and inhaled steroids, n = 40) were obtained from commercial sources. DPP4 levels (ng/mL) were measured using the QUANTIKINE® ELISA from R&D Systems. Median DPP4 values in each population are indicated with black bars. Of the patients evaluated, the median DPP4 level was lower in patients taking oral and inhaled steroids.

FIG. 7 shows the design of a Tralokinumab Phase 2b Study (CD-RI-CAT-354-1049) and a summary of key questions, dosing frequency, entry criteria, and key primary and secondary endpoints (EP). Q2W=every 2 weeks; Q4W=every 4 weeks; Wk=Week; SC=Sub-cutaneous administration.

FIG. 8 shows the change from baseline in pre-bronchodilator FEV1 over time (ITT Population). Relative increases in FEV1 were observed in both treatment groups during the first 17 weeks of the study compared to placebo. These increases were maintained through to Week 53 in the tralokinumab 300 mg Q2W group but were lost in the tralokinumab 300 mg Q2/4W group. FEV1=forced expiratory flow in one second; ITT=intent to treat; MMRM=mixed-effect model repeated measure; SEM=standard error of the mean; CAT-354=tralokinumab.

FIG. 9 presents forest plots showing annual Acute Exacerbation Rate Reductions (AERR) and change from baseline in pre-bronchodilator at Week 53 by FEV1 reversibility and serum periostin level. FIG. 9A is a forest plot showing the percentage reduction in annual % AERR by FEV1 reversibility and serum periostin level for CAT-354 Q2W or CAT-354 Q4W cohorts compared to placebo (PBO). FIG. 9B is a forest plot showing the difference vs placebo in change from baseline in pre-bronchodilator FEV1 by FEV1 reversibility and serum periostin level for CAT-354 Q2W or CAT-354 Q4W cohorts. CAT-354=tralokinumab; FEV1=forced expiratory volume in one second; Q2W=every 2 weeks; Q2/4W=2 injections Q2W for 12 weeks followed by Q4W for 38 weeks.

FIG. 10 presents forest plots showing ACQ and AQLQ(S) at Week 53 By FEV1 reversibility and serum periostin level. FIG. 10A is a forest plot showing the difference vs placebo for ACQ by FEV1 reversibility and periostin for CAT-354 Q2W or CAT-354 Q4W cohorts. FIG. 10B is a forest plot showing the difference vs placebo for AQLQ(S) by FEV1 reversibility and periostin for CAT-354 Q2W or CAT-354 Q4W groups. ACQ-6=Asthma Control Questionnaire 6; AQLQ(S)=Asthma Quality of Life Questionnaire-Standardized Version; CI=confidence interval; FEV1=forced expiratory volume in 1 second; ITT=intent-to-treat; Q2W=every 2 weeks; Q2/4W=2 injections Q2W for 12 weeks followed by Q4W for 38 weeks.

FIG. 11 is a forest plot showing the effect of subgroup analysis on annual Acute Exacerbation Rate (AER). In the ITT population, a reduction in the primary endpoint, the annual AER, was not observed in either tralokinumab treatment cohort compared to placebo; however, trends towards reductions in AER in patients receiving tralokinumab were observed in a number of pre-specified subgroups including: the presence of FEV1 reversibility to SABA ≥ 12% at baseline, high periostin, high eosinophil, and high Th2 subgroups. Reductions in AER were not observed in the subgroups receiving chronic OCS in either tralokinumab treatment cohort. AERR=asthma exacerbation rate reduction; CAT-354=tralokinumab; Eos=eosinophil; FEV1=forced expiratory volume in one second; ITT=intent to treat; OCS=oral corticosteroid; PBO=placebo; Q2W=every 2 weeks; Q2/4W=2 injections Q2W for 12 weeks followed by Q4W for 38 weeks; Th2=T helper 2.

FIG. 12 is a forest plot showing the effect of subgroup analysis on pre-bronchodilator (pre-BD) FEV1. In the ITT population, a statistically significant increase from baseline in pre-bronchodilator FEV1 at Week 53 compared to placebo was observed in the tralokinumab 300 mg Q2W cohort. Within the tralokinumab 300 mg Q2W cohort at Week 53, increases in pre-bronchodilator FEV1 compared to placebo were closely matched in both the high and low periostin subgroups and were numerically higher in the high reversible, high eosinophil and high Th2 subgroups than in the corresponding low subgroups. No increase in pre-bronchodilator FEV1 was observed in the subgroups receiving chronic OCS in either tralokinumab treatment cohort. CAT-354=tralokinumab; Eos=eosinophil; FEV1=forced expiratory volume in one second; ITT=intent to treat; OCS=oral corticosteroid; PBO= placebo; Q2W=every 2 weeks; Q2/4W=2 injections Q2W for 12 weeks followed by Q4W for 38 weeks; Th2=T helper 2.

FIG. 13 shows the asthma exacerbation rate reduction and mean percent change from baseline in pre-bronchodilator FEV1 at Week 53 for patients by baseline serum periostin level (Tralokinumab Q2W vs Placebo). FIG. 13A is a continuous representation of AER reduction (95% CI) by serum periostin level showing the median periostin value used in the analysis and the effect of changing the median periostin value (baseline periostin cutpoint) on AER Reduction at week 53. FIG. 13B is a continuous representation of percent change from baseline in pre-bronchodilator FEV1 (95% CI) by serum periostin level showing the median periostin value used in the analysis and the effect of changing the median periostin value (baseline periostin cutpoint) on the percent change from baseline in pre-bronchodilator FEV1 at Week 53. Q2W=every 2 weeks; AER=Asthma Exacerbation Rate; FEV1=forced expiratory volume in 1 second; CI=confidence interval.

FIG. 14A shows the percent change from baseline in pre-bronchodilator FEV1 over time, when periostin >= median (ITT population). Relative increases in FEV1 were observed in the 300 mg Q2W group through to Week 53. FEV1=forced expiratory flow in one second; ITT=intent to treat; Q2W=every 2 weeks; Q2/4W=2 injections Q2W for 12 weeks followed by Q4W for 38 weeks; CAT-354=tralokinumab.

FIG. 14B shows the percent change from baseline in pre-bronchodilator FEV1 over time, when periostin < median (ITT population). No significant increases in FEV1 were observed in the 300 mg Q2W group or the Q2/4W through to Week 53. FEV1=forced expiratory flow in one second; ITT=intent to treat; Q2W=every 2 weeks; Q2/4W=2 injections Q2W for 12 weeks followed by Q4W for 38 weeks; CAT-3 54=tralokinumab.

FIG. 15 presents data showing that DPP4 (DPP4-high classifier; i.e., DPP4 >= median) outperforms periostin (periostin-high classifier; i.e., periostin level >= median) in the Intention to Treat (ITT) population, 300 mg tralokinumab Q2W group compared to placebo in various endpoints including acute exacerbation rate reduction, percentage change from baseline in FEV1, ACQ-6 change from baseline, and AQLQ change from baseline. FEV1=forced expiratory flow in one second; ACQ-6 = Asthma Control Questionnaire 6; AQLQ(S) = Asthma Quality of Life Questionnaire-Standardized Version; CI=confidence interval; Q2W=every 2 weeks.

FIG. 16 compares acute exacerbation rate reduction, percentage change from baseline in FEV1, ACQ-6 change from baseline, and AQLQ change from baseline in the Intention to Treat (ITT) population, 300 mg tralokinumab Q2W group, stratified according to 4 classifiers: periostin-high (periostin level >= Median), periostin-low (periostin level < Median), DPP4-high (DPP4 level >= Median), and DPP4-low (DPP4 level < Median). FEV1=forced expiratory flow in one second; ACQ-6 = Asthma Control Questionnaire 6; AQLQ = Asthma Quality of Life Questionnaire; CI=confidence interval; Q2W=every 2 weeks.

FIG 17A-17I show the percent change from baseline for different endpoints depending of the DPP4 classifier user (i.e., DPP4 level >= Median or DPP4 level < Median) in the tralokinumab 300 mg Q2W group or the Q2/4W through to Week 53. Q2W=every 2 weeks; Q2/4W=2 injections Q2W for 12 weeks followed by Q4W for 38 weeks; CAT-354=tralokinumab; BSL=baseline; WX=Week X; FEV1=forced expiratory flow in one second; ACQ-6 = Asthma Control Questionnaire 6; AQLQ(S) = Asthma Quality of Life Questionnaire-Standardized Version. FIG. 17A (percent change from baseline in pre-bronchodilator FEV1 over time and DPP4 >= Median), FIG. 17B (percent change from baseline in pre-bronchodilator FEV1 over time and DPP4 < Median), FIG. 17C (change from baseline in mean ACQ-6 over time and DPP4 >= Median), FIG. 17D (change from baseline in mean ACQ-6 over time and DPP4 < Median), FIG. 17E (change from baseline in mean AQLQ(S) over time and DPP4 >= Median), FIG. 17F (change from baseline in mean AQLQ(S) over time and DPP4 < Median), FIG. 17G (percent change from baseline in pre-Bronchodilator FEV1 over time, baseline FEV1 reversibility >= 12% and DPP4 >= Median), FIG. 17H (change from baseline in mean ACQ-6 over time, baseline FEV1 reversibility >= 12% and DPP4 >= Median), and FIG. 17I (change from baseline in mean AQLQ(S) over time, baseline FEV1 reversibility >= 12% and DPP4 >= Median). An increase in the percent change from baseline in pre-bronchodilator FEV1 (17A), a decrease in the change from Baseline in Mean ACQ-6 (17C), and an increase in the change from baseline in mean AQLQ(S) (17E) compared to placebo at week 53 were observed in the tralokinumab 300 mg Q2W cohort for DPP4 >= Median. (Figures 17A, 17C, 17E, respectively). These changes were lost in the DPP4 level < Median group (Figures 17B, 17D, 17F, respectively). Similarly, an increase in the percent change from baseline in pre-bronchodilator FEV1 (17G), a decrease in the change from Baseline in Mean ACQ-6 (17H), and an increase in the change from baseline in mean AQLQ(S) (171) compared to placebo at week 53 were observed in the tralokinumab 300 mg Q2W cohort for the baseline FEV1 reversibility to a short-acting β2 agonist >= 12% + DPP4 >= Median group.

FIG. 18 is a continuous representation of asthma exacerbation rate reduction by DPP4 level showing an asthma exacerbation rate reduction (95% CI) for subjects with baseline DPP4 >= cut-point treated with CAT-354 Q2W compared to placebo. The median DPP4 value used in the analysis and the effect of changing the median DPP4 value (baseline DPP4 cut-point) on asthma exacerbation rate reduction are shown. Q2W=every 2 weeks; CAT-354=tralokinumab; CI=confidence interval.

FIG. 19 is a continuous representation of the mean percent change from baseline in pre-BD FEV1 at week 53 (95% CI) for subjects with baseline DPP4 >= cut-point treated with CAT-354 Q2W compared to placebo. The median DPP4 value used in the analysis and the effect of changing the median DPP4 value (baseline DPP4 cut-point) on mean percent change from baseline in pre-BD FEV1 are shown. Q2W=every 2 weeks; CAT-354=tralokinumab; CI=confidence interval; pre-BD=pre-bronchodilator; FEV1= FEV1=forced expiratory flow in one second.

FIG. 20 is a continuous representation of mean change from baseline in mean ACQ-6 score at week 53 (95% CI) for subjects with baseline DPP4 >= cut-point treated with CAT-354 Q2W compared to placebo. The median DPP4 value used in the analysis and the effect of changing the median DPP4 value (baseline DPP4 cut-point) on the mean change from baseline in ACQ-6 are shown. Q2W=every 2 weeks; CAT-354=tralokinumab; ; CI=confidence interval; ACQ-6 = Asthma Control Questionnaire 6.

FIG. 21 shows the relative distribution of subjects classified as DPP4-high (serum DPP4 ≥ median); DPP-low (serum DPP4 below median); periostin-high (serum periostin ≥ median); and periostin-low (serum periostin below median) irrespective of treatment group. In each quadrant, the upper number corresponds to the number of subjects while the lower number corresponds to the % of total subjects (e.g. DPP4-high, periostin-High = 125 patients or 27.84% of the study subjects). There is a partial overlap between the DPP4-high and periostin-high groups.

FIG. 22 shows the relative distribution of subjects classified as DPP4-high (serum DPP4 ≥ median); DPP-low (serum DPP4 below median); Th2-high (IgE > 100 IU/mL and blood eosinophils ≥ 0.14 × 109/L); and Th2-low (subjects not classified as Th-2 high) irrespective of treatment group. In each quadrant, the upper number corresponds to the number of subjects while the lower number corresponds to the % of total subjects (e.g. DPP4-high, Th2-High = 114 patients or 27.67% of the study subjects). There is a partial overlap between the DPP4-high and Th2-high groups.

FIG. 23 shows the relative distribution of subjects classified as DPP4-high (serum DPP4 ≥ median); DPP-low (serum DPP4 below median); EOS-high (blood eosinophil count ≥ 300 cells/μL); and Eos-low (blood eosinophil count < 300 cells/μL) irrespective of treatment group. In each quadrant, the upper number corresponds to the number of subjects while the lower number corresponds to the % of total subjects (e.g. DPP4-high, Eos-High = 84 patients or 19.86% of the study subjects). There is a partial overlap between the DPP4-high and Eos-high groups.

FIG. 24 shows that mean and median serum DPP4 levels for subjects with (positive) or without (negative) chronic OCS use. The mean and median serum DPP4 levels are reduced in patients chronically treated with OCS. OCS=oral corticosteroid; N= number of subjects.

FIG. 25 shows AERR, Exacerbation rates, mean percent change from baseline FEV1, mean change from baseline for ACQ-6 and the mean change from baseline for AQLQ for CAT-354 compared to placebo in subjects not chronically treated with OCS with baseline FEV1 reversibility to a short-acting β2 agonist (≥ 12% or < 12%) and high (≥ median) or low (< median) serum DPP4. OCS=oral corticosteroid; CAT-354=tralokinumab; BL=baseline; FEV1=forced expiratory flow in one second; ACQ-6 = Asthma Control Questionnaire 6; AQLQ = Asthma Quality of Life Questionnaire; pbo=placebo; N= number of subjects; CI=confidence interval.

FIG. 26 shows AERR, Exacerbation rates, mean percent change from baseline FEV1, mean change from baseline for ACQ-6 and the mean change from baseline for AQLQ for CAT-354 compared to placebo in subjects not chronically treated with OCS with baseline FEV1 reversibility to a short-acting β2 agonist (≥ 12% or < 12%) and high (≥ median) or low (< median) serum periostin. OCS=oral corticosteroid; CAT-354=tralokinumab; BL=baseline; FEV1=forced expiratory flow in one second; ACQ-6 = Asthma Control Questionnaire 6; AQLQ = Asthma Quality of Life Questionnaire; pbo=placebo; N= number of subjects; CI=confidence interval.

FIG. 27 shows periostin (POSTN) mRNA expression intensity levels in normal skin and atopic dermatitis (AD) skin measured using two probes, probe 1555778 (Panel A) and probe 210809 (Panel B), respectively, using whole genome microarray (Affymetrix). Total RNA was isolated from either normal skin (31 donors) or skin from subjects diagnosed with atopic dermatitis (4 donors). Biotin-labeled amplified cRNA was generated from total RNA and fragmented for hybridization on Affymetrix Human Genome U133 Plus 2.0 GeneChip® arrays. Median expression intensity levels in each population are indicated with black bars. POSTN mRNA expression is elevated in AD skin samples compared to normal skin.

FIG. 28 shows DPP4 mRNA expression intensity levels in normal skin and atopic dermatitis (AD) skin measured using two probes, probe 203717 (Panel A) and probe 203716 (Panel B), respectively, using whole genome microarray (Affymetrix). Total RNA was isolated from either normal skin (31 donors) or skin from subjects diagnosed with atopic dermatitis (4 donors). Biotin-labeled amplified cRNA was generated from total RNA and fragmented for hybridization on Affymetrix Human Genome U133 Plus 2.0 GeneChip® arrays. Median expression intensity levels in each population are indicated with black bars. DPP4 mRNA expression is elevated in AD skin samples compared to normal skin.

FIG. 29 shows representative computed tomography (CT) images of lungs from a patient at visit 4 (Panel A) and at visit 30 (Panel B) showing various bronchial airways.

FIG. 30 shows relative changes in lumen area (LA) in patients treated with placebo or tralokinumab from baseline (visit 4) and visit 30 as measured using VIDA APOLLO® software of 3D computed tomography imaging scans of the lungs. Relative changes in LA corresponding to RB1 bronchial airway (Panel A), segmental airways (Panel B), and subsegmental airways (Panel C) are presented. A significant increase in lumen area of subsegmental airways was observed in patients treated with tralokinumab compared to placebo (p value =0.021). Tralo= tralokinumab; p= p value.

FIG. 31 shows relative changes in bronchial wall area percentage (WA%) in patients treated with placebo or tralokinumab (Tralo) from baseline (visit 4) and visit 30 as measured using VIDA APOLLO® software of 3D computed tomography imaging scans of the lungs. Relative changes in WA% corresponding to RB1 bronchial airway (Panel A), segmental airways (Panel B), and subsegmental airways (Panel C) are presented. A significant decrease in wall area percentage (WA%) of subsegmental airways was observed in patients treated with tralokinumab compared to placebo (p value =0.0049). Tralo= tralokinumab; p= p value.

FIG. 32 shows relative changes in airway resistance in patients treated with placebo or tralokinumab (Tralo) from baseline (visit 4) and visit 30 as measured using VIDA APOLLO® software of 3D computed tomography imaging scans of the lungs. *See* Example 6 for more details. Relative changes in airway resistance corresponding to RB1 bronchial airway (Panel A), segmental airways (Panel B), and subsegmental airways (Panel C) are presented. The dashed rectangle indicates the airway resistance data set that was reanalyzed in FIG. 33A according to a baseline WA% threshold value (median WA%). A significant decrease in airway resistance of subsegmental airways was observed in patients treated with tralokinumab compared to placebo (p value =0.0081). Tralo= tralokinumab; p= p value.

FIG. 33 shows relative changes from baseline (visit 4) and visit 30 in airway resistance of subsegmental airways (Panel A) and pre-bronchodilator FEV1 (Panel B) in patients treated with tralokinumab. Patients having a wall percentage (WA%) of subsegmental airways higher than 68% at baseline had significant reductions in airway resistance (p value = 0.0037) and significant improvements in FEV1 response (p value = 0.045) compared to patients having less than a 68% wall percentage (WA%) at baseline. Tralo= tralokinumab; p= p value.

FIG. 34 shows IL-13 specific up-regulation of CCL-26 (Panel A), DPP4 (Panel B), Periostin POSTN-745 (Panel C), and Periostin POST-815(Panel D) transcripts from highly differentiated bronchial epithelial cells grown at air liquid interfaces (EPIAIRWAY™ model). Samples corresponded to 3 normal donors, designated Normal-25, Normal-21 and Normal-30, respectively (bars 1-3 of each set, left to right), and 3 COPD donors, designated COPD-15, COPD-12 and COPD-18, respectively (bars 4-6 of each set, left to right).

FIG. 35 shows periostin (Panel A) and DPP4 (Panel B) expression levels in atopic dermatitis patients suffering from moderate (Mod) or severed (Sev) atopic dermatitis. Data is expressed as geometric mean (number next to central dot) with 95% confidence intervals (positive and negative bars). Each individual patient observation is shown as a separate dot. Horizontal lines and associated numbers represent the upper and lower confidence intervals around the moderate patient point estimate.

FIG. 36 shows serum levels of periostin in healthy controls (n=20 subjects), stable COPD (n=101 subjects) and acute exacerbations of COPD (AECOPD; n=61 subjects) measured by immunoassay. Serum periostin levels are significantly elevated in patients suffering from COPD (both stable and acute exacerbations of COPD) compared to healthy controls. P= p value; NS= no significant difference.

FIG. 37 shows serum levels of DPP4 in healthy controls (n=20 subjects), stable COPD (n=104 subjects) and acute exacerbations of COPD (AECOPD; n=72 subjects) measured by immunoassay. Serum DPP4 levels are significantly elevated in patients suffering from COPD (both stable and acute exacerbations of COPD) compared to healthy controls. P= p value; NS= no significant difference.

DETAILED DESCRIPTION

The disclosure relates to the use of DPP4 (SEQ ID: 5, membrane bound form protein sequence; SEQ ID NO: 6, soluble form protein sequence; SEQ ID NO: 7, *DPP4* gene cDNA sequence) as a biomarker for IL-13 mediated disease or disorders, e.g., asthma, IPF, COPD (e.g., stable COPD or acute exacerbations of COPD), or atopic dermatitis. Accordingly, the disclosure provides methods for diagnosing and treating a subject as having an IL-13-mediated disease or disorder, comprising administering an IL-13 antagonist, for example, an anti-IL-13 antibody, to the patient if the DPP4 level in a sample taken from the patient is above a predetermined DPP4 threshold level, or if the DPP4 level is elevated relative to the DPP4 level in one or more control samples. In particular, the presence of levels of the DPP4 biomarker above or below a predetermined DPP4 threshold level can be used, e.g., (i) to determine whether a patient suffering an IL-13-mediated disease or disorder is eligible or non-eligible for a specific treatment with an IL-13 antagonist (e.g., an antibody such as tralokinumab), (ii) to determine whether a specific treatment of an IL-13-mediated disease or disorder with an IL-13 antagonist should commence, be suspended, or be modified, (iii) to diagnose whether an IL-13-mediated disease or disorder is treatable or not treatable with a specific IL-13 antagonist, (iv) to prognosticate the outcome of treatment of an IL-13-mediated disease or disorder with a specific IL-13 antagonist, etc.

In some aspects, the presence of DPP4 levels above or below a predetermined DPP4 threshold level in samples (e.g., blood serum or skin) obtained from a patient suffering from an IL-13-mediated pulmonary disease or disorder (e.g., asthma, IPF or COPD) or an IL-13-mediated chronic inflammatory skin disease or disorder (e.g., atopic dermatitis) can be used, e.g., (i) to determine whether the patient is eligible or non-eligible for treatment with a specific therapeutic agent, (ii) to determine whether a specific treatment should commence, be suspended, or be modified, (iii) to diagnose whether the disease or disorder is treatable or not treatable with a specific therapeutic agent, (iv) to prognosticate the outcome of treatment of the disease or disorder (e.g., asthma, IPF, COPD or atopic dermatitis) with a specific therapeutic agent, etc.

In some aspects, the presence of DPP4 levels above or below a predetermined DPP4 threshold level in samples (e.g., blood serum or skin) obtained from a patient suffering from an IL-13-mediated disease or disorder (e.g., asthma, IPF or COPD) or an IL-13-mediated chronic inflammatory skin disease or disorder (e.g., atopic dermatitis) in combination with one or more of: (i) high periostin ($\geq$ median serum periostin or about 23 ng/mL, (ii) high eosinophil cell count (blood eosinophil count $\geq$ 300 cells/$\mu$L), (iii) high Th2 (high Th2 defined as IgE > 100 IU/mL and blood eosinophils $\geq 0.14 \times 10^9$/L), (iv) FEV1 reversibility to a short-acting $\beta$2 agonist $\geq$ 12%, (v) wall area % (WA%) of subsegmental airways above about 68% as measured via CT scan of the lungs, or (vi) combinations thereof, can be used, e.g., (i) to determine whether a patient suffering an IL-13-mediated disease or disorder is eligible or non-eligible for a specific treatment with an IL-13 antagonist (e.g., an antibody such as tralokinumab or lebrikizumab), (ii) to determine whether a specific treatment should commence, be suspended, or be modified, (iii) to diagnose whether the disease or disorder is treatable or not treatable with a specific therapeutic agent, (iv) to prognosticate the outcome of treatment of the disease or disorder (e.g., asthma, IPF, COPD or atopic dermatitis) with a specific therapeutic agent, etc.

In order that the present disclosure can be more readily understood, certain terms are first defined. Additional definitions are set forth throughout the detailed description.

I. Definitions

In this specification and the appended claims, the singular forms "a", "an" and "the" include plural referents unless the context clearly dictates otherwise. The terms "a" (or "an"), as well as the terms "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Wherever aspects are described herein with the language "comprising," otherwise analogous aspects described in terms of "consisting of' and/or "consisting essentially of' are also provided.

The term "about" as used in connection with a numerical value throughout the specification and the claims denotes an interval of accuracy, familiar and acceptable to a person skilled in the art. In general, such interval of accuracy is $\pm$ 15 %.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, amino acid sequences are written left to right in amino to carboxy orientation. The headings provided herein are not limitations of the various aspects or aspects of the disclosure, which can be had by reference to the specification as a whole. Accordingly, the terms defined immediately below are more fully defined by reference to the specification in its entirety.

As used herein, the term "antibody" (or a fragment, variant, or derivative thereof) refers to at least the minimal portion of an antibody which is capable of binding to antigen, e.g., at least the variable domain of a heavy chain (VH) and the variable domain of a light chain (VL) in the context of a typical antibody produced by a B cell. Basic antibody structures in vertebrate systems are relatively well understood. See, *e.g.,* Harlow et al., Antibodies: A Laboratory Manual, (Cold Spring Harbor Laboratory Press, 2nd ed. 1988).

Antibodies or antigen-binding fragments, variants, or derivatives thereof include, but are not limited to, polyclonal, monoclonal, human, humanized, or chimeric antibodies, single chain antibodies, epitope-binding fragments, *e.g.,* Fab, Fab' and F(ab')2, Fd, Fvs, single-chain Fvs (scFv), single-chain antibodies, disulfide-linked Fvs (sdFv), fragments comprising either a VL or VH domain, fragments produced by a Fab expression library. ScFv molecules are known in the art and are described, *e.g.,* in US patent 5,892,019. Immunoglobulin or antibody molecules encompassed by this disclosure can be of any type (*e.g.,* IgG, IgE, IgM, IgD, IgA, and IgY), class (*e.g.,* IgG1, IgG2, IgG3, IgG4, IgA1 and IgA2) or subclass of immunoglobulin molecule.

By "specifically binds," it is generally meant that an antibody or fragment, variant, or derivative thereof binds to an epitope via its antigen-binding domain, and that the binding entails some complementarity between the antigen binding domain and the epitope. According to this definition, an antibody is said to "specifically bind" to an epitope when it binds to that epitope via its antigen-binding domain more readily than it would bind to a random, unrelated epitope.

An antibody or fragment, variant, or derivative thereof is said to competitively inhibit binding of a reference antibody or antigen binding fragment to a given epitope if it preferentially binds to that epitope to the extent that it blocks, to some degree, binding of the reference antibody or antigen binding fragment to the epitope. Competitive inhibition can be determined by any method known in the art, for example, competition ELISA assays. A binding molecule can be said to competitively inhibit binding of the reference antibody or antigen-binding fragment to a given epitope by at least 90%, at least 80%, at least 70%, at least 60%, or at least 50%.

Antibodies or antigen-binding fragments, variants, or derivatives thereof disclosed herein can be described or specified in terms of the epitope(s) or portion(s) of an antigen, *e.g.,* a target polysaccharide that they recognize or specifically bind. For example, the portion of human DPP4 that specifically interacts with the antigen-binding domain of an anti-DPP4 antibody is an "epitope."

As used herein, the term "IL-13-mediated disease or disorder" refers to any pathology caused by (alone or in association with other mediators), exacerbated by, associated with, or prolonged by abnormal levels of IL-13 in the subject having the disorder. Non-limiting examples of IL-13-mediated diseases or disorders include asthma, idiopathic pulmonary fibrosis (IPF), chronic obstructive pulmonary disease (COPD), ulcerative colitis (UC), allergic rhinitis, chronic rhinosinusitis, and atopic dermatitis.

As used herein, the term "pulmonary disease or disorder" refers to any pathology affecting at least in part the lungs or respiratory system. Non-limiting examples include asthma, IPF, COPD, allergic rhinitis, or chronic rhinosinusitis. In certain aspects, the pulmonary disease or disorder is IL-13-mediated.

As used herein, the term "chronic inflammatory skin disease or disorder" refers to any pathology affecting at least in part the skin. Non-limiting examples include atopic dermatitis, skin fibrosis, allergic contact dermatitis, eczema or psoriasis. In certain aspects, the chronic inflammatory skin disease or disorder is IL-13-mediated.

The term "asthma" refers to diseases that present as reversible airflow obstruction and/or bronchial hyper-responsiveness that may or may not be associated with underlying inflammation. Examples of asthma include allergic asthma, atopic asthma, corticosteroid naive asthma, chronic asthma, corticosteroid resistant asthma, corticosteroid refractory asthma, asthma due to smoking, asthma uncontrolled on corticosteroids and other asthmas as mentioned, *e.g.,* in the Expert Panel Report 3: Guidelines for the Diagnosis and Management of Asthma, National Asthma Education and Prevention Program (2007) ("NAEPP Guidelines"), incorporated herein by reference in its entirety.

The term "COPD" as used herein refers to chronic obstructive pulmonary disease. The term "COPD" includes two main conditions: emphysema and chronic obstructive bronchitis. Thus, in the broadest sense, the term COPD as used herein refers to COPD itself and also its subconditions chronic bronchitis and emphysema. The Global Initiative for Chronic Obstructive Lung Disease (GOLD) has classified 4 different stages of COPD. GOLD classification for COPD Stage 0: At Risk for COPD. Symptoms of chronic cough and sputum production may be present, but patients have normal spirometry readings. Stage I: Mild COPD. Characterized by $FEV_1$ >= 80%, $FEV_1$/FVC < 70%. Patients may have or not have chronic cough and increased sputum production. Stage II: Moderate COPD. Characterized by a worsening of airflow (30% >= $FEV_1$ > 80%). Patients with Stage II disease often are symptomatic, seek medical attention, and have shortness of breath with exertion. Stage II has 2 subcategories: IIA and IIB. IIA patients have a $FEV_1$ between 50% and 80%; stage IIB patient have a $FEV_1$ between 30% and 50%. Patients with $FEV_1$ below 50% are especially prone to acute exacerbations of disease. Stage III: Severe COPD. Characterized by an $FEV_1$ below 30%. Patients are also included in stage III if they have respiratory failure or right heart failure. The quality of life is severely affected in these patients. Acute exacerbations in this patient population often require hospitalization and are frequently life threatening.

As used herein, "early stage" COPD is intended to mean GOLD Stage 0 or a precursor condition thereto.

In some aspects, COPD is stable COPD. In other aspects, COPD refers to a COPD exacerbation. As used herein, the term "exacerbation" refers to a worsening of symptoms of COPD, relative to a patient's baseline condition. In certain embodiments, a COPD exacerbation may be defined as an event in the natural course of the disease characterized by a change in the patient's baseline lung function, dyspnea, cough, and/or sputum that is beyond normal day-to-day variations, is acute in onset and may warrant a change in medication in a patient with underlying COPD. In certain embodiments, exacerbation of COPD may be an abrupt increase in symptoms of shortness of breath and/or wheezing, and/or increase in production of purulent sputum.

The term "Idiopathic Pulmonary Fibrosis" (IPF) refers to a disease characterized by progressive scarring, or fibrosis, of the lungs. It is a specific type of interstitial lung disease in which the alveoli gradually become replaced by fibrotic tissue. With IPF, progressive scarring causes the normally thin and pliable tissue to thicken and become stiff, making it more difficult for the lungs to expand, preventing oxygen from readily getting into the bloodstream. See, *e.g.,* Am. J. Respir. Crit. Care Med. 2000. 161:646-664.

As used herein, the term "atopic dermatitis" refers to a chronic inflammatory, relapsing, non-contagious and itchy skin disorder that is often associated with other atopic disorders such as allergic rhinitis and asthma (Bieber, New England Journal of Medicine, 2008, 358: 1483-1494). The term "atopic dermatitis" is equivalent to "neurodermatitis", "atopic eczema" or "endogenous eczema". Particular forms of atopic dermatitis, which get their names from the place where they occur or from their appearance or from the stress factors which provoke them, are, according to the present disclosure also comprised by the term "atopic dermatitis". These include, but are not limited to, eczema flexurarum, eczema mulluscatum, eczema verrucatum, eczema vaccinatum, eczema dyskoides, dyshydrotic eczema, microbial eczema, nummular eczema, seborrhobic eczema and other forms of eczema; perioral dermatitis and periorbital dermatitis. As used herein, the term atopic dermatitis also comprises the frequently occurring bacterial secondary infections such as those due to e.g. Staphylococcus aureus infections, pyodermas such as impetigo contagiosa and its derivatives as well as the follicularis barbae or viral secondary infections. IL-13 is involved in the pathogenesis of the disease and is an important in vivo inducer. See, e.g., Oh et al., J. Immunol. 186:7232-42 (2011); Tazawa et al., Arch. Dermatol. Res. 295:459-464 (2004); Metwally et al. Egypt J. Immunol. 11:171-7 (2004).

As used herein the terms "treat," "treatment, " or "treatment of" (*e.g.,* in the phrase "treating a patient having an IL-13-mediated disease or disorder") refers to reducing the potential for an IL-13-mediated disease or disorder, reducing the occurrence of the IL-13-mediated disease or disorder, and/or a reduction in the severity of the IL-13-mediated disease or disorder, preferably, to an extent that the subject no longer suffers discomfort and/or altered function due to it (for example, a relative reduction in asthma exacerbations when compared to untreated patients). For example, treating can refer to the ability of a therapy when administered to a subject, to prevent an IL-13-mediated disease or disorder from occurring and/or to cure or to alleviate IL-13-mediated disease symptoms, signs, or causes. Treating also refers to mitigating or decreasing at least one clinical symptom and/or inhibition or delay in the progression of the condition and/or prevention or delay of the onset of a disease or illness. Thus, the terms "treat," "treating" or "treatment of' (or grammatically equivalent terms) refer to both prophylactic and therapeutic treatment regimes.

The present disclosure provides methods and systems providing therapeutic benefit in the treatment of an IL-13-mediated disease or disorder. A therapeutic benefit is not necessarily a cure for a particular IL-13-mediated disease or disorder, but rather encompasses a result which most typically includes alleviation of the IL-13-mediated disease or disorder or increased survival, elimination of the IL-13-mediated disease or disorder, reduction of a symptom associate with the IL-13-mediated disease or disorder, prevention or alleviation of a secondary disease, disorder or condition resulting from the occurrence of a primary IL-13-mediated disease or disorder, and/or prevention of the IL-13-mediated disease or disorder.

The terms "subject" or "patient" as used herein refer to any subject, particularly a mammalian subject, for whom diagnosis, prognosis, or therapy of an IL-13-mediated disease or disorder is desired. As used herein, the terms "subject" or "patient" include any human or nonhuman animal. The term "nonhuman animal" includes all vertebrates, e.g., mammals and non-mammals, such as nonhuman primates, sheep, dogs, cats, horses, cows, bears, chickens, amphibians, reptiles, *etc.* As used herein, phrases such as "a patient having an IL-13-mediated disease or disorder" includes subjects, such as mammalian subjects, that would benefit from the administration of a therapy, imaging or other diagnostic procedure, and/or preventive treatment for that IL-13-mediated disease or disorder. In some aspects of the present disclosure, a subject is a naive subject. A naive subject is a subject that has not been administered a therapy, for example a therapeutic agent. In some aspects, a naive subject has not been treated with a therapeutic agent prior to being diagnosed as having an IL-13-mediated disease or disorder, for example, asthma, IFP, COPD, UC, or atopic dermatitis. In another aspect, a subject has received therapy and/or one or more doses of a therapeutic agent (*e.g.,* a therapeutic agent capable of modulating an inflammatory response associated with an IL-13-mediated disease or disorder, a pulmonary disease or disorder, an inflammatory bowel disease or disorder or a chronic inflammatory skin disease or disorder) prior to being diagnosed as having an IL-13-mediated disease or disorder. In one aspect, the therapeutic agent is a small molecule drug. In a specific aspect, the agent is a corticosteroid. In another aspect, the agent can be a leukotriene modifier such as montelukast, zafirlukast or zileuton. In a further aspect, the therapeutic agent can be a methylxanthine (*e.g.,* theophylline) or a cromone (*e.g.,* sodium cromolyn and nedocromil). In another aspect, the therapeutic agent can be a long-acting beta-2 agonist such as salmeterol, fomoterol, or indacaterol. In a further aspect, the agent can be methotrexate or cyclosporin.

In certain aspects, the therapeutic agent can be an agent used for preventing, treating, managing, or ameliorating asthma or COPD. Non-limiting examples of therapies for asthma or COPD include anti-cholinergics (*e.g.*, ipratropium bromide and oxitropium bromide), beta-2 antagonists (*e.g.*, albuterol (PROVENTIL® or VENTOLIN®), bitolterol (TOMALATE®), fenoterol, formoterol, isoetharine, metaproterenol, pibuterol (MAXAIR®), salbutamol, salbutamol terbutaline, and salmeterol, terbutlaine (BRETHAIRE®)), corticosteroids (*e.g.*, prednisone, beclomethasone dipropionate (VANCERIL® or BECLOVENT®), triamcinolone acetonide (AZMACORF®), flunisolide (AEROBID®), and fluticasone propionate (FLOVENT®)), leukotriene antagonists (*e.g.*, montelukast, zafirlukast, and zileuton), theophylline (THEO-DUR®, UNIDUR® tablets, and SLO-BID® Gyrocaps), and salmeterol (SEREVENT®), cromolyn, and nedorchromil (INTAL® and TILADE®)), IgE antagonists, IL-4 antagonists (including antibodies), IL-5 antagonists (including antibodies), PDE4 inhibitors, NF-Kappa-B inhibitors, IL-13 antagonists (including antibodies), CpG, CD23 antagonists, selectin antagonist (*e.g.*, TBC 1269), mast cell protease inhibitors (*e.g.,* tryptase kinase inhibitors (*e.g.,* GW-45, GW-58, and genisteine), phosphatidylinositide-3' (PI3)-kinase inhibitors (*e.g.*, calphostin C), and other kinase inhibitors (*e.g.,* staurosporine), C2a receptor antagonists (including antibodies), and supportive respiratory therapy, such as supplemental and mechanical ventilation.

In some aspects, a subject has received at least one therapeutically effective dose of oral or inhaled corticosteroids. In some aspects, a subject has received multiple therapeutically effective doses of oral or inhaled corticosteroids. In some aspects, a subject is a chronic oral corticosteroid (OCS) user.

In certain aspects the subject has received a long-acting beta2-adrenergic agonist, *e.g.,* salmeterol xinafoate. In some aspects the subject has received a synthetic glucocorticoid, *e.g.,* fluticasone propionate. In certain aspects the subject has received a combination of salmeterol xinafoate and fluticasone propionate (ADVAIR®). In certain aspects the subject has received a beta2-adrenergic bronchodilator, *e.g.,* albuterol sulfate.

In one aspect, the therapeutic agent used according to methods disclosed herein is an antibody, *e.g.,* an anti-IL-13 antibody or an antigen-binding fragment thereof. Accordingly, in some aspects, a subject has received or is a candidate to receive at least one therapeutically effective dose of an antibody (*e.g.*, an anti-IL-13 antibody or an antigen-binding fragment thereof) capable of neutralizing IL-13-mediated pathology. In some aspects, the anti-IL-13 antibody is tralokinumab (SEQ ID NOS: 3 and 4) or an antigen-binding fragment thereof. *See* US Patent 7,829,090, herein incorporated by reference in its entirety. Other anti-IL-13 monoclonal antibodies that can be used include those described in U.S. Pat. Appl. Publ. No. 2012-0052060, published March 1, 2012. Other IL-13 antagonists include, without limitation: (a) an anti-human-IL-13 antibody, for example, Lebrikizumab (MILR1444A / RG3637, Roche / Genentech) (SEQ ID NOS: 1 and 2) or an antigen-binding fragment thereof, ABT-308 (Abbott), GSK679586 (GlaxoSmithKline) or QAX576 (Novartis); (b) an anti-human-IL-13R$\alpha$1 antibody, for example, Merck MK6105; (c) an IL-13-toxin conjugate such as IL-13-PE38QQR (NeoPharm, Inc.); (d) an IL-4 mutein AEROVANT™ (Aerovance, Inc.); (e) an anti-IL-4R$\alpha$ antibody such as dupilumab/REGN668 (Regeneron); (f) a double-stranded oligonucleotide directed against IL-4R$\alpha$ such as AIR645 (Isis); or (g) an IL-4 / IL-13 bispecific antibody such as GSK2434735 (Glaxo SmithKline). In some aspects, a subject can be administered at least one therapeutically effective dose of an anti-IL-13 antibody or an antigen-binding fragment thereof disclosed herein if the subject's DPP4 level is above a predetermined DPP4 threshold level, or if the DPP4 level is elevated relative to the DPP4 level in one or more control samples. In other aspects, a subject can be deemed eligible to receive at least one therapeutically effective dose of an anti-IL-13 antibody or an antigen-binding fragment thereof disclosed herein if the subject's DPP4 level is above a predetermined DPP4 threshold level, or if the DPP4 level is elevated relative to the DPP4 level in one or more control samples.

As used herein, the term "IL-13 antagonist" refers to any agent that can affect the expression, activity, or half-life of IL-13 either *in vitro* or *in vivo,* or symptoms, pathology, or sequelae caused by or exacerbated by IL-13 in a subject with an IL-13-mediated disease or disorder, e.g., asthma. An IL-13 antagonist can be any "therapeutic agent" as defined herein, which either directly or indirectly can inhibit, lessen, or neutralize IL-13 activity, inhibit or reduce IL-13 expression, reduce IL-13 half-life, or can prevent exacerbation of symptoms due to IL-13. In certain aspects, an IL-13 antagonist is an anti-IL-13 monoclonal antibody, *e.g.,* tralokinumab, or other anti-IL-13 monoclonal antibodies described, *e.g.,* in U.S. Pat. Appl. Publ. No. 2012-0052060, published March 1, 2012.

The term "therapy" as used herein includes any means for curing, mitigating, or preventing an IL-13-mediated disease or disorder, including, for example, therapeutic agents, instrumentation, supportive measures, and surgical or rehabilitative procedures. In this respect, the term therapy encompasses any protocol, method and/or therapeutic or diagnostic that can be used in prevention, management, treatment, and/or amelioration of an IL-13-mediated disease or disorder.

The term "therapeutic agent" as used herein refers to any therapeutically active substance that is administered to a subject having an IL-13-mediated disease or disorder to produce a desired, usually beneficial, effect. The term therapeutic agent includes, *e.g.,* classical low molecular weight therapeutic agents commonly referred to as small molecule drugs and biologics including but not limited to: antibodies or active fragments thereof, peptides, lipids, protein drugs, protein conjugate drugs, enzymes, oligonucleotides, ribozymes, genetic material, prions, virus, bacteria, and eukaryotic cells. A therapeutic agent can also be a pro-drug, which metabolizes into the desired therapeutically active substance when administered to a subject. In some aspects, the therapeutic agent is a prophylactic agent. In addition, a therapeutic agent can be pharmaceutically formulated. A therapeutic agent can also be a radioactive isotope or agent activated by some other form of energy such as light or ultrasonic energy, or by other circulating molecules that can be systemically administered.

A "therapeutically effective" amount as used herein is an amount of therapeutic agent that provides some improvement or benefit to a subject having an IL-13-mediated disease or disorder, e.g., an IL-13-mediated pulmonary disease or disorder such as asthma, IPF or COPD; or a chronic inflammatory skin disease or disorder such as atopic dermatitis. Thus, a "therapeutically effective" amount is an amount that provides some alleviation, mitigation, and/or decrease in at least one clinical symptom of the IL-13-mediated disease or disorder, e.g., an IL-13-mediated pulmonary disease or disorder such as asthma, IPF, or COPD; or a chronic inflammatory skin disease or disorder such as atopic dermatitis. Clinical symptoms associated with the IL-13-mediated disease or disorders, e.g., IL-13-mediated pulmonary disease or disorders such as asthma, IPF or COPD; or a chronic inflammatory skin disease or disorder such as or atopic dermatitis that can be treated by the methods and systems of the disclosure are well known to those skilled in the art. Further, those skilled in the art will appreciate that the therapeutic effects need not be complete or curative, as long as some benefit is provided to the subject. In some aspects, the term "therapeutically effective" refers to an amount of a therapeutic agent therapeutic agent that is capable of reducing IL-13 activity in a patient in need thereof.

As used herein, a "sufficient amount" or "an amount sufficient to" achieve a particular result in a patient having an IL-13-mediated disease or disorder refers to an amount of a therapeutic agent (*e.g.,* an antibody such as tralokinumab) that is effective to produce a desired effect, which is optionally a therapeutic effect (*i.e.,* by administration of a therapeutically effective amount). In some aspects, such particular result is a reduction in IL-13 activity in a patient in need thereof.

The term "sample" as used herein includes any biological fluid or tissue, such as whole blood, serum, muscle, saliva, or skin obtained from a subject. Samples include any biological fluid or tissue, such as whole blood, serum, muscle, saliva, urine, synovial fluid, bone marrow, cerebrospinal fluid, nasal secretions, sputum, amniotic fluid, bronchoalveolar lavage fluid, lung tissue, peripheral blood mononuclear cells, total white blood cells, lymph node cells, spleen cells, tonsil cells, or skin. In some specific aspects, that sample is blood or a fraction thereof, muscle, skin, or a combination thereof. Samples can be obtained by any means known in the art. In some aspects, a sample is a computed tomography (CT) scan of a patient's organ or tissue including, but not limited to the lungs. In some aspects, a sample can be derived by taking biological samples from a number of subjects and pooling them or pooling an aliquot of each subjects' biological sample. The pooled sample can be treated as a sample from a single subject. The term sample also includes experimentally separated fractions of all of the preceding. For example, a blood sample can be fractionated into serum or into fractions containing particular types of cells. In some aspects, a sample can be a combination of samples from an individual, such as a combination of a tissue and fluid sample.

In order to apply the methods and systems of the disclosure, samples from a patient can be obtained before or after the administration of a therapy to treat an IL-13-mediated disease or disorder. In some cases, successive samples can be obtained from the patient after therapy has commenced or after therapy has ceased. Samples can, for example, be requested by a healthcare provider (*e.g.,* a doctor) or healthcare benefits provider, obtained and/or processed by the same or a different healthcare provider (*e.g.,* a nurse, a hospital) or a clinical laboratory, and after processing, the results can be forwarded to the original healthcare provider or yet another healthcare provider, healthcare benefits provider or the patient. Similarly, the measuring/determination of one or more scores, comparisons between scores, evaluation of the scores and treatment decisions can be performed by one or more healthcare providers, healthcare benefits providers, and/or clinical laboratories.

As used herein, the term "healthcare provider" refers to individuals or institutions that directly interact and administer to living subjects, *e.g.,* human patients. Non-limiting examples of healthcare providers include doctors, nurses, technicians, therapist, pharmacists, counselors, alternative medicine practitioners, medical facilities, doctor's offices, hospitals, emergency rooms, clinics, urgent care centers, alternative medicine clinics/facilities, and any other entity providing general and/or specialized treatment, assessment, maintenance, therapy, medication, and/or advice relating to all, or any portion of, a patient's state of health, including but not limited to general medical, specialized medical, surgical, and/or any other type of treatment, assessment, maintenance, therapy, medication and/or advice.

As used herein, the term "clinical laboratory" refers to a facility for the examination or processing of materials derived from a living subject, *e.g.,* a human being. Non-limiting examples of processing include biological, biochemical, serological, chemical, immunohematological, hematological, biophysical, cytological, pathological, genetic, or other examination of materials derived from the human body for the purpose of providing information, *e.g.,* for the diagnosis, prevention, or treatment of any disease or impairment of, or the assessment of the health of living subjects, *e.g.,* human beings. These examinations can also include procedures to collect or otherwise obtain a sample, prepare, determine, measure, or otherwise describe the presence or absence of various substances in the body of a living subject, *e.g.,* a human being, or a sample obtained from the body of a living subject, *e.g.,* a human being.

As used herein, the term "healthcare benefits provider" encompasses individual parties, organizations, or groups providing, presenting, offering, paying for in whole or in part, or being otherwise associated with giving a patient access to one or more healthcare benefits, benefit plans, health insurance, and/or healthcare expense account programs.

In some aspects, a healthcare provider can administer or instruct another healthcare provider to administer a therapy to treat an IL-13-mediated disease or disorder. A healthcare provider can implement or instruct another healthcare provider or patient to perform the following actions: obtain a sample, process a sample, submit a sample, receive a sample, transfer a sample, analyze or measure a sample, quantify a sample, provide the results obtained after analyzing/measuring/quantifying a sample, receive the results obtained after analyzing/measuring/quantifying a sample, compare/score the results obtained after analyzing/measuring/quantifying one or more samples, provide the comparison/score from one or more samples, obtain the comparison/score from one or more samples, administer a therapy (*e.g.,* a therapeutic agent that treats an IL-13-mediated disease or disorder such as asthma, IPF, COPD, ulcerative colitis, or atopic dermatitis), commence the administration of a therapy, cease the administration of a therapy, continue the administration of a therapy, temporarily interrupt the administration of a therapy, increase the amount of an administered therapeutic agent, decrease the amount of an administered therapeutic agent, continue the administration of an amount of a therapeutic agent, increase the frequency of administration of a therapeutic agent, decrease the frequency of administration of a therapeutic agent, maintain the same dosing frequency on a therapeutic agent, replace a therapy or therapeutic agent by at least another therapy or therapeutic agent, combine a therapy or therapeutic agent with at least another therapy or additional therapeutic agent.

In some aspects, a healthcare benefits provider can authorize or deny, for example, collection of a sample, processing of a sample, submission of a sample, receipt of a sample, transfer of a sample, analysis or measurement a sample, quantification a sample, provision of results obtained after analyzing/measuring/quantifying a sample, transfer of results obtained after analyzing/measuring/quantifying a sample, comparison/scoring of results obtained after analyzing/measuring/quantifying one or more samples, transfer of the comparison/score from one or more samples, administration of a therapy or therapeutic agent, commencement of the administration of a therapy or therapeutic agent, cessation of the administration of a therapy or therapeutic agent, continuation of the administration of a therapy or therapeutic agent, temporary interruption of the administration of a therapy or therapeutic agent, increase of the amount of administered therapeutic agent, decrease of the amount of administered therapeutic agent, continuation of the administration of an amount of a therapeutic agent, increase in the frequency of administration of a therapeutic agent, decrease in the frequency of administration of a therapeutic agent, maintain the same dosing frequency on a therapeutic agent, replace a therapy or therapeutic agent by at least another therapy or therapeutic agent, or combine a therapy or therapeutic agent with at least another therapy or additional therapeutic agent.

In addition a healthcare benefits provides can, *e.g.,* authorize or deny the prescription of a therapy, authorize or deny coverage for therapy, authorize or deny reimbursement for the cost of therapy, determine or deny eligibility for therapy, etc.

In some aspects, a clinical laboratory can, for example, collect or obtain a sample, process a sample, submit a sample, receive a sample, transfer a sample, analyze or measure a sample, quantify a sample, provide the results obtained after analyzing/measuring/quantifying a sample, receive the results obtained after analyzing/measuring/quantifying a sample, compare/score the results obtained after analyzing/measuring/quantifying one or more samples, provide the comparison/score from one or more samples, obtain the comparison/score from one or more samples, or other related activities.

As used herein, the term "Computed Tomography" or "CT" refers to an imaging method using tomographic images (virtual 'slices') of specific areas of a scanned organ, tissue or object. Digital geometry processing is used to generate a three-dimensional (3D) image of the inside of an object or organ from a series of two-dimensional (2D) radiographic images taken around a single axis of rotation.

As used herein, the term "Computed Tomography scan" or "CT scan" refers to the production of tomographic images obtained using any method suitable including, but not limited to, x-rays, multidetector computed tomography (MDCT), high-resolution computed tomography (HRCT), positron emission tomography (PET), positron emission tomography computed tomography (PET-CT) single-photon emission computed tomography (SPECT), magnetic resonance imaging (MRI), computed axial tomography (CAT scan), computer-assisted tomography, xenon ventilation computed tomography, and hyperpolarized gas lung MRI ventilation imaging.

## II. DPP4 as a Biomarker

The term "DPP4" as used herein refers to the dipeptidyl peptidase IV protein (EC 3.4.14.5; Uniprot: P27487) encoded by the DPP4 gene. DPP4 is also known as DPP-IV, adenosine deaminase complexing protein 2, or CD26 (cluster of differentiation 26). DPP4 is related to attractin, FAP, DPP8 and DPP9. DPP4 is a highly conserved multifunctional type II transmembrane glycoprotein, which is present both in circulation (plasma) and on the surface of several cell types, including epithelial, endothelial and lymphoid cells. DPP4 is part of the serine protease family that is involved in T-cell co-stimulation, chemokine biology, type II diabetes, and tumor biology (Zhong et al., Atherosclerosis 2013;226:305-314). The endogenous substrates of DPP4 include a wide variety of proline-containing peptides such as growth factors, chemokines, neuropeptides and vasoactive peptides (Gorrell, M., Clin. Sci. 108, 277-292, 2005; McIntosh, C. H. S., et al. Int. J. Biochem. Cell Biol. 38, 860-872, 2006). A role for DPP4 in inflammatory respiratory diseases like asthma is suggested by Giovannini-Chami (Giovannini-Chami et al., European Respiratory Journal. 2012 May;39(5):1197-205), who found elevated DPP4 transcripts (and other Th2 signature genes) in the nasal epithelia of children with dust mite allergic rhinitis, associated with uncontrolled asthma. The term DPP4 also includes fragments, variants (e.g., the K1R, V7I, S437I, T557I, D663E variants known in the arts), and derivatives thereof (e.g., glycosylated or aglycosilated protein forms of the DPP4 protein, or otherwise chemically modified forms of the protein). In some aspects, the term DPP4 refers to the DPP4 gene, which includes genomic DNA, cDNA, mRNA, and fragments thereof. In some aspects, the term DPP4 also refers to oligonucleotides capable of specifically hybridizing to the DPP4 gene under stringent conditions. In some aspects, the oligonucleotides comprise nucleobases different from A, T, C, G, or U, for example, universal bases.

The term "level", e.g., as in "DPP4 level" refers to a measurement that is made using any analytical method for detecting presence or expression of DPP4 (protein expression or gene expression) in a biological sample and that indicates the presence, absence, absolute amount or concentration, relative amount or concentration, titer, expression level, ratio of measured levels, or the like, of, for, or corresponding to DPP4 in the biological sample. The exact nature of the "value" or "level" depends on the specific designs and components of the particular analytical method employed to detect DPP4 (e.g., immunoassays, mass spectrometry methods, *in vivo* molecular imaging, gene expression profiling, aptamer-based assays, etc.). See, e.g., U.S. 2010/00221752.

As used herein with reference to DPP4, the terms "elevated DPP4," "high DPP4," "elevated DPP4 level," or "high DPP4 level" refer to a level in a biological sample (e.g., blood serum) that is higher than a normal level or range. The normal level or range for DPP4 is defined in accordance with standard practice. Thus, the level measured in a particular biological sample can be compared with level or range of levels determined in similar normal samples. In this context, a normal sample would be a sample obtained from an individual with no detectable IL-13-mediated disease symptoms. The level of DPP4 is said to be elevated wherein the DPP4 is present in the test sample at a higher level or range than in a normal sample.

The methods disclosed herein can be carried out using any sample that may contain soluble DPP4, as well as samples containing the membrane bound form of DPP4, its intracellular, transmembrane, or extracellular moieties, or any peptide fraction thereof. Convenient samples include, for example, blood, blood cells, serum, plasma, urine, etc. In some aspects, the sample can be pretreated as necessary by dilution in an appropriate buffer solution or concentrated. Any of a number of standard aqueous buffer solutions and/or protease inhibitor, employing any of a variety of buffers, such as phosphate, Tris, or the like, at physiological pH, can be used.

DPP4 levels (either expressed protein levels, or nucleic acid levels such as mRNA levels) can be detected and quantified by any of a number of methods well known to those of skill in the art. These methods include analytic biochemical methods such as electrophoresis, capillary electrophoresis, high performance liquid chromatography (HPLC), thin layer chromatography (TLC), hyperdiffusion chromatography, mass spectroscopy and the like, or various immunological methods such as fluid or gel precipitin reactions, immunodiffusion (single or double), immunohistochemistry, affinity chromatography, immunoelectrophoresis, radioimmunoassay (RIA), enzyme-linked immunosorbent assays (ELISAs), immunofluorescent assays, Western blotting, and the like.

In one aspect, DPP4 can be detected and/or quantified in an electrophoretic polypeptide separation (e.g., a 1- or 2-dimensional electrophoresis). Means of detecting polypeptides using electrophoretic techniques are well known to those skilled in the art (see generally, R. Scopes (1982) Polypeptide Purification, Springer-Verlag, N.Y.; Deutscher, (1990) Methods in Enzymology Vol. 182: Guide to Polypeptide Purification, Academic Press, Inc., N.Y.). A variation of this aspect utilizes a Western blot (immunoblot) analysis to detect and quantify the presence of DPP4 in the sample. This technique generally comprises separating sample polypeptides by gel electrophoresis on the basis of molecular weight, transferring the separated polypeptides to a suitable solid support (such as a nitrocellulose filter, a nylon filter, or derivatized nylon filter), and incubating the sample with antibodies that specifically bind the analyte. Antibodies that specifically bind to the analyte may be directly labeled or alternatively may be detected subsequently using labeled antibodies (e.g., labeled sheep anti-mouse antibodies) that specifically bind to a domain of the primary antibody.

In some aspects, the sample and/or DPP4 is transformed in some manner in the course of the detection and/or quantitation assay. For example, the sample can be fractionated such that DPP4 is separated from at least one other sample component. DPP4 can be recovered in a liquid fraction or can be detected while embedded in a separation medium, such as a gel. For mass spectroscopy, DPP4 is volatilized for detection.

In a specific aspect, DPP4 is detected and/or quantified in the biological sample using an immunoassay. For a general review of immunoassays, see also Methods in Cell Biology Volume 37: Antibodies in Cell Biology, Asai, ed. Academic Press, Inc. New York (1993); Basic and Clinical Immunology 7th Edition, Stites & Terr, eds. (1991). In some aspects, the immunoassay can use one or more anti-DPP4 antibodies or antigen binding fragments thereof which recognize human DDP4.

In certain aspects, the immunoassay comprises a sandwich immunoassay, e.g., an enzyme-linked immunosorbent assay (ELISA) or a sandwich electrochemiluminescent (ECL) assay, in which a first anti-DPP4 "capture" antibody or antigen-binding fragment thereof is attached to a solid support, antigen from a sample or standard is allowed to bind to the capture antibody, and then a second anti-DPP4 "detection" antibody or antigen binding fragment thereof is added and detected either by an enzymatic reaction, an ECL reaction, radioactivity, or other detection method.

In certain aspects, the immunoassay comprises the following steps: First, the capture antibody or fragment thereof is allowed to bind to a solid support, *e.g.,* a multi-well plate or other assay device known to those of ordinary skill in the art. The capture antibody is allowed to attach for a period of time, *e.g.,* overnight, and then unbound antibody is removed. The plate can then be washed to remove any unbound capture antibody. The plate can then be treated with a blocking solution to allow non-specific protein to bind to any unbound regions of the solid support.

Typical blocking solutions include an unrelated protein, *e.g.,* nonfat dry milk or serum albumin. The plate can then again be washed to remove any unbound blocking solution. Next, a sample suspected of containing DPP4 is added to the plate. Samples are typically serially diluted and plated in duplicate or triplicate. Controls, including standard amounts of DPP4 or a suitable fragment thereof and various negative controls are also included. The antigen is allowed to bind to the capture antibody for a period of time, *e.g.,* one hour at room temperature. Following incubation, the plate can then be washed to remove any unbound antigen.

Next, a detection antibody is added. The detection antibody is typically an anti-DPP4 antibody that binds to a different DPP4 epitope than the capture antibody. The detection antibody can be labeled or unlabeled. Where the detection antibody is unlabeled, an addition step of addition a labeled secondary antibody will be required, as is well known by those of ordinary skill in the art. The detection antibody can be directly labeled with an enzyme, *e.g.,* horseradish peroxidase or alkaline phosphatase, or can be labeled with a tag that will allow an enzyme to bind. For example the detection antibody can be conjugated to biotin, and the enzyme attached in a subsequent step by allowing enzyme-conjugated streptavidin to bind to the biotin tag.

Alternatively the detection antibody can be conjugated to a chemiluminescent, fluorescent, or ECL tag. An example of the latter is a ruthenium chelate. Following incubation, the plate can then be washed to remove any unbound detection antibody.

Detection of the detection antibody can be accomplished by methods that vary based on the type of detection antibody that is used. If the detection antibody is tagged with biotin, then enzyme-conjugated streptavidin is added, unbound streptavidin is washed away, and a substrate is added which provides a colorimetric reaction that can be read, *e.g.,* on a spectrophotometer. If the detection antibody is conjugated to a ruthenium chelate, the plate is subjected to electrical current, and light emission is measured.

In certain aspects, the method directly measures DPP4 levels in a patient sample, where absolute levels are calculated by plotting the immunoassay results on a standard curve using, *e.g.,* purified full length or a DPP4 fragment. The detected signal from the detection antibody can then be quantitated based on the various standards and controls included on the plate. By plotting the results on a standard curve, the absolute levels of DPP4 in the test samples can be calculated, *e.g.,* in ng DPP4/mL or ng DPP4/mg protein.

Based on comparison to known control samples, a "DPP4 threshold level" can be determined, and test samples that fall above that DPP4 threshold level (e.g., a DPP4 protein expression and/or gene expression threshold level) can indicate that the patient from whom the sample of taken may benefit from treatment with an IL-13 antagonist, for example, an anti-IL-13 antibody such as tralokinumab. DPP4 threshold levels (e.g., protein expression levels or gene expression levels) must be predetermined, and must be matched as to the type of sample (*e.g.,* serum, lung tissue, skin), the type of disease (*e.g.,* asthma, IPF, COPD, UC, or atopic dermatitis), and in some instances, the assay used. In some aspects, the predetermined DPP4 threshold level in a serum sample can be a DPP4 median or DPP4 mean level as depicted in FIGS. 5-6, 18-20 or 24. In some aspects, the predetermined DPP4 threshold level in a serum sample can be at least about 100 ng DPP4/mL serum to about 1000 ng DPP4/mL serum, *e.g.,* at least about 100 ng DPP4/mL serum, at least about 150 ng DPP4/mL serum, at least about 200 ng DPP4/mL serum, at least about 250 ng DPP4/mL serum, about 300 ng DPP4/mL serum, at least about 350 ng DPP4/mL serum, at least about 400 ng DPP4/mL serum, at least about 450 ng DPP4/mL serum, at least about 500 ng DPP4/mL serum, at least about 550 ng DPP4/mL serum, at least about 600 ng DPP4/mL serum, at least about 650 ng DPP4/mL serum, at least about 700 ng DPP4/mL serum, at least about 750 ng DPP4/mL serum, at least about 800 ng DPP4/mL serum, at least about 850 ng DPP4/mL serum, or at least about 900 ng DPP4/mL serum. In some aspects, the predetermined DPP4 threshold level is at least about 300 ng DPP4/mL, at least about 310 ng DPP4/mL, at least about 320 ng DPP4/mL, at least about 330 ng DPP4/mL, at least about 340 ng DPP4/mL, at least about 350 ng DPP4/mL, at least about 360 ng DPP4/mL, at least about 370 ng DPP4/mL, at least about 380 ng DPP4/mL, at least about 390 ng DPP4/mL, at least 400 ng DPP4/mL, at least about 410 ng DPP4/mL, at least about 420 ng DPP4/mL, at least about 430 ng DPP4/mL, at least about 440 ng DPP4/mL, at least about 450 ng DPP4/mL, at least about 460 ng DPP4/mL, at least about 470 ng DPP4/mL, at least about 480 ng DPP4/mL, at least about 490 ng DPP4/mL, at least 500 ng DPP4/mL, at least about 510 ng DPP4/mL, at least about 520 ng DPP4/mL, at least about 530 ng DPP4/mL, at least about 540 ng DPP4/mL, at least about 550 ng DPP4/mL, at least about 560 ng DPP4/mL, at least about 570 ng DPP4/mL, at least about 580 ng DPP4/mL, at least about 590 ng DPP4/mL, or at least 600 ng DPP4/mL.

In some aspects, the predetermined DPP4 threshold level in a serum sample can be at least about 200 ng DPP4/mL serum to about 500 ng DPP4/mL serum. In some aspects, the predetermined DPP4 threshold level in a serum sample can be at least about 300 ng DPP4/mL serum to about 400 ng DPP4/mL serum. In some aspects, the predetermined DPP4 threshold level in a serum sample can be at least about 315 ng DPP4/mL serum to about 380 ng DPP4/mL serum. In some aspects, DPP4 levels in serum are measured using ELISA. In some specific aspects, the ELISA is a QUANTIKINE® assay.

DPP4 levels quantified obtained using a QUANTIKINE® DPP4 assay (Example 2) and serum samples from a population chronic oral corticosteroid users indicated that the median value was 371 ng/mL, with a minimum value of 134 ng/mL, and a maximum value of 905 ng/mL. See FIG. 24. Accordingly, in some aspects, the predetermined DPP4 threshold is such median value, i.e., about 371 ng DPP4/mL of serum.

DPP4 levels quantified obtained using a QUANTIKINE® DPP4 assay (Example 2) and serum samples from a population of non-users or chronic oral corticosteroid (OCS) users indicated that the median value was 321 ng/mL, with a minimum value of 169 ng/mL, and a maximum value of 540 ng/mL. See FIG. 24. Accordingly, in some aspects, the predetermined DPP4 threshold is such median value, i.e., about 321 ng DPP4/mL of serum.

DPP4 levels quantified obtained using a QUANTIKINE® DPP4 assay (Example 2) and serum samples from 437 subjects enrolled in a clinical study (see Example 3) indicated that the median value was 364 ng/mL, with a minimum value of 134 ng/mL, and a maximum value of 905 ng/mL. Accordingly, in some aspects, the predetermined DPP4 threshold is such median value, i.e., about 364 ng DPP4/mL of serum. In the placebo group, the median value was 343 ng DPP4 /mL of serum. *See* Table 5. Thus, in some aspects, the predetermined DPP4 threshold is about 343 ng DPP4/mL of serum. In the group treated with tralokinumab every two weeks (Q2W) the median value was 372 ng DPP4/mL of serum. *See* Table 5. Accordingly, in some aspects the predetermined DPP4 threshold is about 372 ng DPP4/mL of serum. In the group treated with tralokinumab every 4 weeks (Q4W) the median value was 375 DPP4 ng/mL of serum. *See* Table 5. Thus, in some aspects, the predetermined DPP4 threshold is about 375 DPP4 ng/mL of serum.

As indicated in the QUANTIKINE® DPP4 assay manufacturer's manual, normal DPP4 levels in serum, EDTA plasma, or heparin plasma using a QUANTIKINE® human DPP4 immunoassay are 197-615 ng/mL, 187-604 ng/mL, and 159-588 ng/mL respectively. For urine, normal DPP4 levels are 2.26-13.3 ng/mL. For saliva, normal DPP4 levels measured are 13.0-69.9 ng/mL.

The DPP4 threshold level (e.g., a protein expression level or a gene expression level) can vary based on the nature of the assay, *e.g.,* the capture and detection antibodies used, the source, purity, and composition of the DPP4 standard, and the like. In one aspect, instead of using an arbitrary threshold level to determine whether a patient can benefit from treatment with an IL-13 antagonist (e.g., an anti-IL-13 antibody such as tralokinumab), the patient's DPP4 levels can be compared to one or more control DPP4 levels. According to this aspect, the test sample (*e.g.,* a sample from a patient suffering from an IL-13-mediated disease or disorder) is compared to one or more control samples (*e.g.,* samples taken from normal healthy individuals, earlier samples taken from the same patient, samples taken from patients with a non-IL-13-mediated subset of the patient's disease, *e.g.,* asthma, COPD, IPF, UC, or atopic dermatitis, a predetermined standard amount of isolated DPP4, or a combination thereof).

The results can be expressed as a ratio with the control samples to determine a percent increase or a percent decrease in the patient's DPP4 levels (e.g., a protein expression level or a gene expression level) compared to the control DPP4 levels. According to this aspect, the control sample can be a matched pair with the patient sample, *e.g.,* one or more of whole blood if the patient sample is whole blood, serum if the patient sample is serum, plasma if the patient sample is plasma, saliva if the patient sample is saliva, urine if the patient sample is urine, sputum if the patient sample is sputum, bronchoalveolar lavage fluid if the patient sample is bronchoalveolar lavage fluid, lung tissue if the patient sample is lung tissue, or skin if the patient sample is skin.

A DPP4 level (e.g., a protein expression level or a gene expression level) is considered to be increased if it is at least about 10%, at least 20%, at least about 30%, at least about 40%, at least about 50%, at least about 60%, at least about 70%, at least about 80%, at least about 90%, or at least about 100% higher than the control DPP4 level. A DPP4 is considered to be decreased if it is at least about 10%, at least 20%, at least about 30%, at least about 40%, at least about 50%, at least about 60%, at least about 70%, at least about 80%, at least about 90%, or at least about 100% lower than the control DPP4 level.

Immunoassays for detecting DPP4 can be either competitive or noncompetitive. Noncompetitive immunoassays are assays in which the amount of captured analyte is directly measured. In competitive assays, the amount of analyte in the sample is measured indirectly by measuring the amount of an added (exogenous) labeled analyte displaced (or competed away) from a capture agent by the analyte present in the sample. In one competitive assay, a known amount of, in this case, labeled DPP4 is added to the sample, and the sample is then contacted with a capture agent. The amount of labeled DPP4 bound to the antibody is inversely proportional to the concentration of DPP4 present in the sample.

DPP4 detection assays can be scored (as positive or negative or quantity of analyte) according to standard methods well known to those of skill in the art. The particular method of scoring will depend on the assay format and choice of label. For example, a Western Blot assay can be scored by visualizing the colored product produced by the enzymatic label. A clearly visible colored band or spot at the correct molecular weight is scored as a positive result, while the absence of a clearly visible spot or band is scored as a negative. The intensity of the band or spot can provide a quantitative measure of analyte concentration.

Once determined, a DPP4 level (e.g., a protein expression level or a gene expression level) can be recorded in a patient medical record. In some aspects, the methods disclosed herein include making a diagnosis, often a differential diagnosis, based at least in part on the DPP4 level.

As used herein, the term "differential diagnosis" refers to the determination of which of two or more diseases with similar symptoms is likely responsible for a subject's symptom(s), based on an analysis of the clinical data. The term can also refer to the determination of whether a patient is susceptible to treatment with an IL-13-antagonist depending on whether the measured DPP4 level (e.g., a protein expression level or a gene expression level) in a sample from the patient sample is above a predetermined DPP4 threshold level, or is elevated relative to the DPP4 level in one or more control samples.

In particular aspects, the methods disclosed herein include informing the subject of a result of the DPP4 assay and/or of a diagnosis based at least in part on the DPP4 level. The patient can be informed verbally, in writing, and/or electronically.

This diagnosis can also be recorded in a patient medical record. For example, in various aspects, the diagnostic of an IL-13-mediated disease or disorder treatable with a specific IL-13 antagonist is recorded in a medical record. The term "medical record" or "patient medical record" refers to an account of a patient's examination and/or treatment that typically includes one or more of the following: the patient's medical history and complaints, the physician's physical findings, the results of diagnostic tests and procedures, and patient medications and therapeutic procedures. A medical record is typically made by one or more physicians and/or physicians' assistants and it is a written, transcribed or otherwise recorded record and/or history of various illnesses or injuries requiring medical care, and/or inoculations, and/or allergies, and/or treatments, and/or prognosis, and/or frequently health information about parents, siblings, and/or occupation. The record may be reviewed by a physician in diagnosing the condition.

The medical record can be in paper form and/or can be maintained in a computer-readable medium. The medical record can be maintained by a laboratory, physician's office, a hospital, a healthcare maintenance organization, an insurance company, and/or a personal medical record website. In some aspects, a diagnosis, based at least in part on the DPP4 level, is recorded on or in a medical alert article such as a card, a worn article, and/or a radiofrequency identification (RFID) tag. As used herein, the term "worn article" refers to any article that can be worn on a subject's body, including, but not limited to, a tag, bracelet, necklace, arm band, or head band.

The methods disclosed herein also include prescribing, initiating, and/or altering prophylaxis and/or therapy, e.g., for an IL-13 mediated disease or disorder (e.g., asthma, IPF, COPD or atopic dermatitis). In certain aspects, the methods can entail ordering and/or performing one or more additional assays. For example, if the DPP4 level (e.g., a protein expression level or a gene expression level) is determined to be within a normal range (i.e., not elevated), the DPP4 assay may be repeated to rule out a false negative result, and/or one or more additional DPP4 assays may be performed to monitor the subject's status. If the DPP4 level (e.g., a protein expression level or a gene expression level) is determined to be elevated, it may be desirable repeat the DPP4 assay to rule out a false positive result. In certain aspects, it will be desirable to assay another indicator of, e.g., IL-13 mediated disease (e.g., asthma, IPF, COPD or atopic dermatitis), to confirm a diagnosis.

A person skilled in the art would understand that DPP4 levels (e.g., a protein expression level or a gene expression level) can be used according to the methods disclosed herein, including but not limited to treatment, diagnostic, and monitoring methods, as (i) positive selectors, *i.e.,* a specific action would be taken (e.g., treating a patient having an IL-13-mediated disease or disorder with an IL-13 antagonist) if the DPP4 level (e.g., a protein expression level or a gene expression level) in a sample taken from the patient is above a predetermined DPP4 threshold level, or is elevated relative to the DPP4 level in one or more control samples; or (ii) negative selectors, *i.e.,* a specific action would be taken (e.g., treating a patient having an IL-13-mediated disease or disorder with an IL-13 antagonist) if the DPP4 level in a sample taken from the patient is below a predetermined DPP4 threshold level, or is lower relative to the DPP4 level in one or more control samples; or (iii) both positive and negative selectors, for example, a specific treatment could cease (e.g., oral corticosteroid treatment) and a different treatment could commence (e.g., treatment with an anti-IL-13 antibody) if the DPP4 level in a sample taken from the patient is above/below a predetermined DPP4 threshold level, or is higher/lower relative to the DPP4 level in one or more control samples

III. Methods of Diagnosis and Treatment

This disclosure provides a method of treating a patient having an IL-13-mediated disease or disorder, or a patient with a pulmonary disease or disorder, inflammatory bowel disease or disorder, or chronic inflammatory skin disease or disorder of unknown etiology which might be IL-13-mediated, comprising administering an IL-13 antagonist to the patient if the DPP4 level in a sample (e.g., a protein expression level or a gene expression level) taken from the patient is above a predetermined DPP4 threshold level, or is elevated relative to the DPP4 level in one or more control samples. In one aspect, the patient's DPP4 level is measured in an immunoassay employing one or more anti-DPP4 antibodies or antigen binding fragments thereof which recognize human DPP4, or antigen-binding fragments, variants or derivatives thereof.

This disclosure also provides methods, assays, and kits to facilitate a determination by a healthcare provider, a healthcare benefits provider, or a clinical laboratory to as to whether a patient will benefit from treatment with an IL-13 antagonist, *e.g.,* an ant-IL-13 antibody or antigen-binding fragment thereof, *e.g.,* tralokinumab, or a fragment, variant, or derivative thereof, an antibody or fragment thereof that binds to the same IL-13 epitope as tralokinumab, or an antibody or fragment thereof that competitively inhibits binding of tralokinumab to IL-13. The methods assays and kits provided herein will also facilitate a determination by a healthcare provider, a healthcare benefits provider, or a clinical laboratory to as to whether a patient will benefit from treatment with any other IL-13 antagonist IL-13 disclosed herein, or known to those of ordinary skill in the art.

The present disclosure provides a method of treating a patient having an interleukin-13 (IL-13)-mediated disease or disorder (e.g., asthma, IPF, COPD or atopic dermatitis), comprising administering an IL-13 antagonist to the patient if the level of DPP4 (e.g., a protein expression level or a gene expression level) in a sample taken from the patient is above a predetermined DPP4 threshold level, or is above the DPP4 level in one or more control samples. In some aspects, the sample is obtained from the patient and is submitted for measurement of the level of DPP4 in the sample, for example, to a clinical laboratory.

Also provided is a method of treating a patient having an IL-13-mediated disease or disorder comprising (a) submitting a sample taken from the patient for measurement of the DPP4 level in the sample, wherein the patient's DPP4 level is measured, for example, in an immunoassay employing one or more anti-DPP4 antibodies or antigen binding fragments thereof which recognize human DPP4; and, (b) administering an IL-13 antagonist to the patient if the patient's DPP4 level in the sample is above a predetermined DPP4 threshold level, or is above the DPP4 level in one or more control samples.

The disclosure also provides a method of treating a patient having an IL-13-mediated disease or disorder comprising (a) measuring the DPP4 level in a sample obtained from a patient having an IL-13-mediated disease or disorder, wherein the patient's DPP4 level in the sample is measured, for example, in an immunoassay employing one or more anti-DPP4 antibodies or antigen binding fragments thereof which recognize human DPP4; (b) determining whether the patient's DPP4 level in the sample is above a predetermined DPP4 threshold level, or is above the DPP4 level in one or more control samples; and, (c) advising a healthcare provider to administer an IL-13 antagonist to the patient if the patient's DPP4 level is above a predetermined DPP4 threshold level, or is above the DPP4 level in one or more control samples.

In some aspects, the patient's DPP4 level is measured in an immunoassay employing one or more anti-DPP4 antibodies or antigen binding fragments thereof which recognize human DPP4. In other aspects, the patients DPP4 level (e.g., DNA or RNA level) is measured in an assay employing one or more oligonucleotide probes capable of specifically measuring the expression of the DPP4 gene.

In certain aspects, the DPP4 detection assay (e.g., an immunoassay) is performed on a sample obtained from the patient, by the healthcare professional treating the patient (e.g., using an immunoassay as described herein, formulated as a "point of care" diagnostic kit). In some aspects, a sample is obtained from the patient and is submitted, e.g., to a clinical laboratory, for measurement of the DPP4 level in the sample according to the healthcare professional's instructions (e.g., using an immunoassay as described herein). In certain aspects, the clinical laboratory performing the assay will advise the healthcare provide as to whether the patient can benefit from treatment with an IL-13 antagonist based on whether the patient's DPP4 level is above a predetermined DPP4 threshold value or is elevated relative to one or more control samples.

In certain aspects, this disclosure provides a method of treating a patient having an IL-13-mediated disease or disorder over a period of time, comprising: measuring a first DPP4 level (e.g., protein expression level or gene expression level) in a first sample taken from the patient, or submitting a first sample taken from the patient for measurement of a first DPP4 level in the sample, wherein the patient's DPP4 level is, for example, measured in an immunoassay employing one or more anti-DPP4 antibodies or antigen binding fragments thereof which recognize human DPP4, and administering an IL-13 antagonist to the patient if the patient's DPP4 level in the first sample is above a predetermined DPP4 threshold level, or is elevated relative to the DPP4 level in one or more control samples. The test can be performed by a healthcare provider or a clinical laboratory as noted above.

According to this aspect, the method can further comprise: measuring a second DPP4 level (e.g., protein expression level or gene expression level) in a second sample taken from the patient, or submitting a second sample taken from the patient for measurement of a second DPP4 level in the sample, wherein the patient's DPP4 level is again measured, for example, in an immunoassay employing one or more anti-DPP4 antibodies or antigen binding fragments thereof which recognize human DPP4; comparing the first and second DPP4 levels in the patient, and altering the dose, *e.g.,* increasing or maintaining the amount or frequency of the IL-13 antagonist administered to the patient, or even discontinuing IL-13 antagonist therapy if the patient's DPP4 level in the second sample is higher than the DPP4 level in the first sample, or maintaining or reducing the amount or frequency of the IL-13 antagonist administered to the patient if the patient's DPP4 level in the second sample is lower than or about the same as the DPP4 level in the first sample.

In certain aspects of all method of treatment aspects provided herein, a "loading" dose of an IL-13 antagonist is administered to achieve a desired therapeutic level in the patient. If the loading dose does not affect the patient's DPP4 levels (e.g., protein expression levels or gene expression levels) significantly or the patient's DPP4 levels rise, a decision could be made to discontinue treatment - *e.g.,* to use a non-IL-13 antagonist therapy. If the loading dose results in steady or reduced DPP4 levels in the patient a decision could be made to reduce the dose size or frequency to a "maintenance" dose. It is important to note that the methods provided here are guidelines for a healthcare provider to administer treatment, and the ultimate treatment decision will be based on the healthcare provider's sound judgment.

In certain aspects, results of an immunoassay as provided herein can be submitted to a healthcare benefits provider for determination of whether the patient's insurance will cover treatment with an IL-13 antagonist.

In certain aspects this disclosure provides a method of treating a patient having an IL-13-mediated disease or disorder comprising: measuring, *e.g.,* in a clinical laboratory, the DPP4 level (e.g., protein expression level or gene expression level) in a first sample obtained from a patient having an IL-13-mediated disease or disorder, *e.g.,* a sample provided by a healthcare provider, wherein the patient's DPP4 level in the first sample is, for example, measured in an immunoassay employing one or more anti-DPP4 antibodies or antigen binding fragments thereof which recognize human DPP4, determining whether the patient's DPP4 level in the first sample is above a predetermined DPP4 threshold level, or is elevated relative to the DPP4 level in one or more control samples; and advising a healthcare provider to administer an IL-13 antagonist to the patient if the patient's DPP4 level is above a predetermined DPP4 threshold level, or is elevated relative to the DPP4 level in one or more control samples.

In certain aspects, this method can further comprise: measuring the DPP4 level (e.g., protein expression level or gene expression level) in a second sample obtained from the patient, *e.g.,* a sample provided by a healthcare provider, wherein the patient's DPP4 level is again measured, for example, in an immunoassay employing one or more anti-DPP4 antibodies or antigen binding fragments thereof which recognize human DPP4; determining whether the patient's DPP4 level in the second sample is higher than, about the same as, or lower than the DPP4 level measured in the first sample; and advising a healthcare provider to adjust the IL-13 antagonist therapy if indicated, *e.g.,* to increase or maintain the amount or frequency of the IL-13 antagonist administered to the patient, or discontinuing IL-13 antagonist therapy, if the patient's DPP4 level in the second sample is higher than the DPP4 level in the first sample, or to maintain or reduce the amount or frequency of the IL-13 antagonist administered to the patient if the patient's DPP4 level in the second sample is lower than or about the same as the DPP4 level in the first sample.

In some aspects, a sample is obtained from the patient and is submitted, *e.g.,* to a clinical laboratory, for measurement of the DPP4 level (e.g., protein expression level or gene expression level) in the sample, *e.g.,* using an immunoassay. In certain aspects, the clinical laboratory performing the assay will advise the healthcare provide as to whether the patient can benefit from treatment with an IL-13 antagonist based on whether the patient's DPP4 level (e.g., protein expression level or gene expression level) is above a predetermined DPP4 threshold value or is elevated relative to one or more control samples.

Similarly, this disclosure provides a method of monitoring the therapeutic efficacy of an IL-13 antagonist therapeutic regimen in a patient having an IL-13-mediated disease or disorder comprising: measuring, or instructing a clinical laboratory to measure the DPP4 level (e.g., protein expression level or gene expression level) in a first sample obtained from a patient having an IL-13-mediated disease or disorder, wherein the patient's DPP4 level is measured, for example, in an immunoassay employing one or more anti-DPP4 antibodies or antigen binding fragments thereof which recognize human DPP4; administering, or advising a healthcare professional to administer an IL-13 antagonist to a patient if the patient's DPP4 level in the first sample is above a predetermined DPP4 threshold level, or is elevated relative to the DPP4 level in one or more control samples; measuring the DPP4 level in a second sample obtained from the patient, wherein the patient's DPP4 level is again measured, for example, in an immunoassay employing one or more anti-DPP4 antibodies or antigen binding fragments thereof which recognize human DPP4, and determining, or obtaining results indicating whether the patient's DPP4 level in the second sample is higher than, about the same as, or lower than the DPP4 level measured in the first sample; wherein the IL-13 antagonist therapeutic regimen is effective if the patient's DPP4 level in the second sample is lower than or about the same as the DPP4 level in the first sample.

In certain aspects, a patient is diagnosed with a pulmonary disease or disorder, and in the course of diagnosis a determination can be made as whether to treat the patient with an IL-13 antagonist. Accordingly, in certain aspects this disclosure provides a method of treating a patient diagnosed with a pulmonary disease or disorder, comprising administering an IL-13 antagonist to the patient if the DPP4 level (e.g., protein expression level or gene expression level) in a sample taken from the patient is above a predetermined DPP4 threshold level, or is above the DPP4 level in one or more control samples; wherein the patient's DPP4 level is measured, for example, in an immunoassay employing one or more anti-DPP4 antibodies or antigen binding fragments thereof which recognize human DPP4.

In certain aspects this disclosure provides a method of treating a patient diagnosed with a pulmonary disease or disorder (e.g., asthma, IPF or COPD) or a chronic inflammatory skin disease or disorder (e.g., or atopic dermatitis) comprising:

(a) submitting a sample taken from the patient for measurement of the DPP4 level (e.g., protein expression level or gene expression level) in the sample, wherein the patient's DPP4 level is, for example, measured in an immunoassay employing one or more anti-DPP4 antibodies or antigen binding fragments thereof which recognize human DPP4; and,
(b) administering an IL-13 antagonist to a patient if the patient's DPP4 level in the sample is above a predetermined DPP4 threshold level, or is above the DPP4 level in one or more control samples.

In certain aspects this disclosure also provides a method of determining whether to treat a patient diagnosed with a pulmonary disease or disorder (e.g., asthma, IPF or COPD) or a chronic inflammatory skin disease or disorder (e.g., or atopic dermatitis) with an IL-13 antagonist therapeutic regimen comprising:

(a) measuring, or instructing a clinical laboratory to measure the DPP4 level (e.g., protein expression level or gene expression level) in a sample obtained from a patient diagnosed with a pulmonary disease or disorder (e.g., asthma, IPF or COPD) or a chronic inflammatory skin disease or disorder (e.g., or atopic dermatitis), wherein the patient's DPP4 level is measured, for example, in an immunoassay employing one or more anti-DPP4 antibodies or antigen binding fragments thereof which recognize human DPP4; and,
(b) treating, or instructing a healthcare provider to treat the patient with an IL-13 antagonist therapeutic regimen if the patient's DPP4 level in the sample is above a predetermined DPP4 threshold level, or is above the DPP4 level in one or more control samples.

In certain aspects, this disclosure provides a method of treating a patient diagnosed with a pulmonary disease or disorder (e.g., asthma, IPF or COPD) or a chronic inflammatory skin disease or disorder (e.g., or atopic dermatitis) comprising: submitting a first sample taken from the patient for measurement of a first DPP4 level (e.g., protein expression level or gene expression level) in the sample, wherein the patient's DPP4 level is measured, for example, in an immunoassay employing one or more anti-DPP4 antibodies or antigen binding fragments thereof which recognize human DPP4; administering an IL-13 antagonist to a patient if the patient's DPP4 level in the first sample is above a predetermined DPP4 threshold level, or is elevated relative to the DPP4 level in one or more control samples. The DPP4 levels can be measured by a healthcare professional or by a clinical laboratory that obtains a patient sample from a healthcare professional, and is instructed to measure the DPP4 in the sample by the healthcare professional.

In certain aspects the method of treatment provided above can further comprise submitting a second sample taken from the patient for measurement of a second DPP4 level (e.g., protein expression level or gene expression level) in the sample, wherein the patient's DPP4 level is again measured, for example, in an immunoassay employing one or more anti-DPP4 antibodies or antigen binding fragments thereof which recognize human DPP4; increasing or maintaining the amount or frequency of the IL-13 antagonist administered to the patient, or even discontinuing IL-13 antagonist therapy if the patient's DPP4 level in the second sample is higher than the DPP4 level in the first sample, or maintaining or reducing the amount or frequency of the IL-13 antagonist administered to the patient if the patient's DPP4 level in the second sample is lower than or about the same as the DPP4 level in the first sample. It is important to note that the methods provided here are guidelines for a healthcare provider to administer treatment, and the ultimate treatment decision will be based on the healthcare provider's sound judgment.

In certain aspects, this disclosure provides a method of determining whether to treat a patient diagnosed with a pulmonary disease or disorder (e.g., asthma, IPF or COPD); or a chronic inflammatory skin disease or disorder (e.g., or atopic dermatitis) with an IL-13 antagonist therapeutic regimen comprising measuring, or instructing a clinical laboratory to measure the DPP4 level (e.g., protein expression level or gene expression level) in a first sample obtained from a patient diagnosed with a pulmonary disease or disorder (e.g., asthma, IPF or COPD); or a chronic inflammatory skin disease or disorder (e.g., or atopic dermatitis), wherein the patient's DPP4 level is measured, for example, in an immunoassay employing one or more anti-DPP4 antibodies or antigen binding fragments thereof which recognize human DPP4; and treating, or instructing a healthcare provider to treat the patient with an IL-13 antagonist therapeutic regimen if the patient's DPP4 level in the first sample is above a predetermined DPP4 threshold level, or is elevated relative to the DPP4 level in one or more control samples. In certain aspects, the results of the DPP4 level measuring assay (e.g.,

an immunoassay) can be submitted to a healthcare benefits provider to determine whether the patient's insurance will cover treatment with an IL-13 antagonist.

In some aspects, the methods disclosed herein can be used to diagnose COPD. In other aspects, the methods disclosed herein can be used to prevent progression of COPD in a subject from one stage to a subsequent stage in the COPD GOLD classification. In another aspect, the methods disclosed herein allow monitoring the progression of COPD disease from one stage to a subsequent stage in the GOLD classification. The COPD markers disclosed herein are also suited to discriminate between humans suffering from COPD stage I/II and COPD stage III/IV as defined above. The discrimination between these COPD stages is important to determine the appropriate therapy. In another aspect, the methods disclosed herein allow monitoring the progression of CPOD from one stage to a subsequent stage in the GOLD classification. In another aspect, the methods disclosed herein allows monitoring the progress of a COPD therapy. In yet another aspect, the methods disclosed herein can be used to prevent or ameliorate the progression of COPD in a subject from one stage to a subsequent stage in the COPD GOLD classification. In some aspects, the methods disclosed herein can be used to prevent, treat, or ameliorate COPD exacerbations.

In certain aspects, the patient has been treated or is being treated with one or more additional medications, either before, during, or after administration of an IL-13 antagonist. Various other medications useful for treating, e.g., asthma, IPF, COPD, UC and atopic dermatitis are described elsewhere herein. In certain aspects the patient has been treated, continues to be treated, or will be treated with one or more additional medications comprising, e.g., a steroid, a bronchodilator, or a combination thereof. In certain aspects, the steroid is a corticosteroid. In some aspects, the corticosteroid is an oral corticosteroid. In some aspects, the steroid is fluticasone or budesonide. In some aspects, the bronchodilator is salbutamol or salmeterol. In certain aspects, the additional medication comprises at least one steroid, wherein the steroid is fluticasone or budesonide, and at least one bronchodilator, wherein the bronchodilator is salbutamol or salmeterol. In certain aspects, the one or more additional medications are administered by inhalation, by oral administration, by injection, or a combination thereof. In some aspect inhalation administration is conducted using a metered dose inhaler (MDI) or a dry powder inhaler (DPI).

In some aspects, the steroid is administered at a high dose. The term high dose when application to an inhaled corticosteroid (ICS) can refer, for example, to a total daily dose of at least 500 $\mu$g of ICS (e.g., fluticasone) DPI or at least 440 $\mu$g ICS MDI. In some aspects, the high ICS total daily dose is at least about 300 $\mu$g, at least about 350 $\mu$g, at least about 400 $\mu$g, at least about 450 $\mu$g, at least about 500 $\mu$g, at least about 550 $\mu$g, at least about 600 $\mu$g, at least about 650 $\mu$g, at least about 700 $\mu$g, at least about 750 $\mu$g, at least about 800 $\mu$g, at least about 850 $\mu$g, at least about 900 $\mu$g, at least about 950 $\mu$g, or at least 1000 $\mu$g of ICS (e.g., fluticasone) DPI. In some aspects, the high ICS total daily dose is at least about 300 $\mu$g, at least about 350 $\mu$g, at least about 400 $\mu$g, at least about 450 $\mu$g, at least about 500 $\mu$g, at least about 550 $\mu$g, at least about 600 $\mu$g, at least about 650 $\mu$g, at least about 700 $\mu$g, at least about 750 $\mu$g, at least about 800 $\mu$g, at least about 850 $\mu$g, at least about 900 $\mu$g, at least about 950 $\mu$g, or at least 1000 $\mu$g of ICS (e.g., fluticasone) MPI.

The term "high dose" when application to an inhaled corticosteroid (ICS) (e.g., fluticasone) in combination treatments (e.g., with a bronchodilator such as salmeterol) can refer, for example, to about 230 $\mu$g fluticasone and about 21 $\mu$g salmeterol as MDI at a dose of 2 inhalations twice per day, or to about 500 $\mu$g fluticasone and about 50 $\mu$g salmeterol as single dose DPI. Concentrations of corticosteroids considered to be high-dose alone as well as in combination with other therapeutic agents are well known in the art.

In certain aspects, the IL-13 antagonist comprises one or more of an anti-IL-13 antibody or antigen-binding fragment thereof *e.g.,* tralokinumab, an IL-13 mutein, *e.g.,* IL-13E13K (Kioi M, et al., Cell Immunol. 2004 229:41-51), an IL-4 mutein, *e.g.,* Pitrakinra (AER-001, BAY-16-9996) (Antoniu SA., Curr Opin Investig Drugs. 2010 11:1286-94), an anti-IL-13R$\alpha$1 antibody or antigen-binding fragment thereof, or an anti-IL-4R$\alpha$ antibody or antigen-binding fragment thereof. In certain aspects, the IL-13 antagonist is an anti-IL13 antibody, or antigen-binding fragment thereof. In certain aspects, the anti-IL-13 antibody or fragment thereof binds to the same IL-13 epitope as tralokinumab or competitively inhibits binding of tralokinumab to IL-13, or both. In certain aspects the antibody comprises tralokinumab or an antigen-binding fragment thereof. In other aspects, the antibody or fragment thereof consists of tralokinumab or an antigen-binding fragment thereof.

In some aspects, the anti-IL-13 antibody or fragment thereof binds to the same IL-13 epitope as lebrikizumab or competitively inhibits binding of lebrikizumab to IL-13, or both. In some aspects, the anti-IL-13 antibody or fragment thereof comprises lebrikizumab or an antigen-binding fragment thereof. In some aspects, the anti-IL-13 antibody or fragment thereof consists of lebrikizumab or an antigen-binding fragment thereof.

In some aspects, the samples used in the methods disclosed herein are taken from a patient and comprise one or more of whole blood, serum, plasma, saliva, sputum, bronchoalveolar lavage fluid, urine, lung epithelial cells, skin, or nasal polyps. In particular aspects, the sample taken from the patient is blood serum.

In some aspects, the airway dimensions at baseline (i.e. prior to administration of an IL-13 antagonist), for example, Wall Area % as determined using a CT scan of the lungs of subsegmental airways (WA%) can be used to predict treatment response (for example, improvements in airway resistant and/or $FEV_1$) in patients treated or candidates for treatment with an IL-13 antagonist (for example an anti-IL-13 antibody such as tralokinumab or lebrikizumab). The term "wall area" as used herein refers to the cross-sectional area of a bronchial tube wall (*e.g.* segmental and subsegmental bronchi in the upper lobes). Wall area percentage (WA%) is calculated as follows: 100*wall area/(wall area + lumen area). Tools to measure wall area and wall area percentage are well known in the art. *See, e.g.,* Gupta et al., J Allergy Clin Immunol. 133(3): 729-738 (2014); Gupta et al., Thorax. 65(9):775-81 (2010). In some aspects, airway dimensions are measured from Computed Tomography (CT) imaging data of the lungs. Such imaging data can be processed, for example, using commercially available software such as VIDA Apollo (*e.g.,* the Volumetric Information Display and Analysis (VIDA) Pulmonary Workstation, VIDA Diagnostics, Coralville, Iowa). In some aspects, WA% of subsegmental airways from CT scan data of the lungs can be used to determine, for example, whether to treat, to modify the treatment, or to monitor the treatment of a patient suffering from an IL-13-mediated disease, *e.g.*, asthma, COPD, emphysema, IPF, UC, or atopic dermatitis. In some aspects, WA% of subsegmental airways from CT scan data can be used alone or in combination with other biomarkers (e.g., periostin, DPP4, and /or clinical characteristics such as $FEV_1$ reversibility) to identify a patient population suffering from and IL-13-mediated disease, *e.g.,* asthma, COPD, emphysema, IPF, UC, or atopic dermatitis, that is responsive to anti-IL-13 therapeutic agents (e.g., tralokinumab or lebrikizumab). Accordingly, the methods provided in the present disclosure comprise evaluating WA% measured from CT scan imaging data of the lungs of a patient, and determining whether WA% of subsegmental airways is above or below a predetermined WA% threshold level, or it is above or below the WA% in one or more control CT scans, wherein patients suffering from an IL-13-mediated disease (*e.g.,* asthma, COPD, emphysema, IPF, UC, or atopic dermatitis) having a WA% value of subsegmental airways above a predetermined WA% threshold level or above the WA% in one or more control CT scans are treated with an IL-13 antagonist (for example an anti-IL-13 antibody such as tralokinumab or lebrikizumab). WA% can be used in the methods disclosed herein independently or in combination with periostin levels, DPP4 levels and /or clinical characteristics such as $FEV_1$ reversibility.

In some aspects, the predetermined WA% threshold level useful in the methods disclosed herein is about 40%, about 45%, about 50%, about 55%, about 60%, about 65%, about 70%, about 75%, about 80%, about 85%, or about 90%. In some aspects, the predetermined WA% threshold level useful in the methods disclosed herein is between about 60% and about 80%. In other aspects, the predetermined WA% threshold level useful in the methods disclosed herein is between about 65% and about 75%. In other aspects, the predetermined WA% threshold level useful in the methods disclosed herein is about 60%. In other aspects, the predetermined WA% threshold level useful in the methods disclosed herein is about 68%. In specific aspects, the predetermined WA% threshold level useful in the methods disclosed herein is 68%. In some aspects, the predetermined threshold WA% level useful in the methods disclosed herein is the mean WA% value of a population of patients. In other aspects, the predetermined threshold WA% level useful in the methods disclosed herein is the median WA% value of a population of patients. In some aspects, the patients have been treated with an IL-13 antagonist (e.g., tralokinumab or lebrikizumab). In some aspects, the patients have not been treated with an IL-13 antagonist (e.g., they have been treated with a non-IL-13 antagonist therapeutic agent, or not treated with any therapeutic agent).

In some aspects, WA% is measured using 3D airway analysis of CT scan data of lung scans using segmental bronchi. In other aspects, WA% is measured using 3D airway analysis of CT scan data of lung scans using subsegmental bronchi. In some aspects, the segmental or subsegmental bronchi are from the upper lobes. In some aspects, the segmental or subsegmental bronchi are from the entire lung. In some aspects, the segmented airways are right apical (RB1), right anterior (RB2), right posterior (RB3), left apicoposterior (LB1+2), LB3 (left anterior), or combinations thereof. In some aspects, the subsegmental airways are RB1a, RB1b, RB2a, RB2b, RB3a, RB3b, LB1, LB1a, LB1b, LB2, LB2a, LB2b, LB3a, LB3b, or combinations thereof. *See* Naidich, et al, Imaging of the Airways - Functional and Radiologic Correlations, 2005. In some aspects, airway parameters are calculated for each airway segment separately, and then averaged over segmental and/or subsegmental airways in each subject.

In some aspects, patients with WA% above the specified threshold (*e.g.,* WA% at least 60% at subsegmental level) display a statistically significant improvement in airway resistance. In some aspects, patients with WA% above the specified threshold (e.g., WA% at least 60% at subsegmental level) display a statistically significant improvement in pre-bronchodilator FEV1. In some aspects, WA% can be combined with other biomarkers obtained using 3D airway analysis of CT scan data for example lumen area (LA), wall area (WA), wall thickness area (WT), airway resistance, or combinations thereof.

In some aspects, in addition to the determination of the level of DPP4 (e.g., protein expression level or gene expression level), the method of the present disclosure can further comprise determining, submitting a sample taken from the patient for determination, or instructing a clinical laboratory to determine:

(i) the level of the patient's IgE levels,
(ii) the patient's eosinophil count,
(iii) the patient's Fraction of Exhaled Nitric Oxide ($FE_{NO}$),
(iv) the patient's Eosinophil/Lymphocyte and Eosinophil/Neutrophil (ELEN) index (see WO2012158954, which is herein incorporated by reference in its entirety),
(v) the patient's EOS index (see WO2012158954, which is herein incorporated by reference in its entirety),
(vi) the patient's wall area percentage (WA%) of subsegmental airways from CT scan data of the lungs, or
(vii) a combination of two or more thereof.

Accordingly, in certain aspects described above, the patient having an IL-13-mediated disease or disorder has been diagnosed with a pulmonary disease or disorder an inflammatory bowel disease or disorder or a chronic inflammatory skin disease or disorder, which, in a subset of differential diagnoses, can be IL-13-mediated. See, e.g., U.S. Pat. Appl. Publication 2012-0328606 incorporated herein by reference in its entirety. In certain aspects, the disease or disorder suspected of having IL-13-mediated pathology is asthma, idiopathic pulmonary fibrosis (IPF), chronic obstructive pulmonary disease (COPD), ulcerative colitis (UC), or atopic dermatitis.

In some aspects, in addition to the determination of the level of DPP4 (e.g., protein expression level or gene expression level), the methods disclosed herein can comprise determining, submitting a sample taken from the patient for determination, or instructing a clinical laboratory to determine the expression level or activity of isoforms 1, 2, 3, or 4 of human periostin, or combinations thereof. The use periostin as a biomarker for IL-13-mediated diseases has been disclosed, e.g., in Jia, et al., J Allergy Clin. Immunol 2012 130:647-654; Takayama, et al., J Allergy Clin Immunol 2006 118:98-104; and PCT Publ. No. WO 2012/083132, each herein incorporated by reference in their entirety.

The term "periostin" as used herein refers to the osteoblast specific factor protein (Uniprot: Q15063) encoded by the *POSTN* gene. Periostin is also known as osteoblast-specific factor 2 (OSF-2). Periostin functions as a ligand for alpha-V/beta-3 and alpha-V/beta-5 integrins to support adhesion and migration of epithelial cells. Periostin is a gla domain vitamin K dependent factor.

The term periostin also includes fragments, variants (e.g., isoforms produced by alternative splicing), and derivatives thereof (e.g., glycosylated or aglycosilated protein forms of the protein, or otherwise chemically modified forms of the protein). Seven isoforms produced by alternative splicing are known in the art: Isoform 1 (Uniprot: Q15063-1), also known as OSF-2OS, which is 836 amino acids long; Isoform 2 (Uniprot: Q15063-2), also known as OSF-2p1, which is 779 amino acids long; Isoform 3 (Uniprot: Q15063-3), which is 781 amino acids long; Isoform 4 (Uniprot: Q15063-4), which is 751 amino acids long; Isoform 5 (Uniprot: Q15063-5), which is 809 amino acids long; Isoform 6 (Uniprot: Q15063-6), which is 749 amino acids long; and Isoform 7 (Uniprot: Q15063-7), which is 721 amino acids long. Known periostin variants include those with any of the following sequence differences with respect to the canonical Isoform-1 sequence: I290F, D421V, T339I, or V814M.

In some aspects, the term periostin refers to the periostin gene, which includes genomic DNA, cDNA, mRNA, and fragments thereof. In some aspects, the term periostin also refers to oligonucleotides capable of specifically hybridizing to the periostin gene under stringent conditions. In some aspects, the oligonucleotides comprise nucleobases different from A, T, C, G, or U, for example, universal bases. See Takeshita et al. Biochem J. 294:271-278 (1993); Sasaki et al. Cancer 92:843-848 (2001); Blanchard et al. Mucosal Immunol. 1:289-296 (2008); Blanchard & Rothenberg, Immunol. Allergy Clin. North. Am. 29:141-148 (2009); Sidhu et al., Proc. Natl. Acad. Sci. USA 107:14170-14175 (2010); Kanemitsu et al., J. Allergy Clin. Immunol. 132:305-12 (2013), which are herein incorporated by reference in their entireties.

In other aspects, in addition to the determination of the level of DPP4 (e.g., protein expression level or gene expression level) and/or periostin (e.g., protein expression level or gene expression level), the methods disclosed herein can comprise determining, submitting a sample taken from the patient for determination, or instructing a clinical laboratory to determine a patient's blood eosinophil cell count, the level of the patient's IgE levels, pre- or post-bronchodilator FEV1 reversibility, the wall area percentage (WA%) of subsegmental airways from CT scan data of the lungs, or combinations thereof.

In other aspects, in addition to the determination of the level of DPP4 (e.g., protein expression level or gene expression level), the methods disclosed herein can comprise determining, submitting a sample taken from the patient for determination, or instructing a clinical laboratory to determine the expression level or activity of sCTLA-3 (soluble CTLA-3; also known as Cytotoxic T-Lymphocyte-Associated serine Esterase 3, granzyme A, or granzyme 1; Uniprot: P12544), sCD28 (soluble CD28; also known as cluster of differentiation 28 or Tp44; Uniprot: P10747), CCL5 (chemokine C-C motif ligand 5; also known as RANTES; Uniprot: P13501), CCL11 (C-C motif chemokine 11; also known as eosinophil chemotactic protein or eotaxin-1; Uniprot: P51671), CCL22 (C-C motif chemokine 22; Uniprot: O00626), or combinations thereof. These biomarkers have been disclosed in IL-13 mediated disease, e.g., in Lun et al., J. Clin. Immunol. 2007 27:430-437.

In some aspects, in addition to the determination of the level of DPP4, the methods disclosed herein can further comprising determining, submitting a sample taken from the patient for determination, or instructing a clinical laboratory to determine the expression level or activity of CCL26, FZD5, DOK1, CST2, ZNF436, C20orf100, NAGS, CST1, CDH13, HRH1, TMEM132B, NTRK1, SLCO2A1, IgE, FETUB, KRT31IKRT34, C6orf138, ATP5J, TUBAL3, JAM2, NOVA2, NOS2A, HS3ST4, GRM8, IL1R2, CTDSPL, CEP72, LOC199800, LYPD1, DISP1, NKX1-2, C4orf38, LOXL4, PRKD1, PAM124B, GPR44, HIGD1B, CLCA1, SEPT11, CYYR1, CD36, ALOX15, AADAC, ACTA1, ODC1, DKFZp434F142, ACHE, CSF3, LOC100132552, C12orf27, ZNF331, GK5, DUSP1IDUSP4, LRWD1, PGLYRP4, GUSBL2, CLGN, NR1I2, EST, LRRC37B, SAA4, SLC12A3, TMEM45A, FLJ37464, MUC5B, CXCL6, GLRB, DKFp686K01114, FOLR1, TSPAN6, AKR1C1, KIAA0232, PTP4A1, PCYT2, RHOV, PROS1, C11orf63, TCTN1, PIP5K1B, OSBPL6, NSUM7, GJB7, IRS2, or combinations thereof. These genes are part of the Th-2 signature as disclosed in Choi et al,, J. Immunol. 186(3):1861-9 (2011) and WO2009124090, both of which are herein incorporated by reference in their entireties.

In some aspects, in addition to the determination of the level of DPP4 (e.g., protein expression level or gene expression level), the methods disclosed herein can further comprising determining, submitting a sample taken from the patient for determination, or instructing a clinical laboratory to determine the expression level or activity of POSTN (SEQ ID NO:8), CSTI (SEQ ID NO:9), CCL26 (SEQ ID NO:10), CLCAI (SEQ ID NO:11), CST2 (SEQ ID NO:12), PRR4 (SEQ ID NO:13), SERPINB2 (SEQ ID NO:14), CEACAM5 (SEQ ID NO:15), iNOS (SEQ ID NO:16), SERPINB4 (SEQ ID NO:17), CST4 (SEQ ID NO:18), PRB4 (SEQ ID NO:19), TPSDI (SEQ ID NO:20), TPSGI (SEQ ID NO: 21), MFSD2 (SEQ ID NO:22), CPA3 (SEQ ID NO:23), GPR105 (SEQ ID NO:24), CDH26 (SEQ ID NO:25), GSN (SEQ ID NO:26), C2ORF32 (SEQ ID NO:27), TRACH2000196 (TMEM71) (SEQ ID NO:28), DNAJC12 (SEQ ID NO:29), RGS13 (SEQ ID NO: 30), SLC18A2 (SEQ ID NO: 31), SERPINBI0 (SEQ ID NO:32), SH3RF2 (SEQ ID NO:33), FCERIB (SEQ ID NO:34), RUNX2 (SEQ ID NO:35), PTGSI (SEQ ID NO:36), ALOX15 (SEQ ID NO:37), and combinations thereof.

Examples of POSTN (periostin) include a polypeptide comprising SEQ ID NO: 8 and other POSTN native sequence polypeptides, such as naturally occurring variants and native sequence polypeptides encoded by a nucleic acid sequence that can hybridize under stringent conditions to SEQ ID NOs:38 and/or 39. Also included are nucleic acids encoding such POSTN and fragments thereof, and their complementary sequences.

Examples of CST1 (cystatin-SN; Uniprot: P01037) include a polypeptide comprising SEQ ID NO:9 and other CSTI native sequence polypeptides, such as naturally occurring variants and native sequence polypeptides encoded by a nucleic acid sequence that can hybridize under stringent conditions to SEQ ID NO:40. Also included are nucleic acids encoding such CST1 and fragments thereof, and their complementary sequences.

Examples of CCL26 (chemokine (C-C motif) ligand 26; Uniprot: Q9Y258) include a polypeptide comprising SEQ ID NO:10 and other CCL26 native sequence polypeptides, such as naturally occurring variants and native sequence polypeptides encoded by a nucleic acid sequence that can hybridize under stringent conditions to SEQ ID NO:41. Also included are nucleic acids encoding such CCL26 and fragments thereof, and their complementary sequences.

Examples of CLCA1 (calcium-activated chloride channel regulator 1; Uniprot: A8K7I4) include a polypeptide comprising SEQ ID NO:11 and other CLCAI native sequence polypeptides, such as naturally occurring variants and native sequence polypeptides encoded by a nucleic acid sequence that can hybridize under stringent conditions to SEQ ID NO:42. Also included are nucleic acids encoding such CLCA1 and fragments thereof, and their complementary sequences.

Examples of CST2 (cystatin-SA; Uniprot: P09228) include a polypeptide comprising SEQ ID NO:12 and other CST native sequence polypeptides, such as naturally occurring variants and native sequence polypeptides encoded by a nucleic acid sequence that can hybridize under stringent conditions to SEQ ID NO:43. Also included are nucleic acids encoding such CST2 and fragments thereof, and their complementary sequences.

Examples of PRR4 (proline-rich protein 4; Uniprot: Q16378) include a polypeptide comprising SEQ ID NO:13 and other PRR4 native sequence polypeptides, such as naturally occurring variants and native sequence polypeptides encoded by a nucleic acid sequence that can hybridize under stringent conditions to SEQ ID NO:44. Also included are nucleic acids encoding such PRR4 and fragments thereof, and their complementary sequences.

Examples of SERPINB2 (plasminogen activator inhibitor-2 (placental PAI), also known as HsT1201, PAI, PAI-2, PAI2 or PLANH2; Uniprot: P05120) include a polypeptide comprising SEQ ID NO:14 and other SERPINB2 native sequence polypeptides, such as naturally occurring variants and native sequence polypeptides encoded by a nucleic acid sequence that can hybridize under stringent conditions to SEQ ID NO:45. Also included are nucleic acids encoding such SERPINB2 and fragments thereof, and their complementary sequences.

Examples of CEACAM5 (carcinoembryonic antigen-related cell adhesion molecule 5 (CEACAM5) also known as CD66e (cluster of differentiation 66); Uniprot: P06731) include a polypeptide comprising SEQ ID NO:15 and other CEACAM5 native sequence polypeptides, such as naturally occurring variants and native sequence polypeptides encoded by a nucleic acid sequence that can hybridize under stringent conditions to SEQ ID NO:46. Also included are nucleic acids encoding such CEACAM5 and fragments thereof, and their complementary sequences.

Examples of iNOS (inducible NOS, known as iNOS or NOS2) include a polypeptide comprising SEQ ID NO:16 and other iNOS native sequence polypeptides, such as naturally occurring variants and native sequence polypeptides encoded by a nucleic acid sequence that can hybridize under stringent conditions to SEQ ID NO:47. Also included are nucleic acids encoding such iNOS and fragments thereof, and their complementary sequences.

Examples of SERPINB4 (serpin peptidase inhibitor, clade B (ovalbumin), member 4, also known as LEUPIN, PI11, SCCA-2, SCCA1, or SCCA2; Uniprot: P48594) include a polypeptide comprising SEQ ID NO:17 and other SERPINB4 native sequence polypeptides, such as naturally occurring variants and native sequence polypeptides encoded by a nucleic acid sequence that can hybridize under stringent conditions to SEQ ID NOs :48 and/or 49. Also included are nucleic acids encoding such SERPINB4 and fragments thereof, and their complementary sequences.

Examples of CST4 (cystatin S; Uniprot: P01036) include a polypeptide comprising SEQ ID NO:18 and other CST4 native sequence polypeptides, such as naturally occurring variants and native sequence polypeptides encoded by a nucleic acid sequence that can hybridize under stringent conditions to SEQ ID NO:50. Also included are nucleic acids encoding such CST4 and fragments thereof, and their complementary sequences.

Examples of PRB4 (basic salivary proline-rich protein 4; Uniprot: P10163) include a polypeptide comprising SEQ ID NO:19 and other PRB4 native sequence polypeptides, such as naturally occurring variants and native sequence polypeptides encoded by a nucleic acid sequence that can hybridize under stringent conditions to SEQ ID NO:51. Also included are nucleic acids encoding such PRB4 and fragments thereof, and their complementary sequences.

Examples of TPSD1 (tryptase Delta 11, also known as delta-tryptase, mast cell MMCP-7 like protein, or HmMCP-3-like tryptase III; Uniprot: Q9BZJ3) include a polypeptide comprising SEQ ID NO:20 and other TPSDl native sequence polypeptides, such as naturally occurring variants and native sequence polypeptides encoded by a nucleic acid sequence that can hybridize under stringent conditions to a sequence selected from the group consisting of SEQ ID NO:52-58. Also included are nucleic acids encoding such TPSD1 and fragments thereof, and their complementary sequences.

Examples of TPSG1 (tryptase gamma 1, also known as TMT, tryptase gamma I, tryptase gamma II, serine protease 31, lung tryptase, mast cell protease II, mast cell tryptase, or skin tryptase; Uniprot: Q9NRR2) include a polypeptide comprising SEQ ID NO:21 and other TPSGl native sequence polypeptides, such as naturally occurring variants and native sequence polypeptides encoded by a nucleic acid sequence that can hybridize under stringent conditions a sequence selected from the group consisting of SEQ ID NO:59-62. Also included are nucleic acids encoding such TPSG1 and fragments thereof, and their complementary sequences.

Examples of MFSD2 (major facilitator superfamily domain containing 2A protein; Uniprot: Q8NA29) include a polypeptide comprising SEQ ID NO:22 and other MFSD2 native sequence polypeptides, such as naturally occurring variants and native sequence polypeptides encoded by a nucleic acid sequence that can hybridize under stringent conditions to SEQ ID NO:63. Also included are nucleic acids encoding such MFSD2 and fragments thereof, and their complementary sequences.

Examples of CPA3 (carboxypeptidase A3, also known as mast cell carboxypeptidase A, tissue carboxypeptidase A, or MC-CPA2; Uniprot: P15088) include a polypeptide comprising SEQ ID NO:23 and other CPA3 native sequence polypeptides, such as naturally occurring variants and native sequence polypeptides encoded by a nucleic acid sequence that can hybridize under stringent conditions to SEQ ID NO:64. Also included are nucleic acids encoding such CPA3 and fragments thereof, and their complementary sequences.

Examples of GPR105 (G-Protein coupled receptor 105, also known as G protein coupled receptor for UDP-Glucose, P2Y purinoceptor 14, BPR105, UDP-Glucose receptor, purinergic receptor P2Y G-Protein coupled 14, or P2RY14; Uniprot: Q15391) include a polypeptide comprising SEQ ID NO:24 and other GPRl05 native sequence polypeptides, such as naturally occurring variants and native sequence polypeptides encoded by a nucleic acid sequence that can hybridize under stringent conditions to SEQ ID NO:65. Also included are nucleic acids encoding such GPR105 and fragments thereof, and their complementary sequences.

Examples of CDH26 (cadherin 26, also known as VR20; Uniprot: Q8IXH8) include a polypeptide comprising SEQ ID NO:25 and other CDH26 native sequence polypeptides, such as naturally occurring variants and native sequence polypeptides encoded by a nucleic acid sequence that can hybridize under stringent conditions to SEQ ID NO:66. Also included are nucleic acids encoding such CDH26 and fragments thereof, and their complementary sequences.

Examples of GSN (gelsolin, also known as brevin, ADF, AGEL, or Actin-Depolymerizing Factor 2; Uniprot: P06396) include a polypeptide comprising SEQ ID NO:26 and other GSN native sequence polypeptides, such as naturally occurring variants and native sequence polypeptides encoded by a nucleic acid sequence that can hybridize under stringent conditions to SEQ ID NO: 67. Also included are nucleic acids encoding such GSN and fragments thereof, and their complementary sequences.

Examples of C2ORF32 (cannabinoid receptor interacting protein 11, also known as CRIP-1; Uniprot: Q96F85) include a polypeptide comprising SEQ ID NO:27 and other C2ORF32 native sequence polypeptides, such as naturally occurring variants and native sequence polypeptides encoded by a nucleic acid sequence that can hybridize under stringent conditions to SEQ ID NO:68. Also included are nucleic acids encoding such C2ORF32 and fragments thereof, and their complementary sequences.

Examples of TRACH2000196 (transmembrane protein 711 or TMEM71; Uniprot: Q6P5X7) include a polypeptide comprising SEQ ID NO:28 and other TRACH2000196 (TMEM71) native sequence polypeptides, such as naturally occurring variants and native sequence polypeptides encoded by a nucleic acid sequence that can hybridize under stringent conditions to SEQ ID NO: 69. Also included are nucleic acids encoding such TRACH2000196 and fragments thereof, and their complementary sequences.

Examples of DNAJC12 (DnaJ (Hsp40) homolog, subfamily C, member 121, also known as JDP1 or J Domain protein 1; Uniprot: Q9UKB3) include a polypeptide comprising SEQ ID NO:29 and other DNAJC12 native sequence polypeptides, such as naturally occurring variants and native sequence polypeptides encoded by a nucleic acid sequence that can hybridize under stringent conditions to SEQ ID NO: 70. Also included are nucleic acids encoding such DNAJC12 and fragments thereof, and their complementary sequences.

Examples of RGS 13 (regulator of G-protein signaling 13; Uniprot: 014921) include a polypeptide comprising SEQ ID NO:30 and other RGS 13 native sequence polypeptides, such as naturally occurring variants and native sequence polypeptides encoded by a nucleic acid sequence that can hybridize under stringent conditions to SEQ ID NO: 71. Also included are nucleic acids encoding such RGS 13 and fragments thereof, and their complementary sequences.

Examples of SLC18A2 (vesicular monoamine transporter 2 (VMAT2) also known as solute carrier family 18 member 2 (SLC18A2); Uniprot: Q05940) include a polypeptide comprising SEQ ID NO:31 and other SLCl8 A2 native sequence polypeptides, such as naturally occurring variants and native sequence polypeptides encoded by a nucleic acid sequence that can hybridize under stringent conditions to SEQ ID NO: 72. Also included are nucleic acids encoding such SLC18A2 and fragments thereof, and their complementary sequences.

Examples of SERPINB10 (serpin peptidase inhibitor, clade B (ovalbumin), member 10; Uniprot: P48595) include a polypeptide comprising SEQ ID NO:32 and other SERPINBl0 native sequence polypeptides, such as naturally occurring variants and native sequence polypeptides encoded by a nucleic acid sequence that can hybridize under stringent conditions to SEQ ID NO: 73. Also included are nucleic acids encoding such SERPINB10 and fragments thereof, and their complementary sequences.

Examples of SH3RF2 (SH3 ring finger 2 protein) include a polypeptide comprising SEQ ID NO:33 and other SH3RF2 native sequence polypeptides, such as naturally occurring variants and native sequence polypeptides encoded by a nucleic acid sequence that can hybridize under stringent conditions to SEQ ID NO: 74. Also included are nucleic acids encoding such SH3RF2 and fragments thereof, and their complementary sequences.

Examples of FCER1B (high affinity immunoglobulin epsilon receptor subunit beta or MS4A2; Uniprot: Q01362) include a polypeptide comprising SEQ ID NO:34 and other FCERlB native sequence polypeptides, such as naturally occurring variants and native sequence polypeptides encoded by a nucleic acid sequence that can hybridize under stringent conditions to SEQ ID NO:75. Also included are nucleic acids encoding such FCER1B and fragments thereof, and their complementary sequences.

Examples of RUNX2 (runt-related transcription factor 2; also known as core-binding factor subunit alpha-1 or CBF-alpha-1; Uniprot: Q13950) include a polypeptide comprising SEQ ID NO:35 and other RUNX2 native sequence polypeptides, such as naturally occurring variants and native sequence polypeptides encoded by a nucleic acid sequence that can hybridize under stringent conditions to SEQ ID NO: 76. Also included are nucleic acids encoding such RUNX2 and fragments thereof, and their complementary sequences.

Examples of PTGSl (cyclooxygenase-1, COX-1, also known as prostaglandin G/H synthase 1, prostaglandin-endoperoxide synthase 1 or prostaglandin H2 synthase 1; Uniprot: P23219) include a polypeptide comprising SEQ ID NO:36 and other PTGSl native sequence polypeptides, such as naturally occurring variants and native sequence polypeptides encoded by a nucleic acid sequence that can hybridize under stringent conditions to SEQ ID NO: 77. Also included are nucleic acids encoding such PTGS1 and fragments thereof, and their complementary sequences.

Examples of ALOX15 (arachidonate 15-lipoxygenase; Uniprot: P16050) include a polypeptide comprising SEQ ID NO:37 and other ALOX 15 native sequence polypeptides, such as naturally occurring variants and native sequence polypeptides encoded by a nucleic acid sequence that can hybridize under stringent conditions to SEQ ID NO:78. Also included are nucleic acids encoding such ALOX15 and fragments thereof, and their complementary sequences.

In some aspects, the IL-13 antagonist is administered at a fixed dose. In some specific aspects, the IL-antagonist is tralokinumab and the fixed dose is about 300 mg/dose. In some aspects, the IL-13 antagonist, e.g., an anti-IL-13 antibody such as tralokinumab, is administered in two or more doses. In some aspects, the IL-13 antagonist, e.g., an anti-IL-13 antibody such as tralokinumab, is administered weekly, biweekly or monthly. In some aspects, the IL-13 antagonist, e.g., an anti-IL-13 antibody such as tralokinumab, is administered biweekly. In some aspects, the IL-13 antagonist, e.g., an anti-IL-13 antibody such as tralokinumab, is administered intravenously, intramuscularly, subcutaneously, or a combination thereof.

In some aspects, the one or more control samples are obtained from normal healthy individuals; patients with a non-IL-13-mediated subset of asthma; asthma patients naive for corticosteroid treatment; asthma patients treated with corticosteroids; a pre-determined standard amount of isolated DPP4 (e.g., protein expression level or gene expression level); or a combination thereof.

In some aspects, the administration of the IL-13 antagonist, e.g., an anti-IL-13 antibody such as tralokinumab, to the patient results in:

(a) AER (Acute Exacerbation Rate) reduction;
(b) $FEV_1$ (Forced Expiratory Volume in one second) increase;
(c) improved ACQ-6 (Asthma Control Questionnaire, 6-item version) results;
(d) improved AQLQ (Asthma Quality of Life Questionnaire) results; or,

(e) a combination thereof.

In some aspects the AER reduction after administration of an IL-13 antagonist (e.g., biweekly administration of a 300 mg/dose fixed dose of tralokinumab) is at least about 5%, at least about 10%, at least about 15%, at least about 20%, at least about 25%, at least about 30%, at least about 35%, at least about 40%, at least about 45%, at least about 50%, at least about 55%, or at least 60% compared to the AER observed in a population of patients treated with a placebo. In some specific aspects the AER reduction after administration of an IL-13 antagonist (e.g., biweekly administration of a 300 mg/dose fixed dose of tralokinumab) is about 28% compared to the mean AER observed in a population of patients treated with a placebo.

In some aspects the $FEV_1$ increase after administration of an IL-13 antagonist (e.g., biweekly administration of a 300 mg/dose fixed dose of tralokinumab) is at least about 3%, at least about 5%, at least about 7%, at least about 9%, at least about 11%, at least about 13%, at least about 15%, least about 17%, or at least about 19% compared to the $FEV_1$ observed in a population of patients treated with a placebo. In some aspects the $FEV_1$ increase after administration of an IL-13 antagonist (e.g., biweekly administration of a 300 mg/dose fixed dose of tralokinumab) is about 10% compared to the mean $FEV_1$ observed in a population of patients treated with a placebo.

In some aspects the ACQ-6 change after administration of an IL-13 antagonist (e.g., biweekly administration of a 300 mg/dose fixed dose of tralokinumab) is about -0.5 compared to the mean ACQ-6 observed in a population of patients treated with a placebo. In some aspects the AQLQ change after administration of an IL-13 antagonist (e.g., biweekly administration of a 300 mg/dose fixed dose of tralokinumab) is about -0.5 compared to the mean AQLQ observed in a population of patients treated with a placebo.

In certain aspects this disclosure provides a method of identifying a patient as a candidate for treatment with an IL-13 antagonist (e.g., anti-IL13 antibody including tralokinumab, or an antigen-binding fragment thereof, or lebrikizumab, or an antigen-binding fragment thereof) comprising measuring the level of DPP4 (dipeptidyl peptidase-4) in a sample taken from the patient, wherein a level of DPP4 above a predetermined DPP4 threshold level, or above the DPP4 level in one or more control samples identifies the patient as a candidate for treatment with the IL-13 antagonist.

In some aspects, the methods of identifying a patient as a candidate for treatment with an IL-13 antagonist (e.g., anti-IL13 antibody including tralokinumab, or an antigen-binding fragment thereof, or lebrikizumab, or an antigen-binding fragment thereof) further comprise measuring one or more of periostin, eosinophil cell count, IgE and FEV1 reversibility, wherein a level of DPP4 above a predetermined DPP4 threshold level, or above the DPP4 level in one or more control samples and one or more of the following: (i) high periostin ($\geq$ median serum periostin or about 23 ng/mL), (ii) high eosinophil cell count (blood eosinophil count $\geq$ 300 cells/$\mu$L), (iii) high Th2 (high Th2 defined as IgE > 100 IU/mL and blood eosinophils $\geq 0.14 \times 109$/L), (iv) FEV1 reversibility to a short-acting $\beta 2$ agonist $\geq$ 12%, or (v) patient's wall area percentage (WA%) of subsegmental airways from a CT scan of the lungs above about 68% identifies the patient as a candidate for treatment with the IL-13 antagonist.

In certain aspects, the patient identified as a candidate for treatment with the IL-13 antagonist (e.g., anti-IL13 antibody including tralokinumab, or an antigen-binding fragment thereof, or lebrikizumab, or an antigen-binding fragment thereof) has asthma, IPF, COPD, chronic rhinosinusitis, allergic rhinitis, or atopic dermatitis. In certain aspects, the patient identified as a candidate for treatment with the IL-13 antagonist has allergic asthma, atopic asthma, corticosteroid naive asthma, chronic asthma, corticosteroid resistant asthma, corticosteroid refractory asthma, asthma due to smoking, or asthma uncontrolled on corticosteroids.

In some aspects, the predetermined DPP4 threshold level (e.g., protein expression level or gene expression level) in a serum sample used to identify the patient as a candidate for treatment with an IL-13 antagonist (e.g., anti-IL13 antibody including tralokinumab, or an antigen-binding fragment thereof, or lebrikizumab, or an antigen-binding fragment thereof) is at least about 250 ng/ml, at least about 350 ng/mL, at least about 365 ng/mL, at least about 375 ng/mL, at least about 400 ng/mL, at least about 450 ng/mL, at least about 500 ng/mL, at least 550 ng/mL, or at least about 600 ng/mL, as measured in serum using an ELISA (e.g. a QUANTIKINE® assay).

In certain aspects the one or more control samples used to identify the patient as a candidate for treatment with a IL-13 antagonist are obtained from normal healthy individuals; patients with a non-IL-13-mediated subset of asthma; asthma patients naive for corticosteroid treatment; asthma patients treated with corticosteroids; a pre-determined standard amount of isolated DPP4; or a combination thereof; and can comprise one or more of whole blood, serum, plasma, saliva, sputum, bronchoalveolar lavage fluid, lung epithelial cells, urine, or a combination thereof.

In certain aspects this disclosure provides a method of diagnosing an IL-13 mediated disease or disorder in a patient comprising measuring the level of DPP4 (dipeptidyl peptidase-4) in a sample taken from the patient, wherein the patient is diagnosed with the IL-13 mediated disease or disorder if the level of DPP4 is above a predetermined DPP4 threshold level, or above the DPP4 level in one or more control samples.

In addition this disclosure further provides methods of diagnosing an IL-13 mediated disease or disorder in a patient comprising measuring the level of DPP4 (dipeptidyl peptidase-4) in a sample taken from the patient, and one or more of periostin, eosinophil cell count, IgE, FEV1 reversibility or wall area percentage (WA%) of subsegmental airways from a CT scan of the lungs, wherein the patient is diagnosed with the IL-13 mediated disease or disorder if the level of DPP4 is above a predetermined DPP4 threshold level, or above the DPP4 level in one or more control samples and the patient has one or more of the following: (i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ 0.14 × 109/L), (iv) FEV1 reversibility to a short-acting β2 agonist ≥ 12%, or (v) patient's wall area percentage (WA%) of subsegmental airways from a CT scan of the lungs is above about 68%.

In certain aspects, the IL-13 mediated disease or disorder diagnosed using the methods disclosed herein is asthma, IPF, COPD, chronic rhinosinusitis, allergic rhinitis, allergic asthma, atopic asthma, corticosteroid naive asthma, chronic asthma, corticosteroid resistant asthma, corticosteroid refractory asthma, asthma due to smoking, asthma uncontrolled on corticosteroids, or atopic dermatitis.

In some aspects, the predetermined DPP4 threshold level in a sample used to diagnose the patient with an IL-13 mediated disease or disorder in a patient is at least about 250 ng/ml, at least about 350 ng/mL, at least about 365 ng/mL, at least about 375 ng/mL, at least about 400 ng/mL, at least about 450 ng/mL, at least about 500 ng/mL, at least 550 ng/mL, or at least about 600 ng/mL, as measured in serum using an ELISA (e.g. a QUANTIKINE® assay).

In certain aspects the one or more control samples used to diagnose the patient as having an IL-13 mediated disease or disorder are obtained from normal healthy individuals; patients with a non-IL-13-mediated subset of asthma; asthma patients naive for corticosteroid treatment; asthma patients treated with corticosteroids; a pre-determined standard amount of isolated DPP4; or a combination thereof; and can comprise one or more of whole blood, serum, plasma, saliva, sputum, bronchoalveolar lavage fluid, lung epithelial cells, urine, skin or a combination thereof.

IV. DPP4 Detection Assays and Kits

This disclosure also provides kits for detecting DPP4 (e.g., protein expression level or gene expression level), for example, through an immunoassay method. Such kits can comprise containers, each with one or more of the various reagents (e.g., in concentrated form) utilized in the method, including, for example, one or more anti-DPP4 antibodies. One or more anti-DPP4 antibodies, e.g., capture antibodies, can be provided already attached to a solid support. One or more antibodies, e.g., detection antibodies, can be provided already conjugated to a detectable label, e.g., biotin or a ruthenium chelate. The kit can also provide reagents for coupling a detectable label to an antibody (as well as the label itself), buffers, and/or reagents and instrumentation to support the practice of the assays provided herein. In certain aspects, a labeled secondary antibody can be provided that binds to the detection antibody. A kit provided according to this disclosure can further comprise suitable containers, plates, and any other reagents or materials necessary to practice the assays provided herein.

In some aspects, a kit comprises one or more nucleic acid probes (e.g., oligonucleotides comprising naturally occurring and/or chemically modified nucleotide units) capable of hybridizing a subsequence of the *DPP4* gene sequence (SEQ ID NO: 7) under high stringency conditions. In some aspects, one or more nucleic acid probes (e.g., oligonucleotides comprising naturally occurring and/or chemically modified nucleotide units) capable of hybridizing a subsequence of the *DPP4* gene sequence (SEQ ID NO: 7) under high stringency conditions are attached to a microarray chip.

A kit provided according to this disclosure can also comprise brochures or instructions describing the process. For DPP4 detection immunoassays, and in particular sandwich immunoassays, e.g., an ELISA assay or an ECL assay, the sandwich immunoassay process comprises a first anti-DPP4 "capture" antibody or antigen-binding fragment thereof attached to a solid support, and a second anti-DPP4 "detection" antibody or antigen binding fragment thereof. The immunoassay can be performed by methods provided herein or methods well known and understood by those of ordinary skill in the art. In one aspect, the immunoassay comprises attaching a capture antibody or fragment thereof to a solid support; applying the test sample or a control sample, allowing DPP4, if present in the sample, to bind to the capture antibody or fragment thereof; applying the detection antibody or fragment thereof, which can bind to DPP4 already bound to the capture antibody or fragment thereof; and measuring the amount of detection antibody or fragment thereof bound to DPP4. In certain aspects, the assay can further include washing steps, blocking steps and incubation steps.

Test kits can include instructions for carrying out one or more DPP4 detection assays, e.g., immunoassays or nucleic acid detection assays. Instructions included in the kits can be affixed to packaging material or can be included as a package insert. While the instructions are typically written or printed materials they are not limited to such. Any medium capable of storing such instructions and communicating them to an end user is contemplated. Such media include, but are not limited to, electronic storage media (e.g., magnetic discs, tapes, cartridges, chips), optical media (e.g., CD ROM), and the like. As used herein, the term "instructions" can include the address of an internet site that provides the instructions.

V. Computer Methods and Software

The methods disclosed herein can comprise collecting or otherwise obtaining a biological sample and performing an analytical method to detect and measure DPP4 levels (e.g., protein expression levels or gene expression levels) alone or in combination with other biomarkers (e.g., a panel a genes used to derive a gene signature, such as a Th-2 signature). Biomarkers that can be combined with DPP4 include isoforms 1, 2, 3, or 4 of human periostin, sCTLA-3, sCD28, CCL5, CCL11, CCL22, CCL26, FZD5, DOK1, CST2, ZNF436, C20orf100, NAGS, CST1, CDH13, HRH1, TMEM132B, NTRK1, SLCO2A1, IgE, FETUB, KRT31IKRT34, C6orf138, ATP5J, TUBAL3, JAM2, NOVA2, NOS2A, HS3ST4, GRM8, IL1R2, CTDSPL, CEP72, LOC199800, LYPD1, DISP1, NKX1-2, C4orf38, LOXL4, PRKD1, PAM124B, GPR44, HIGD1B, CLCA1, SEPT11, CYYR1, CD36, ALOX15, AADAC, ACTA1, ODC1, DKFZp434F142, ACHE, CSF3, LOC100132552, C12orf27, ZNF331, GK5, DUSP1IDUSP4, LRWD1, PGLYRP4, GUSBL2, CLGN, NR1I2, EST, LRRC37B, SAA4, SLC12A3, TMEM45A, FLJ37464, MUC5B, CXCL6, GLRB, DKFp686K01114, FOLR1, TSPAN6, AKR1C1, KIAA0232, PTP4A1, PCYT2, RHOV, PROS1, C11orf63, TCTN1, PIP5K1B, OSBPL6, NSUM7, GJB7, IRS2, or combinations thereof. See Lun et al., J. Clin. Immunol. 27:430-437 (2007), Choi et al,, J. Immunol. 186(3):1861-9 (2011), and WO2009124090A1, which are herein incorporated by reference in their entireties. Standard names, aliases, etc. of proteins and genes designated by identifiers used throughout this application (e.g., PIP5K1B) can be identified, for example, via Genecards (www.genecards.org) or Uniprot (www.uniprot.org).

Biomarkers that can be combined with DPP4 include POSTN (SEQ ID NO:8), CSTl (SEQ ID NO:9), CCL26 (SEQ ID NO:10), CLCAl (SEQ ID NO:11), CST2 (SEQ ID NO:12), PRR4 (SEQ ID NO:13), SERPINB2 (SEQ ID NO:14), CEACAM5 (SEQ ID NO:15), iNOS (SEQ ID NO:16), SERPINB4 (SEQ ID NO:17), CST4 (SEQ ID NO:18), PRB4 (SEQ ID NO:19), TPSDl (SEQ ID NO:20), TPSGl (SEQ ID NO: 21), MFSD2 (SEQ ID NO:22), CPA3 (SEQ ID NO:23), GPR105 (SEQ ID NO:24), CDH26 (SEQ ID NO:25), GSN (SEQ ID NO:26), C2ORF32 (SEQ ID NO:27), TRACH2000196 (TMEM71) (SEQ ID NO:28), DNAJC12 (SEQ ID NO:29), RGS13 (SEQ ID NO: 30), SLC18A2 (SEQ ID NO: 31), SERPINBl0 (SEQ ID NO:32), SH3RF2 (SEQ ID NO:33), FCERlB (SEQ ID NO:34), RUNX2 (SEQ ID NO:35), PTGSl (SEQ ID NO:36), ALOX15 (SEQ ID NO:37), and combinations thereof.

DPP4 levels (e.g., protein expression levels or gene expression levels) or normalized scores derived from measured DPP4 levels can be used alone (e.g., for treatment, diagnostic, prognostic, or monitoring purposes), or in combination with levels or normalized scores derived from other biomarkers (e.g., a panel a genes used to derive a gene signature, such as a Th-2 signature). These scores can also be combined with scores corresponding, for example, to (i) the level of the patient's IgE levels, (ii) the patient's eosinophil count, (iii) the patient's Fraction of Exhaled Nitric Oxide ($FE_{NO}$), (iv) the patient's Eosinophil/Lymphocyte and Eosinophil/Neutrophil (ELEN) index, (v) the patient's EOS index, (vi) the patient's IgE levels, (vii), pre- or post-bronchodilator FEV1, FVC measurements or reversibility, (viii) wall area percentage (WA%) of subsegmental airways from CT scan of the lungs, or (ix) a combination of two or more thereof, to yield a diagnostic score. In this approach, the diagnostic score may be a single number determined from the sum of all the marker calculations that is compared to a preset DPP4 threshold value that is an indication of the presence or absence of disease. Or the diagnostic score may be a series of bars that each represent a biomarker value and the pattern of the responses may be compared to a pre-set pattern for determination of the presence or absence of disease.

At least some aspects of the methods described herein, due to the complexity of the calculations involved, a method comprising the use of DPP4 as a biomarker can be implemented with the use of a computer. In some aspects, the computer system comprises hardware elements that are electrically coupled via bus, including a processor, input device, output device, storage device, computer-readable storage media reader, communications system, processing acceleration (e.g., DSP or special-purpose processors), and memory. The computer-readable storage media reader can be further coupled to computer-readable storage media, the combination comprehensively representing remote, local, fixed and/or removable storage devices plus storage media, memory, etc. for temporarily and/or more permanently containing computer-readable information, which can include storage device, memory and/or any other such accessible system resource.

A single architecture might be utilized to implement one or more servers that can be further configured in accordance with currently desirable protocols, protocol variations, extensions, etc. However, it will be apparent to those skilled in the art that embodiments may well be utilized in accordance with more specific application requirements. Customized hardware might also be utilized and/or particular elements might be implemented in hardware, software or both. Further, while connection to other computing devices such as network input/output devices (not shown) may be employed, it is to be understood that wired, wireless, modem, and/or other connection or connections to other computing devices might also be utilized.

In one aspect, the system further comprises one or more devices for providing input data to the one or more processors. The system further comprises a memory for storing a data set of ranked data elements. In another aspect, the device for providing input data comprises a detector for detecting the characteristic of the data element, e.g., such as a fluorescent plate reader, mass spectrometer, or gene chip reader.

The system additionally may comprise a database management system. User requests or queries can be formatted in an appropriate language understood by the database management system that processes the query to extract the relevant information from the database of training sets. The system may be connectable to a network to which a network server and one or more clients are connected. The network may be a local area network (LAN) or a wide area network (WAN), as is known in the art. Preferably, the server includes the hardware necessary for running computer program products (e.g., software) to access database data for processing user requests. The system can be in communication with an input device for providing data regarding data elements to the system (e.g., expression values). In one aspect, the input device can include a gene expression profiling system including, e.g., a mass spectrometer, gene chip or array reader, and the like.

Some aspects described herein can be implemented so as to include a computer program product. A computer program product may include a computer readable medium having computer readable program code embodied in the medium for causing an application program to execute on a computer with a database. As used herein, a "computer program product" refers to an organized set of instructions in the form of natural or programming language statements that are contained on a physical media of any nature (e.g., written, electronic, magnetic, optical or otherwise) and that may be used with a computer or other automated data processing system. Such programming language statements, when executed by a computer or data processing system, cause the computer or data processing system to act in accordance with the particular content of the statements.

Computer program products include without limitation: programs in source and object code and/or test or data libraries embedded in a computer readable medium. Furthermore, the computer program product that enables a computer system or data processing equipment device to act in pre-selected ways may be provided in a number of forms, including, but not limited to, original source code, assembly code, object code, machine language, encrypted or compressed versions of the foregoing and any and all equivalents.

In one aspect, a computer program product is provided to implemented the treatment, diagnostic, prognostic, or monitoring methods disclosed herein, for example, to determine whether to administer an IL-13 antagonist (e.g., an anti-IL-13 antibody such as tralokinumab) to a patient in need thereof if the level of DPP4 in a sample taken from the patient is above a predetermined DPP4 threshold level.

The computer program product includes a computer readable medium embodying program code executable by a processor of a computing device or system, the program code comprising:

(a) code that retrieves data attributed to a biological sample from a subject, wherein the data comprises DPP4 level values (or data otherwise derived from these level values) alone or combination with values corresponding to other biomarkers in the biological sample (e.g., a panel a genes used to derive a gene signature, such as a Th-2 signature or periostin). These values can also be combined with values corresponding, for example, to (i) the level of the patient's IgE levels, (ii) the patient's eosinophil count, (iii) the patient's Fraction of Exhaled Nitric Oxide ($FE_{NO}$), (iv) the patient's Eosinophil/Lymphocyte and Eosinophil/Neutrophil (ELEN) index, (v) the patient's EOS index, (vi) wall area percentage (WA%) of subsegmental airways from CT scan data of the lungs, (vii) the patient's IgE levels, (viii), pre- or post-bronchodilator FEV1, FVC measurements or reversibility, or (ix) a combination of two or more thereof; and,

(b) code that executes a classification method that indicates, e.g., whether to administer an IL-13 antagonist to a patient in need thereof.

While various aspects have been described as methods or apparatuses, it should be understood that aspects can be implemented through code coupled with a computer, e.g., code resident on a computer or accessible by the computer. For example, software and databases could be utilized to implement many of the methods discussed above. Thus, in addition to aspects accomplished by hardware, it is also noted that these aspects can be accomplished through the use of an article of manufacture comprised of a computer usable medium having a computer readable program code embodied therein, which causes the enablement of the functions disclosed in this description. Therefore, it is desired that aspects also be considered protected by this patent in their program code means as well.

Furthermore, some aspects can be code stored in a computer-readable memory of virtually any kind including, without limitation, RAM, ROM, magnetic media, optical media, or magneto-optical media. Even more generally, some aspects could be implemented in software, or in hardware, or any combination thereof including, but not limited to, software running on a general purpose processor, microcode, PLAs, or ASICs.

It is also envisioned that some aspects could be accomplished as computer signals embodied in a carrier wave, as well as signals (e.g., electrical and optical) propagated through a transmission medium. Thus, the various types of information discussed above could be formatted in a structure, such as a data structure, and transmitted as an electrical signal through a transmission medium or stored on a computer readable medium.

V. Embodiments

Embodiments are designated according to an "En" schema, where E means "embodiment" and n is the embodiment ordinal number.

E1. A method of treating a patient having an interleukin-13 (IL-13)-mediated disease or disorder, comprising administering an IL-13 antagonist to the patient if the level of DPP4 (dipeptidyl peptidase-4) in a sample taken from the patient is above a predetermined DPP4 threshold level, or is above the DPP4 level in one or more control samples.

E2. A method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents (i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ $0.14\times10^9$/L), (iv) FEV1 reversibility to a short-acting β2 agonist ≥ 12%, (v) wall area percentage (WA%) of subsegmental airways from a CT scan of the lungs ≥ 68%, or (vi) combinations thereof.

E3. A method of treating a patient having an interleukin-13 (IL-13)-mediated disease or disorder, comprising administering an IL-13 antagonist to the patient if (a) the level of DPP4 in a sample taken from the patient is above a predetermined DPP4 threshold level, or is above the DPP4 level in one or more control samples, and (b) the patient presents a level of at least one additional biomarker in a sample taken from the patient which is above a predetermined biomarker threshold level, or is above the biomarker level in one or more control samples, wherein said additional biomarker is selection from the group consisting of POSTN (SEQ ID NO:8), CSTI (SEQ ID NO:9), CCL26 (SEQ ID NO:10), CLCAI (SEQ ID NO:11), CST2 (SEQ ID NO:12), PRR4 (SEQ ID NO:13), SERPINB2 (SEQ ID NO:14), CEACAM5 (SEQ ID NO:15), iNOS (SEQ ID NO:16), SERPINB4 (SEQ ID NO:17), CST4 (SEQ ID NO:18), PRB4 (SEQ ID NO:19), TPSDI (SEQ ID NO:20), TPSGI (SEQ ID NO: 21), MFSD2 (SEQ ID NO:22), CPA3 (SEQ ID NO:23), GPR105 (SEQ ID NO:24), CDH26 (SEQ ID NO:25), GSN (SEQ ID NO:26), C2ORF32 (SEQ ID NO:27), TRACH2000196 (TMEM71) (SEQ ID NO:28), DNAJC12 (SEQ ID NO:29), RGS13 (SEQ ID NO: 30), SLC18A2 (SEQ ID NO: 31), SERPINBl0 (SEQ ID NO:32), SH3RF2 (SEQ ID NO:33), FCERIB (SEQ ID NO:34), RUNX2 (SEQ ID NO:35), PTGSI (SEQ ID NO:36), ALOX15 (SEQ ID NO:37), and combinations thereof.

E4. The method according to embodiments E1 to E3, wherein a sample is obtained from the patient and is submitted for measurement of the level of DPP4 in the sample.

E5. The method according to embodiments E1 to E4, wherein the patient's DPP4 level is measured in an immunoassay.

E6. The method according to embodiment E5, wherein the immunoassay employs one or more anti-DPP4 antibodies or antigen binding fragments thereof which recognize human DDP4.

E7. A method of treating a patient having an IL-13-mediated disease or disorder comprising (a) submitting a sample taken from the patient for measurement of the DPP4 level in the sample, wherein the patient's DPP4 level is measured with one or more anti-DPP4 antibodies or antigen binding fragments thereof which recognize human DPP4; and, (b) administering an IL-13 antagonist to the patient if the patient's DPP4 level in the sample is above a predetermined DPP4 threshold level, or is above the DPP4 level in one or more control samples.

E8. A method of treating a patient having an IL-13-mediated disease or disorder comprising (a) measuring the DPP4 level in a sample obtained from a patient having an IL-13-mediated disease or disorder, wherein the patient's DPP4 level in the sample is measured with one or more anti-DPP4 antibodies or antigen binding fragments thereof which recognize human DPP4; (b) determining whether the patient's DPP4 level in the sample is above a predetermined DPP4 threshold level, or is above the DPP4 level in one or more control samples; and, (c) advising a healthcare provider to administer an IL-13 antagonist to the patient if the patient's DPP4 level is above a predetermined DPP4 threshold level, or is above the DPP4 level in one or more control samples.

E9. The method according to any one of embodiments E1 to E8, wherein the IL-13-mediated disease or disorder is a pulmonary disease or disorder, an inflammatory bowel disease or disorder, or a chronic inflammatory skin disease or disorder.

E10. The method according to embodiment E9, wherein the pulmonary disease or disorder is asthma or allergic rhinitis.

E11. A method of treating a patient diagnosed with a pulmonary disease or disorder or a chronic inflammatory skin disease or disorder comprising administering an IL-13 antagonist to the patient if the DPP4 level in a sample taken from the patient is above a predetermined DPP4 threshold level, or is above the DPP4 level in one or more control samples.

E12. The method according to embodiment E11, wherein the patient's DPP4 level is measured in an immunoassay employing one or more anti-DPP4 antibodies or antigen binding fragments thereof which recognize human DPP4.

E13. A method of treating a patient diagnosed with a pulmonary disease or disorder or a chronic inflammatory skin disease or disorder comprising (a) submitting a sample taken from the patient for measurement of the DPP4 level in the sample, wherein the patient's DPP4 level is measured with one or more anti-DPP4 antibodies or antigen binding fragments thereof which recognize human DPP4; and (b) administering an IL-13 antagonist to a patient if the patient's DPP4 level in the sample is above a predetermined DPP4 threshold level, or is above the DPP4 level in one or more control samples.

E14. A method of determining whether to treat a patient diagnosed with a pulmonary disease or disorder or a chronic inflammatory skin disease or disorder with an IL-13 antagonist therapeutic regimen comprising (a) measuring, or instructing a clinical laboratory to measure the DPP4 level in a sample obtained from a patient diagnosed with a pulmonary disease or disorder or a chronic inflammatory skin disease or disorder, wherein the patient's DPP4 level is measured with one or more anti-DPP4 antibodies or antigen binding fragments thereof which recognize human DPP4; and (b) treating, or instructing a healthcare provider to treat, the patient with an IL-13 antagonist therapeutic regimen if the patient's DPP4 level in the sample is above a predetermined DPP4 threshold level, or is above the DPP4 level in one or more control samples.

E15. A method of selecting a patient diagnosed with a pulmonary disease or disorder or a chronic inflammatory skin disease or disorder as a candidate for treatment with an IL-13 antagonist therapeutic regimen comprising (a) measuring, or instructing a clinical laboratory to measure the DPP4 level in a sample obtained from a patient diagnosed with a pulmonary disease or disorder or a chronic inflammatory skin disease or disorder, wherein the patient's DPP4 level is measured with one or more anti-DPP4 antibodies or antigen binding fragments thereof which recognize human DPP4; and (b) treating, or instructing a healthcare provider to treat the patient with an IL-13 antagonist therapeutic regimen if the patient's DPP4 level in the sample is above a predetermined DPP4 threshold level, or is above the DPP4 level in one or more control samples.

E16. The method according to any one of embodiments E12 to E15, wherein the pulmonary disease or disorder is asthma, IPF, COPD, chronic rhinosinusitis, or allergic rhinitis or wherein the chronic inflammatory skin disease or disorder is atopic dermatitis, allergic contact dermatitis, eczema or psoriasis.

E17. The method according to embodiment E16, wherein the asthma is allergic asthma, atopic asthma, corticosteroid naive asthma, chronic asthma, corticosteroid resistant asthma, corticosteroid refractory asthma, asthma due to smoking, or asthma uncontrolled on corticosteroids.

E18. The method according to any one of embodiments E1 to E17, wherein the IL-13 antagonist comprises one or more of an anti-IL-13 antibody or antigen-binding fragment thereof, an IL-13 mutein, and IL-4 mutein, an anti-IL-13R$\alpha$1 antibody or antigen-binding fragment thereof, or an anti-IL-4R$\alpha$ antibody or antigen-binding fragment thereof.

E19. The method according to any one of embodiments E1 to E18, wherein the patient has been treated with one or more additional medications, either before, during, or after administration of an IL-13 antagonist.

E20. The method according to embodiment E19, wherein the one or more additional medications comprises a steroid.

E21. The method according to embodiment E19 or embodiment E20, wherein the one or more additional medications further comprises a bronchodilator.

E22. The method according to embodiment E20 or embodiment E21, wherein the steroid is fluticasone or budesonide.

E23. The method according to embodiment E21 or embodiment E22, wherein the bronchodilator is salbutamol or salmeterol.

E24. The method according to any one of embodiments E19 to E23, wherein the one or more additional medications are administered by inhalation, by oral administration, by injection, or by a combination thereof.

E25. The method according to embodiment E24, wherein inhalation administration is conducted using a metered dose inhaler (MDI) or a dry powder inhaler (DPI).

E26. The method according to embodiments E20 to E25, wherein the steroid is administered at a high dose.

E27. The method according to any one of embodiments E1 to E26, wherein the IL-13 antagonist is an anti-IL13 antibody, or antigen-binding fragment thereof.

E28. The method according to embodiment E27, wherein the antibody or fragment thereof binds to the same IL-13 epitope as tralokinumab or competitively inhibits binding of tralokinumab to IL-13, or both.

E29. The method according to embodiment E27 or embodiment E28, wherein the antibody or fragment thereof comprises tralokinumab or an antigen-binding fragment thereof.

E30. The method according to any one of embodiments E27 to E29, wherein the antibody or fragment thereof consists of tralokinumab or an antigen-binding fragment thereof.

E31. The method according to embodiment E27, wherein the antibody or fragment thereof binds to the same IL-13 epitope as lebrikizumab or competitively inhibits binding of lebrikizumab to IL-13, or both.

E32. The method according to embodiment E27 or embodiment E31, wherein the antibody or fragment thereof comprises lebrikizumab or an antigen-binding fragment thereof.

E33. The method according to embodiment E27, embodiment E31, or embodiment E32, wherein the antibody or fragment thereof consists of lebrikizumab or an antigen-binding fragment thereof.

E34. The method according to any one of embodiments E1 to E33, wherein the sample taken from the patient comprises one or more of whole blood, serum, plasma, saliva, sputum, bronchoalveolar lavage fluid, lung epithelial cells, urine, skin, or nasal polyps.

E35. The method according to embodiment E34, wherein the sample taken from the patient is blood serum.

E36. The method according to any one of embodiments E1 to E35, further comprising determining, submitting a sample taken from the patient for determination, or instructing a clinical laboratory to determine (i) the level of the patient's IgE levels, (ii) the patient's eosinophil count, (iii) the patient's Fraction of Exhaled Nitric Oxide (FENO), (iv) the patient's Eosinophil/Lymphocyte and Eosinophil/Neutrophil (ELEN) index, (v) the patient's EOS index, (vi) the patients wall area percentage (WA%) of subsegmental airways from a CT scan of the lungs, or (vii) a combination of two or more thereof.

E37. The method according to any one of embodiments E1 to E36, further comprising determining, submitting a sample taken from the patient for determination, or instructing a clinical laboratory to determine the expression level or activity of isoforms 1, 2, 3, or 4 of human periostin, a patient's blood eosinophil cell count, the level of the patient's IgE levels, pre- or post-bronchodilator FEV1 reversibility, wall area percentage (WA%) of subsegmental airways from a CT scan of the lungs, or combinations thereof.

E38. The method according to any one of embodiments E1 to E37, further comprising determining, submitting a sample taken from the patient for determination, or instructing a clinical laboratory to determine the expression level or activity of sCTLA-3, sCD28, CCL5, CCL11, CCL22, or combinations thereof.

E39. The method according to any one of embodiments E1 to E38, further comprising determining, submitting a sample taken from the patient for determination, or instructing a clinical laboratory to determine the expression level or activity of POSTN (SEQ ID NO:8), CSTI (SEQ ID NO:9), CCL26 (SEQ ID NO:10), CLCAI (SEQ ID NO:11), CST2 (SEQ ID NO:12), PRR4 (SEQ ID NO:13), SERPINB2 (SEQ ID NO:14), CEACAM5 (SEQ ID NO:15), iNOS (SEQ ID NO:16), SERPINB4 (SEQ ID NO:17), CST4 (SEQ ID NO:18), PRB4 (SEQ ID NO:19), TPSDI (SEQ ID NO:20), TPSGI (SEQ ID NO: 21), MFSD2 (SEQ ID NO:22), CPA3 (SEQ ID NO:23), GPR105 (SEQ ID NO:24), CDH26 (SEQ ID NO:25), GSN (SEQ ID NO:26), C2ORF32 (SEQ ID NO:27), TRACH2000196 (TMEM71) (SEQ ID NO:28), DNAJC12 (SEQ ID NO:29), RGS13 (SEQ ID NO: 30), SLC18A2 (SEQ ID NO: 31), SERPINBI0 (SEQ ID NO:32), SH3RF2 (SEQ ID NO:33), FCERIB (SEQ ID NO:34), RUNX2 (SEQ ID NO:35), PTGSI (SEQ ID NO:36), ALOX15 (SEQ ID NO:37), and combinations thereof.

E40. The method according to any one of embodiments E1 to E39, wherein the IL-13 antagonist is administered at a fixed dose.

E41. The method according to embodiment E30, wherein tralokinumab is administered at a fixed dose of about 300 mg/dose.

E42. The method according to any one of embodiments E1 to E41, wherein the IL-13 antagonist is administered in two or more doses.

E43. The method according to any one of embodiments E1 to E42, wherein the IL-13 antagonist is administered week, biweekly or monthly.

E44. The method according to any one of embodiments E1 to E43, wherein the IL-13 antagonist is administered biweekly.

E45. The method according to any one of embodiments E1 to E44, wherein the IL-13 antagonist is administered intravenously, intramuscularly, subcutaneously, or a combination thereof.

E46. The method according to any one of embodiments E1 to E45, wherein the predetermined DPP4 threshold level is at least about 250 ng/ml, at least about 350 ng/mL, at least about 375 ng/mL, at least about 400 ng/mL, at least about 450 ng/mL, at least about 500 ng/mL, at least 550 ng/mL, or at least about 600 ng/mL, as measured in serum using an ELISA.

E47. The method according to embodiment E46, wherein the ELISA is a QUANTIKINE® assay.

E48. The method according to embodiment E46 or embodiment E47, wherein the predetermined DPP4 threshold level is about 365 ng/mL.

E49. The method according to any one embodiments E1 to E48, wherein the one or more control samples are obtained from normal healthy individuals; patients with a non-IL-13-mediated subset of asthma; asthma patients naive for corticosteroid treatment; asthma patients treated with corticosteroids; a pre-determined standard amount of isolated DPP4; or a combination thereof.

E50. The method according to embodiment E49, wherein the one or more control samples comprise one or more of whole blood, serum, plasma, saliva, sputum, bronchoalveolar lavage fluid, lung epithelial cells, urine, skin, or a combination thereof.

E51. The method according to any one of embodiments E1 to E50, wherein administration of the IL-13 antagonist results in

(a) AER (Acute Exacerbation Rate) reduction;
(b) FEV1 (Forced Expiratory Volume in one second) increase;
(c) improved ACQ-6 (Asthma Control Questionnaire, 6-item version) results;
(d) improved AQLQ (Asthma Quality of Life Questionnaire) results; or,
(e) a combination thereof.

E52. The method according to embodiment E51, wherein the AER reduction is at least 5%, at least 10%, at least 15%, at least 20%, at least 25%, at least 30%, at least 35%, at least 40%, or at least 45% compared to the AER observed in a population of patients treated with a placebo.

E53. The method according to embodiment E51 or embodiment E52, wherein the mean AER reduction is about 28% compared to the mean AER observed in a population of patients treated with a placebo.

E54. The method according to embodiment E51, wherein the FEV1 increase is at least 3%, at least 5%, at least 7%, at least 9%, at least 11%, at least 13%, at least 15%, least 17%, or at least 19% compared to the FEV1 observed in a population of patients treated with a placebo.

E55. The method according to embodiment E51 or embodiment E54, wherein the mean FEV1 increase is about 10% compared to the mean FEV1 observed in a population of patients treated with a placebo.

E56. The method according to any one embodiments E3 to E55, comprising administering the IL-13 antagonist to the patient if (a) the level of DPP4 in a sample taken from the patient is above a predetermined DPP4 threshold level, or is above the DPP4 level in one or more control samples, and (b) the patient presents (i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ 0.14 × $10^9$/L), (iv) FEV1 reversibility to a short-acting β2 agonist ≥ 12%, (v) wall area percentage (WA%) of subsegmental airways from CT scan of the lungs ≥ 68%, or (vi) combinations thereof.

E57. The method according to any one embodiments E7 to E56, wherein DPP4 is measured in an immunoassay.

E58. A method of identifying a patient as a candidate for treatment with an IL-13 antagonist comprising measuring the level of DPP4 (dipeptidyl peptidase-4) in a sample taken from the patient, wherein a level of DPP4 above a predetermined DPP4 threshold level, or above the DPP4 level in one or more control samples identifies the patient as a candidate for treatment with the IL-13 antagonist.

E59. The method according to embodiment E58 further comprising measuring one or more of periostin, eosinophil cell count, IgE and FEV1 reversibility, wherein a level of DPP4 above a predetermined DPP4 threshold level, or above the DPP4 level in one or more control samples and one or more of the following: (i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ 0.14 × $10^9$/L), (iv) wall area percentage (WA%) of subsegmental airways from a CT scan of the lungs ≥ 68%, or (v) FEV1 reversibility to a short-acting β2 agonist ≥ 12%, identifies the patient as a candidate for treatment with the IL-13 antagonist.

E60. The method according to any one of embodiments E58 or E59, wherein the patient has asthma, IPF, COPD, chronic rhinosinusitis, allergic rhinitis, or atopic dermatitis.

E61. The method according to embodiment E60, wherein the asthma is allergic asthma, atopic asthma, corticosteroid naive asthma, chronic asthma, corticosteroid resistant asthma, corticosteroid refractory asthma, asthma due to smoking, or asthma uncontrolled on corticosteroids.

E62. The method according to any one of embodiments E58 to E61, wherein the IL-13 antagonist is an anti-IL13 antibody, or antigen-binding fragment thereof.

E63. The method according to embodiment E62, wherein the anti-IL13 antibody, or antigen-binding fragment thereof comprises tralokinumab, or an antigen-binding fragment thereof, or lebrikizumab, or an antigen-binding fragment thereof.

E64. The method according to any one of embodiments E58 to E63, wherein the predetermined DPP4 threshold level is at least about 250 ng/ml, at least about 350 ng/mL, at least about 375 ng/mL, at least about 400 ng/mL, at least about 450 ng/mL, at least about 500 ng/mL, at least 550 ng/mL, or at least about 600 ng/mL, as measured in serum using an ELISA.

E65. The method according to embodiment E64, wherein the ELISA is a QUANTIKINE® assay.

E66. The method according to embodiment E64 or embodiment E65, wherein the predetermined DPP4 threshold level is about 365 ng/mL.

E67. The method according to any one embodiments E58 to E66, wherein the one or more control samples are obtained from normal healthy individuals; patients with a non-IL-13-mediated subset of asthma; asthma patients naive for corticosteroid treatment; asthma patients treated with corticosteroids; untreated atopic dermatitis patients; treated atopic dermatitis patients; a pre-determined standard amount of isolated DPP4; or a combination thereof.

E68. The method according to embodiment E67, wherein the one or more control samples comprise one or more of whole blood, serum, plasma, saliva, sputum, bronchoalveolar lavage fluid, lung epithelial cells, urine, skin, or a combination thereof.

E69. A method of diagnosing an IL-13 mediated disease or disorder in a patient comprising measuring the level of DPP4 (dipeptidyl peptidase-4) in a sample taken from the patient, wherein the patient is diagnosed with the IL-13 mediated disease or disorder if the level of DPP4 is above a predetermined DPP4 threshold level, or above the DPP4 level in one or more control samples.

E70. The method according to embodiment E69 further comprising measuring one or more of periostin, eosinophil cell count, IgE and FEV1 reversibility, wherein the patient is diagnosed with the IL-13 mediated disease or disorder if the level of DPP4 is above a predetermined DPP4 threshold level, or above the DPP4 level in one or more control samples and the patient has one or more of the following: (i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ 0.14 × $10^9$/L), (iv) wall area percentage (WA%) of subsegmental airways from a CT scan of the lungs ≥ 68%, or (v) FEV1 reversibility to a short-acting β2 agonist ≥ 12%.

E71. The method according to any one of embodiments E69 or E70, wherein the IL-13 mediated disease or disorder is asthma, IPF, COPD, chronic rhinosinusitis, allergic rhinitis, or atopic dermatitis.

E72. The method according to embodiment E71, wherein the asthma is allergic asthma, atopic asthma, corticosteroid naive asthma, chronic asthma, corticosteroid resistant asthma, corticosteroid refractory asthma, asthma due to smoking, or asthma uncontrolled on corticosteroids.

E73. The method according to any one of embodiments E69 to E72, wherein the predetermined DPP4 threshold level is at least about 250 ng/ml, at least about 350 ng/mL, at least about 375 ng/mL, at least about 400 ng/mL, at least about 450 ng/mL, at least about 500 ng/mL, at least 550 ng/mL, or at least about 600 ng/mL, as measured in serum using an ELISA.

E74. The method according to embodiment E73, wherein the ELISA is a QUANTIKINE® assay.

E75. The method according to embodiment E73 or embodiment E74, wherein the predetermined DPP4 threshold level is about 365 ng/mL.

E76. The method according to any one embodiments E69 to E75, wherein the one or more control samples are obtained from normal healthy individuals; patients with a non-IL-13-mediated subset of asthma; asthma patients naive for corticosteroid treatment; asthma patients treated with corticosteroids; a pre-determined standard amount of isolated DPP4; or a combination thereof.

E77. The method according to embodiment E76, wherein the one or more control samples comprise one or more of whole blood, serum, plasma, saliva, sputum, bronchoalveolar lavage fluid, lung epithelial cells, urine, skin, or a combination thereof.

E78. The method according to any one of embodiments E1 to E77, wherein the method further comprises (a) determining the wall area percentage (WA%) from a Computed Tomography (CT) scan taken from the patient's lungs; (b) submitting a CT scan taken from the patient's lung for determination of WA% from the CT scan; or, (c) instructing a clinical laboratory to determine WA% from the CT scan.

E79. A method of treating a patient having an interleukin-13 (IL-13)-mediated disease or disorder, comprising administering an IL-13 antagonist to the patient if wall area percentage (WA%) measured from a CT scan of the patient's lung is above a predetermined WA% threshold level, or is above a WA% threshold level calculated from one or more control CT scans.

E80. A method of treating a patient having an IL-13-mediated disease or disorder comprising (a) submitting a CT scan of the patient's lungs for measurement of a wall area percentage (WA%) from the CT scan; and, (b) administering an IL-13 antagonist to the patient if the patient's WA% from the CT scan is above a predetermined WA% threshold level, or is above a WA% threshold level calculated from one or more control CT scans.

E81. A method of treating a patient having an IL-13-mediated disease or disorder comprising (a) measuring wall area percentage (WA%) from a CT scan obtained from a patient's lungs having an IL-13-mediated disease or disorder; (b) determining whether the patient's WA% is above a predetermined WA% threshold level, or is above a WA% threshold level calculated from one or more control CT scans; and, (c) advising a healthcare provider to administer an IL-13 antagonist to the patient if the patient's WA% is above a predetermined WA% threshold level, or is above a WA% threshold level calculated from one or more control CT scans.

E82. The method according to any one of embodiments E79 to E81, wherein the IL-13-mediated disease or disorder is a pulmonary disease or disorder, or an inflammatory bowel disease or disorder, or a chronic inflammatory skin disease or disorder.

E83. The method according to embodiment E82, wherein the pulmonary disease or disorder is asthma, IPF, COPD, emphysema, chronic rhinosinusitis, or allergic rhinitis; or wherein the chronic inflammatory skin disease or disorder is atopic dermatitis, allergic contact dermatitis, eczema or psoriasis.

E84. The method according to embodiment E83, wherein the asthma is allergic asthma, atopic asthma, corticosteroid naive asthma, chronic asthma, corticosteroid resistant asthma, corticosteroid refractory asthma, asthma due to smoking, or asthma uncontrolled on corticosteroids.

E85. The method according to any one of embodiments E79 to E84, wherein the IL-13 antagonist is an anti-IL13 antibody, or antigen-binding fragment thereof.

E86. The method according to embodiment E85, wherein the antibody or fragment thereof comprises tralokinumab or an antigen-binding fragment thereof or lebrikizumab or an antigen-binding fragment thereof.

E87. The method according to any one of embodiments E79 to E86, wherein the predetermined wall area percentage (WA%) threshold level is at least about 50%, at least about 55%, at least about 60%, at least about 65%, at least about 70%, at least about 75%, or at least about 80%, as measured using 3D analysis of a CT scan of the subsegmental bronchi in the upper lobes.

E88. The method according to any one of embodiments E79 to E86, wherein the predetermined wall area percentage (WA%) threshold level is about 68% as measured using 3D analysis of a CT scan of the subsegmental bronchi in the upper lobes.

All patents and publications referred to herein are expressly incorporated by reference in their entireties.

Aspects of the present disclosure can be further defined by reference to the following non-limiting examples, which describe in detail preparation of certain antibodies of the present disclosure and methods for using antibodies of the present disclosure. It will be apparent to those skilled in the art that many modifications, both to materials and methods, can be practiced without departing from the scope of the present disclosure.

Examples

**Example 1**

**Identification of Peripheral Markers of IL-13 Activation in the Lung**

To identify genes that are regulated by IL-13 in the lung, human lung epithelial cells, and an air-liquid interface model of normal human bronchial epithelial cells were stimulated with IL-13 and the resulting transcriptional alterations were analyzed by whole genome array (WGA) and TAQMAN® PCR (see FIG. 1). Results from these stimulations revealed a number of potential markers for IL-13 pathway activation. WGA results for the EpiAirway Model and normal human bronchial epithelial cells are shown in FIG. 2 and FIG. 3, respectively. The results obtained via WGA were confirmed using TAQMAN® qPCR as shown in FIG. 4. As expected, periostin was found to be considerably up-regulated in response to IL-13. In addition to periostin up-regulation, other genes altered by IL-13 were identified, namely DPP4, CCL26, FETUB, and CST1. Accordingly, selecting one or more of these genes (or their respective expressed proteins) as biomarkers could be useful in selecting patients likely to be responsive to IL-13 antagonist therapy, for example treatment with an anti-IL-13 antibody such as tralokinumab.

One such up-regulated gene was dipeptidyl peptidase-4 (DPP4)/CD26, a highly conserved type II transmembrane glycoprotein that also exists as a shed or secreted form. DPP4 has been found in the circulation in several disease settings and DPP4 inhibitors are currently used in the treatment of type II diabetes. Additionally, DPP4 is expressed on multiple cell types in the lung and has previously been shown to be up-regulated in plasma from allergic asthmatic patients, independently of inhaled corticosteroid treatment (Lun et al. Increased expression of plasma and CD4+ T lymphocyte co-stimulatory molecule CD26 in adult patients with allergic asthma. J Clin Immunol 2007; 27:430-437).

Levels of DPP4 were determined in serum samples from asthma patients using a commercially available test (R&D Systems QUANTIKINE® Human DPPIV ELISA). A statistically significant increase in DPP4 levels in severe asthma patients compared to normal donors ($p < 0.05$) was found, with a trend toward increasing DPP4 levels in asthma patients with moderate disease. FIG. 5. Conversely, DPP4 protein levels were lower in serum from asthma patients taking oral and inhaled steroids compared to patients taking no medication, ADVAIR® only, or Albuterol and inhaled steroids (FIG. 6). The findings that IL-13 regulates DPP4, that serum DPP4 is increased in asthma patients with moderate or severe disease, and that oral and inhaled steroids lower serum DPP4 levels suggested that DPP4: (1) could be used as a peripheral marker of IL-13 pathway activation in asthmatic lungs; (2) could be informative in electing potential therapies for asthma patients, and (3) could be useful in selecting patients responsive to therapy using an IL-13 antagonist, for example, an anti-IL-13 antibody such as tralokinumab.

**Example 2**

**Method for DPP4 Quantification in Human Serum**

DPP4 was determined from human serum samples using a modified human DPP4/CD26 QUANTIKINE® ELISA kit (R&D Systems; Cat. No. DC260). The QUANTIKINE® immunoassay can be used for quantitative determination of human DPP4 concentrations in cell culture supernatants, serum, plasma, saliva, and urine. The immunoassay contains NS0-expressed recombinant human DPP4, and antibodies raised against the recombinant factor. The assay employed the quantitative sandwich enzyme immunoassay technique. A monoclonal antibody specific for DPP4 was pre-coated onto a microplate. Standards and samples were pipetted into the wells, and any DPP4 present was bound by the immobilized antibody. After washing away any unbound substances, an enzyme-linked polyclonal antibody specific for DPP4 was added to the wells. Following a wash to remove any unbound antibody-enzyme reagent, a substrate solution was added to the wells and color developed in proportion to the amount of DPP4 bound in the initial step. The color development was stopped and the intensity of the color is measured.

**DPPIV Reagents:**

(1) DPPIV Microplate, R&D Part 892951: 96 well polystyrene microplate (12 strips of 8 wells) coated with a rat monoclonal antibody against DPP4.
(2) DPPIV Conjugate, R&D Part 892952: 21 mL of polyclonal antibody against DPP4 conjugated to horseradish peroxidase with preservatives.
(3) DPPIV Standard, R&D Part 892953: 200 ng of recombinant human DPP4 in a buffer with preservatives, lyophilized.
(4) Assay Diluent RD1-57, R&D Part 895207: 11 mL of a buffered protein base with blue dye and preservatives.
(5) Calibrator Diluent RD5-33, R&D Part 895813: 3 vials (21 mL/vial) of a buffered protein base with preservatives, for serum/plasma samples (and alternative calibrator diluent RD5K, R&D Part 895119, consisting of 21 mL of an animal serum with preservatives can be used for cell culture supernatant, saliva, and urine samples).
(6) Color Reagent A, R&D Part 895000: 12.5 mL of stabilized hydrogen peroxide.
(7) Color Reagent B, R&D Part 895001: 12.5 mL of stabilized chromogen (tetramethylbenzidine).
(8) Stop Solution, R&D Part 895032: 6 mL of 2 N sulfuric acid.
(9) Plate Covers: 4 adhesive strips.

Sample collection and storage: sample were collected and stored by the following methods.

(1) Cell culture supernatants: Particulates were removed by centrifugation and assayed immediately, or samples were aliquoted and stored at a temperate of -20°C or lower. Repeated freeze-thaw cycles were avoided.
(2) Serum: A serum separator tube (SST) was used. Samples were allowed to clot for 30 minutes before centrifugation for 15 minutes at 1000xg. Serum was removed and assayed immediately or samples were aliquoted and stored at a temperature of -20°C or lower. Repeated freeze-thaw cycles were avoided.
(3) Plasma: Plasma was collected using heparin or EDTA as anticoagulant. Plasma was centrifuged for 15 minutes at 1000 x g within 30 minutes of collection. Plasma samples were assayed immediately or aliquoted and stored at a temperature of -20°C or lower. Repeated freeze-thaw cycles were avoided.
(4) Saliva: Saliva was collected using a collection device such as SALIVETTE® or equivalent. Saliva samples were assayed immediately or aliquoted and stored at a temperature of -20°C or lower. Repeated freeze-thaw cycles were avoided.
(5) Urine: The first urine of the day (mid-stream) was aseptically collected and voided directly into a sterile container. The samples was centrifuged to remove particulate matter and assayed immediately, or aliquoted and stored at a temperature of -20°C or lower. Repeated freeze-thaw cycles were avoided.

**DPP4 Assay Procedure:** All reagents and samples were brought to room temperature before use. All samples, standards, and controls were assayed in duplicate.
(1) All reagents, standard dilutions, and samples were prepared as indicated in the standard Quantikine® manufacturer's protocol.
(2) Samples were tested at 1:50 MRD (minimal required dilution). All the samples were tested at 2% serum matrix. Samples were thawed at room temperature and diluted with Calibrator Diluent RD5-33.

TABLE 1. Standard dilution scheme

| Solution ID | Target Concentration (ng/mL) | Source Solution | Source Solution Vol (μL) | Calibrator Diluent RD5-33 Vol (μL) | Dilution Factor |
|---|---|---|---|---|---|
| STD1 | 20 ng/mL | RS Pre-Dil | 40 | 360 | 10 |
| STD2 | 10 ng/mL | STD1 | 200 | 200 | 2 |
| STD3 | 5 ng/mL | STD2 | 200 | 200 | 2 |
| STD4 | 2.5 ng/mL | STD3 | 200 | 200 | 2 |
| STD5 | 1.25 ng/mL | STD4 | 200 | 200 | 2 |
| STD6 | 0.62 ng/mL | STD5 | 200 | 200 | 2 |
| STD7 | 0.31 ng/mL | STD6 | 200 | 200 | 2 |

(continued)

| Solution ID | Target Concentration (ng/mL) | Source Solution | Source Solution Vol (μL) | Calibrator Diluent RD5-33 Vol (μL) | Dilution Factor |
|---|---|---|---|---|---|
| Blank | 0.01 ng/Ml | N/A | N/A | 200 | N/A |

TABLE 2. QC dilution scheme

| Solution ID | Target Concentration (ng/mL) | Source Solution | Source Solution Vol (μL) | Calibrator Diluent RD5-33 Vol (μL) | Dilution Factor |
|---|---|---|---|---|---|
| Pre-Dilution A | 200 | QCS 200 ng/mL | N/A | N/A | N/A |
| Pre-Dilution B | 15 | Pre-Dilution A | 20 | 246 | 13.3 |
| QC1 (High QC) | 10 | Pre-Dilution A | 20 | 380 | 20 |
| QC2 (Medium QC) | 5 | Pre-Dilution B | 70 | 140 | 3 |
| QC3 (Low QC) | 1.3 | Pre-Dilution B | 20 | 210 | 11.5 |

(3) 100 μL of Assay Diluent RD1-57 was added to each well.
(4) 50 μL of Standard, control, or sample were added to each well. Samples were covered with a plate sealer, and incubated at room temperature for 2 hours.
(5) Each well was aspirated and washed, repeating the process 3 times for a total of 4 washes.
(6) 200 μL of DPP4 Conjugate was added to each well. Samples were covered with a new plate sealer, and incubated at room temperature for 2 hours.
(7) Well contents were aspirated and wells were washed 4 times.
(8) 200 μL Substrate Solution was added to each well. Samples were incubated at room temperature for 30 minutes, protected from light.
(9) 50 μL of Stop Solution was added to each well. Optical density was measured for each well at 450 nm within 30 minutes.

**Example 3**

**A Phase 2b, Randomized, Double-blind Study to Evaluate the Efficacy and Safety of SC Tralokinumab in Adults with Uncontrolled, Severe Asthma**

TABLE 3. List of Abbreviations

| | |
|---|---|
| ACQ-6 | Asthma Control Questionnaire (6-item version) |
| AER | asthma exacerbation rate |
| AHR | airway hyperresponsiveness |
| AQLQ(S) | Standardized Asthma Quality of Life Questionnaire |
| AQLQ(s)+ 12 | Standardized Asthma Quality of Life Questionnaire for 12 years and older |
| BASE | baseline $FEV_1$ |
| CI | confidence interval |
| CPAP | continuous positive airway pressure |

(continued)

| | |
|---|---|
| DPI | dry powder inhaler |
| ePRO | electronic patient reported outcome |
| $FEV_1$ | forced expiratory volume in one second |
| HRQoL | health-related quality of life |
| $IC_{50}$ | half-maximal inhibitory concentration |
| ICS | inhaled corticosteroids |
| IgE | immunoglobulin E |
| IL-13 | interleukin- 13 |
| LABA | long-acting β2 agonist |
| MCID | minimal clinical important change |
| MDI | metered dose inhaler |
| MRD | minimum required dilution |
| OCS | oral corticosteroid(s) |
| PEF | peak expiratory flow |
| PEFR | peak expiratory flow rate |
| Q2W | every 2 weeks |
| Q2/4W | every 2 weeks for 12 weeks followed by every 4 weeks |
| Q4W | every 4 weeks |
| SABA | short-acting β2 agonist |
| SC | Subcutaneous |
| SD | standard deviation |
| $t_{1/2}$ | half-life |
| TEAE | treatment-emergent adverse event |
| TESAE | treatment-emergent serious adverse event |
| Th2 | T helper type 2 |

I. Study Objectives & Design

Study CD-RI-CAT-354-1049 was a Phase 2b, randomized, double-blind, placebo-controlled, parallel-arm, multicenter study to evaluate the efficacy and safety of two SC treatment regimens of tralokinumab in adults with uncontrolled, severe asthma requiring high-dose ICS and LABA with or without additional controller medications (high-dose ICS defined as a total daily dose > 500 μg fluticasone DPI or > 440 μg metered dose inhaler (MDI; Global Strategy for Asthma Management and Prevention, Global Initiative for Asthma (GINA) 2012. Available from www.ginasthma.org; National Heart, Lung, and Blood Institute National Asthma Education and Prevention Program Expert Panel Report 3: Guidelines for the Diagnosis and Management of Asthma Full Report 2007.). A 5-week screening/run-in period (Week -5 to -1 [Day -1]) preceded randomisation. Starting at Week -4 (Day -28), patients received a fixed-dose combination product of fluticasone/salmeterol, either as an MDI (230 μg/21 μg) at a dose of 2 inhalations twice per day or as a DPI (500 μg/50 μg) at a dose of one inhalation twice per day. If the patient was also taking additional asthma controller medications (including leukotriene modifiers, theophylline, cromones, a secondary ICS, or oral prednisolone ≤ 20 mg/day or equivalent OCS), then these medications were to be continued at a stable dose during the screening/run-in and treatment period.

Key inclusion criteria:

(i) Documented physician-diagnosed asthma for at least 12 months prior to the screening/run-in period and either:

(a) Proof of post-bronchodilator reversibility of FEV1 ≥ 12% and ≥ 200 mL to a SABA documented within 36 months prior to Visit 1; OR

(b) Proof of a positive response to a methacholine (20% fall in $FEV_1$ [$PC_{20}$] ≤ 8 mg/mL), histamine or mannitol challenge documented within 36 months prior to Visit 1; OR

(c) A post-bronchodilator increase in $FEV_1$ ≥ 12% and ≥ 200 mL at Visit 1 or 2. (A maximum of 400 μg salbutamol administered for the reversibility assessment.)

(ii) An asthma controller regimen consistent with that described at Step 4 or 5 of the GINA guidelines (GINA, 2009) for at least 6 of the 12 months prior to the screening/run-in period and must have used physician prescribed high-dose ICS in combination with LABA for at least 30 days prior to the screening/run-in period

(iii) A history of at least 2 but no more than 6 documented asthma exacerbation events within the 12 months prior to the screening/run-in period

(iv) At least one of the following; a morning prebronchodilator FEV1 value of between 40% and 80% predicted or an ACQ-6 score for the preceding week of ≥ 1.5 at both screening and randomisation visits.

At least 390 patients aged between 18-75 years of age were planned to be randomised in a 1:1 ratio to one of 2 cohorts (Cohort 1 or Cohort 2). Within each cohort, patients were randomised in a 2:1 ratio to receive tralokinumab (300 mg) or placebo as follows:

Cohort 1: Tralokinumab 300 mg (n = 130) or Placebo (n = 65) as 2 SC injections Q2W for 50 weeks for a total of 26 doses.

Cohort 2: Tralokinumab 300 mg (n = 130) or Placebo (n = 65) as 2 SC injections Q2W for 12 weeks followed by Q4W for 38 weeks for a total of 16 doses.

Patients were stratified at screening by the number of asthma exacerbations in the past 12 months (2 versus > 2 but ≤ 6 exacerbations) and by chronic OCS use (presence versus absence).

The primary objective of this study was to evaluate the effect of two SC treatment regimens of tralokinumab (300 mg Q2W and 300 mg Q2/4W) on AER over 52 weeks. Secondary objectives were to evaluate the safety and tolerability of tralokinumab, the effect of tralokinumab on pulmonary function, patient reported outcomes (including ACQ-6 score and HRQoL using AQLQ[S], and asthma symptoms using the asthma daily diary). The design of the trial and key primary and secondary endpoints is summarized in FIG. 7.

Asthma exacerbation was defined as a progressive increase of asthma symptoms (cough, wheeze, chest tightness, and/or shortness of breath) that did not resolve after the initiation of rescue medications and remained troublesome for the patient resulting in either:

1. Use of systemic corticosteroids (tablets, suspension, or injection) or increase of a stable systemic maintenance dose for a duration of at least 3 consecutive days OR
2. Patient initiation of systemic corticosteroids for a duration of at least 3 consecutive days.

The trial was powered to detect a 40% reduction in annual AER for each tralokinumab treatment group compared to combined placebo from Cohorts 1 and 2 assuming an annual exacerbation rate in placebo group of 1.2 with 80% power and a significance level of 0.15. The sample size was adequate for prespecified subanalysis to explore the relationship between the clinical response to tralokinumab and the presence of peripheral blood biomarkers associated with upregulation of IL-13 in the asthmatic lung including serum periostin, peripheral eosinophil count, and Th2 status (Th2 high defined as IgE > 100 IU/mL and blood eosinophils ≥ 0.14 × 109/L; Woodruff et al., Am. J. Respir. Crit. Care Med. 2009; 180:388-395).

A total of 452 patients were randomised from 15 countries (Argentina, Canada, Chile, Czech Republic, France, Germany, Japan, Mexico, Philippines, Poland, Russia, South Korea, Spain, UK and US). All the efficacy and safety data collected through Week 53 have been analysed.

II. Disease Evaluation and Methods

***Pre- and Post-bronchodilator FEV1 and FVC Measurements Including Reversibility Calculations***

Pre- and post-bronchodilator FEV1 and FVC were performed at each spirometry assessment. The reversibility assessment was performed as follows:

(1) Prebronchodilator FEV1 measurement was assessed using spirometry. Spirometry was performed on equipment provided by a central vendor according to American Thoracic Society (ATS)/European Respiratory Society (ERS) guidelines (Miller et al., Eur. Respir. J. 2005 26:153-61). The following values were captured: pre- and postbronchodilator FEV1, FEV6, FVC, and IC. Spirometry testing was performed in the morning between 6:00 AM and 11:00 AM. On treatment days, spirometry testing was performed before administration of investigational product. All morning spirometry testing was completed between 6:00 AM and 11:00 AM and within ± 1 hour of the time the screening spirometry was completed. For example, if the screening spirometry was at 8:00 AM, then all spirometry testing at subsequent visits had to be completed between 7:00 AM and 9:00 AM. Subjects were required to refrain from strenuous exercise for 30 minutes prior to spirometry testing.

(2) After a gentle and incomplete expiration, a dose of 100 μg of salbutamol (or equivalent short acting bronchodilator) was inhaled in one breath to total lung capacity from a spacer device.

(3) Breath was then held for 5-10 seconds before the subject exhaled. Four separate doses of 100 μg of salbutamol were delivered at 30 second intervals.

(4) Subjects waited for 15-30 minutes (30 minutes if a short-acting anticholinergic agent was used).

(5) Postbronchodilator FEV1 measurement was assessed and the 2 best efforts that meet the ATS/ERS acceptability and reproducibility criteria were recorded. The maximum FEV1 of the 2 best efforts was used for the analysis. Each subject used the same dose and type of short acting bronchodilator throughout the study. Total doses of less than 400 μg of salbutamol or equivalent were used for the reversibility assessment. Reversibility was calculated as follows:

$$\% \text{ Reversibility} = (\text{post-bronchodilator FEV1- pre- bronchodilator FEV1}) \times 100 / \text{pre-bronchodilator FEV1}$$

***ACQ-6: Asthma Control Questionnaire***

The ACQ is a patient-reported questionnaire assessing asthma symptoms (night-time waking, symptoms on waking, activity limitation, shortness of breath, wheezing) and daily rescue bronchodilator use and FEV1 (Juniper et al., Eur. Respir. J. 1999 14:902-7). The ACQ-6 is a shortened version of the ACQ that assesses asthma symptoms (night-time waking, symptoms on waking, activity limitation, shortness of breath, wheezing, and short-acting β2 agonist use) omitting the FEV1 measurement from the original ACQ score. The ACQ-6 was completed during Visit 1 (Week -5). Subjects were provided with the ePRO device at Visit 2 and completed the ACQ-6 at home weekly between Visits 2 and 4, and every 4 weeks thereafter through Visit 33 (Week 75). Subjects were asked to recall how their asthma had been during the previous week by responding to one bronchodilator use question and 5 symptom questions. Questions were weighted equally and scored from 0 (totally controlled) to 6 (severely uncontrolled). The mean ACQ score was the mean of the responses. Mean scores of ≤ 0.75 indicated well-controlled asthma, scores between 0.75 and ≤ 1.5 indicated partly-controlled asthma, and a score > 1.5 indicated uncontrolled asthma (Juniper et al., Respir. Med. 2006 100:616-21). Individual changes of at least 0.5 were considered to be clinically meaningful (Juniper et al., Respir. Med. 2005 99:553-8).

***AQLQ: Asthma Quality of Life Questionnaire (Standardized Version)***

The AQLQ(S) is a 32-item questionnaire that measures the HRQoL experienced by asthma patients (Juniper et al., Chest. 1999 May; 115(5):1265-70) and was completed at the Week 1 visit, and then every 4 weeks at home through the Week 75 visit using an ePRO device. The questionnaire comprised 4 separate domains (symptoms, activity limitations, emotional function, and environmental stimuli). Subjects were asked to recall their experiences during the previous 2 weeks and to score each of the 32 questions on a 7-point scale ranging from 7 (no impairment) to 1 (severe impairment). The overall score was calculated as the mean response to all questions. The 4 individual domain scores (symptoms, activity limitations, emotional function, and environmental stimuli) were the means of the responses to the questions in each of the domains. Individual improvements in both the overall score and individual domain scores of 0.5 were identified as a minimally important change, with score changes of > 1.5 identified as large meaningful changes (Juniper et al., J. Clin. Epidemiol. 1994; 47: 81-7).

***Asthma Exacerbations***

For the purpose of this study, an asthma exacerbation occurring after Visit 1 was defined as a progressive increase of asthma symptoms (cough, wheeze, chest tightness, and/or shortness of breath) that did not resolve after the initiation of rescue medications and remained troublesome for the subject resulting in either (1) use of systemic corticosteroids (tablets, suspension or injection) or increase of a stable systemic maintenance dose for a duration of at least 3 consecutive days; or (2) initiation of systemic corticosteroids for a duration of at least 3 consecutive days.

An asthma exacerbation event was considered resolved 7 days after the last dose of OCS was administered (10 days after administration of an injectable corticosteroid). Courses of corticosteroids initiated after this time period were considered a separate new asthma exacerbation.

Asthma exacerbation severity was classified as follows:

(a) Moderate: Worsening symptoms requiring systemic corticosteroids for at least 3 consecutive days; and,
(b) Severe: Worsening symptoms requiring systemic corticosteroids and requiring urgent care evaluation and/or hospital admission.

III. Endpoints

***Effect of Tralokinumab on Pulmonary Function***

The effect of tralokinumab on pulmonary function was measured by pre- and post-bronchodilator FEV1, FEV6, FVC, and IC at clinic visits (morning); and PEF and FEV1 measured at home. Change from baseline in the mean values and percent change from baseline at various time points were summarized using descriptive statistics. Two-sample t-test were used to compare the changes from baseline and percent changes from baseline in the subject's pulmonary function between the individual tralokinumab treatment group and combined placebo.

***Effect of Tralokinumab on Patient Reported Outcomes***

The change from baseline in the mean ACQ-6 score was analyzed. The proportion of subjects achieving ACQ-6 $\leq$ 0.75, ACQ-6 $\leq$ 1.5, and a reduction from baseline in the mean ACQ-6 score $\geq$ 0.5 during the study was compared between the individual tralokinumab treatment group and the combined placebo using the Fisher's exact test. A stratified log-rank test was conducted to compare the time to first asthma control, defined as a reduction from baseline in the mean ACQ-6 score $\geq$ 0.5 was first observed. Health-related quality of life was evaluated using the AQLQ(S) and EQ-5D. The overall and 4 domain scores from the AQLQ(S) responses along with their respective changes from baseline were summarized using descriptive statistics. Additionally, the proportion of AQLQ(S) responders was reported; subjects with > 0.5 improvement and subjects with > 1.5 improvement from baseline in AQLQ(S) scores at each visit were reported separately.

The EQ-5D questionnaire assessed 5 dimensions: mobility, self-care, usual activities, pain/discomfort, and anxiety/depression. Each dimension had 3 response options (no problem, some or moderate problems, and unable or extreme problems) that reflect increasing levels of difficulty. The questionnaire also included a visual analog scale, where the patients were asked to rate their current health on a scale of 0-100, with 0 being the worst imaginable health state. The responses from each dimension and the visual analog scale were summarized by treatment group and visits. The shift tables were provided for each dimension. The change from baseline in visual analog scale was summarized with descriptive statistics by visit.

IV. Results

**Efficacy**

The primary efficacy analysis was based on the Intent-to-Treat (ITT) population (n = 452). The ITT population included all patients who were randomised into the study. Treatment arm was assigned according to the initial randomisation, regardless of whether patients received any investigational product or received an investigational product different from that to which they were randomised.

**Demography and Background Disease Characteristics**

Baseline demographic characteristics were generally similar between the placebo and tralokinumab groups (TABLE 4). The mean age of the patient population was 50.3 years (range 18-75 years). The majority of patients were not Hispanic or Latino, approximately half of patients were White, and a third were Asian. Approximately two thirds of the population was female.

TABLE 4: Summary of Baseline Demographic Characteristics (ITT Population)

| Characteristic | Placebo (n = 151) | 300 mg Tralokinumab Q2W (n = 150) | 300 mg Tralokinumab Q2/4W (n = 151) |
|---|---|---|---|
| **Age (years)** | | | |
| Mean (SD) | 50.3 (12.9) | 49.7 (12.2) | 50.5 (11.8) |
| Range (min-max) | 18-75 | 19-75 | 18-74 |
| **Gender** | | | |
| Male | 54 (35.8%) | 50 (33.3%) | 51 (33.8%) |
| Female | 97 (64.2%) | 100 (66.7%) | 100 (66.2%) |
| **Ethnicity** | | | |
| Hispanic or Latino | 30 (19.9%) | 38 (25.3%) | 35 (23.2%) |
| Not Hispanic or Latino | 121 (80.1%) | 112 (74.7%) | 116 (76.8%) |
| **Race** | | | |
| American Indian or Alaskan Native | 10 (6.6%) | 9 (6.0%) | 8 (5.3%) |
| Asian | 52 (34.4%) | 53 (35.3%) | 53 (35.1%) |
| Black or African American | 4 (2.6%) | 4 (2.7%) | 3 (2.0%) |
| White | 84 (55.6%) | 83 (55.3%) | 86 (57.0%) |
| Other | 1 (0.7%) | 1 (0.7%) | 1 (0.7%) |
| **BMI ($kg/m^2$)** | | | |
| Mean (SD) | 27.944 (5.154) | 26.512 (4.314) | 27.411 (5.047) |
| Range (min-max) | 18.90-40.00 | 16.00-38.54 | 17.27-39.90 |
| BMI = body mass index; ITT = intent-to-treat; Q2W = every 2 weeks; Q2/4W = 2 injections Q2W for 12 weeks followed by Q4W for 38 weeks; SD = standard deviation | | | |

Baseline disease characteristics were generally similar between the placebo and tralokinumab groups (TABLE 5), including serum periostin levels, blood eosinophil counts, and Th2 status. Between 16% and 18% of patients were reported to receive chronic OCS. DPP4 was measured as described in Example 2.

Mean FEV1 % reversibility ranged between 10.0% and 12.7%, with approximately a third of patients showed FEV1 reversibility ≥ 12% at baseline. The mean ACQ-6 scores and % predicted pre-bronchodilator FEV1 reflect a patient population with asthma that was not well controlled at baseline.

TABLE 5: Summary of Baseline Disease Characteristics (ITT Population

| Parameter | Measure | Placebo (n = 151) | 300 mg Tralokinumab Q2W (n = 150) | 300 mg Tralokinumab Q2/4W (n = 151) |
|---|---|---|---|---|
| **Markers of Asthma Control** | | | | |
| Pre-BD $FEV_1$ (L) | N | 149 | 146 | 149 |
| | Mean (SD) | 1.924 (0.599) | 1.922 (0.682) | 1.939 (0.706) |
| Pre-BD $FEV_1$ % Predicted | N | 149 | 146 | 149 |
| | Mean (SD) | 68.1 (16.2) | 68.3 (19.6) | 69.3 (18.6) |

(continued)

| Parameter | Measure | Placebo (n = 151) | 300 mg Tralokinumab Q2W (n = 150) | 300 mg Tralokinumab Q2/4W (n = 151) |
|---|---|---|---|---|
| **Markers of Asthma Control** | | | | |
| $FEV_1$ Reversibility (%) | N | 148 | 144 | 146 |
| | Mean (SD) | 12.734 (16.994) | 10.763 (14.404) | 10.060 (13.183) |
| | Median | 9.042 | 7.902 | 7.312 |
| Proportion of patients with $FEV_1$ Reversibility | ≥ 12% | 57 (38.5%) | 43 (28.7%) | 49 (33.6%) |
| | < 12% | 91 (61.5%) | 101 (70.1%) | 97 (66.4%) |
| | Missing | 3 | 6 | 5 |
| Mean ACQ-6[a] | N | 146 | 146 | 147 |
| | Mean (SD) | 2.54 (0.88) | 2.59 (1.07) | 2.52 (0.96) |
| Overall AQLQ(S) | N | 131 | 130 | 127 |
| | Mean (SD) | 4.01 (1.03) | 3.96 (1.05) | 4.02 (1.00) |
| Asthma Daily Diary Symptom Score | N | 151 | 147 | 145 |
| | Mean (SD) | 1.60 (0.71) | 1.49 (0.77) | 1.56 (0.69) |
| **Population Descriptors** | | | | |
| Chronic OCS Use | Negative | 124 (82.1%) | 124 (82.7%) | 127 (84.1%) |
| | Positive | 27 (17.9%) | 26 (17.3%) | 24 (15.9%) |
| Atopic Asthma Status | Non-atopic | 50 (34.2%) | 42 (28.6%) | 55 (37.7%) |
| | Atopic | 96 (65.8%) | 105 (71.4%) | 91 (62.3%) |
| | Missing | 5 | 3 | 5 |
| Periostin | N | 147 | 150 | 149 |
| | Mean (SD) | 23.959 (9.137) | 25.531 (10.656) | 25.480 (10.037) |
| | Median | 22.040 | 23.600 | 23.250 |
| Blood Eosinophil Count ($10^3$/UL) | N | 142 | 141 | 142 |
| | Mean (SD) | 0.370 (0.578) | 0.383 (0.388) | 0.344 (0.430) |
| Th2 Status [b] | Low | 73 (51.4%) | 61 (45.5%) | 70 (51.1%) |
| | High | 69 (48.6%) | 73 (54.5%) | 67 (48.9%) |
| | Missing | 9 | 16 | 14 |

(continued)

| Population Descriptors | | | | |
|---|---|---|---|---|
| DPP4 (ng/mL) | Median | 343.0 | 372.0 | 375.0 |
| ACQ-6 = Asthma Control Questionnaire 6; AQLQ(S) = Asthma Quality of Life Questionnaire - Standardised Version; $FEV_1$ = forced expiratory volume in 1 second; ITT = intent-to-treat; OCS = oral corticosteroid; Pre-BD = pre-bronchodilator; Q2W = every 2 weeks; Q2/4W = 2 injections Q2W for 12 weeks followed by Q4W for 38 weeks; SD = standard deviation; Th2 = T helper type 2<br>[a] Mean ACQ-6 score: ≤ 0.75 = well-controlled; scores > 0.75 and < 1.5 = partly controlled; scores ≥ 1.5 = uncontrolled<br>[b] Th2-high: IgE > 100 IU/mL and blood eosinophils ≥ 0.14 x $10^9$/L (Woodruff et. al. Am J Respir Crit Care Med. 180: 388-395 (2009)). | | | | |

Approximately a third of patients had childhood asthma with a median age of adult onset asthma 35-37 years (TABLE 6). Allergies were reported as an asthma trigger for over half of the patients. Over a third of patients reported 3-6 asthma exacerbations in the prior year (patients were required to have between 2-6 exacerbations in the prior year at study entry) and approximately 17% to 21% of patients reported an asthma-related hospital admission.

TABLE 6: Summary of Asthma History (ITT Population)

| Parameter | Measure | Placebo (n = 151) | 300 mg Tralokinumab Q2W (n = 150) | 300 mg Tralokinumab Q2/4W (n =151) |
|---|---|---|---|---|
| Childhood Asthma | Yes | 53 (35.1%) | 50 (33.3%) | 49 (32.5%) |
| Age of Adult Asthma Onset (years) | Median | 35.00 | 36.00 | 37.00 |
| | Range | 18.0-73.0 | 18.0-65.0 | 18.0-73.0 |
| Triggers: Allergies | Yes | 87 (57.6%) | 95 (63.3%) | 91 (60.3%) |
| Symptoms: Night-time Awakening (Last 3 Months) | > 2/week | 59 (39.1 %) | 77 (51.3%) | 73 (48.3%) |
| Symptoms: SABA Use (Last 3 Months) | 7 days/ week | 63 (41.7%) | 67 (44.7%) | 72 (47.7%) |
| Symptoms: Duration on High Dose ICS/LABA (Months) | Median | 12.00 | 12.00 | 12.00 |
| Exacerbation in the last 12 months [a] | > 2 - 6 | 35.8% | 35.3% | 37.1% |
| Hospital Admissions: Last 12 Months | Yes | 17.9% | 20.7% | 16.6% |
| Smoking History: Have You Ever Smoked | Yes | 38 (25.2%) | 46 (30.7%) | 38 (25.2%) |
| Comorbidity: Obesity | Current | 36 (23.8%) | 21 (14.0%) | 26 (17.2%) |
| ICS = inhaled corticosteroids; ITT = intent-to-treat; LABA = long-acting beta agonist; Q2W = every 2 weeks; Q2/4W = 2 injections Q2W for 12 weeks followed by Q4W for 38 weeks; SABA = short-acting beta agonist<br>[a] Patients were required to have between 2-6 exacerbations in the prior year at study entry | | | | |

**Analyses of the Primary and Key Secondary Efficacy Endpoints: ITT population**

**Summary of Annual Asthma Exacerbation Rate at Week 53**

In the ITT population, the annual AERs at Week 53 were similar between the placebo and tralokinumab groups (TABLE 7).

TABLE 7: Summary of Annual Asthma Exacerbation Rate at Week 53 (ITT Population)

| Parameter | Placebo (n = 151) | 300 mg Tralokinumab Q2W (n= 150) | 300 mg Tralokinumab Q2/4W (n = 151) |
|---|---|---|---|
| AER Rate[a] | 0.90 | 0.90 | 0.97 |
| 95% CI of Rate [a] | 0.75,1.08 | 0.75, 1.07 | 0.81, 1.14 |
| Rate Ratio (RR)[b] | -- | 0.93 | 1.02 |
| 95% CI of RR [b] | -- | 0.67, 1.30 | 0.71, 1.46 |
| P-value [c] | -- | 0.669 | 0.909 |

AER = asthma exacerbation rate; CI = confidence interval; ITT = intent-to-treat; Q2W = every 2 weeks; Q2/4W = 2 injections Q2W for 12 weeks followed by Q4W for 38 weeks; RR = rate ratio

[a] Rate = Total number of asthma exacerbations in each group / Total person-year follow-up in each group; and 95% CI rate is based on the exact 95% Poisson CI

[b] Rate ratio and 95% CI for the rate ratio were estimated from the Poisson regression (Pearson correction) with treatment group, age, gender, number of exacerbations in past year (2 vs > 2 but ≤ 6), atopic asthma status (atopic/non-atopic), chronic OCS use (presence versus absence) and geographical region as the covariates

[c] P-value from the Poisson regression based on pairwise comparison against placebo

**Key Secondary Efficacy Endpoints: Effect of Tralokinumab on FEV1**

A key secondary efficacy endpoint of the study was to evaluate the effect of tralokinumab on pulmonary function as assessed by changes from baseline in spirometry variables, in particular pre- and post-bronchodilator FEV1. Statistically significant mean increases from baseline were observed for both pre- and post-bronchodilator FEV1 at Week 53 for the tralokinumab 300 mg Q2W group compared with placebo ($p < 0.004$; TABLE 8). No statistically significant mean changes from baseline were observed for either pre- or post-bronchodilator FEV1 at Week 53 for the tralokinumab 300 mg Q2/4W group compared with placebo.

TABLE 8: Summary of Change from Baseline in FEV1 at Week 53 (ITT Population)

| Parameter | Placebo (n = 151) | 300 mg Tralokinumab Q2W (n = 150) | 300 mg Tralokinumab Q2/4W (n = 151) |
|---|---|---|---|
| **Pre-BD $FEV_1$ Change from Baseline (%)** | | | |
| **N** | 125 | 129 | 121 |
| Mean Estimate | 1.55 | 8.65 | 3.12 |
| Difference vs Placebo | -- | 7.10 | 1.57 |
| 95% CI of Difference | -- | 2.35,11.84 | -3.22,6.35 |
| P-value [c] | -- | 0.003 | 0.521 |
| **Post-BD $FEV_1$ Change from Baseline (%)** | | | |
| N | 125 | 125 | 119 |
| Mean Estimate | -1.84 | 5.75 | 0.99 |
| Difference vs Placebo | -- | 7.60 | 2.83 |
| 95% CI of Difference | -- | 3.87, 11.32 | -0.92, 6.58 |
| P-value [c] | -- | ≤ 0.001 | 0.139 |
| **Pre-BD $FEV_1$ Change from Baseline (L)** | | | |
| N | 125 | 129 | 121 |
| Mean Estimate | 0.02 | 0.13 | 0.04 |

(continued)

| **Pre-BD $FEV_1$ Change from Baseline (L)** | | | |
|---|---|---|---|
| Difference vs Placebo | -- | 0.12 | 0.03 |
| 95% CI of Difference | -- | 0.04, 0.20 | -0.05, 0.11 |
| P-value [c] | -- | 0.004 | 0.495 |
| **Post-BD $FEV_1$ Change from Baseline (L)** | | | |
| N | 125 | 125 | 119 |
| Mean Estimate | -0.05 | 0.09 | 0.01 |
| Difference vs Placebo | -- | 0.14 | 0.06 |
| 95% CI of Difference | -- | 0.08, 0.21 | -0.01, 0.13 |
| P-value | -- | < 0.001 | 0.073 |

CI = confidence interval; $FEV_1$ = forced expiratory volume in 1 second; ITT = intent-to-treat; Post-BD = Post-bronchodilator; Pre-BD = Pre-bronchodilator; Q2W = every 2 weeks; Q2/4W = 2 injections Q2W for 12 weeks followed by Q4W for 38 weeks

Change from baseline = current visit value - baseline value

Percent change from baseline = (current visit value - baseline value) / baseline value × 100

P-values are from a mixed effects repeated measure model comparing treatment effect between tralokinumab and placebo within each cohort at Week 53

Relative increases in FEV1 were observed in both treatment groups during the first 17 weeks of the study compared to placebo (FIG. 8). These increases were maintained through to Week 53 in the tralokinumab 300 mg Q2W group but were lost in the tralokinumab 300 mg Q2/4W group suggesting that Q4W maintenance dosing is inadequate to maintain improvements in airflow obstruction.

**Key Secondary Efficacy Endpoints: Effect of Tralokinumab on Patient Reported Outcomes ACQ-6 and AQLQ(S)**

The ACQ-6 is a shortened version of the ACQ that assesses asthma symptoms (night-time waking, symptoms on waking, activity limitation, shortness of breath, wheezing, and SABA use) omitting the FEV1 measurement from the original ACQ score. Questions were weighted equally and scored from 0 (totally controlled) to 6 (severely uncontrolled). The mean ACQ score is the mean of the responses. An ACQ score of ≥ 1.5 has a positive predictive value of 0.88 that the patient has inadequately controlled asthma (Juniper et al, 2006). Mean scores of ≤ 0.75 indicate well-controlled asthma, scores between 0.75 and < 1.5 indicate partly-controlled asthma, and a score ≥ 1.5 indicates uncontrolled asthma (Juniper et al., Respir Med. 2006 100:616-21). Individual changes of at least 0.5 are considered to be clinically meaningful (Juniper et al., Respir. Med. 2005 99:553-8.).

In this study, the ACQ-6 was completed at home using an ePRO device Q4W during the treatment period. The changes from baseline to Week 53 were evaluated. No statistically significant differences in ACQ-6 scores were found when comparing the placebo and tralokinumab groups at Week 53 (TABLE 9).

The AQLQ(S) is a 32-item questionnaire that measures the HRQoL experienced by asthma patients (Juniper et al., Chest. 1999 115:1265-70). The questionnaire comprised 4 separate domains (symptoms, activity limitations, emotional function, and environmental stimuli). Patients were asked to recall their experiences during the previous 2 weeks and to score each of the 32 questions on a 7-point scale ranging from 7 (no impairment) to 1 (severe impairment). The overall score was calculated as the mean response to all questions. Individual improvement in the overall score of 0.5 has been identified as a minimally important change, with score changes of > 1.5 identified as large meaningful changes (Juniper et al., J. Clin. Epidemiol. 1994 47:81-7).

In this study, the AQLQ(S) was completed at home using an ePRO device Q4W during the treatment period. The changes from baseline to Week 53 were evaluated. No statistically significant differences in AQLQ(S) scores were found when comparing the placebo and tralokinumab groups at Week 53 (TABLE 9).

TABLE 9: Summary of Change from Baseline in Mean ACQ-6 and AQLQ(S) at Week 53 (ITT Population)

| Parameter | Placebo (n = 151) | 300 mg Tralokinumab Q2W (n = 150) | 300 mg Tralokinumab Q2/4W (n = 151) |
|---|---|---|---|
| **Mean ACQ-6: Change from Baseline** | | | |
| N | 118 | 115 | 112 |
| Mean Estimate | -0.66 | -0.84 | -0.78 |
| Difference vs Placebo | -- | -0.18 | -0.12 |
| 95% CI of Difference | -- | -0.43,0.06 | -0.36, 0.12 |
| P-value | -- | 0.137 | 0.335 |
| **AQLQ(S) Overall Score: Change from Baseline** | | | |
| N | 107 | 109 | 101 |
| Mean Estimate | 0.70 | 0.91 | 0.89 |
| Difference vs Placebo | -- | 0.21 | 0.19 |
| 95% CI of Difference | -- | -0.05, 0.46 | -0.07, 0.45 |
| P-value | -- | 0.114 | 0.159 |
| ACQ-6 = Asthma Control Questionnaire 6; AQLQ(S) = Asthma Quality of Life Questionnaire-Standardised Version; CI = confidence interval; ITT = intent-to-treat; Q2W = every 2 weeks; Q2/4W = 2 injections Q2W for 12 weeks followed by Q4W for 38 weeks<br>P-values are from a mixed effects repeated measure model comparing treatment effect between tralokinumab and placebo within each cohort at Week 53 | | | |

**Key Secondary Efficacy Endpoints: Effect of Tralokinumab on the Asthma Daily Diary**

The asthma daily diary is a 13-item questionnaire. The asthma daily diary was assessed each morning from Visit 2 (Week -4) through the Week 75 visit using an ePRO device. Patients were asked to recall their experience with daytime and night-time symptom frequency and severity, activity avoidance and limitation, asthma-related anxiety, and fatigue, as well as rescue medication use. The overall symptom score was calculated as the average of daytime severity score, daytime frequency score, and night-time severity score, and ranges from zero (no symptom) to 4 (worst possible symptom).

The changes from baseline to Week 53 were evaluated. No statistically significant differences in asthma daily diary overall asthma symptom scores were found when comparing the placebo and tralokinumab groups at Week 53 (TABLE 10).

TABLE 10: Summary of Change from Baseline in Asthma Daily Diary at Week 53 (ITT Population)

| Parameter | Placebo (n = 151) | 300 mg Tralokinumab Q2W (n = 150) | 300 mg Tralokinumab Q2/4W (n = 151) |
|---|---|---|---|
| Overall Asthma Symptom Score | | | |
| N | 112 | 108 | 108 |
| Mean Estimate | -0.30 | -0.36 | -0.39 |
| Difference vs Placebo | -- | -0.06 | -0.09 |
| 95% CI of Difference | -- | -0.21, 0.10 | -0.25, 0.06 |

(continued)

| Parameter | Placebo (n = 151) | 300 mg Tralokinumab Q2W (n = 150) | 300 mg Tralokinumab Q2/4W (n = 151) |
|---|---|---|---|
| Overall Asthma Symptom Score | | | |
| P-value | -- | 0.454 | 0.234 |
| CI = confidence interval; ITT - intent to treat; Q2W = every 2 weeks; Q2/4W = 2 injections Q2W for 12 weeks followed by Q4W for 38 weeks<br>P-values are from a mixed effects repeated measure model comparing treatment effect between tralokinumab and placebo within each cohort at Week 53 | | | |

**Subgroup Analyses of the Key Primary and Secondary Efficacy Endpoints**

A number of pre-specified subgroup analyses were defined to explore the relationship between the clinical response to tralokinumab and baseline clinical criteria. Subgroups defined by baseline FEV1 % reversibility (≥ 12% vs < 12%), and OCS use (presence vs absence) are presented. In addition, subgroup analysis by prior exacerbation in the past 12 months at baseline (2 vs > 2 ≤ 6) was performed, no relationship with response to tralokinumab was observed. To explore the relationship between the clinical response to tralokinumab and peripheral blood biomarkers associated with upregulation of IL-13, subgroups defined by baseline serum periostin (≥ median vs < median), peripheral eosinophil count (≥ 300 cells/μL vs < 300 cells/μL), and Th2 status (Th2 high defined vs Th2 low) are presented.

**Subgroup Analysis: Baseline FEV1 Reversibility**

Subgroup analysis was undertaken based on the degree of FEV1 reversibility to SABA following administration of bronchodilator at the randomisation visit. A high reversible subgroup (≥ 12% change in FEV1 from baseline after SABA) and low reversible subgroup (< 12% change) were defined.

Analysis at Week 53 showed a reduction in the annual AER (34% [95% CI: -32, 67%]) in the tralokinumab 300 mg Q2W cohort compared with placebo in the high reversible group. No reduction in annual AER was observed in the low reversible group (TABLE 11).

Within the tralokinumab 300 mg Q2/4W cohort, the reduction in annual AER in the high reversible subgroup (24% [95% CI: -54, 63%]) was also numerically greater than in the low reversible group (2% [95% CI: -60, 40%]).

TABLE 11: Summary of Annual Asthma Exacerbation Rate at Week 53 By FEV1 Reversibility at Baseline (ITT Population)

| $FEV_1$ Reversibility Cut-point | Treatment Group | N | Rate | 95% CI of Rate | RR | 95% CI of RR | P-value |
|---|---|---|---|---|---|---|---|
| AER | | | | | | | |
| ≥ 12% | Placebo | 57 | 0.88 | 0.65, 1.18 | -- | -- | -- |
| | Tralokinumab 300 mg Q2W | 43 | 0.68 | 0.45, 0.99 | 0.66 | 0.33, 1.32 | 0.245 |
| | Tralokinumab 300 mg Q2/4W | 49 | 1.08 | 0.80, 1.42 | 0.76 | 0.37, 1.54 | 0.438 |

(continued)

| $FEV_1$ Reversibility Cut-point | Treatment Group | N | Rate | 95% CI of Rate | RR | 95% CI of RR | P-value |
|---|---|---|---|---|---|---|---|
| AER | | | | | | | |
| < 12% | Placebo | 91 | 0.93 | 0.73, 1.16 | -- | -- | -- |
| | Tralokinumab 300 mg Q2W | 101 | 0.98 | 0.79, 1.20 | 1.00 | 0.65, 1.55 | 0.993 |
| | Tralokinumab 300 mg Q2/4W | 97 | 0.90 | 0.71, 1.12 | 0.98 | 0.60, 1.60 | 0.931 |
| CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks; RR = rate ratio<br>Rate = Total number of asthma exacerbations in each group / Total person-year follow-up in each group; and 95% CI rate is based on the exact 95% Poisson CI<br>Rate ratio and 95% CI for the rate ratio were estimated from the Poisson regression (Pearson correction) with treatment group, age, gender, number of exacerbations in past year (2 vs > 2 but ≤ 6), atopic asthma status (atopic/non-atopic), chronic OCS use (presence versus absence), and geographical region as the covariates P-value from the Poisson regression based on pairwise comparison against placebo | | | | | | | |

Analysis of Week 53 showed an increase from baseline in FEV1 in the tralokinumab 300 mg Q2W compared with placebo in both the high (11.07% [95% CI: 0.99, 21.14) and low reversible (7.48% [2.55, 12.40]) subgroups; this increase was numerically higher in the high reversible subgroup. Clinically relevant changes in FEV1 were not observed in the tralokinumab 300 mg Q2/4W cohort in either high or low reversible subgroup (TABLE 12).

TABLE 12: Summary of Change from Baseline in Key Secondary Efficacy Endpoints at Week 53 By FEV1 Reversibility at Baseline (ITT Population)

| $FEV_1$ Reversibility Cut-point | Treatment Group | N | Mean Estimate | Difference vs Placebo | 95% CI | P-value |
|---|---|---|---|---|---|---|
| **Change from Pre-bronchodilator Baseline $FEV_1$ (%)** | | | | | | |
| ≥ 12% | Placebo | 49 | 8.21 | -- | -- | -- |
| | Tralokinumab 300 mg Q2W | 35 | 19.28 | 11.07 | 0.99,21.14 | 0.031 |
| | Tralokinumab 300 mg Q2/4W | 40 | 8.84 | 0.63 | -9.30, 10.56 | 0.901 |
| < 12% | Placebo | 76 | -3.58 | -- | -- | -- |
| | Tralokinumab 300 mg Q2W | 92 | 3.90 | 7.48 | 2.55, 12.40 | 0.003 |
| | Tralokinumab 300 mg Q2/4W | 80 | -1.13 | 2.45 | -2.51, 7.42 | 0.332 |
| **Change from Pre-bronchodilator Baseline $FEV_1$ (L)** | | | | | | |
| ≥ 12% | Placebo | 49 | 0.12 | -- | -- | -- |
| | Tralokinumab 300 mg Q2W | 35 | 0.30 | 0.18 | 0.02, 0.34 | 0.031 |
| | Tralokinumab 300 mg Q2/4W | 40 | 0.15 | 0.03 | -0.13, 0.19 | 0.709 |

(continued)

| | | | | | | |
|---|---|---|---|---|---|---|
| **Change from Pre-bronchodilator Baseline $FEV_1$ (L)** | | | | | | |
| < 12% | Placebo | 76 | -0.07 | -- | -- | -- |
| | Tralokinumab300 mg Q2W | 92 | 0.06 | 0.13 | 0.05, 0.22 | 0.002 |
| | Tralokinumab 300 mg Q2/4W | 80 | -0.03 | 0.04 | -0.05, 0.12 | 0.386 |
| **ACQ-6** | | | | | | |
| ≥ 12% | Placebo | 43 | -0.33 | -- | -- | -- |
| | Tralokinumab 300 mg Q2W | 33 | -0.77 | -0.44 | -0.89, 0.01 | 0.055 |
| | Tralokinumab 300 mg Q2/4W | 35 | -0.71 | -0.37 | -0.82, 0.08 | 0.104 |
| < 12% | Placebo | 73 | -0.86 | -- | -- | -- |
| | Tralokinumab 300 mg Q2W | 78 | -0.92 | 0.06 | -0.37, 0.24 | 0.685 |
| | Tralokinumab 300 mg Q2/4W | 75 | -0.83 | 0.03 | -0.27, 0.33 | 0.846 |
| **AQLQ(S) Overall Score** | | | | | | |
| ≥ 12% | Placebo | 39 | 0.56 | -- | -- | -- |
| | Tralokinumab 300 mg Q2W | 29 | 1.15 | 0.59 | 0.10, 1.09 | 0.020 |
| | Tralokinumab 300 mg Q2/4W | 32 | 0.85 | 0.29 | -0.18, 0.77 | 0.226 |
| < 12% | Placebo | 66 | 0.81 | -- | -- | -- |
| | Tralokinumab 300 mg Q2W | 76 | 0.81 | 0.00 | -0.32, 0.33 | 0.983 |
| | Tralokinumab 300 mg Q2/4W | 67 | 0.89 | 0.08 | -0.24, 0.41 | 0.610 |
| **Asthma Daily Diary Overall Symptom Score** | | | | | | |
| ≥ 12% | Placebo | 40 | -0.19 | -- | -- | -- |
| | Tralokinumab 300 mg Q2W | 30 | -0.42 | -0.23 | -0.51, -0.04 | 0.094 |
| | Tralokinumab 300 mg Q2/4W | 34 | -0.32 | -0.13 | -0.40, 0.15 | 0.358 |
| < 12% | Placebo | 70 | -0.34 | -- | -- | -- |
| | Tralokinumab 300 mg Q2W | 74 | -0.36 | -0.02 | -0.22, 0.19 | 0.859 |
| | Tralokinumab 300 mg Q2/4W | 72 | -0.43 | -0.08 | -0.29, 0.12 | 0.407 |
| ACQ-6 = Asthma Control Questionnaire 6; AQLQ(S) = Asthma Quality of Life Questionnaire-Standardised Version; CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks<br>P-values are from a mixed effects repeated measure model comparing treatment effect between tralokinumab and placebo within each cohort at Week 53 | | | | | | |

In patients receiving tralokinumab 300 mg Q2W, the mean change in ACQ-6 score compared to placebo was numerically larger in the high reversible subgroup (-0.44 [95% CI: -0.89, 0.01]), and approximated the minimal clinical important change (MCID; -0.5) compared to the low reversible subgroup (0.06 [95% CI: -0.37, 0.24]). The same relationship was observed between the subgroups within the tralokinumab 300 mg Q2/4W cohort but the MCID was not reached in the high reversible subgroup (TABLE 12).

In patients receiving tralokinumab 300 mg Q2W, improvement in mean change in AQLQ(S) Overall score compared to placebo was observed in the high reversible subgroup (0.59 [95% CI: 0.10, 1.09]) and reached the MCID of 0.5. No improvement in AQLQ(S) was observed in the low reversible subgroup. The same relationship was observed between the subgroups within the tralokinumab 300 mg Q2/4W cohort, but the MCID was not reached in the high reversible subgroup (TABLE 12).

In patients receiving tralokinumab 300 mg Q2W a numerically greater reduction from baseline at Week 53 in the overall asthma daily diary symptom score compared to placebo was observed in the high reversible subgroup (-0.23 [95% CI: -0.51, -0.04]) compared with the low reversible subgroup (-0.02 [95% CI: -0.22, 0.19]). The same relationship was observed between the subgroups within the tralokinumab 300 mg Q2/4W cohort (TABLE 12).

**Subgroup Analysis: OCS Use**

Analysis at Week 53 showed that in patients without chronic OCS use there was a reduction in the annual AER compared to placebo in both the tralokinumab 300 mg Q2W (21% [95% CI: -17, 47%]) and the Q2/4W cohorts (13% [95% CI: -34, 43%]). No reduction in annual AER was observed for the tralokinumab 300 mg Q2W and Q2/4W cohorts compared with placebo in patients with chronic OCS use (TABLE 13).

TABLE 13: Summary of Annual Asthma Exacerbation Rate at Week 53 By Chronic Oral Corticosteroid Use (ITT Population)

| Chronic OCS Use | Treatment Group | N | Rate | 95% CI of Rate | RR | 95% CI of RR | P-value |
|---|---|---|---|---|---|---|---|
| AER | | | | | | | |
| With chronic OCS use | Placebo | 27 | 1.37 | 0.93, 1.94 | --- | --- | --- |
| | Tralokinumab 300 mg Q2W | 26 | 1.99 | 1.47, 2.64 | 1.18 | 0.58,2.41 | 0.647 |
| | Tralokinumab 300 mg Q2/4W | 24 | 2.20 | 1.62, 2.91 | 1.29 | 0.61,2.74 | 0.506 |
| Without chronic OCS use | Placebo | 124 | 0.81 | 0.66, 1.00 | --- | --- | --- |
| | Tralokinumab 300 mg Q2W | 124 | 0.68 | 0.54, 0.84 | 0.79 | 0.53, 1.17 | 0.240 |
| | Tralokinumab 300 mg Q2/4W | 127 | 0.74 | 0.59, 0.91 | 0.87 | 0.57, 1.34 | 0.525 |

CI = confidence interval; ITT = intent-to-treat; OCS = oral corticosteroid use; Q2W = every 2 weeks; Q2/4W = 2 injections Q2W for 12 weeks followed by Q4W for 38 weeks; RR = rate ratio
Rate = Total number of asthma exacerbations in each group / Total person-year follow-up in each group; and 95% CI rate is based on the exact 95% Poisson CI
Rate ratio and 95% CI for the rate ratio were estimated from the Poisson regression (Pearson correction) with treatment group, age, gender, number of exacerbations in past year (2 vs > 2 but ≤ 6), atopic asthma status (atopic/non-atopic), chronic OCS use (presence versus absence), and geographical region as the covariates
P-value from the Poisson regression based on pairwise comparison against placebo

Analysis at Week 53 showed a numerically greater increase from baseline in pre-bronchodilator FEV1 in the tralokinumab 300 mg Q2W cohort compared with placebo in patients without chronic OCS use (7.33% [95% CI: 2.52, 12.14]) compared with patients with chronic OCS use (0.87% [95% CI: -14.70, 16.44]; TABLE 14). Clinically relevant changes in FEV1 were not observed in the tralokinumab 300 mg Q2/4W cohort in either the with or without chronic OCS use subgroups.

Clinically important changes from placebo for ACQ-6, AQLQ(S), and asthma daily diary symptom scores were not observed in the with or without chronic OCS use subgroups for either tralokinumab cohort.

TABLE 14: Summary of Change from Baseline in Key Secondary Efficacy Endpoints at Week 53 By Chronic Oral Corticosteroid Use (ITT Population)

| Chronic OCS Use | Treatment Group | N | Mean Estimate | Difference vs Placebo | 95% CI | P-value |
|---|---|---|---|---|---|---|
| **Change from Pre-bronchodilator Baseline $FEV_1$ (%)** | | | | | | |
| With chronic OCS use | Placebo | 20 | 4.63 | --- | --- | --- |
| | Tralokinumab 300 mg Q2W | 21 | 5.51 | 0.87 | -14.70, 16.44 | 0.912 |
| | Tralokinumab 300 mg Q2/4W | 16 | 3.17 | -1.46 | -17.76, 14.84 | 0.860 |
| Without chronic OCS use | Placebo | 105 | 3.66 | --- | --- | --- |
| | Tralokinumab 300 mg Q2W | 108 | 10.99 | 7.33 | 2.52, 12.14 | 0.003 |
| | Tralokinumab 300 mg Q2/4W | 105 | 4.58 | 0.92 | -3.89, 5.73 | 0.708 |
| **Change from Pre-bronchodilator Baseline $FEV_1$ (L)** | | | | | | |
| With chronic OCS use | Placebo | 20 | 0.07 | --- | --- | --- |
| | Tralokinumab 300 mg Q2W | 21 | 0.06 | -0.01 | -0.26, 0.24 | 0.932 |
| | Tralokinumab 300 mg Q2/4W | 16 | -0.03 | -0.10 | -0.36,0.16 | 0.464 |
| Without chronic OCS use | Placebo | 105 | 0.04 | --- | --- | --- |
| | Tralokinumab300 mg Q2W | 108 | 0.16 | 0.13 | 0.04, 0.21 | 0.003 |
| | Tralokinumab 300 mg Q2/4W | 105 | 0.06 | 0.03 | -0.06, 0.11 | 0.530 |
| **ACQ-6** | | | | | | |
| With chronic OCS use | Placebo | 20 | -0.79 | --- | --- | --- |
| | Tralokinumab 300 mg Q2W | 17 | -1.16 | -0.37 | -0.97, 0.23 | 0.226 |
| | Tralokinumab 300 mg Q2/4W | 18 | -0.63 | 0.15 | -0.44, 0.75 | 0.613 |
| Without chronic OCS use | Placebo | 98 | -0.83 | --- | --- | --- |
| | Tralokinumab 300 mg Q2W | 98 | -1.00 | -0.17 | -0.43,0.09 | 0.208 |
| | Tralokinumab 300 mg Q2/4W | 94 | -1.01 | -0.18 | -0.44,0.09 | 0.186 |
| **AQLQ(S) Overall Score** | | | | | | |
| With chronic OCS use | Placebo | 17 | 0.54 | --- | --- | --- |
| | Tralokinumab 300 mg Q2W | 16 | 0.82 | 0.28 | -0.37,0.93 | 0.398 |
| | Tralokinumab 300 mg Q2/4W | 16 | 0.22 | -0.32 | -0.95, 0.32 | 0.327 |

(continued)

| Chronic OCS Use | Treatment Group | N | Mean Estimate | Difference vs Placebo | 95% CI | P-value |
|---|---|---|---|---|---|---|
| **AQLQ(S) Overall Score** | | | | | | |
| Without chronic OCS use | Placebo | 90 | 0.79 | --- | --- | --- |
| | Tralokinumab 300 mg Q2W | 93 | 1.02 | 0.23 | -0.05, 0.52 | 0.109 |
| | Tralokinumab 300 mg Q2/4W | 85 | 1.09 | 0.30 | 0.02, 0.59 | 0.039 |
| **Asthma Daily Diary Overall Symptom Score** | | | | | | |
| With chronic OCS use | Placebo | 20 | -0.36 | --- | --- | --- |
| | Tralokinumab 300 mg Q2W | 17 | -0.42 | -0.06 | -0.42, 0.30 | 0.749 |
| | Tralokinumab 300 mg Q2/4W | 18 | -0.12 | 0.24 | -0.12, 0.60 | 0.196 |
| Without chronic OCS use | Placebo | 92 | -0.39 | --- | --- | --- |
| | Tralokinumab 300 mg Q2W | 91 | -0.46 | -0.07 | -0.24, 0.11 | 0.456 |
| | Tralokinumab 300 mg Q2/4W | 90 | -0.55 | -0.15 | -0.32, 0.02 | 0.084 |
| **Chronic OCS Use** | **Treatment Group** | **N** | **Mean Estimate** | **Difference vs Placebo** | **95% CI** | **P-value** |
| **Change from Pre-bronchodilator Baseline $FEV_1$ (%)** | | | | | | |

ACQ-6 = Asthma Control Questionnaire 6; AQLQ(S) = Asthma Quality of Life Questionnaire-Standardised Version; CI = confidence interval; $FEV_1$ = forced expiratory volume in 1 second; OCS = oral corticosteroids; ITT= intent-to-treat; Q2W = every 2 weeks; Q2/4W = 2 injections Q2W for 12 weeks followed by Q4W for 38 weeks
P-values are from a mixed effects repeated measure model comparing treatment effect between tralokinumab and placebo within each cohort at Week 53

## Subgroup Analysis: Baseline Serum Periostin Level

Subgroup analysis at Week 53 by serum periostin level at baseline showed that reductions in the annual AER were observed in the tralokinumab 300 mg Q2W cohort compared with placebo in the high periostin group (≥ median serum periostin level at baseline; 25% [95% CI: -19, 53%]). No reduction in AER was observed in the low periostin group (< median serum periostin level at baseline; TABLE 15).

TABLE 15: Summary of Asthma Exacerbation Rate Through Week 53 By Serum Periostin Level at Baseline (ITT Population)

| Baseline Serum Periostin Cut-point | Treatment Group | N | Rate | 95% CI of Rate | RR | 95% CI of RR | P-value |
|---|---|---|---|---|---|---|---|
| AER | | | | | | | |
| ≥ Median | Placebo | 67 | 1.13 | 0.88, 1.43 | -- | -- | -- |
| | Tralokinumab 300 mg Q2W | 80 | 0.84 | 0.65, 1.08 | 0.75 | 0.47, 1.19 | 0.219 |
| | Tralokinumab 300 mg Q2/4W | 78 | 1.29 | 1.04, 1.57 | 0.97 | 0.58, 1.64 | 0.914 |

(continued)

| Baseline Serum Periostin Cut-point | Treatment Group | N | Rate | 95% CI of Rate | RR | 95% CI of RR | P-value |
|---|---|---|---|---|---|---|---|
| AER | | | | | | | |
| < Median | Placebo | 83 | 0.74 | 0.56, 0.96 | -- | -- | -- |
| | Tralokinumab 300 mg Q2W | 70 | 0.97 | 0.75, 1.23 | 1.08 | 0.68, 1.73 | 0.742 |
| | Tralokinumab 300 mg Q2/4W | 72 | 0.62 | 0.44, 0.84 | 0.89 | 0.56, 1.46 | 0.645 |
| CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks; RR = rate ratio<br>Rate = Total number of asthma exacerbations in each group / Total person-year follow-up in each group; and 95% CI rate is based on the exact 95% Poisson CI<br>Rate ratio and 95% CI for the rate ratio were estimated from the Poisson regression (Pearson correction) with treatment group, age, gender, number of exacerbations in past year (2 vs > 2 but ≤ 6), atopic asthma status (atopic/non-atopic), chronic OCS use (presence versus absence) and geographical region as the covariates<br>P-value from the Poisson regression based on pairwise comparison against placebo | | | | | | | |

Improvements from baseline in FEV1 compared to placebo was observed in both the high periostin (6.75% [95% CI-0.31, 13.82]) and the low periostin subgroups (7.06% [95% CI: 0.51, 13.60]; TABLE 16).

Clinically relevant changes in FEV1 were not observed in the tralokinumab 300 mg Q2/4W cohort in either periostin subgroup. Clinically important changes from placebo for ACQ-6, AQLQ(S), and asthma daily diary symptom scores were not observed in the high or low periostin subgroups for either tralokinumab cohort.

TABLE 16: Summary of Change from Baseline in Key Secondary Efficacy Endpoints at Week 53 By Serum Periostin Level at Baseline (ITT Population)

| Baseline Serum Periostin Cut-point | Treatment Group | N | Mean Estimate | Difference vs Placebo | 95% CI | P-value |
|---|---|---|---|---|---|---|
| **Change from Pre-bronchodilator Baseline $FEV_1$ (%)** | | | | | | |
| ≥ Median | Placebo | 54 | 4.68 | -- | -- | -- |
| | Tralokinumab 300 mg Q2W | 69 | 11.43 | 6.75 | -0.31, 13.82 | 0.061 |
| | Tralokinumab 300 mg Q2/4W | 63 | 5.31 | 0.64 | -6.55, 7.83 | 0.862 |
| < Median | Placebo | 70 | -1.27 | -- | -- | -- |
| | Tralokinumab 300 mg Q2W | 60 | 5.79 | 7.06 | 0.51, 13.60 | 0.035 |
| | Tralokinumab 300 mg Q2/4W | 57 | -0.76 | 0.51 | -6.06, 7.07 | 0.879 |
| **Change from Pre-bronchodilator Baseline $FEV_1$ (L)** | | | | | | |
| ≥ Median | Placebo | 54 | 0.07 | -- | -- | -- |
| | Tralokinumab 300 mg Q2W | 69 | 0.17 | 0.10 | -0.01, 0.21 | 0.062 |
| | Tralokinumab 300 mg Q2/4W | 63 | 0.09 | 0.02 | -0.09, 0.13 | 0.762 |

(continued)

| Change from Pre-bronchodilator Baseline $FEV_1$ (L) | | | | | | |
|---|---|---|---|---|---|---|
| < Median | Placebo | 70 | -0.03 | -- | -- | -- |
| | Tralokinumab 300 mg Q2W | 60 | 0.10 | 0.13 | 0.01, 0.25 | 0.0.29 |
| | Tralokinumab 300 mg Q2/4W | 57 | -0.02 | 0.01 | -0.11, 0.13 | 0.846 |
| **ACQ-6** | | | | | | |
| ≥ Median | Placebo | 51 | -0.71 | -- | -- | -- |
| | Tralokinumab 300 mg Q2W | 64 | -0.94 | -0.23 | -0.56, 0.09 | 0.163 |
| | Tralokinumab 300 mg Q2/4W | 59 | -0.65 | 0.06 | -0.27, 0.39 | 0.711 |
| < Median | Placebo | 66 | -0.66 | -- | -- | -- |
| | Tralokinumab 300 mg Q2W | 51 | -0.68 | -0.02 | -0.38, 0.34 | 0.919 |
| | Tralokinumab 300 mg Q2/4W | 53 | -0.94 | -0.28 | -0.64, 0.08 | 0.125 |
| **AQLQ(S) Overall Score** | | | | | | |
| ≥ Median | Placebo | 46 | 0.65 | -- | -- | -- |
| | Tralokinumab 300 mg Q2W | 62 | 0.87 | 0.22 | -0.15, 0.59 | 0.245 |
| | Tralokinumab 300 mg Q2/4W | 55 | 0.81 | 0.16 | -0.22, 0.53 | 0.412 |
| < Median | Placebo | 60 | 0.74 | -- | -- | -- |
| | Tralokinumab 300 mg Q2W | 47 | 0.95 | 0.21 | -0.16, 0.58 | 0.272 |
| | Tralokinumab 300 mg Q2/4W | 46 | 0.99 | 0.25 | -0.13,0.62 | 0.193 |
| **Asthma Daily Diary Overall Symptom Score** | | | | | | |
| ≥ Median | Placebo | 48 | -0.29 | -- | -- | -- |
| | Tralokinumab 300 mg Q2W | 56 | -0.35 | -0.06 | -0.29, 0.16 | 0.595 |
| | Tralokinumab 300 mg Q2/4W | 58 | -0.34 | -0.06 | -0.28, 0.17 | 0.619 |
| < Median | Placebo | 64 | -0.33 | -- | -- | -- |
| | Tralokinumab 300 mg Q2W | 52 | -0.37 | -0.04 | -0.26,0.17 | 0.685 |
| | Tralokinumab 300 mg Q2/4W | 50 | -0.43 | -0.10 | -0.32, 0.12 | 0.368 |

**Subgroup Analysis: Baseline Peripheral Blood Eosinophil Count**

Subgroup analysis at Week 53 by blood eosinophil count at baseline showed a reduction in the AER in the tralokinumab 300 mg Q2W cohort compared with placebo in the high eosinophil group (blood eosinophil count ≥ 300 cells/μL at baseline; 22% [95% CI: -31, 54%]). No reduction in the annual AER in the 300 mg Q2W cohort was observed for the low eosinophil group (blood eosinophil count < 300 cells/μL). See TABLE 17.

Conversely, no reduction in annual AER was observed in the high eosinophil subgroup for the tralokinumab 300 mg Q2/4W cohort; whereas, in the low eosinophil subgroup there was a reduction in the annual AER (23%).

TABLE 17: Summary of Asthma Exacerbation Rate Through Week 53 By Peripheral Blood Eosinophil Count at Baseline (ITT Population)

| Blood Eosinophil Count Cut-point | Treatment Group | N | Rate | 95% CI of Rate | RR | 95% CI of RR | P-value |
|---|---|---|---|---|---|---|---|
| AER | | | | | | | |
| ≥ 300 cells/μL | Placebo | 53 | 1.02 | 0.76, 1.35 | -- | -- | -- |
| | Tralokinumab 300 mg Q2W | 59 | 0.96 | 0.72, 1.26 | 0.78 | 0.46, 1.31 | 0.350 |
| | Tralokinumab 300 mg Q2/4W | 50 | 1.56 | 1.23, 1.97 | 1.26 | 0.67, 2.35 | 0.476 |
| < 300 cells/μL | Placebo | 89 | 0.89 | 0.70, 1.12 | -- | -- | -- |
| | Tralokinumab 300 mg Q2W | 82 | 0.83 | 0.64, 1.06 | 1.06 | 0.67, 1.68 | 0.794 |
| | Tralokinumab 300 mg Q2/4W | 92 | 0.63 | 0.47, 0.83 | 0.77 | 0.48, 1.22 | 0.258 |
| CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks; RR = rate ratio<br>Rate = Total number of asthma exacerbations in each group / Total person-year follow-up in each group; and 95% CI rate is based on the exact 95% Poisson CI<br>Rate ratio and 95% CI for the rate ratio were estimated from the Poisson regression (Pearson correction) with treatment group, age, gender, number of exacerbations in past year (2 vs > 2 but ≤ 6), atopic asthma status (atopic/non-atopic), chronic OCS use (presence versus absence) and geographical region as the covariates<br>P-value from the Poisson regression based on pairwise comparison against placebo | | | | | | | |

In the high eosinophil subgroup the percentage increase from baseline in FEV1 compared to placebo was numerically higher in patients receiving tralokinumab 300 mg Q2W (13.49% [95% CI: 4.99, 22.00]) compared to the low eosinophil subgroup (4.38% [95% CI: -1.51, 10.273]); TABLE 18). Clinically relevant changes in FEV1 were not observed in the tralokinumab 300 mg Q2/4W cohort in the high eosinophil subgroup.

The mean change in ACQ-6 score compared to placebo was larger in the high eosinophil subgroup in patients receiving tralokinumab 300 mg Q2W (-0.49 [95% CI: - 0.88, -0.09]) compared to the low eosinophil subgroup (-0.02 [95% CI: -0.34, 0.290]) and approximated the MCID of -0.50. This difference between subgroups was not apparent in the tralokinumab 300 mg Q2/4W cohort.

Clinically important changes from placebo for AQLQ(S) scores were not observed for either tralokinumab treatment cohort in the high eosinophil subgroup.

Numerically greater reductions in asthma daily diary symptom score compared with placebo were observed for both the 300 mg tralokinumab Q2W and Q2/4W cohorts in the high eosinophil subgroup (-0.21 [95% CI: -0.47, 0.04] and -0.19 [95% CI: -0.45, 0.07], respectively) compared with the low eosinophil subgroup (0.03 [95% CI: -0.17, 0.23] and -0.12 [95% CI: -0.32, 0.07]).

TABLE 18: Summary of Change from Baseline in Key Efficacy Endpoints at Week 53. By Peripheral Blood Eosinophil Count at Baseline (ITT Population)

| Blood Eosinophil Count Cut-point | Treatment Group | N | Mean Estimate | Difference vs Placebo | 95% CI | P-value |
|---|---|---|---|---|---|---|
| **Change from Pre-bronchodilator Baseline $FEV_1$ (%)** | | | | | | |
| ≥ 300 cells/μL | Placebo | 44 | 1.78 | -- | -- | -- |
| | Tralokinumab 300 mg Q2W | 50 | 15.27 | 13.49 | 4.99, 22.00 | 0.002 |
| | Tralokinumab 300 mg Q2/4W | 39 | 6.79 | 5.01 | -3.80, 13.82 | 0.264 |
| < 300 cells/μL | Placebo | 75 | 1.67 | -- | -- | -- |
| | Tralokinumab 300 mg Q2W | 72 | 6.05 | 4.38 | -1.51, 10.27 | 0.145 |
| | Tralokinumab 300 mg Q2/4W | 73 | 2.15 | 0.48 | -5.32, 6.28 | 0.871 |
| **Change from Pre-bronchodilator Baseline $FEV_1$ (L)** | | | | | | |
| ≥ 300 cells/μL | Placebo | 44 | 0.01 | -- | -- | -- |
| | Tralokinumab 300 mg Q2W | 50 | 0.24 | 0.23 | 0.09,0.37 | 0.001 |
| | Tralokinumab 300 mg Q2/4W | 39 | 0.11 | 0.10 | -0.04, 0.24 | 0.170 |
| < 300 cells/μL | Placebo | 75 | 0.03 | -- | -- | -- |
| | Tralokinumab 300 mg Q2W | 72 | 0.10 | 0.07 | -0.03,0.17 | 0.177 |
| | Tralokinumab 300 mg Q2/4W | 73 | 0.03 | 0.00 | -0.10, 0.10 | 0.992 |
| **ACQ-6** | | | | | | |
| ≥ 300 cells/μL | Placebo | 42 | -0.71 | -- | -- | -- |
| | Tralokinumab 300 mg Q2W | 44 | -1.20 | -0.49 | -0.88, -0.09 | 0.016 |
| | Tralokinumab 300 mg Q2/4W | 37 | -0.92 | -0.21 | -0.62,0.20 | 0.322 |
| < 300 cells/μL | Placebo | 71 | -0.64 | -- | -- | -- |
| | Tralokinumab 300 mg Q2W | 65 | -0.66 | -0.02 | -0.34, 0.29 | 0.888 |
| | Tralokinumab 300 mg Q2/4W | 68 | -0.86 | -0.22 | -0.53,0.09 | 0.164 |
| **AQLQ(S) Overall Score** | | | | | | |
| ≥ 300 cells/μL | Placebo | 38 | 0.72 | -- | -- | -- |
| | Tralokinumab 300 mg Q2W | 40 | 0.91 | 0.19 | -0.24,0.62 | 0.376 |
| | Tralokinumab 300 mg Q2/4W | 35 | 0.78 | 0.07 | -0.38, 0.51 | 0.765 |

(continued)

| AQLQ(S) Overall Score | | | | | | |
|---|---|---|---|---|---|---|
| < 300 cells/μL | Placebo | 64 | 0.67 | -- | -- | -- |
| | Tralokinumab 300 mg Q2W | 63 | 0.92 | 0.24 | -0.08, 0.57 | 0.146 |
| | Tralokinumab 300 mg Q2/4W | 59 | 1.04 | 0.37 | 0.04, 0.69 | 0.028 |
| **Asthma Daily Diary Overall Symptom Score** | | | | | | |
| ≥ 300 cells/μL | Placebo | 41 | -0.26 | -- | -- | -- |
| | Tralokinumab 300 mg Q2W | 42 | -0.47 | -0.21 | -0.47, 0.04 | 0.097 |
| | Tralokinumab 300 mg Q2/4W | 37 | -0.45 | -0.19 | -0.45,0.07 | 0.152 |
| **Blood Eosinophil Count Cut-point** | **Treatment Group** | **N** | **Mean Estimate** | **Difference vs Placebo** | **95% CI** | **P-value** |
| **Change from Pre-bronchodilator Baseline $FEV_1$ (%)** | | | | | | |
| < 300 cells/μL | Placebo | 67 | -0.33 | -- | -- | -- |
| | Tralokinumab 300 mg Q2W | 61 | -0.30 | 0.03 | -0.17, 0.23 | 0.777 |
| | Tralokinumab 300 mg Q2/4W | 64 | -0.45 | -0.12 | -0.32, 0.07 | 0.220 |
| ACQ-6 = Asthma Control Questionnaire 6; AQLQ(S) = Asthma Quality of Life Questionnaire-Standardised Version; CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks<br>P-values are from a mixed effects repeated measure model comparing treatment effect between tralokinumab and placebo within each cohort at Week 53 | | | | | | |

**Subgroup Analysis: Baseline Th2 Status**

Subgroup analysis at Week 53 by Th2 status at baseline showed a reduction in the annual AER in the tralokinumab 300 Q2W cohort compared with placebo in the high Th2 group at baseline (23% [95% CI: -25, 52%). No reduction in annual AER was observed in the low Th2 group (TABLE 19).

No increase in treatment effect for annual AER in the high or low Th2 subgroups was observed in the tralokinumab 300 mg Q2/4W cohort.

TABLE 19: Summary of Annual Asthma Exacerbation Rate at Week 53 By Th2 Status at Baseline (ITT Population)

| Th2 Status | Treatment Group | N | Rate [a] | 95% CI of Rate | RR[b] | 95% CI of RR[b] | P-value [c] |
|---|---|---|---|---|---|---|---|
| AER | | | | | | | |
| Th2 High | Placebo | 69 | 0.98 | 0.75, 1.25 | -- | -- | -- |
| | Tralokinumab 300 mg Q2W | 73 | 0.90 | 0.69, 1.16 | 0.77 | 0.48,1.25 | 0.298 |
| | Tralokinumab 300 mg Q2/4W | 67 | 1.09 | 0.85, 1.39 | 0.97 | 0.56, 1.67 | 0.900 |

(continued)

| Th2 Status | Treatment Group | N | Rate [a] | 95% CI of Rate | RR[b] | 95% CI of RR[b] | P-value [c] |
|---|---|---|---|---|---|---|---|
| AER | | | | | | | |
| Th2 Low | Placebo | 73 | 0.90 | 0.69, 1.16 | -- | -- | -- |
| | Tralokinumab 300 mg Q2W | 61 | 0.88 | 0.66, 1.16 | 1.11 | 0.67, 1.84 | 0.685 |
| | Tralokinumab 300 mg Q2/4W | 70 | 0.86 | 0.65, 1.12 | 1.06 | 0.66, 1.70 | 0.820 |

CI = confidence interval; $FEV_1$ = forced expiratory volume in one second; ITT = intent-to-treat; Q2W = every 2 weeks; Q2/4W = 2 injections Q2W for 12 weeks followed by Q4W for 38 weeks; RR = rate ratio
[a] Rate = Total number of asthma exacerbations in each group / Total person-year follow-up in each group; and 95% CI rate is based on the exact 95% Poisson CI
[b] Rate ratio and 95% CI for the rate ratio were estimated from the Poisson regression (Pearson correction) with treatment group, age, gender, number of exacerbations in past year (2 vs > 2 but ≤ 6), atopic asthma status (atopic/non-atopic), chronic OCS use (presence versus absence) and geographical region as the covariates
[c] P-value from the Poisson regression based on pairwise comparison against placebo

Improvement in the percentage increase from baseline in FEV1 compared to placebo was observed patients receiving tralokinumab 300 mg Q2W in both the high Th2 subgroup (8.64% [95% CI: 1.57, 15.716]) and the low Th2 subgroup (3.42% [95% CI: - 2.61, 9.44]). Clinically relevant changes in FEV1 were not observed in the tralokinumab 300 mg Q2/4W cohort in the high or low Th2 subgroup (TABLE 20).

Clinically important changes from placebo for ACQ-6, AQLQ(S), and asthma daily diary symptom scores were not observed in the high or low Th2 subgroups for either tralokinumab treatment cohort (TABLE 20).

TABLE 20: Summary of Change from Baseline in Key Secondary Efficacy Endpoints at Week 53 By Th2 Status at Baseline (ITT Population)

| Th2 Status | Treatment Group | N | Mean Estimate | Difference vs Placebo | 95% CI | P-value |
|---|---|---|---|---|---|---|
| **Change from Pre-bronchodilator Baseline $FEV_1$ (%)** | | | | | | |
| Th2 High | Placebo | 62 | 4.13 | -- | -- | -- |
| | Tralokinumab 300 mg Q2W | 61 | 12.77 | 8.64 | 1.57, 15.71 | 0.017 |
| | Tralokinumab 300 mg Q2/4W | 56 | 7.55 | 3.42 | -3.71, 10.56 | 0.346 |
| Th2 Low | Placebo | 57 | -1.57 | -- | -- | -- |
| | Tralokinumab 300 mg Q2W | 52 | 1.85 | 3.42 | -2.61, 9.44 | 0.266 |
| | Tralokinumab 300 mg Q2/4W | 53 | -2.82 | -1.25 | -7.14, 4.64 | 0.677 |
| **Change from Pre-bronchodilator Baseline $FEV_1$ (L)** | | | | | | |
| Th2 High | Placebo | 62 | 0.05 | -- | -- | -- |
| | Tralokinumab 300 mg Q2W | 61 | 0.21 | 0.16 | 0.04, 0.27 | 0.008 |
| | Tralokinumab 300 mg Q2/4W | 56 | 0.13 | 0.08 | -0.04, 0.20 | 0.188 |

(continued)

| Th2 Status | Treatment Group | N | Mean Estimate | Difference vs Placebo | 95% CI | P-value |
|---|---|---|---|---|---|---|
| **Change from Pre-bronchodilator Baseline $FEV_1$ (L)** | | | | | | |
| Th2 Low | Placebo | 57 | -0.03 | -- | -- | -- |
| | Tralokinumab 300 mg Q2W | 55 | 0.01 | 0.04 | -0.07, 0.15 | 0.439 |
| | Tralokinumab 300 mg Q2/4W | 53 | -0.06 | -0.03 | -0.14, 0.07 | 0.522 |
| **ACQ-6** | | | | | | |
| Th2 High | Placebo | 58 | -0.73 | -- | -- | -- |
| | Tralokinumab 300 mg Q2W | 56 | -0.99 | -0.25 | -0.61, 0.10 | 0.154 |
| | Tralokinumab 300 mg Q2/4W | 55 | -0.98 | -0.25 | -0.60, 0.11 | 0.170 |
| Th2 Low | Placebo | 55 | -0.55 | - | - | - |
| | Tralokinumab 300 mg Q2W | 47 | -0.66 | -0.11 | -0.47, 0.25 | 0.537 |
| | Tralokinumab 300 mg Q2/4W | 48 | -0.66 | -0.11 | -0.46, 0.24 | 0.538 |
| **AQLQ(S) Overall Score** | | | | | | |
| Th2 High | Placebo | 53 | 0.61 | -- | -- | -- |
| | Tralokinumab 300 mg Q2W | 52 | 0.93 | 0.31 | -0.06, 0.69 | 0.102 |
| | Tralokinumab 300 mg Q2/4W | 50 | 0.92 | 0.30 | -0.07, 0.68 | 0.115 |
| Th2 Low | Placebo | 49 | 0.73 | -- | -- | -- |
| | Tralokinumab 300 mg Q2W | 45 | 0.74 | 0.01 | -0.36, 0.38 | 0.973 |
| | Tralokinumab 300 mg Q2/4W | 42 | 0.91 | 0.18 | -0.18, 0.55 | 0.329 |
| **Asthma Daily Diary Overall Symptom Score** | | | | | | |
| Th2 High | Placebo | 55 | -0.31 | -- | -- | -- |
| | Tralokinumab 300 mg Q2W | 54 | -0.44 | -0.13 | -0.37, 0.10 | 0.261 |
| | Tralokinumab 300 mg Q2/4W | 52 | -0.41 | -0.11 | -0.34, 0.13 | 0.366 |
| Th2 Low | Placebo | 53 | -0.29 | -- | -- | -- |
| | Tralokinumab 300 mg Q2W | 43 | -0.20 | 0.08 | -0.14, 0.31 | 0.468 |
| | Tralokinumab 300 mg Q2/4W | 47 | -0.40 | -0.11 | -0.33, 0.10 | 0.309 |

(continued)

| Th2 Status | Treatment Group | N | Mean Estimate | Difference vs Placebo | 95% CI | P-value |
|---|---|---|---|---|---|---|
| Change from Pre-bronchodilator Baseline $FEV_1$ (%) | | | | | | |
| ACQ-6 = Asthma Control Questionnaire 6; AQLQ(S) = Asthma Quality of Life Questionnaire-Standardised Version; CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks<br>P-values are from a mixed effects repeated measure model comparing treatment effect between tralokinumab and placebo within each cohort at Week 53 | | | | | | |

**Post-hoc Subgroup Analyses**

In the tralokinumab 300 mg Q2W cohort, the presence of FEV1 reversibility to short acting bronchodilator was identified as an important patient characteristic indicating the potential for clinically important benefit on the annual AER, FEV1, ACQ-6, AQLQ(S), and asthma daily diary symptom score. In addition, in the subgroups postulated to be associated with upregulated IL-13 (high periostin, high eosinophils, high Th2) the reductions in annual AER were numerically greater than in the corresponding 'low' subgroups.

In order to further explore the clinical response to tralokinumab and identify a group of patients with the optimal response to tralokinumab, further subgroup analysis explored the combination of high vs low FEV1 reversibility based on the 12% cut-point and peripheral blood biomarkers.

**Post-hoc Subgroup Analyses: Baseline FEV1 Reversibility and Serum Periostin Level**

Within the high reversible group, analysis by serum periostin level at baseline showed that reductions in the annual AER were numerically greater in the tralokinumab 300 mg Q2W cohort compared with placebo in the high periostin group (54% [95% CI: - 65, 87%) compared to the low periostin group (4% [95% CI: -140, 61%]; TABLE 21).

TABLE 21: Summary of Annual Asthma Exacerbation Rate at Week 53 By FEV1 Reversibility and Serum Periostin Level (ITT Population)

| Baseline $FEV_1$ Reversibility | Baseline Serum Periostin Level | Treatment Group | N | Rate [a] | 95% CI of Rate [a] | RR[b] | 95% CI of RR [b] | P-value [c] |
|---|---|---|---|---|---|---|---|---|
| AER | | | | | | | | |
| ≥ 12% | ≥ median | Placebo | 26 | 1.17 | 0.77, 1.71 | -- | -- | -- |
| | | Tralokinumab 300 mg Q2W | 22 | 0.64 | 0.34, 1.10 | 0.46 | 0.13, 1.65 | 0.236 |
| | | Tralokinumab 300 mg Q2/4W | 28 | 1.43 | 1.02, 1.96 | 0.82 | 0.28, 2.37 | 0.713 |
| | < median | Placebo | 31 | 0.65 | 0.39, 1.02 | -- | -- | -- |
| | | Tralokinumab 300 mg Q2W | 21 | 0.72 | 0.39, 1.21 | 0.96 | 0.39, 2.40 | 0.932 |
| | | Tralokinumab 300 mg Q2/4W | 21 | 0.57 | 0.28, 1.02 | 0.59 | 0.18, 1.92 | 0.383 |

(continued)

| Baseline $FEV_1$ Reversib ility | Baseline Serum Periostin Level | Treatment Group | N | Rate [a] | 95% CI of Rate [a] | RR[b] | 95% CI of RR[b] | P-value [c] |
|---|---|---|---|---|---|---|---|---|
| < 12% | ≥ median | Placebo | 39 | 1.13 | 0.81, 1.54 | -- | -- | -- |
| | | Tralokinumab 300 mg Q2W | 57 | 0.91 | 0.67, 1.20 | 0.81 | 0.46, 1.43 | 0.468 |
| | | Tralokinumab 300 mg Q2/4W | 48 | 1.15 | 0.86, 1.50 | 0.86 | 0.41, 1.80 | 0.694 |
| | < median | Placebo | 51 | 0.79 | 0.56, 1.09 | -- | -- | -- |
| | | Tralokinumab 300 mg Q2W | 44 | 1.07 | 0.79, 1.43 | 1.01 | 0.55, 1.88 | 0.964 |
| | | Tralokinumab 300 mg Q2/4W | 48 | 0.65 | 0.43, 0.94 | 0.81 | 0.43, 1.50 | 0.501 |

CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks RR = rate ratio

[a] Rate = Total number of asthma exacerbations in each group / Total person-year follow-up in each group; and 95% CI rate is based on the exact 95% Poisson CI

[b] Rate ratio and 95% CI for the rate ratio were estimated from the Poisson regression (Pearson correction) with treatment group, age, gender, number of exacerbations in past year (2 vs > 2 but ≤ 6), atopic asthma status (atopic/non-atopic), chronic OCS use (presence versus absence) and geographical region as the covariates

[c] P-value from the Poisson regression based on pairwise comparison against placebo

In addition, in the high periostin subgroup the percentage increase from baseline in $FEV_1$ compared to placebo was numerically higher (13.85% [95% CI: -0.18, 27.87]) compared to the low periostin subgroup (7.62% [95% CI: -7.60, 22.84]; TABLE 22).

TABLE 22: Subgroup Analysis: Change from Baseline in Pre-bronchodilator at Week 53 By $FEV_1$ Reversibility and Serum Periostin Level (ITT Population)

| Baseline $FEV_1$ Reversibility | Baseline Serum Periostin Level | Treatment Group | N | Mean Estimate | Difference vs Placebo | 95% CI | P-value |
|---|---|---|---|---|---|---|---|
| **Change from Pre-bronchodilator Baseline $FEV_1$ (%)** | | | | | | | |
| ≥ 12% | ≥ median | Placebo | 21 | 8.34 | -- | -- | -- |
| | | Tralokinumab 300 mg Q2W | 18 | 22.18 | 13.85 | -0.18, 27.87 | 0.053 |
| | | Tralokinumab 300 mg Q2/4W | 23 | 3.01 | -5.33 | -19.09, 8.43 | 0.446 |
| | < median | Placebo | 28 | 8.24 | -- | -- | -- |
| | | Tralokinumab 300 mg Q2W | 17 | 15.86 | 7.62 | -7.60, 22.84 | 0.323 |
| | | Tralokinumab 300 mg Q2/4W | 17 | 12.53 | 4.29 | -10.99, 19.56 | 0.579 |

(continued)

| Baseline $FEV_1$ Reversibility | Baseline Serum Periostin Level | Treatment Group | N | Mean Estimate | Difference vs Placebo | 95% CI | P-value |
|---|---|---|---|---|---|---|---|
| **Change from Pre-bronchodilator Baseline $FEV_1$ (%)** | | | | | | | |
| < 12% | ≥ median | Placebo | 33 | 1.08 | -- | -- | -- |
| | | Tralokinumab 300 mg Q2W | 51 | 6.06 | 4.98 | -2.89, 12.85 | 0.214 |
| | | Tralokinumab 300 mg Q2/4W | 40 | 2.54 | 1.46 | -6.59, 9.51 | 0.721 |
| | < median | Placebo | 42 | -6.23 | -- | -- | -- |
| | | Tralokinumab 300 mg Q2W | 41 | 2.21 | 8.44 | 2.31, 14.57 | 0.007 |
| | | Tralokinumab 300 mg Q2/4W | 39 | -5.86 | 0.37 | -5.78, 6.52 | 0.906 |
| CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks<br>P-values are from a mixed effects repeated measure model comparing treatment effect between tralokinumab and placebo within each cohort at Week 53 | | | | | | | |

In patients in the tralokinumab 300 mg Q2W cohort with FEV1 reversibility ≥ 12% at baseline, improvements in AER and pre-bronchodilator FEV1 were observed; this treatment effect was enhanced in those patients that also had periostin > median. Within tralokinumab 300 mg Q2/4W cohort effect sizes on these endpoints were generally lower and the effect of periostin > median at baseline was not observed (TABLE 22 and FIG. 9).

On the endpoints of ACQ-6, AQLQ(S), and asthma daily diary for both the tralokinumab 300 mg Q2W and Q2/4W cohorts, the treatment effect observed in those patients that had FEV1 reversibility ≥ 12% at baseline was not further enhanced in those that also had periostin > median (TABLE 23 and FIG. 10).

Note that for the tralokinumab 300 mg Q2W cohort, changes in ACQ-6 and AQLQ(S) reached or approximated the MCID for patients that had FEV1 reversibility ≥ 12% at baseline (TABLE 23); this was not observed for the Q2/4W cohort.

TABLE 23: Subgroup Analysis - Change from Baseline in Patient Reported Outcomes at Week 53 By FEV1 Reversibility and Serum Periostin Level (ITT Population)

| Baseline $FEV_1$ Reversibility | Baseline Serum Periostin Level | Treatment Group | N | Mean Estimate | Difference vs Placebo | 95% CI | P-value |
|---|---|---|---|---|---|---|---|
| **Change from Pre-bronchodilator Baseline $FEV_1$ (%)** | | | | | | | |
| **ACQ-6** | | | | | | | |
| ≥ 12% | ≥ median | Placebo | 19 | -0.59 | -- | -- | -- |
| | | Tralokinumab 300 mg Q2W | 18 | -0.92 | -0.34 | -0.93, 0.25 | 0.260 |
| | | Tralokinumab 300 mg Q2/4W | 20 | -0.44 | 0.15 | -0.45, 0.75 | 0.626 |
| | < median | Placebo | 24 | -0.15 | -- | -- | -- |
| | | Tralokinumab 300 mg Q2W | 15 | -0.65 | -0.50 | -1.21, 0.21 | 0.164 |
| | | Tralokinumab 300 mg Q2/4W | 15 | -0.98 | -0.83 | -1.52, -0.14 | 0.019 |

(continued)

| Baseline $FEV_1$ Reversibility | Baseline Serum Periostin Level | Treatment Group | N | Mean Estimate | Differencevs Placebo | 95% CI | P-value |
|---|---|---|---|---|---|---|---|
| **Change from Pre-bronchodilator Baseline $FEV_1$ (%)** | | | | | | | |
| **ACQ-6** | | | | | | | |
| < 12% | ≥ median | Placebo | 31 | -0.73 | -- | -- | -- |
| | | Tralokinumab 300 mg Q2W | 46 | -0.95 | -0.23 | -0.65, 0.19 | 0.291 |
| | | Tralokinumab 300 mg Q2/4W | 39 | -0.72 | 0.00 | -0.42, 0.43 | 0.986 |
| | < median | Placebo | 41 | -0.96 | -- | -- | -- |
| | | Tralokinumab 300 mg Q2W | 32 | -0.79 | 0.17 | -0.28, 0.63 | 0.460 |
| | | Tralokinumab 300 mg Q2/4W | 36 | -0.92 | 0.04 | -0.40, 0.48 | 0.857 |
| **AQLQ(S) Overall Score** | | | | | | | |
| ≥ 12% | ≥ median - | Placebo | 17 | 0.57 | -- | -- | -- |
| | | Tralokinumab 300 mg Q2W | 16 | 1.15 | 0.58 | -0.11, 1.26 | 0.097 |
| | | Tralokinumab 300 mg Q2/4W | 18 | 0.47 | -0.10 | -0.76, 0.56 | 0.771 |
| | < median | Placebo | 22 | 0.62 | -- | -- | -- |
| | | Tralokinumab 300 mg Q2W | 13 | 1.26 | 0.63 | -0.13, 1.39 | 0.103 |
| | | Tralokinumab 300 mg Q2/4W | 14 | 1.28 | 0.66 | -0.05, 1.36 | 0.067 |
| < 12% | ≥ median | Placebo | 28 | 0.64 | -- | -- | -- |
| | | Tralokinumab 300 mg Q2W | 46 | 0.73 | 0.09 | -0.38, 0.55 | 0.713 |
| | | Tralokinumab 300 mg Q2/4W | 37 | 0.92 | 0.27 | -0.21, 0.75 | 0.262 |
| | < median | Placebo | 37 | 0.94 | -- | -- | -- |
| | | Tralokinumab 300 mg Q2W | 30 | 0.90 | -0.04 | -0.50, 0.43 | 0.876 |
| | | Tralokinumab 300 mg Q2/4W | 30 | 0.89 | -0.05 | -0.50, 0.40 | 0.836 |

(continued)

| Asthma Daily Diary Overall Symptom Score | | | | | | | |
|---|---|---|---|---|---|---|---|
| ≥ 12% | ≥ median | Placebo | 16 | -0.27 | -- | -- | -- |
| | | Tralokinumab 300 mg Q2W | 17 | -0.47 | -0.20 | -0.59, 0.19 | 0.305 |
| | | Tralokinumab 300 mg Q2/4W | 20 | -0.25 | 0.02 | -0.36, 0.41 | 0.899 |
| | < median | Placebo | 24 | -0.08 | -- | -- | -- |
| | | Tralokinumab 300 mg Q2W | 13 | -0.37 | -0.29 | -0.70, 0.12 | 0.160 |
| | | Tralokinumab 300 mg Q2/4W | 14 | -0.31 | -0.23 | -0.65,0.20 | 0.293 |
| **Baseline $FEV_1$ Reversibility** | **Baseline Serum Periostin Level** | **Treatment Group** | **N** | **Mean Estimate** | **Differencevs Placebo** | **95% CI** | **P-value** |
| < 12% | ≥ median | Placebo | 31 | -0.28 | -- | -- | -- |
| | | Tralokinumab 300 mg Q2W | 39 | -0.30 | -0.02 | -0.33, 0.28 | 0.874 |
| | | Tralokinumab 300 mg Q2/4W | 38 | -0.37 | -0.09 | -0.39, 0.22 | 0.580 |
| | < median | Placebo | 39 | -0.45 | -- | -- | -- |
| | | Tralokinumab 300 mg Q2W | 35 | -0.44 | 0.00 | -0.27, 0.28 | 0.992 |
| | | Tralokinumab 300 mg Q2/4W | 34 | -0.46 | -0.02 | -0.29, 0.25 | 0.891 |
| ACQ-6 = Asthma Control Questionnaire 6; AQLQ(S) = Asthma Quality of Life Questionnaire-Standardised Version; CI = confidence interval; $FEV_1$ = forced expiratory volume in 1 second; ITT = intent-to-treat; Q2W = every 2 weeks; Q2/4W = 2 injections Q2W for 12 weeks followed by Q4W for 38 weeks<br>P-values are from a mixed effects repeated measure model comparing treatment effect between tralokinumab and placebo within each cohort at Week 53 | | | | | | | |

**Summary of Efficacy**

In the ITT population, a reduction in the primary endpoint, the annual AER, was not observed in either tralokinumab treatment cohort compared to placebo; however, trends towards reductions in AER in patients receiving tralokinumab were observed in a number of prespecified subgroups (FIG. 11). In particular, the presence of FEV1 reversibility to SABA ≥12% at baseline was identified as an important clinical characteristic with 34% (95% CI: -32, 67%) reduction in the annual AER observed in the high reversible subgroup in the tralokinumab 300 mg Q2W cohort and 24% [95% CI: -54, 63%] reduction in the Q2/4W cohort; reductions in AER were not observed in the low reversible subgroups in either cohort.

In the high periostin, high eosinophil, and high Th2 subgroups, reductions in annual AER of 25% (95% CI: -19, 53%), 22% (95% CI: -31, 54%), and 23% (95% CI: - 25, 52%), respectively, were observed in the tralokinumab 300 mg Q2W cohort with no reductions in corresponding low subgroups. Reductions in annual AER were not observed in Q2/4W cohort in the high or low biomarker subgroups.

Reductions in AER were not observed in the subgroups receiving chronic OCS in either tralokinumab treatment cohort.

In the ITT population, a statistically significant increase from baseline in pre-bronchodilator FEV1 at Week 53 compared to placebo 7.10% (95% CI: 2.35, 11.84%) was observed in the tralokinumab 300 mg Q2W cohort. The effect size in the tralokinumab 300 mg Q2/4W cohort was lower 1.57% (95% CI: -3.22, 6.35%) indicating a dose-response relationship; this relationship was also observed across the prespecified subgroups with increases in pre-bronchodilator FEV1 at Week 53 consistently higher in the tralokinumab 300 mg Q2W cohort compared to the Q2/4W cohort (FIG. 12). Within the tralokinumab 300 mg Q2W cohort at Week 53, increases in pre-bronchodilator FEV1 compared to placebo were closely matched in both the high and low periostin subgroups and were numerically higher in the high reversible, high eosinophil and high Th2 subgroups than in the corresponding low subgroups.

No increase in pre-bronchodilator FEV1 was observed in the subgroups receiving chronic OCS in either tralokinumab treatment cohort.

In the ITT population, clinically important changes in ACQ-6, AQLQ(S), and asthma daily diary symptom score were not observed in either tralokinumab treatment cohort compared to placebo. In the high reversible subgroup, the mean changes compared to placebo for ACQ-6 and AQLQ scores approximated the MCID in the tralokinumab 300 mg Q2W cohort and were numerically higher than in the Q2/4W cohort; these improvements were not seen in the low reversible subgroup in either dose regimen. The largest reduction in the asthma daily diary symptom score was also observed in the high reversible subgroup in tralokinumab 300 mg Q2W cohort. In high periostin, high eosinophil and high Th2 subgroups clinically important changes in ACQ-6, AQLQ(S), and ASMA symptom score were not consistently observed in either tralokinumab cohort.

Three main conclusions were reached from the pre-specified ITT and subgroup analysis:

(i) In the ITT population and in the majority of subgroups tested, an increase in pre-bronchodilator $FEV_1$ was observed in the the tralokinumab 300 mg Q2W cohort. A dose response was evident with effects on this endpoint either smaller or absent in the Q2/4W cohort.

(ii) Subgroup analysis within the tralokinumab 300mg Q2W cohort identified potential responder populations in which reductions in annual AER were observed. The key subgroups identified were:

(a) Patients with $FEV_1$ reversibility to SABA ≥ 12% at baseline reduction. In this subgroup, consistent improvements in key secondary endpoints were observed (increase in $FEV_1$ 11.07% (95% CI: 0.99, 21.14), reduction in mean ACQ-6 -0.44 (95% CI: -0.89, 0.01), and increase in AQLQ(S) 0.59 (95% CI: 0.10, 1.09).

(b) Patients in those subgroups postulated to be associated with the presence of up-regulated airway IL-13 (high periostin, high eosinophils, high Th2) suggesting that blood biomarkers associated with up-regulation of IL-13 may be important in identifying patients that will derive most benefit.

(iii) Reductions in AER were not clearly replicated in the same key subgroups in the tralokinumab 300 mg Q2/4W cohorts.

Post hoc analysis explored the hypothesis that patients with FEV1 reversibility ≥ 12% and serum periostin ≥ median at baseline had an enhanced treatment response to tralokinumab 300 mg Q2W. In this subset of patients the reduction in AER was 54% (95% CI: -65, 87%) and the percentage increase from baseline in pre-bronchodilator FEV1 compared to placebo 13.85% (95% CI: -0.18, 27.87), was numerically greater than in those subjects with FEV1 reversibility ≥ 12% and serum periostin < median (reduction in AER 4% [95% CI: -140, 61%] and increase in FEV1 7.62% [95% CI: -7.60, 22.84]).

In summary, the addition of tralokinumab to high dose ICS and other asthma controller therapies at a dose of 300 mg Q2W results in an increase in pre-bronchodilator $FEV_1$ in the ITT population and reduction in AER in biologically relevant subgroups. Clinically important improvements in these endpoints were observed in patients responsive to bronchodilator at baseline and these improvements were enhanced further in those patients that also had serum periostin ≥ median at baseline. The maintenance dosing regimen of tralokinumab 300 mg Q4W was shown to be inadequate in this study.

**Summary of Safety**

The assessment of the overall safety data available from the study has not identified medically important risks associated with tralokinumab at either the 300 mg Q2W or 300 mg Q2/4W regimen. The frequencies of TEAEs were the same between the placebo (84.8%) and tralokinumab 300 mg Q2/4W cohort (84.8%) and slightly higher in the tralokinumab 300 mg Q2W cohort (89.3%). The majority of patients had TEAEs that were mild or moderate in severity and not related to investigational product. The rate of injection site TEAEs was higher in subjects in the Q2W tralokinumab cohort (23.3%) compared to patients receiving placebo Q2W (9.2%) but similar to patients receiving either tralokinumab Q2/4W (20.5%) or placebo Q2/4W (18.7%) and the majority of events were mild to moderate in severity and few patients discontinued investigational product as a result. The frequencies of TESAEs were similar between the placebo (13.9%) and tralokinumab 300 mg Q2W cohort (12.0%) and slightly higher in the tralokinumab 300 mg Q2/4W cohort (16.6%), with few patients having TESAEs related to investigational product. As expected in a study of this duration in a population of patients with severe asthma, there were a number of asthma exacerbations reported as TESAEs but the frequencies of these events were balanced between placebo (4.0%), tralokinumab 300 mg Q2W (6.0%), and tralokinumab 300 mg Q2/4W cohort (6.6%) with only 2 events in the Q2/4W cohort considered related to the product.

**PERIOSTIN**

Periostin levels were measured in baseline serum samples, i.e., prior to tralokinumab treatment, that were collected from patients randomised in the Phase 2b study. Key study endpoints including AER reduction, $FEV_1$, and ACQ-6 stratified by the median serum periostin level to determine if patients with baseline serum periostin levels at or above the median derive greater benefit from tralokinumab compared with those below the median. An increase in $FEV_1$ (6.75% for patients with baseline serum periostin at or above median vs 8.65% for patients regardless of serum periostin level) and greater AER reduction (25% for patients with serum periostin at or above median vs 7% for patients regardless of serum periostin level) with tralokinumab 300 mg Q2W were observed at Week 53. FIG. 14A and FIG. 14B. The median periostin level used in the study to define high periostin was a baseline serum periostin of ≥ 23 ng/mL (i.e., high periostin) as measured by the ARCHITECT platform from Abbott Diagnostics. For a continuous representation of AER reduction by periostin level and percent change from baseline in pre-bronchodilator $FEV_1$ by serum periostin level, see FIG. 13.

In post-hoc analysis, reversible patients (post-bronchodilator reversibility of $FEV_1 \geq 12\%$) with baseline serum periostin levels ≥ median had a greater increase in $FEV_1$ (13.85% for reversible patients with serum periostin at or above median vs 11.07% for reversible patients regardless of serum periostin level) and greater AER reduction (54% for reversible patients with serum periostin at or above median vs 34% for reversible patients regardless of serum periostin level; TABLES 21 and 22) were observed.

Serum periostin levels were substantially reduced by tralokinumab soon after the first dose and remained low for the duration of the study. Greater reduction in serum periostin levels was observed in those patients whose serum periostin levels at baseline were above the median compared to those below the median. These results provided further support for the hypothesis that serum periostin is a surrogate marker for the IL-13 pathway.

**Example 4**

**DPP4 as a Peripheral Asthma Biomarker**

To identify other potential novel peripheral biomarkers of IL-13 in asthmatics beyond periostin, experiments were conducted to identify a panel of genes upregulated in IL-13 stimulated cultures of bronchial cells from normal human subjects. FIGs.1-4. Within this IL-13-induced panel, normal and asthmatic serum samples were then interrogated to identify proteins with different levels in the serum of asthmatics and with plausible asthma biology. FIGs. 4-5. Elevated levels of dipeptidyl peptidase 4 (DPP4 [CD 26]) were observed in asthma serum samples compared to normal serum, similar to findings of Lun (Lun et al., J Clin Immunol. 2007; 430-37), who showed DPP4 elevations in plasma from asthma patients compared to control serum that correlated with other Th2 cytokines. In addition, they found increased membrane DPP4 expression on asthmatic CD4+ T cells.

In addition, it was observed that serum DPP4 levels were reduced in subjects taking oral and inhaled steroids (FIG. 6) and in subjects chronically treated with oral corticosteroids (see FIG. 24).

As a preliminary post-hoc analysis, we evaluated primary and secondary endpoints from the tralokinumab Phase 2B study (see Example 3) including AER reduction, FEV1, and ACQ-6 stratified by the median serum DPP4 level (FIGS. 18, 19, and 20) to determine if patients with baseline serum DPP4 levels at or above the median derive greater benefit from tralokinumab compared with those below the median. Serum DPP4 was measured as described in Example 2.

A statistically significant increase in percent change from baseline in pre-bronchodilator FEV1 (see FIG. 17A and FIG. 17B), change from baseline in mean ACQ-6 (see FIG. 17C and FIG. 17D), and change from baseline in mean AQLQ(S) (see FIG. 17E and FIG. 17F) in patients with serum DPP4 at or above median treated with tralokinumab (300 mg Q2W) were observed at Week 53 (TABLE 24). Additionally, unlike periostin, statistically significant changes in ACQ-6 and AQLQ were also observed for patients with serum DPP4 at or above median (TABLE 24). The evaluation of various DPP4 cut-points for ACQ-6, FEV1, and AER Reduction (FIGS. 18-20) supported use of the median value for analysis and showed that altering from the median would not result in significantly greater efficacy for these endpoints.

In post-hoc analysis, reversible patients (post-bronchodilator reversibility of FEV1 to a short-acting beta agonist ≥ 12%) with baseline serum DPP4 levels >= median saw increases in FEV1 (see FIG. 17G) and greater AER reduction. See also FIG. 17H, FIG. 17I, and FIG. 25.

DPP4 outperformed periostin in a variety of endpoints including: acute exacerbation rate reduction, percent change from baseline in pre-bronchodilator FEV1, change from baseline in mean ACQ-6, and change from baseline in mean AQLQ(S). FIGs. 15-16. These findings strongly support the utility of serum DPP4 (e.g. baseline serum DPP4 levels >= median) for identifying patients more likely to benefit from treatment with an IL-13 antagonist (e.g. an anti-IL13 antibody such as tralokinumab or lebrikizumab). These results also strongly support the utility of serum DPP4 (e.g.

baseline serum DPP4 levels >= median) to predict exacerbations, exacerbation rate, FEV1 response and asthma symptoms (e.g., night-time waking, symptoms on waking, activity limitation, shortness of breath, wheezing, and SABA use) in asthma patients.

Observations of greater reductions in AER in subgroups defined by both biomarker and clinical characteristics led to exploration of the population of subjects reversible at baseline not receiving OCS (n=33). In this group, a reduction in AER and significant improvements in FEV1, ACQ-6, and AQLQ(S) vs placebo were observed (TABLE 25). Evidence of further improvements in efficacy was observed in those subjects with elevated periostin or DPP4 at baseline (TABLE 25). *See also* FIG. 25 and FIG. 26 to compare DPP4 and periostin in reversible patients (post bronchodilator reversibility of FEV1 to a short-acting beta agonist ≥ 12%) with baseline serum DPP4 or Periostin levels >= median and not on chronic oral corticoid steroid treatment.

DPP4 can be combined with other markers/classifiers to identify patients more likely to benefit from treatment with an IL-13 antagonist (e.g. an anti-IL13 antibody such as tralokinumab or lebrikizumab), including, e.g., high periostin (Periostin-high), i.e., ≥ median serum periostin or about 23 ng/mL; high eosinophil cell count (Eos-high), i.e., blood eosinophil count ≥ 300 cells/μL; or high Th2 (th2-high), i.e., IgE > 100 IU/mL and blood eosinophils ≥ 0.14 × $10^9$/L. The partial overlap between the DPP4-high (DPP4 levels >= median) group and other groups, namely, periostin-high (periostin levels >=median) (FIG. 21), Th2-high (FIG. 22), and EOS-high (Eosinophil count >= 300) (FIG. 23) supports the notion that DPP4 can be used in combination with one or more of these biomarkers (e.g., Th2, periostin, and/or Eos) to identify patients more likely to benefit from treatment with an IL-13 antagonist (e.g. an anti-IL13 antibody such as tralokinumab or lebrikizumab).

TABLE 24: Summary of Primary and secondary efficacy endpoints for tralokinumab 300 mg Q2W ITT and subgroups (FEV1 reversibility, periostin and DPP4)

| | **ITT (N=150)** | **$FEV_1$ reversibility ≥12% (N=43)** | **Periostin ≥ Median (N=80)** | **DPP4 ≥ Median (N=77)** | **$FEV_1$ reversibility ≥12% (N=43) & Periostin ≥ Median (N=22)** |
|---|---|---|---|---|---|
| **Primary endpoint** | | | | | |
| Asthma exacerbation rate reduction[a] (95% CI) | 7% (-30%, 33%) *P*=0.669 | 34% (-32%, 67%) *P*=0.245 | 25% (-19%, 53%) *P*=0.219 | 34% (-6%, 59%) *P*=0.083 | 54% (-65%, 87%) P=0.236 |
| **Secondary endpoints (difference from placebo)** | | | | | |
| Percent change from baseline in $FEV_1$ (95% CI) | 7.1 (2.35, 11.84) *P*=0.003 | 11.1 (0.99, 21.14) *P*=0.031 | 6.8 (-0.31, 13.82) *P*=0.061 | 10.8 (3.27, 18.23) *P*=0.005 | 13.8 (-0.18, 27.87) P=0.053 |
| Change from baseline in ACQ-6 (95% CI) | -0.18 (-0.43, 0.06) *P*=0.137 | -0.44 (-0.89, 0.01) *P*=0.055 | -0.23 (-0.56, 0.09) *P*=0.163 | -0.50 (-0.86, -0.14) *P*=0.007 | -0.34 (-0.93, 0.25) P=0.260 |
| Change from baseline in AQLQ (95% CI) | 0.21 ( -0.05, 0.46) *P*=0.114 | 0.59 (0.10, 1.09) *P*=0.020 | 0.22 (-0.15, 0.59) P=0.245 | 0.69 (0.30, 1.08) *P*<0.001 | 0.58 (-0.11, 1.26) P=0.097 |

Abbreviations: CI, confidence interval; $FEV_1$, forced expiratory volume at 1 second; ITT, intent-to-treat. ACQ-6, asthma control questionnaire, AQLQ, asthma quality of life questionnaire

[a]Asthma exacerbation rate reductions were calculated using Poisson regression model adjusted for over dispersion with treatment group, age, gender, number of asthma exacerbations in the past year, atopic asthma status, presence or absence of chronic OCS use and geographical region as covariates and the log of number of days in the study as offset

N is in each base the number of subjects in the 300 mg Q2W group

TABLE 25: AER reduction, FEV1, ACQ-6, and AQLQ(S) for tralokinumab Q2W in subjects reversible at baseline and not receiving chronic OCS vs placebo (post hoc exploratory analyses)

| Parameter vs placebo | Tralokinumab 300 mg (Q2W) reversible without OCS use | | | | |
|---|---|---|---|---|---|
| | (n=33) | Periostin-high (n=18) | Periostin-low (n=15) | DPP4-high (n=24) | DPP4-low (n=8) |
| AER reduction, % (95% CI)<br>P-value | 44<br>(-22, 74)<br>0.147 | 67<br>(2,89)<br>0.046 | -32<br>(-273, 53)<br>0.597 | 57<br>(-30,86)<br>0.134 | -7<br>(-886,88)<br>0.950 |
| FEV1 % change from baseline (95% CI)<br>P-value | 12<br>(1.5,22.5)<br>0.025 | 14.7<br>(-0.2, 29.5)<br>0.054 | 8.0<br>(-7.0, 23.0)<br>0.294 | 20.3<br>(1.2,39.5)<br>0.038 | -0.9<br>(-15.4, 13.5)<br>0.897 |
| ACQ-6 change from baseline (95% CI)<br>P-value | -0.55<br>(-1.07, -0.04)<br>0.036 | -0.68<br>(-1.31, -0.06)<br>0.033 | -0.23<br>(-1.10, 0.64)<br>0.596 | -0.89<br>(-1.63, -0.14)<br>0.020 | -0.43<br>(-1.41, 0.56)<br>0.390 |
| AQLQ(S) change from baseline (95% CI)<br>P-value | 0.70<br>(0.12, 1.28)<br>0.019 | 0.64<br>(-0.11, 1.39)<br>0.095 | 0.71<br>(-0.23, 1.65)<br>0.138 | 1.26<br>(0.48, 2.04)<br>0.002 | 0.24<br>(-0.87, 1.35)<br>0.663 |

In conclusion, this double-blind phase 2b study enrolled adults with severe asthma, post-bronchodilator forced expiratory volume in 1 second (FEV1) reversibility ≥12% and ≥200 mL within 3 years/at screening and ≥2 asthma exacerbations in the previous year. Subjects received fluticasone/salmeterol 500 μg/50μg bid (or equivalent) and continued pre-study controller medications. Following 5-week run-in, subjects with FEV1 40-80% predicted or Asthma Control Questionnaire 6 (ACQ-6) score ≥1.5 were randomized to tralokinumab 300 mg/placebo (2:1) every 2 weeks (Q2W) or tralokinumab 300 mg/placebo (2:1) Q2W for 12 weeks followed by every 4 weeks (Q4W). The primary endpoint was asthma exacerbation rate (AER) over 52 weeks. Secondary endpoints included FEV1, ACQ-6, Asthma Quality of Life Questionnaire (AQLQ), and safety. The trial was powered to detect a 40% reduction in AER for each tralokinumab group (Q2W or Q4W) vs. combined placebo groups with 80% power and significance level 0.15. Subjects with baseline FEV1 reversibility ≥12 % defined a "reversible" subgroup. Baseline levels of serum DPP4 and periostin, genes whose expression is highly induced by IL-13, were assessed as potential surrogate biomarkers with subgroups defined by median levels.

Analyses were based on intent-to-treat population (ITT, N=452). Baseline characteristics, mean (SD): age: 50.2 (12.3); ACQ-6: 2.55 (0.97); FEV1 % predicted: 68.6 (18.1). AER at week 53 was similar in both tralokinumab groups vs. placebo. Trends towards AER reduction in Q2W were observed in reversible, periostin-high, and DPP4-high subgroups (TABLE 24). Reversible and periostin-high subgroup AER reductions were 54% (-65, 87%), and when excluding subjects receiving oral corticosteroid, 67% (2, 89%). At week 53, a statistically significant increase in pre-bronchodilator FEV1 was observed for Q2W and increases were evident in all subgroups (TABLE 24). ACQ-6 and AQLQ were significantly different from placebo in the reversible and DPP4-high subgroups for Q2W (TABLE 24). No significant differences vs. placebo were observed for secondary endpoints in Q4W group or subgroups. Frequencies of treatment emergent serious adverse events/adverse events were similar within the safety population (tralokinumab Q2W: 12.0/89.3%; Q4W: 16.6/84.8%; placebo: 13.9/84.8%).

## Example 5

### Identification of Peripheral Markers of IL-13 Activation in Atopic Dermatitis

To examine whether periostin and/or DPP4 are also up-regulated in the human skin of patients suffering from atopic dermatitis, transcriptional alterations in four atopic dermatitis skin samples and 31 normal skin samples were analyzed using whole genome microarray. Briefly, biotin-labeled amplified cRNA was generated from total RNA using cDNA Synthesis and IVT Labeling kits and fragmented for hybridization on Affymetrix Human Genome U133 Plus 2.0 GeneChip® arrays. Data capture and quality assessments were performed with the GeneChip Operating Software tool. The R statistical analysis tool was used to calculate probe-level summaries (frma) from the array CEL files. Expression intensity data (linear) from whole genome array analysis showed that mRNA expression of periostin (FIG. 27) and DPP4 (FIG. 28) were elevated in atopic dermatitis skin compared to normal skin.

The finding that DPP4 and periostin expression levels are increased in the skin of atopic dermatitis patients indicates that DPP4 and/or periostin gene expression levels: (1) could be used as a peripheral markers of IL-13 pathway activation in atopic dermatitis patients; (2) could be informative in electing potential therapies for atopic dermatitis patients, and (3) could be useful in selecting patients responsive to therapy using an IL-13 antagonist, for example, an anti-IL-13 antibody such as tralokinumab or lebrikizumab.

The results obtained using skin samples from atopic dermatitis patients suggest that expression levels (e.g., gene expression and/or protein expression) of DPP4 and/or periostin in serum can also be used as biomarkers in atopic dermatitis. Protein levels of DPP4 and periostin were also elevated in serum of atopic dermatitis patients (See Example 8). Accordingly, these findings that serum and skin DPP4 and/or periostin protein levels are increased in atopic dermatitis suggest that DPP4 and/or periostin levels: (1) could be used as a peripheral marker of IL-13 pathway activation in atopic dermatitis patients; (2) could be informative in electing potential therapies for atopic dermatitis patients, and (3) could be useful in selecting patients responsive to therapy using an IL-13 antagonist, for example, an anti-IL-13 antibody such as tralokinumab or lebrikizumab.

## Example 6

### CAT-354-1049 Computed Tomography (CT) Image Data Analysis: 3D Airway Analysis

As discussed herein, there is a need to identify patients who would benefit from therapeutic intervention with an IL-13 antagonist and predict the outcome of the treatment with IL-13 antagonists such as anti-IL-13 antibodies. This is achieved using biochemical biomarkers such as DPP4, periostin and/or clinical characteristics such as FEV1 reversibility, or combinations thereof, as described above in Examples 3-5. Another approach is using Computed Tomography (CT) imaging data as high-performance CT scanners are available at most hospitals, and standardized image analysis can be obtained as a service. A change in airway dimensions, and in particular airway resistance that can be estimated from the subsegmental airway dimensions, should relate to the improvements in lung function, and be a more objective measure than standard lung function tests such as FEV1.

Other groups have studied the change in dimensions for large airways (RB1) as one of the indicators of treatment effect (*see, e.g.,* Haldar, et al 2009). Here, we investigated the treatment effect following Tralokinumab administration as measured by changes in subsegmental airway dimensions from baseline, as quantifying the dimensions of more peripheral airways, such as the subsegmental bronchi, should have a greater potential to reflect efficacy since these airways are not as rigid as the larger airways, including RB1.

Computed tomography (CT) imaging data of lung scans obtained from patients enrolled in the CAT-354-1049 clinical trial (described in Example 3) were analyzed using VIDA APOLLO® software (version 1.2.001_Investigator; VIDA Diagnostics, Inc., Coralville, IA) using methods well known in the art. *See, e.g.,* Gupta et al., J Allergy Clin Immunol. 133(3):729-738 (2014). In the clinical trial, CT scanning was used to determine the effects of tralokinumab administration on airway wall structural change. The image data was obtained using spiral/helical MSCT (multislice computed tomography) imaging. CT scanners from GE Healthcare, Philips and Siemens were used in the multicenter trial. Imaging and reconstruction parameters were standardized with tube voltage 120kVp, slice thickness<=1.0mm, recon kernel Standard (GE), B (Philips) and B30f (Siemens), respectively. All images were acquired at full inspiration (TLC). In this study, only the upper part of the lung was imaged, allowing the analysis of the segmental and sub-segmental airways in the upper lobes. The VIDA APOLLO® software allowed the 3D analysis of parameters related to airway dimensions, in particular, bronchial tubes. As used herein, the term "bronchial tube" means a bronchus or any of its branches, including bronchia and bronchioles. The parameters measured for each bronchial tube (or segment) were average cross-sectional lumen area (LA), wall area (WA), wall area percentage (WA%), and wall thickness (WT). The term "lumen" refers to the inner open space or cavity of a bronchial tube. The term "wall area" refers to the cross-sectional area of a bronchial tube wall. Wall area percentage was calculated as follows: 100*wall area/(wall area + lumen area). Measurements were performed in all imaged segmental and subsegmental bronchi in the upper lobes. Segmental airways (up to five in each subject) were right apical (RBI), right anterior (RB2), right posterior (RB3); left apicoposterior (LB1+2), and left anterior (LB3). Subsegmental airways (up to 14 in each subject) were RB1a & b, RB2a & b, RB3a & b, LB1, LB1a* & b*, LB2, LB2a* & b*, LB3a & b, with the areas labeled with an asterisk being sub-subsegmental airways. LB1 and LB2 could alternatively be named LB1+2a and LB1+2b, respectively. The corresponding sub-subsegmental airways (labeled with an asterisk above) can alternatively be named LB1+2ai, LB1+2aii, LB1+2bi, LB1+2bi, respectively. *See e.g.* Naidich, et al, Imaging of the Airways - Functional and Radiologic Correlations, 2005.

The baseline measurements disclosed herein were consistent with the baseline measurements observed in other CT studies in asthma, including Gupta et al., J Allergy Clin Immunol. 133(3): 729-738 (2014).

Changes in the airway parameters described above were calculated for each airway segment separately, and then averaged over segmental and subsegmental airways in each subject. Calculations of airway resistance and averages of cross-sectional airways were performed in Matlab (Matlab R2010a (MathWorks, Natick, MA)). Only relative changes between baseline (visit 4) (see FIG. 29, panel A) and follow up (visit 30) (see FIG. 29, panel B) were calculated. Group differences were calculated only between total tralokinumab (i.e. 300 mg Q2 + 300 mgQ2/4W cohorts) and total placebo.

The analysis dataset used all subjects that had CT scans at both baseline, i.e., visit 4, and follow-up, i.e., visit 30. The most severe CT protocol deviations were excluded (i.e., change in image slice thickness or reconstruction kernel between visits 4 and 30).

Airway Resistance was calculated assuming laminar airflow and airway segments that were substantially longer than their diameter. Thus, airway resistance was theoretically calculated as:

$$R = \frac{8\mu l V}{\pi r^4}$$

where r is the radius of the airway ($\mu$=viscosity, l=length, V=flow rate). Since area is approximately $r^2$, relative change in resistance can consequently be estimated as:

$$\frac{R_2 - R_1}{R_1} = \frac{1/LA_2^2 - 1/LA_1^2}{1/LA_1^2}$$

Relative change in airway resistance was calculated for each airway segment prior to averaging across several bronchi.

The relative changes in Luman Area (LA) from baseline to visit 30 are shown in TABLE 26 and FIG. 30.

TABLE 26: Relative change in Lumen Area (LA) from baseline to visit 30

| | Total Placebo, n=12 Mean (S.D.) | Total Tralokinumab, n=14 Mean (S.D.) | Group difference | P value |
|---|---|---|---|---|
| RB1 | +4.15% (19.06%) | +9.54% (24.58%) | +5.39% | 0.54 |
| Segmental | +3.25% (10.41%) | +11.16% (19.20%) | +7.90% | 0.22 |
| Subsegmental | +0.28% (13.24%) | +16.77% (19.52%) | +16.49% | 0.021 |
| All | +1.12% (10.52%) | +15.34% (18.36%) | +14.22% | 0.026 |

Relative change in LA should not be affected by normalization with body surface area (BSA) at baseline, i.e.

$$\frac{LA_2/BSA - LA_1/BSA}{LA_1/BSA} = \frac{LA_2 - LA_1}{LA_1}$$

The relative changes in Wall Area (WA) from baseline to visit 30 are shown in TABLE 27.

TABLE 27: Relative change in Wall Area (WA) from baseline to visit 30

| | Total Placebo, n=12 Mean (S.D.) | Total Tralokinumab, n=14 Mean (S.D.) | Group difference | P value |
|---|---|---|---|---|
| RB1 | +5.17% (20.06%) | +17.17% (32.69%) | +11.99% | 0.28 |
| Segmental | +8.59% (12.02%) | +12.18% (16.67%) | +3.59% | 0.54 |
| Subsegmental | +1.89% (10.04%) | +10.29% (14.40%) | +8.40% | 0.10 |
| All | +4.02% (9.19%) | +11.19% (12.79%) | +7.17% | 0.12 |

Relative change in WA should not be affected by normalization with body surface area (BSA) at baseline , i.e.

$$\frac{WA_2/BSA - WA_1/BSA}{WA_1/BSA} = \frac{WA_2 - WA_1}{WA_1}$$

The relative changes in Wall Area Percentage (WA%) from baseline to visit 30 are shown in TABLE 28 and FIG. 31.

TABLE 28: Relative change in (Wall Area Percentage) WA% from baseline to visit 30

| | Total Placebo, n=12 Mean (S.D.) | Total Tralokinumab, n=14 Mean (S.D.) | Group difference | P value |
|---|---|---|---|---|
| RB1 | +0.43% (4.67%) | +2.27% (6.34%) | +1.83% | 0.42 |
| Segmental | +1.96% (2.38%) | +0.39% (3.10%) | -1.57% | 0.17 |
| Sub segmental | +0.62% (1.82%) | -1.63% (1.87%) | -2.25% | 0.0049 |
| All | +1.08% (1.48%) | -1.01% (1.82%) | -2.09% | 0.0040 |

The relative changes in Wall Thickness (WT) from baseline to visit 30 are shown in TABLE 29.

TABLE 29: Relative change in Wall Thickness (WT) from baseline to visit 30

| | Total Placebo, n=12 Mean (S.D.) | Total Tralokinumab, n=14 Mean (S.D.) | Group difference | P value |
|---|---|---|---|---|
| RB1 | +2.70% (11.29%) | +9.71% (18.27%) | +7.00% | 0.26 |
| Segmental | +6.32% (8.21%) | +5.38% (7.72%) | -0.94% | 0.77 |
| Sub segmental | +1.99% (5.81%) | +3.41% (9.31%) | +1.41% | 0.65 |
| All | +3.40% (5.58%) | +4.22% (7.00%) | +0.81% | 0.75 |

The relative changes in Airway Resistance from baseline to visit 30 are shown in TABLE 30 and FIG. 32. The dataset for tralokinumab (dashed box in FIG. 32) was split into two sub-groups according to WA% at baseline, for subsequent analysis of relative improvement in sub-segmental airway resistance and FEV1%. A median cut-off based on WA% was used, resulting in a cut-off level of 68% (see FIG. 33).

TABLE 30: Relative change in Airway Resistance from baseline to visit 30

| | Total Placebo, n=12 Mean (S.D.) | Total Tralokinumab, n=14 Mean (S.D.) | Group difference | P value |
|---|---|---|---|---|
| RB1 | -0.06% (31.89%) | -6.47% (35.06%) | -6.42% | 0.63 |
| Segmental | +1.51% (19.45%) | -5.80% (30.76%) | -7.31 % | 0.48 |
| Subsegmental | +14.09% (24.87%) | -12.56% (22.16%) | -26.65% | 0.0081 |
| All | +10.42% (20.20%) | -10.68% (22.37%) | -21.10% | 0.019 |

**Conclusions:**

A number of conclusions can be made from these studies including:

(i) 3D measurements of WA% were more consistent with published data, and with less variability than 2D measurements;

(ii) 2D and 3D measurements in the right apical segmental bronchus (RB1) were relatively consistent for lumen area, but more variable for wall measurements;

(iii) Averaging relative change in each parameter over multiple airways in the 3D analysis reduced the variability;

(iv) Tralokinumab had a greater effect on LA and WA% in smaller (subsegmental) airways than in segmental bronchi;

(v) Lumen area, wall area percentage, airway resistance in subsegmental bronchi were significantly improved with tralokinumab compared to placebo ($p=0.021$, $p<0.005$ and $p<0.01$ respectively);

(vi) No significant treatment effects were seen in wall area and wall thickness;

(vii) The effect of Tralokinumab treatment on airway resistance was significantly higher ($p=0.037$) in the half of the tralokinumab group with the highest wall area percentage (WA% of subsegmental airways higher than the 68% median cut-off) at baseline compared to the half with the lowest wall area percentage (WA% of subsegmental airways lower than the 68% median cut-off). See FIG. 33. Indeed, patients with WA% above the specified threshold (e.g., WA% at least 68% at subsegmental level) display a statistically significant improvement in airway resistance and display a statistically significant improvement in pre-bronchodilator FEV1. See FIG. 33.

Taken together, these studies suggest that Wall Area % as determined using a CT scan of the lungs of subsegmental airways (WA%) can be used to predict treatment response (for example, improvements in airway resistance and/or FEV1) in patients treated or candidates for treatment with an IL-13 antagonist (for example an anti-IL-13 antibody such as tralokinumab or lebrikizumab). Moreover, these studies suggest that patients suffering from an IL-13-mediated disease (e.g., asthma, COPD, IPF, UC, or atopic dermatitis) having a WA% value above a predetermined WA% threshold level or above the WA% in one or more control samples (e.g., a WA% threshold level of about 68%, above about 60% or between 60%-80% of subsegmental airways) prior to treatment are good candidates for treatment with an IL-13 antagonist (for example an anti-IL-13 antibody such as tralokinumab or lebrikizumab). In addition, wall area percentage (WA%) can be combined with other measurements obtained using 3D airway analysis of CT scan data for example lumen area (LA), wall area (WA), wall thickness area (WT), airway resistance, or combinations thereof to identify populations of patients amenable for treatment with an IL-13 antagonist (for example an anti-IL-13 antibody such as tralokinumab or lebrikizumab).

Wall area percentage (WA%) at baseline describes how constricted/thickened the airways are and consequently the potential improvement that can be achieved with treatment with an IL-13 antagonist such as tralokinumab or lebrikizumab. Since WA% is computed as a ratio, it is automatically normalized with the airway dimensions for an individual patient, making this parameter a logical choice for baseline characterization.

In addition to the use in severe asthma, this method would be applicable in other pulmonary diseases, including but not limited to, COPD, emphysema, and IPF.

**Example 7**

**Induction of Periostin and DPP4 Expression in Airway Epithelium from COPDSubjects**

Airway inflammation within COPD is heterogeneous and modulated by a variety of inflammatory mediators including IL-17, IL-33 and IL-13. Differentiated normal and COPD -bronchial epithelial cells at (EPIAIRWAY™ tissue) air-liquid interfaces were procured from MATTEK (MA,TTEK Corporation, MA) and cultured for 24 hours at 37°C in 5% $CO_2$-rich incubator. The tissues were then rinsed twice with PBS and cultured in medium devoid of serum or steroids for an additional 24 hours. Following this period the cells were stimulated with 25ng/mL of IL-13, IL-17A, IL-17F, IL-17E, IL-13, TSLP or IL-33 (PeproTech, NJ) for an additional 24h. Total RNA was then extracted using mirVana Isolation protocols (Life Technologies, MD), reverse transcribed by The SuperScript® III First-Strand Synthesis System (Life Technologies, MD) and quantified by $TA_Q$MAN® gene expression PCR assays (Life Technologies, MD).

IL-13 specific up-regulation of CCL-26, DPP4, periostin POSTN-745, and periostin POST-815 was observed in transcripts obtained from highly differentiated bronchial epithelial cells from normal subjects and COPD subjects. The bronchial epithelial cells were grown at air liquid interfaces (EPIAIRWAY™ model). See FIG. 34. The data presented in FIG. 34 represent log2 fold change (fc) in CCL-26, DPP4, periostin POSTN-745, and periostin POST-815 gene transcripts, relative to basal/untreated basal condition after stimulation with 25ng/mL of IL-17A, IL-17F, IL-17E, IL-13, TSLP or IL-33 for 24 hours as indicated above. These findings indicate that periostin and DPP4 are specific markers for IL-13 mediated COPD. This experimental data, corresponding to differentiated airway epithelial cells, shows that IL-13 mediated inflammation can be distinguished from other phenotypes by specific expression of CCL-26, DPP4 and periostin. The data also shows that IL-13 (but not IL-17A/F/E, TSLP, or IL-33) significantly induce periostin and DPP4 expression in airway epithelium from COPD subjects. The preservation of these outcomes across normal and diseased epithelium, indicates that induced periostin and DPP4 can be used as biomarkers for identifying COPD patients affected by IL-13 mediated airway inflammation.

### Example 8

### Expression of Periostin and DPP4 in Atopic Dermatitis Patients

**Subject selection:** Atopic dermatitis patients provided serum samples and clinical characteristics with informed consent. Samples were accessed and selected anonymously corresponding to 100 patients with moderate atopic dermatitis and 100 patients with severe atopic dermatitis. In order to balance between the moderate and severe comparison groups, available patient samples were matched with respect to gender and age.

**DPP4 Quantification:** DPP4 was quantified using the Human DPPIV/CD26 Quantikine ELISA Kit. The Reference Standard Stock Solution (RS) was the Human DPPIV Standard from the kit. The QC Sample Stock Solution (QCS) was Human DPPIV Standard from the kit. Quality Controls (QCs) were prepared the day prior to the start of an assay. At least 2 Reference Standard (DPPIV Standard, Part 892953) (RS) vials provided in the kits were reconstituted. Each RS was reconstituted with 1000μL of diH2O as per product insert. The reconstituted concentration produced a stock solution of 200ng/mL. Test Samples were prepared at 1:70 MRD. Samples were thawed at room temperature and diluted with Calibrator Diluent RD5-33. To prepare Reference Standard Stock Solution (RS), RS was reconstituted with 1000 μL of diH2O. The reconstituted concentration produced a stock solution of 200 ng/mL. Reference Standards were prepared starting with Reference Standard Stock (RS). Quality Control (QC) Samples were prepared starting with frozen QC Stock (QCS). 50 μL of prepared Reference Standards, QCs, and diluted Test Samples were pipetted into appropriate wells. The plate(s) were sealed and incubated for 2 hour ± 15 minutes at room temperature with shaking at approximately 450 rpm on an orbital plate shaker. The plate(s) were washed four times using a plate washer. After the wash, any remaining Wash Buffer was removed by blotting against clean paper towels. 200 μL of DPPIV Conjugate were added to each well. The plate(s) were sealed and incubated for 2 hour ± 15 minutes at room temperature with shaking at approximately 450 rpm on an orbital plate shaker. The plate(s) were washed again four times using a plate washer. Substrate Reagent was prepared by adding equal volume of Substrate A and B prior to adding it to the wells. 200 μL of Substrate Reagent were added to each well. The plate(s) was sealed and incubated for 30 minutes ± 5 min at room temperature with shaking at approximately 450 rpm on an orbital plate shaker. Samples were protect from light. 50 μL of Stop Solution were added, and absorbance in each well was measured at 450 nm using a spectrophotometric microplate reader. Wells were read within 30 minutes of adding the Stop Solution. Data was analyzed using a 4-PL non-linear fit (SoftMax ProGxP v5.2). Blank values were not subtracted from Standard Curve values when back-calculating to the concentrations. For acceptance of an assay, Standard Curve and Quality Control replicates on the assay plate had to pass the acceptance criteria of 100 ± 30% recovery and ≤ 25% CV. Test sample replicates on the assay plate had to pass the acceptance criteria of ≤ 25% CV.

**Periostin Quantification:** An MSD assay plate (MSD L15XA) (MSD, Gaithersburg, MD) was coated with an anti-human Periostin antibody (MedImmune, clone# 4B4.B11, as disclosed in U.S. Provisional Patent Application No. 61/936,967, herein incorporated by reference, and deposited with the at the American Type Culture Collection, Manassas, VA (the ATCC) under Deposit No. PTA-120210 on April 17, 2013). The Capture Antibody in IX PBS (Lonza, Catalog # 17-516Q or equivalent) to a final concentration of 2 μg/ml. 50 μl/well of 2 μg/ml Capture Antibody was added to each well, and the plate was covered with an adhesive microplate sealer. The plate was incubated overnight at 2°C to 8°C and subsequently washed with ELISA Wash Buffer.

The coated assay plate was washed three times with IX ELISA Wash Buffer (0.05% Tween-20, IX PBS) and blocked with 150 μl/well I-Block Buffer (IBB) (I-Block Buffer: 0.5% Tween-20, IX PBS, 0.2% I-Block Buffer) (Tropix I-BlockTM, Applied Biosystems, Cat# T2015) for a minimum of one hour at room temperature (RT) with gentle shaking (for ≥ 60 minutes but no more than 4 hours).

Recombinant human Periostin (R&D Systems, Catalog # 3548-F2) was used as a standard. Reference standards (RS), quality controls (QC) and negative control (NC) prepared in IBB, and serum test samples diluted to the Minimum Required Dilution of 1:10 in IBB, were added to the plate and incubated for approximately one hour at RT on a plate shaker with gentle shaking. Unbound analyte was removed by washing the plate with ELISA Wash Buffer. To detect bound analyte, Ruthenylated-anti-human Periostin (Ru-7B5, conjugate antibody clone# 7B5.C4, MedImmune, as disclosed in US Provisional Patent Application No. 61/936,967, herein incorporated by reference, and deposited with the at the American Type Culture Collection, Manassas, VA (the ATCC) under Deposit No. PTA-120211 on April 17, 2013) was prepared to a final concentration of 2 μg/ml, and after washing each well with 200 μl/well of IX ELISA Wash Buffer, 30 μl/well of the Detection Antibody was added to each wells and the plate was incubated for approximately one hour (60 minutes ± 10 minutes) on a plate shaker with gentle shaking at RT (protected from light exposure). Unbound detection antibody was removed by washing the plate with ELISA Wash Buffer.

Read Buffer (MSD) was prepared by the diluting "4X Read Buffer T" (4X, MSD, Cat # R92TC-1) stock to "IX" using distilled water. Read Buffer was added to the plate and the plate was read on an MSD Plate Reader. Raw data, in Electrochemiluminescence Units (ECLU), was transferred to the SoftMax Pro software (SoftMax® Pro v5.2 GxP) and to an Excel spreadsheet for further analysis. The reference standard curve for each assay was plotted using the 4-parameter logistic weighted (1/y^2) curve fit method. Periostin concentrations were interpolated for each QC level, NC and for Serum Test Samples from the fitted curve.

**Results and Conclusions:** Periostin was expressed in the sera of severe atopic dermatitis patients at higher levels when compared to moderate atopic dermatitis patients. See FIG. 35, panel A. DPP4 expression was also elevated in the sera of atopic dermatitis patients, but expression levels were not related to disease severity. See FIG. 35, panel B. The finding that DPP4 and periostin expression levels are increased in the serum of severe and moderate atopic dermatitis patients indicates that DPP4: (1) could be used as a peripheral biomarker of IL-13 pathway activation in atopic dermatitis patients; (2) could be informative in electing potential therapies for atopic dermatitis patients, and (3) could be useful in selecting patients responsive to therapy using an IL-13 antagonist, for example, an anti-IL-13 antibody such as tralokinumab or lebrikizumab.

**Example 9**

**Periostin and serum DPP4 in stable COPD and acute exacerbations of COPD**

To determine whether there are differences in the expression levels of periostin and/or DPP4 in acute exacerbations of COPD (AECOPD) with respect to the expression levels observed in stable COPD, levels of periostin (FIG. 36) and serum DPP4 (FIG. 37) were measured in healthy controls, stable COPD patients, and patients experiencing acute exacerbations of COPD. Healthy control sera were obtained from Bioreclamation (Baltimore MD, USA) from 20 nonsmoking subjects (10 females and 10 males, ages 17 to 59). COPD and AECOPD sera were from the MI-CP221 clinical biomarker study, sponsored by MedImmune. Periostin and DPP4 levels were measured by immunoassay, as described above. Data showed that periostin and serum DPP4 were increased in stable COPD and also in AECOPD relative to healthy controls. This indicated that DPP4 and/or periostin can be used as biomarkers for both stable COPD and AECOPD and could be useful in selecting COPD patients responsive to therapy using an IL-13 antagonist, for example, an anti-IL-13 antibody such as tralokinumab or lebrikizumab.

It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s), and thus, are not intended to limit the present invention and the appended claims in any way.

The present invention has been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described exemplary embodiments, but should be defined only in accordance with the following claims and their equivalents.

All publications, patents, patent applications, and/or other documents cited in this application are incorporated by reference in their entirety for all purposes to the same extent as if each individual publication, patent, patent application, and/or other document were individually indicated to be incorporated by reference for all purposes.

The following numbered clauses, describing aspects of the invention, are part of the description.

1. A method of treating a patient having an interleukin-13 (IL-13)-mediated disease or disorder, comprising administering an IL-13 antagonist to the patient if the level of DPP4 (dipeptidyl peptidase-4) in one or more samples taken from the patient is above a predetermined DPP4 threshold level, or is above the DPP4 level in one or more control samples.

2. A method of treating a patient having an interleukin-13 (IL-13)-mediated disease or disorder, comprising administering an IL-13 antagonist to the patient if (a) the level of DPP4 (dipeptidyl peptidase-4) in one or more samples taken from the patient is above a predetermined DPP4 threshold level, or is above the DPP4 level in one or more control samples, and optionally if (b) the patient presents (i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ 0.14 × $10^9$/L), (iv) FEV1 reversibility to a short-acting β2 agonist ≥ 12%, (v) wall area percentage (WA%) of subsegmental airways from a CT scan of the lungs ≥ 68%, or (vi) combinations thereof.

3. The method according to clause 1, wherein the patient's DPP4 level is measured in an immunoassay.

4. The method according to clause 3, wherein the immunoassay employs one or more anti-DPP4 antibodies or antigen binding fragments thereof which recognize human DDP4.

5. The method according to clause 1, wherein the IL-13 antagonist comprises one or more of an anti-IL-13 antibody or antigen-binding fragment thereof, an IL-13 mutein, and IL-4 mutein, an anti-IL-13Rα1 antibody or antigen-binding fragment thereof, or an anti-IL-4Rα antibody or antigen-binding fragment thereof.

6. The method according to clause 1, wherein the patient has been treated with one or more additional medications, either before, during, or after administration of an IL-13 antagonist.

7. The method according to clause 6, wherein the one or more additional medications comprises a steroid, and optionally comprises a bronchodilator.

8. The method according to clause 7, wherein the steroid is fluticasone or budesonide, and the bronchodilator is salbutamol or salmeterol.

9. The method according to clause 6, wherein the one or more additional medications are administered by inhalation, by oral administration, by injection, or by a combination thereof.

10. The method according to clause 9, wherein inhalation administration is conducted using a metered dose inhaler (MDI) or a dry powder inhaler (DPI).

11. The method according to clause 7, wherein the steroid is administered at a high dose.

12. The method according to clause 1, wherein the IL-13 antagonist is an anti-IL13 antibody, or antigen-binding fragment thereof, wherein:

(i) the antibody or antigen-binding fragment thereof binds to the same IL-13 epitope as tralokinumab (VH: SEQ ID NO:3; VL:SEQ ID NO:4) or competitively inhibits binding of tralokinumab to IL-13, or both;
(ii) the antibody or antigen-binding fragment thereof comprises tralokinumab (VH: SEQ ID NO:3; VL:SEQ ID NO:4) or an antigen-binding fragment thereof;
(iii) the antibody or antigen-binding fragment thereof consists of tralokinumab (VH: SEQ ID NO:3; VL:SEQ ID NO:4) or an antigen-binding fragment thereof;
(iv) the antibody or antigen-binding fragment thereof binds to the same IL-13 epitope as lebrikizumab (VH: SEQ ID NO:1; VL:SEQ ID:2) or competitively inhibits binding of lebrikizumab to IL-13, or both;
(v) the antibody or antigen-binding fragment thereof comprises lebrikizumab (VH: SEQ ID NO:1; VL:SEQ ID:2) or an antigen-binding fragment thereof;
(vi) the antibody or antigen-binding fragment thereof consists of lebrikizumab (VH: SEQ ID NO:1; VL:SEQ ID:2) or an antigen-binding fragment thereof; or
(vii) the antibody or antigen-binding fragment thereof comprises SEQ ID NO:3, SEQ ID NO:4, or an antigen-binding fragment thereof.

13. The method according to clause 1, wherein the one or more samples taken from the patient and/or the one or more control samples comprises one or more of whole blood, blood serum, plasma, saliva, sputum, bronchoalveolar lavage fluid, lung epithelial cells, urine, skin, nasal polyps, or a combination thereof.

14. The method according to clause 1, wherein the IL-13 antagonist is administered at a fixed dose.

15. The method according to clause 12, wherein the anti-IL13 antibody is tralokinumab, and wherein tralokinumab is administered at a fixed dose of about 300 mg/dose.

16. The method according to clause 1, wherein the IL-13 antagonist is administered in two or more doses.

17. The method according to clause 1, wherein the IL-13 antagonist is administered week, biweekly or monthly.

18. The method according to clause 1, wherein the IL-13 antagonist is administered intravenously, intramuscularly, subcutaneously, or a combination thereof.

19. The method according to clause 1, wherein the predetermined DPP4 threshold level is at least about 250 ng/ml, at least about 350 ng/mL, at least about 375 ng/mL, at least about 400 ng/mL, at least about 450 ng/mL, at least about 500 ng/mL, at least 550 ng/mL, or at least about 600 ng/mL, as measured in serum using an ELISA QUANTIKINE® assay.

20. The method according to clause 1, wherein the predetermined DPP4 threshold level is about 365 ng/mL.

21. The method according to clause 1, wherein the one or more control samples are (i) a sample or samples obtained from normal healthy individuals; (ii) a sample or samples obtained from patients with a non-IL-13-mediated subset of asthma; (iii) a sample or samples obtained from asthma patients naive for corticosteroid treatment; (iv) a sample or samples obtained from asthma patients treated with corticosteroids; (v) a sample or samples obtained from untreated atopic dermatitis patients; (vi) a sample or samples obtained from treated atopic dermatitis patients; (vii) a pre-determined standard amount of isolated DPP4; or (viii) a combination thereof.

22. The method according to clause 1, wherein administration of the IL-13 antagonist results in:

(a) AER (Acute Exacerbation Rate) reduction, wherein the AER reduction is at least 5%, at least 10%, at least 15%, at least 20%, at least 25%, at least 30%, at least 35%, at least 40%, or at least 45% compared to the AER observed in a population of patients treated with a placebo;
(b) $FEV_1$ (Forced Expiratory Volume in one second) increase, wherein the $FEV_1$ increase is at least 3%, at least 5%, at least 7%, at least 9%, at least 11%, at least 13%, at least 15%, least 17%, or at least 19% compared to the $FEV_1$ observed in a population of patients treated with a placebo;
(c) improved ACQ-6 (Asthma Control Questionnaire, 6-item version) results;
(d) improved AQLQ (Asthma Quality of Life Questionnaire) results; or,
(e) a combination thereof.

23. The method according to clause 1, wherein the IL-13-mediated disease or disorder is a pulmonary disease or disorder, an inflammatory bowel disease or disorder, or a chronic inflammatory skin disease or disorder.

24. The method according to clause 23, wherein the pulmonary disease or disorder is asthma, IPF, COPD, chronic rhinosinusitis, or allergic rhinitis

25. The method according to clause 23, wherein the chronic inflammatory skin disease or disorder is atopic dermatitis, allergic contact dermatitis, eczema or psoriasis.

26. The method according to clause 24, wherein the asthma is allergic asthma, atopic asthma, corticosteroid naive asthma, chronic asthma, corticosteroid resistant asthma, corticosteroid refractory asthma, asthma due to smoking, or asthma uncontrolled on corticosteroids.

27. A method of diagnosing an IL-13 mediated disease or disorder in a patient comprising measuring the level of DPP4 (dipeptidyl peptidase-4) in a sample taken from the patient, wherein the patient is diagnosed with the IL-13 mediated disease or disorder if the level of DPP4 is above a predetermined DPP4 threshold level, or above the DPP4 level in one or more control samples.

28. A method of identifying a patient as a candidate for treatment with an IL-13 antagonist comprising measuring the level of DPP4 (dipeptidyl peptidase-4) in a sample taken from the patient, wherein a level of DPP4 above a predetermined DPP4 threshold level, or above the DPP4 level in one or more control samples identifies the patient as a candidate for treatment with the IL-13 antagonist.

-

## Claims

1. An IL-13 antibody or antigen-binding fragment thereof for use in a method of treating a patient having atopic dermatitis, wherein the method comprises administering an IL-13 antagonist to the patient if

    (a) the level of DPP4 (dipeptidyl peptidase-4) in one or more samples taken from the patient is above a predetermined DPP4 threshold level of at least 250 ng/ml as measured in serum using an enzyme-linked immunosorbent assay (ELISA), and optionally if
    (b) the patient presents (i) high periostin (≥ median serum periostin or about 23 ng/mL), (ii) high eosinophil cell count (blood eosinophil count ≥ 300 cells/μL), (iii) high Th2 (high Th2 defined as IgE > 100 IU/mL and blood eosinophils ≥ 0.14 x $10^9$/L), or (iv) combinations thereof,

    wherein the antibody or antigen-binding fragment thereof comprises tralokinumab (VH: SEQ ID NO:3; VL:SEQ ID NO:4) or an antigen-binding fragment thereof; or the antibody or antigen-binding fragment thereof consists of tralokinumab (VH: SEQ ID NO:3; VL:SEQ ID NO:4) or an antigen-binding fragment thereof.

2. The IL-13 antibody or antigen-binding fragment thereof for use according to claim 1, wherein the patient has been treated with one or more additional medications, either before, during, or after administration of an IL-13 antibody or antigen-binding fragment thereof, wherein the one or more additional medications optionally comprises a steroid, such as fluticasone or budesonide, wherein the steroid is optionally administered at a high dose.

**3.** The IL-13 antibody or antigen-binding fragment thereof for use according to any one of the preceding claims, wherein the IL-13 antibody or antigen-binding fragment thereof is administered at a fixed dose and optionally wherein the IL-13 antibody or antigen-binding fragment thereof is administered at a fixed dose of about 300 mg/dose.

**4.** The IL-13 antibody or antigen-binding fragment thereof for use according to any one of the preceding claims, wherein the IL-13 antibody or antigen-binding fragment thereof is administered in two or more doses, wherein the IL-13 antibody or antigen-binding fragment thereof is optionally administered weekly, biweekly or monthly.

**5.** The IL-13 antibody or antigen-binding fragment thereof for use according to any one of the preceding claims, wherein the IL-13 antibody or antigen-binding fragment thereof is administered intravenously, intramuscularly, subcutaneously, or a combination thereof.

**6.** The IL-13 antibody or antigen-binding fragment thereof for use according to any one of the preceding claims, wherein the predetermined DPP4 threshold level is at least 350 ng/mL, at least 375 ng/mL, at least 400 ng/mL, at least 450 ng/mL, at least 500 ng/mL, at least 550 ng/mL, or at least 600 ng/mL, as measured in serum using an ELISA.

**7.** The IL-13 antibody or antigen-binding fragment thereof for use according to any one of the preceding claims, wherein the predetermined DPP4 threshold level is 365 ng/mL.

FIG. 1

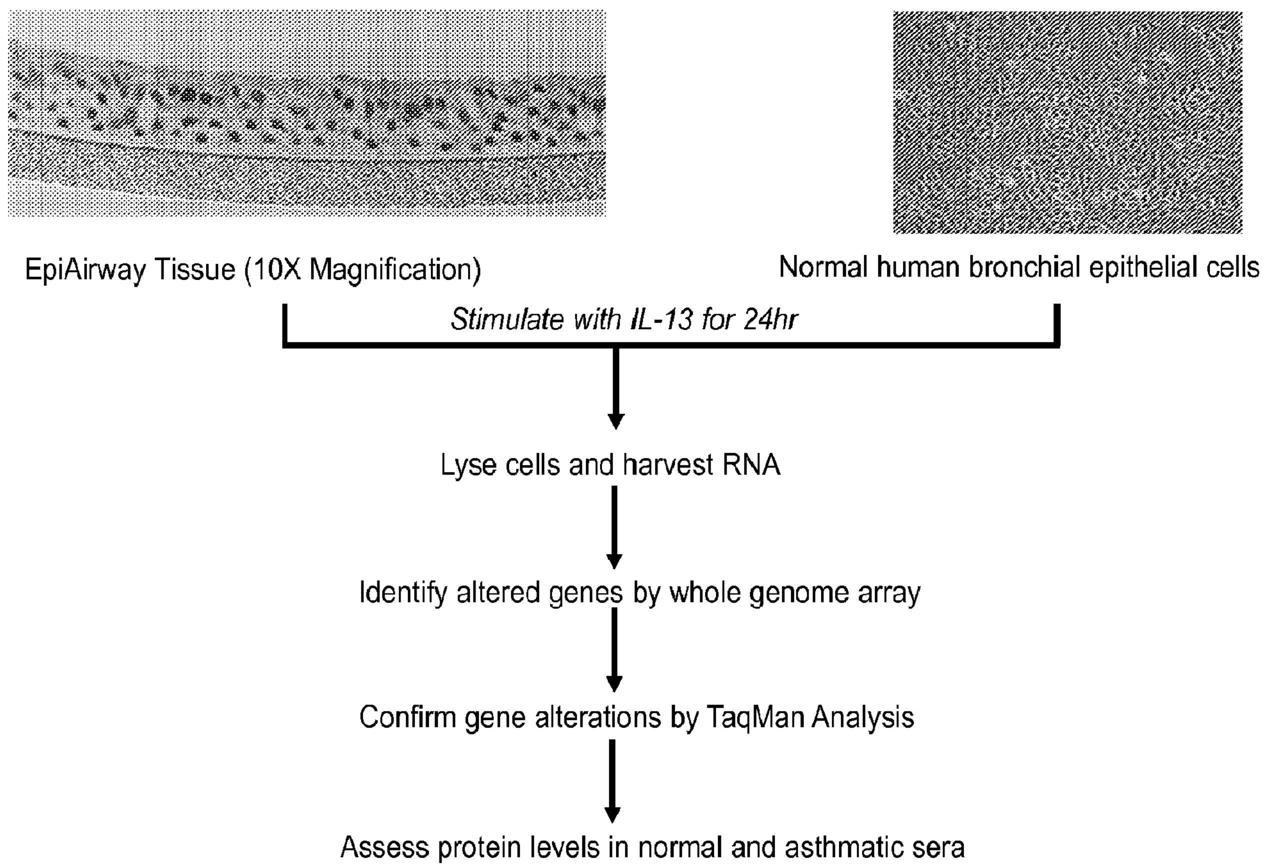
EpiAirway Tissue (10X Magnification)
Normal human bronchial epithelial cells
Stimulate with IL-13 for 24hr
Lyse cells and harvest RNA
Identify altered genes by whole genome array
Confirm gene alterations by TaqMan Analysis
Assess protein levels in normal and asthmatic sera

FIG. 2

| Probe ID | log2.fc | p.value | q.val | UniGene.ID | Gene.Title | Gene.Symbol |
|---|---|---|---|---|---|---|
| 223710_at | 11.704 | 3.48E-06 | 0.007679 | Hs.131342 | chemokine (C-C motif) ligand 26 | CCL26 |
| 1553177_at | 7.373 | 0.003256 | 0.150603 | Hs.350581 | SH2 domain containing 1B | SH2D1B |
| 203717_at | 6.896 | 1.73E-06 | 0.007679 | Hs.368912 | dipeptidyl-peptidase 4 | DPP4 |
| 1564746_at | 6.882 | 0.000149 | 0.033081 | Hs.546482 | Na+/H+ exchanger domain containing 2 | NHEDC2 |
| 1553176_at | 6.785 | 3.36E-05 | 0.015623 | Hs.350581 | SH2 domain containing 1B | SH2D1B |
| 219564_at | 6.680 | 1.94E-05 | 0.012103 | Hs.463985 | potassium inwardly-rectifying channel, subfamily J, member | KCNJ16 |
| 211478_s_at | 6.663 | 2.17E-05 | 0.01218 | Hs.368912 | dipeptidyl-peptidase 4 | DPP4 |
| 229491_at | 6.483 | 6.92E-05 | 0.02249 | Hs.546482 | Na+/H+ exchanger domain containing 2 | NHEDC2 |
| 220622_at | 6.008 | 0.000137 | 0.030929 | Hs.411295 | leucine rich repeat containing 31 | LRRC31 |
| 210521_s_at | 5.971 | 0.00076 | 0.071008 | Hs.81073 | fetuin B | FETUB |
| 203716_s_at | 5.874 | 6.92E-06 | 0.007679 | Hs.368912 | dipeptidyl-peptidase 4 | DPP4 |
| 214539_at | 5.769 | 3.91E-06 | 0.007679 | Hs.158339 | serpin peptidase inhibitor, clade B (ovalbumin), member 10 | SERPINB10 |
| 227480_at | 5.669 | 0.01015 | 0.246439 | Hs.131819 | sushi domain containing 2 | SUSD2 |
| 218804_at | 5.460 | 0.00054 | 0.063169 | Hs.503074 | anoctamin 1, calcium activated chloride channel | ANO1 |
| 223597_at | 5.200 | 0.004137 | 0.166478 | Hs.50813 | intelectin 1 (galactofuranose binding) | ITLN1 |
| 206932_at | 5.171 | 0.000399 | 0.053415 | Hs.47357 | cholesterol 25-hydroxylase | CH25H |
| 223377_x_at | 5.091 | 0.007388 | 0.214469 | Hs.655334 | cytokine inducible SH2-containing protein | CISH |
| 205969_at | 5.057 | 0.00027 | 0.043821 | Hs.506908 | arylacetamide deacetylase (esterase) | AADAC |
| 1557321_a_at | 5.047 | 2.45E-06 | 0.007679 | Hs.468059 | calpain 14 | CAPN14 |
| 210809_s_at | 4.936 | 0.003132 | 0.147591 | Hs.136348 | periostin, osteoblast specific factor | POSTN |
| 207328_at | 4.911 | 0.000227 | 0.041212 | Hs.73809 | arachidonate 15-lipoxygenase | ALOX15 |
| 1555778_a_at | 4.704 | 0.002295 | 0.127657 | Hs.136348 | periostin, osteoblast specific factor | POSTN |

# FIG. 3

| Probe ID | IL13 vs. NT (log2 fc) | IL13 vs. NT (p-value) | IL13 vs. NT (q-value) | UniGene.ID | Gene.Title | Gene.Symbol |
|---|---|---|---|---|---|---|
| 223710_at | 10.5303 | 0.022019802 | 0.100091 | Hs.131342 | chemokine (C-C motif) ligand 26 | CCL26 |
| 206224_at | 10.23784 | 0.007869349 | 0.072181 | Hs.123114 | cystatin SN | CST1 |
| 210809_s_at | 10.08787 | 0.01771057 | 0.093066 | Hs.136348 | periostin, osteoblast specific factor | POSTN |
| 1555778_a_at | 9.920576 | 0.011827893 | 0.081568 | Hs.136348 | periostin, osteoblast specific factor | POSTN |
| 220622_at | 7.874274 | 0.003115017 | 0.054393 | Hs.411295 | leucine rich repeat containing 31 | LRRC31 |
| 1553176_at | 7.818319 | 0.000388042 | 0.036284 | Hs.350581 | SH2 domain containing 1B | SH2D1B |
| 203716_s_at | 7.600052 | 0.002277719 | 0.05012 | Hs.368912 | dipeptidyl-peptidase 4 (CD26, adenosine deaminase complexing protein 2) | DPP4 |
| 211478_s_at | 7.545594 | 0.001764026 | 0.04697 | Hs.368912 | dipeptidyl-peptidase 4 (CD26, adenosine deaminase complexing protein 2) | DPP4 |
| 206994_at | 7.178186 | 0.005964215 | 0.066448 | Hs.654549 | cystatin S | CST4 |
| 206942_s_at | 6.794592 | 0.0008042 | 0.040603 | Hs.707990 | pro-melanin-concentrating hormone | PMCH |
| 1557321_a_at | 6.691411 | 0.008835125 | 0.074483 | Hs.468059 | calpain 14 | CAPN14 |
| 1553177_at | 6.521778 | 0.004185219 | 0.059371 | Hs.350581 | SH2 domain containing 1B | SH2D1B |
| 218804_at | 6.458367 | 0.003943545 | 0.058651 | Hs.503074 | transmembrane protein 16A | TMEM16A |
| 203717_at | 6.245909 | 4.72091E-05 | 0.024165 | Hs.368912 | dipeptidyl-peptidase 4 (CD26, adenosine deaminase complexing protein 2) | DPP4 |
| 210521_s_at | 6.003439 | 0.046524549 | 0.133084 | Hs.81073 | fetuin B | FETUB |
| 232217_at | 5.825 | 0.003617583 | 0.057486 | Hs.660142 | family with sequence similarity 26, member E | FAM26E |
| 206172_at | 5.779177 | 0.007917215 | 0.072332 | Hs.336046 | interleukin 13 receptor, alpha 2 | |

FIG. 4

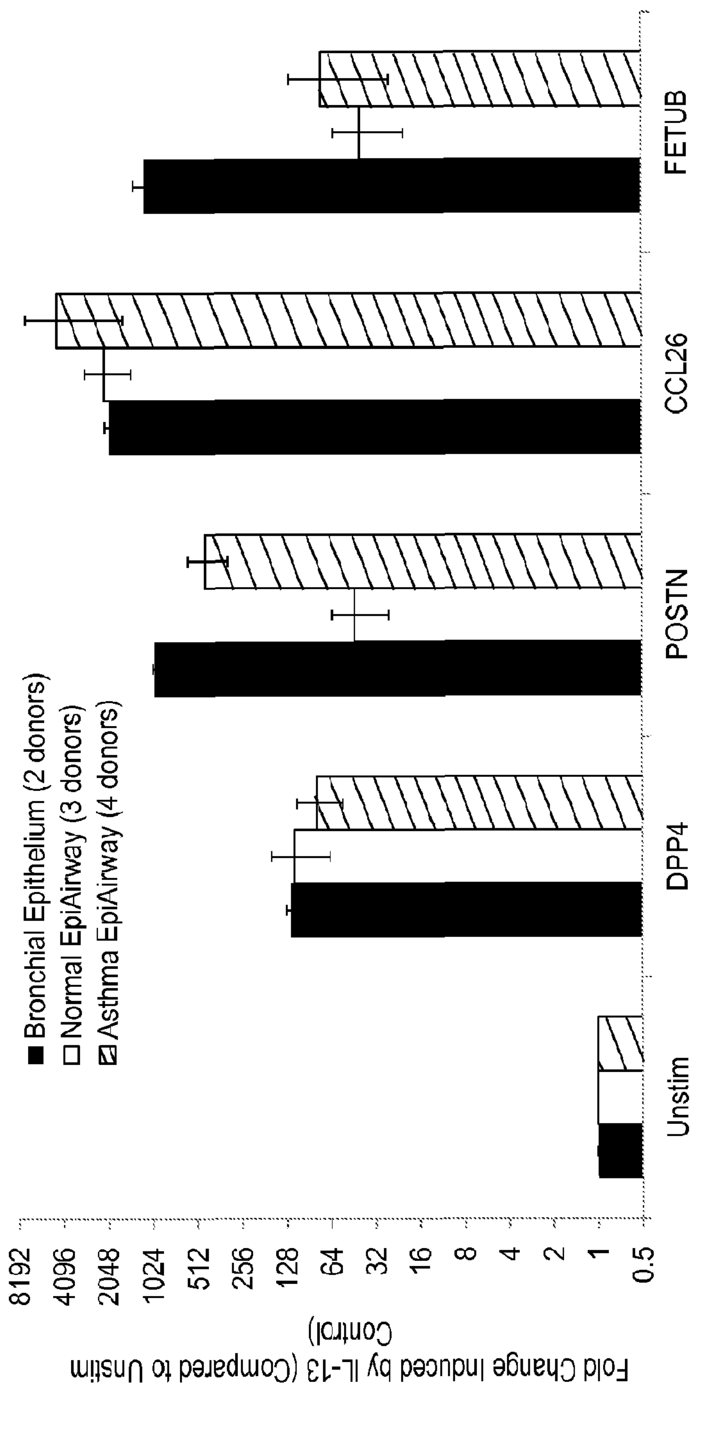
Bronchial Epithelium (2 donors)
Normal EpiAirway (3 donors)
Asthma EpiAirway (4 donors)
Fold Change Induced by IL-13 (Compared to Unstim Control)
8192
4096
2048
1024
512
256
128
64
32
16
8
4
2
1
0.5
Unstim
DPP4
POSTN
CCL26
FETUB

FIG. 5

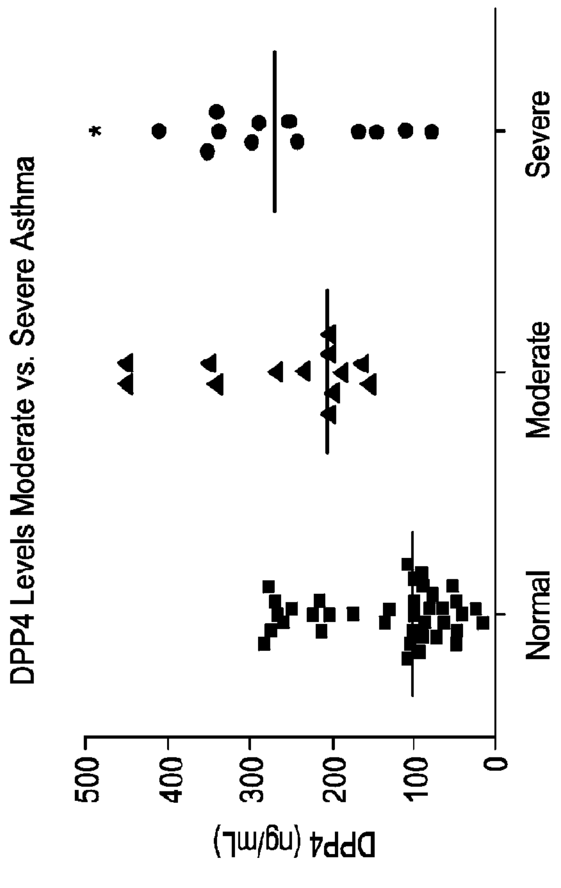
DPP4 Levels Moderate vs. Severe Asthma
DPP4 (ng/mL)
500
400
300
200
100
0
Normal
Moderate
Severe
*

FIG. 6

Serum DDP4 Levels in Asthma Patients by Medication Status

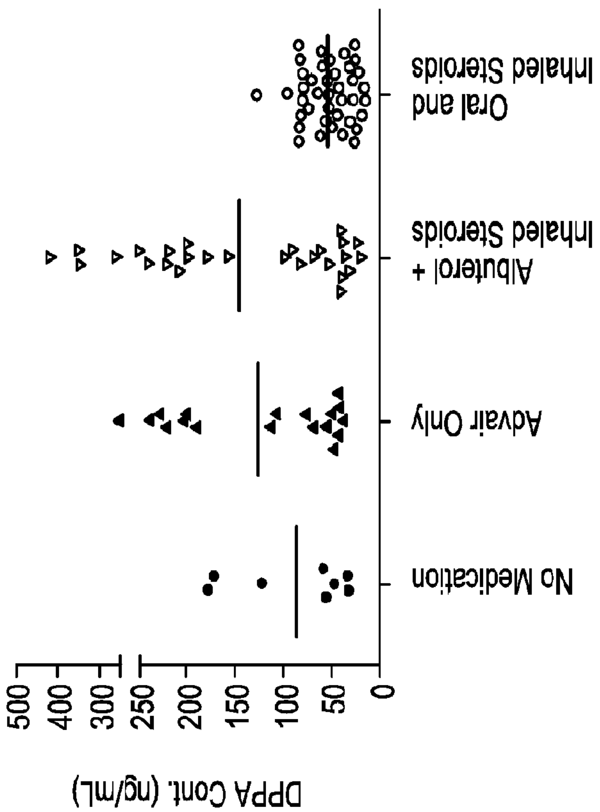

FIG. 7

Tralokinumab Phase 2b Study – CD-RI-Cat-354-1049

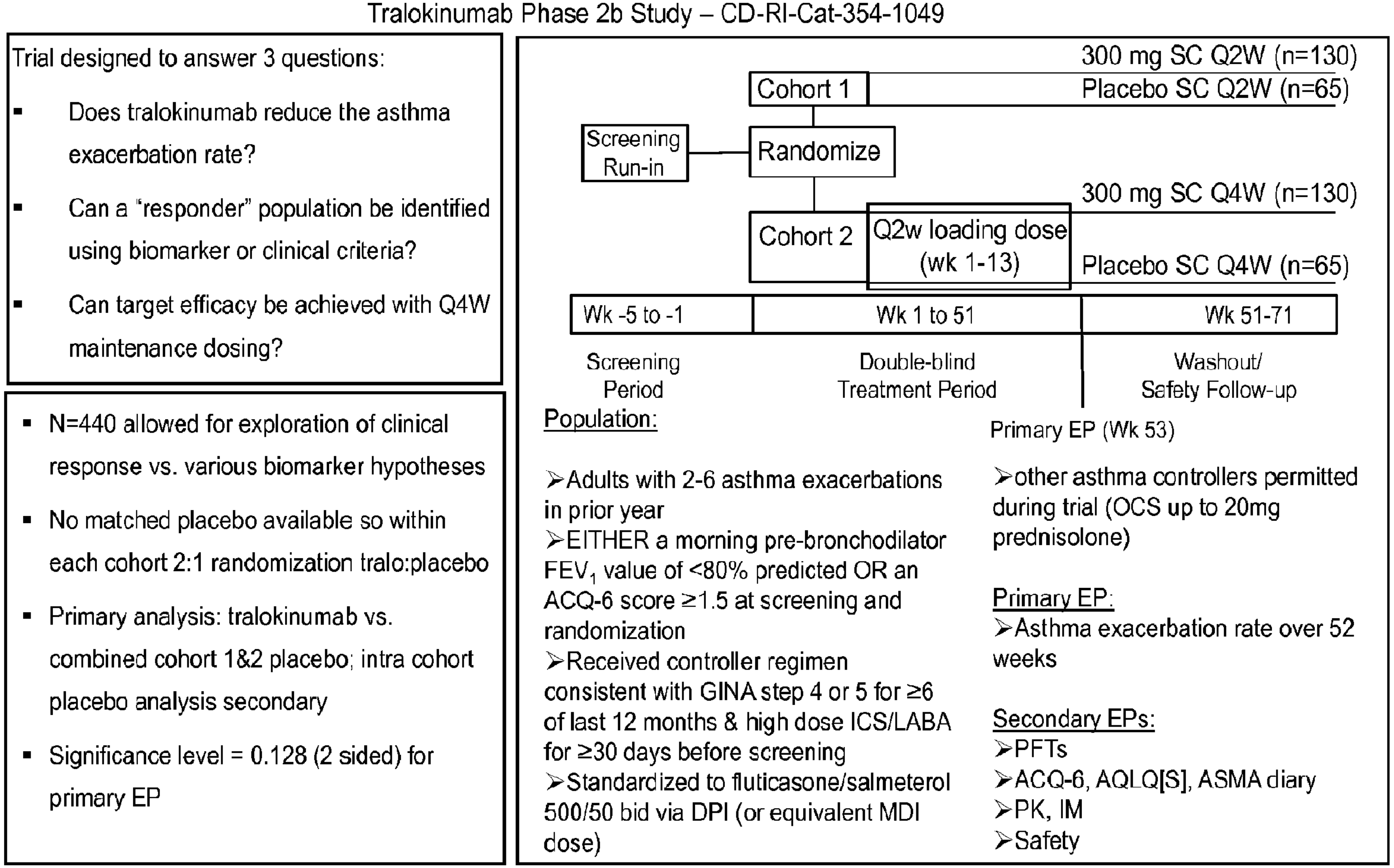

# FIG. 8

Change from Baseline in Pre-bronchodilator FEV1 Over Time (ITT Population)

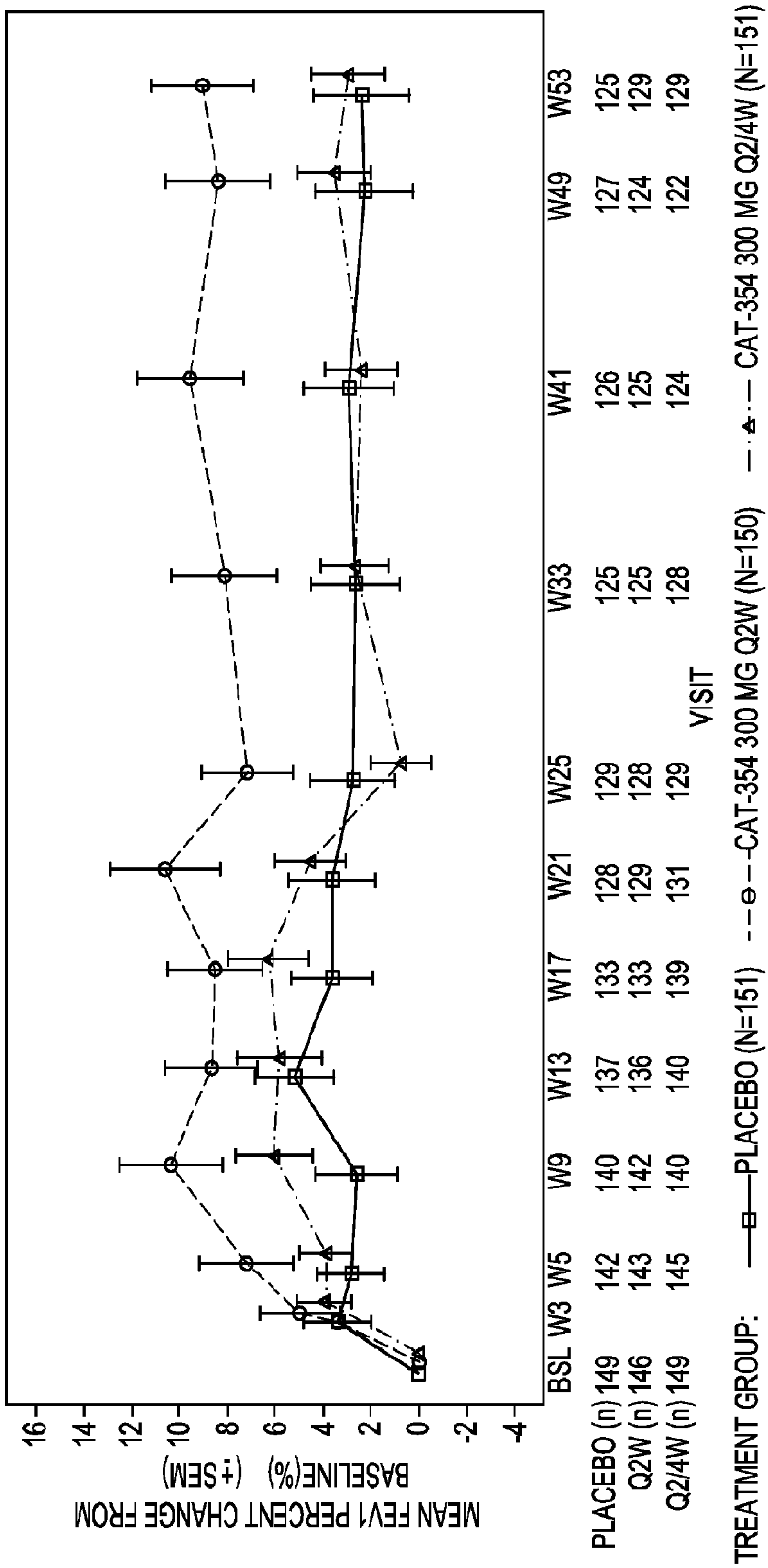

# FIG. 9A

Annual AER and Change from Baseline in Pre-bronchodilator at Week 53 by FEV1 Reversibility and Serum Periostin Level

| | PBO N | CAT354 Q2W N | CAT354 Q2/4W N |
|---|---|---|---|
| ITT Population | 151 | 150 | 151 |
| FEV1 Reversibility >=12% | 57 | 43 | 49 |
| +Periostin >= Median | 26 | 22 | 28 |
| +Periostin < Median | 31 | 21 | 21 |
| FEV1 Reversibility < 12% | 91 | 101 | 97 |
| +Periostin >= Median | 39 | 57 | 48 |
| +Periostin < Median | 51 | 44 | 48 |

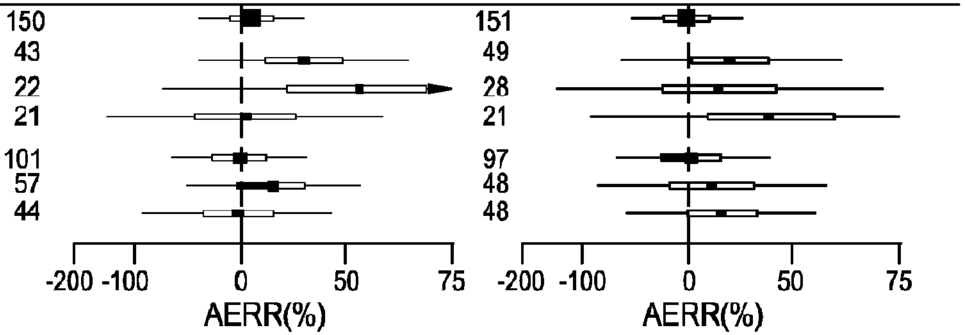


Estimates are from Poisson regression model, adjusted for covariates
Estimates are shown with 95% CI: within these 50% CI are highlighted.

# FIG. 9B

Annual AER and Change from Baseline in Pre-bronchodilator at Week 53 by FEV1 Reversibility and Serum Periostin Level

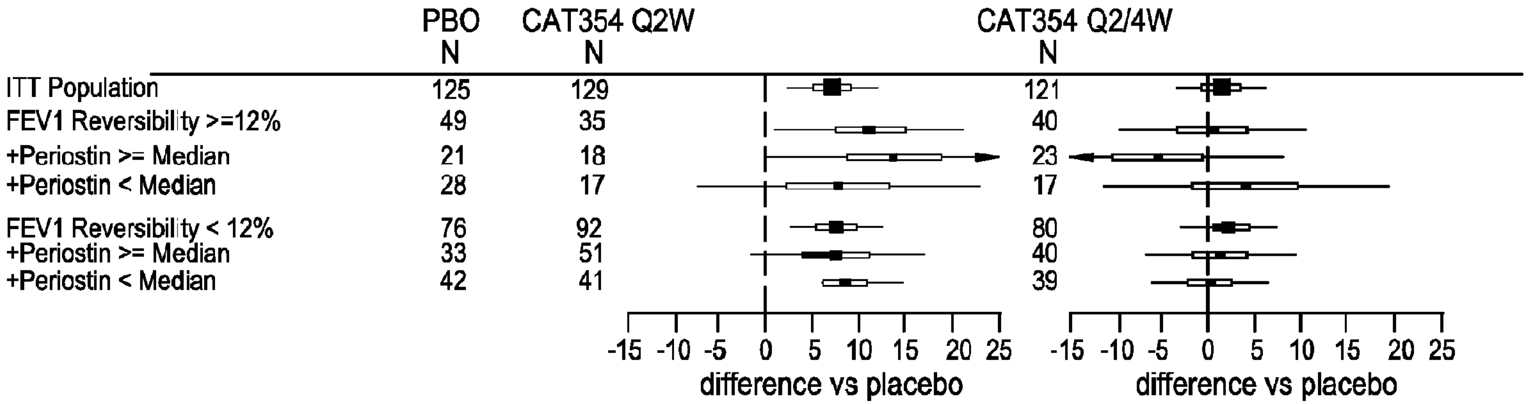


Estimates are from a mixed model repeated measures (MMRM) analysis including all patients, N shows the number of patients with observations at week 53. Estimates are shown with 95% CI: within these 50% CI are highlighted.

# FIG. 10A

ACQ and AQLQ(S) at Week 53 By FEV1 Reversibility and Serum Periostin Level

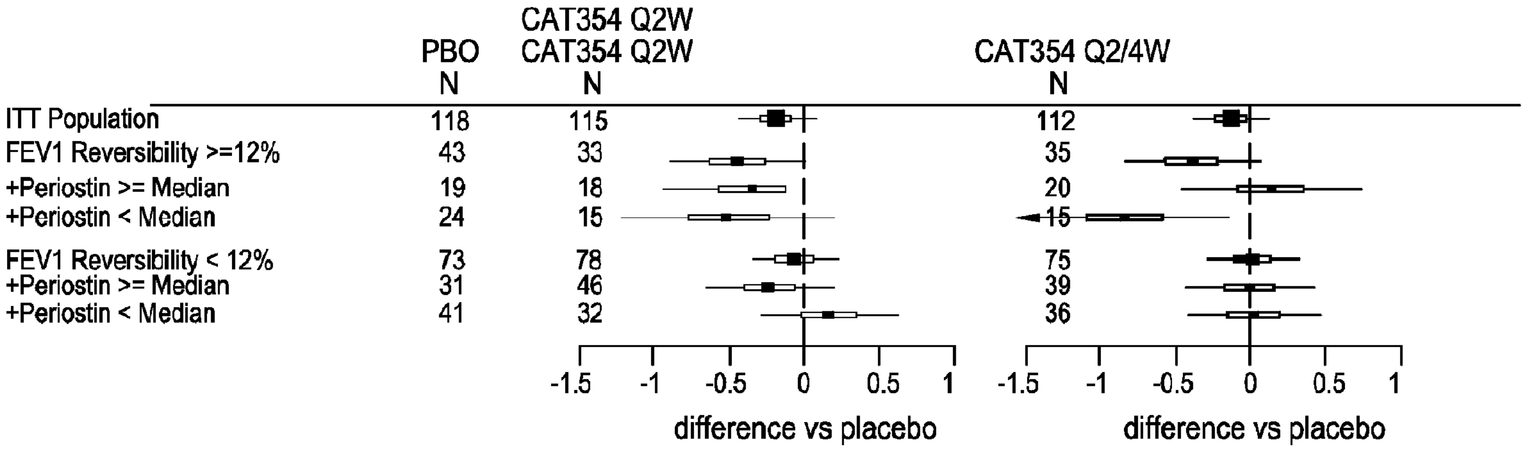


Estimates are from a mixed model repeated measures (MMRM) analysis including all patients, N shows the number of patients with observations at week 53. Estimates are shown with 95% CI: within these 50% CI are highlighted.

# FIG. 10B

ACQ and AQLQ(S) at Week 53 By FEV1 Reversibility and Serum Periostin Level

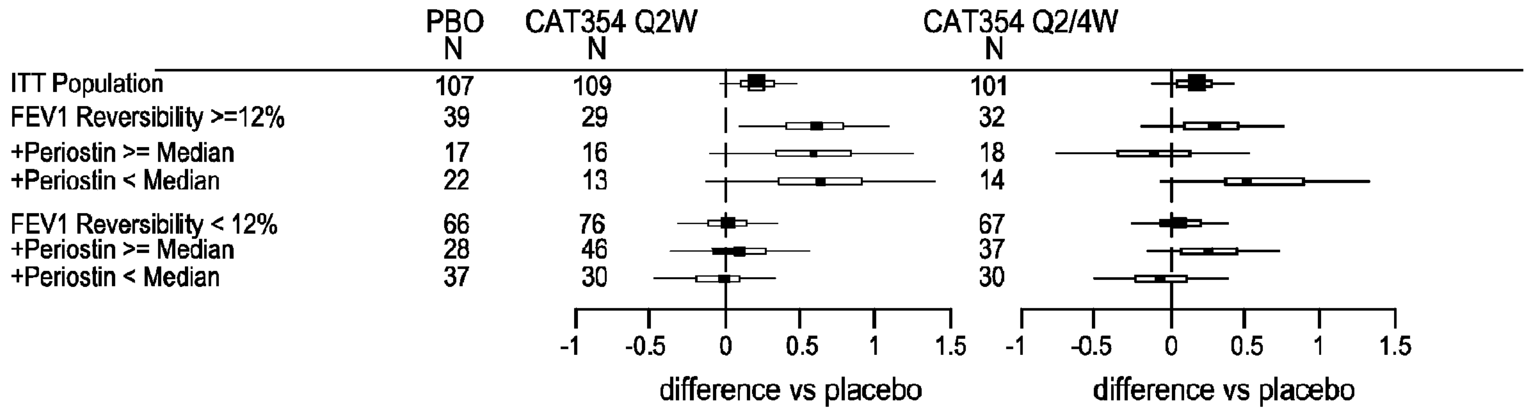


Estimates are from a mixed model repeated measures (MMRM) analysis including all patients, N shows the number of patients with observations at week 53. Estimates are shown with 95% CI: within these 50% CI are highlighted.

FIG. 11

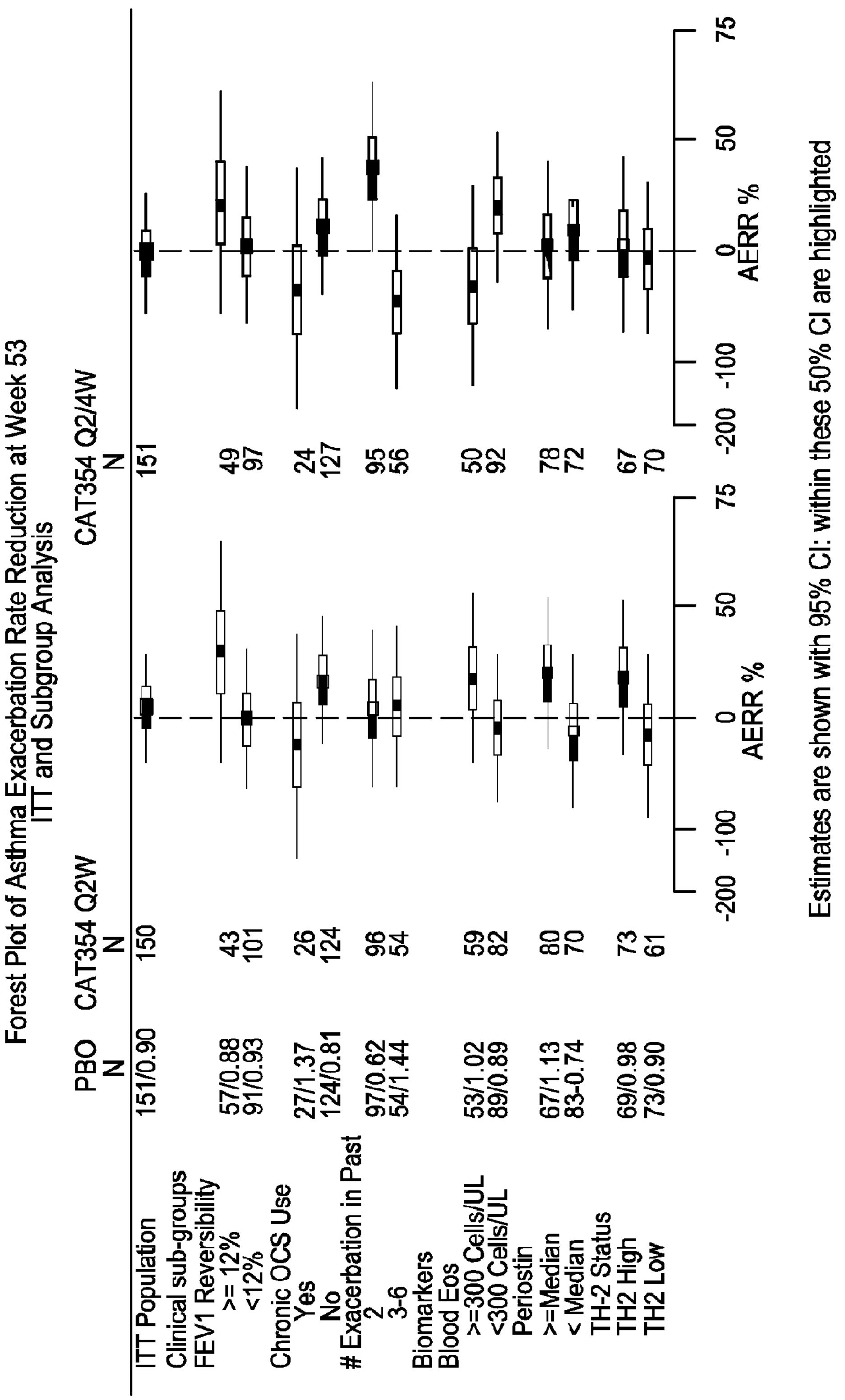
Forest Plot of Asthma Exacerbation Rate Reduction at Week 53
ITT and Subgroup Analysis
PBO N
CAT354 Q2W N
CAT354 Q2/4W N
ITT Population 151/0.90 150 151
Clinical sub-groups
FEV1 Reversibility
>= 12% 57/0.88 43 49
<12% 91/0.93 101 97
Chronic OCS Use
Yes 27/1.37 26 24
No 124/0.81 124 127
# Exacerbation in Past
2 97/0.62 96 95
3-6 54/1.44 54 56
Biomarkers
Blood Eos
>=300 Cells/UL 53/1.02 59 50
<300 Cells/UL 89/0.89 82 92
Periostin
>=Median 67/1.13 80 78
< Median 83-0.74 70 72
TH-2 Status
TH2 High 69/0.98 73 67
TH2 Low 73/0.90 61 70
-200 -100 0 50 75
AERR %
-200 -100 0 50 75
AERR %
Estimates are shown with 95% CI: within these 50% CI are highlighted

FIG. 12

Forest Plot of Percent Change from Baseline in pre-BD FEV1 at week 53
ITT and Subgroup Analysis

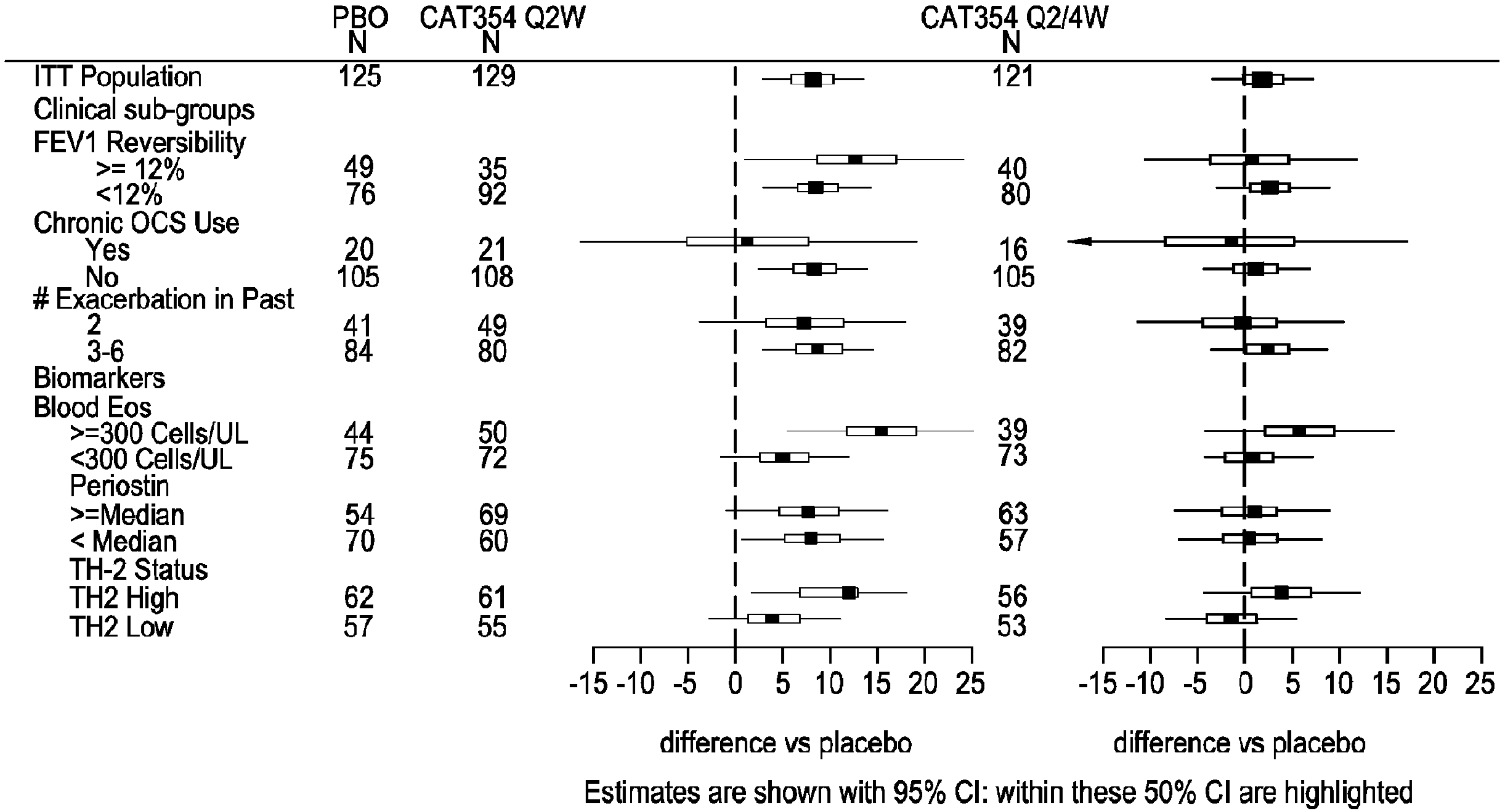

FIG. 13A

Asthma Exacerbation Rate Reduction and Mean Percent Change from Baseline in Pre-bronchodilator FEV1 at Week 53 for Patients by Baseline Serum Periostin Level (Tralokinumab Q2W vs Placebo)

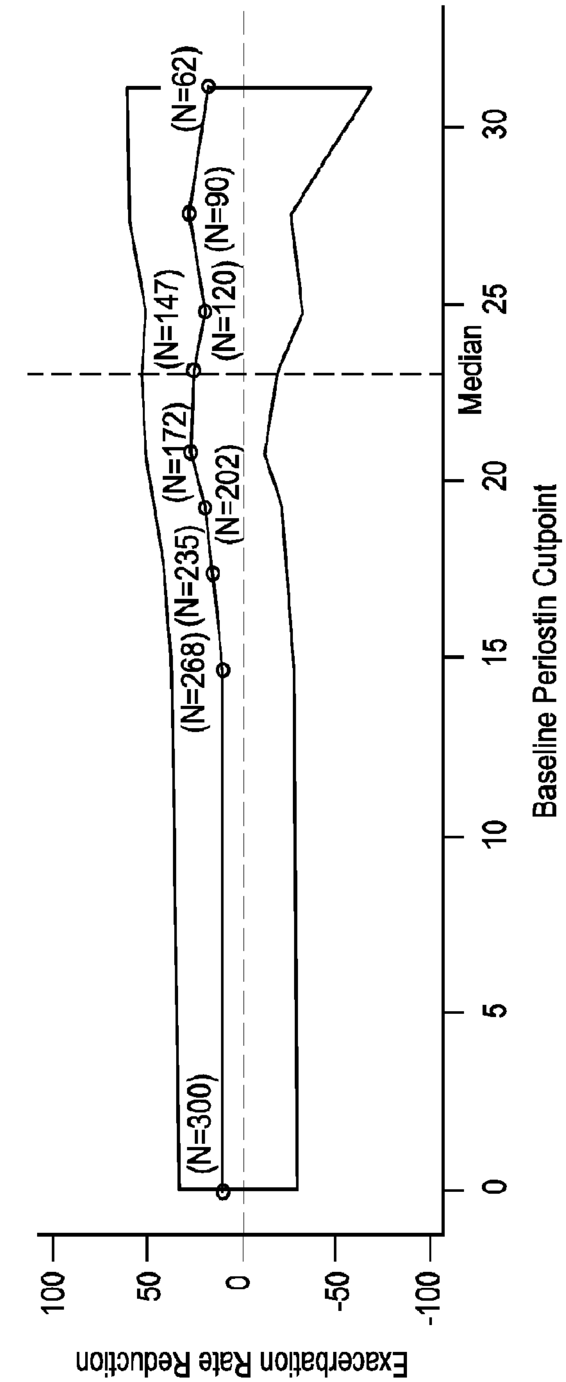

FIG. 13B

Asthma Exacerbation Rate Reduction and Mean Percent Change from Baseline in Pre-bronchodilator FEV1 at Week 53 for Patients by Baseline Serum Periostin Level (Tralokinumab Q2W vs Placebo)

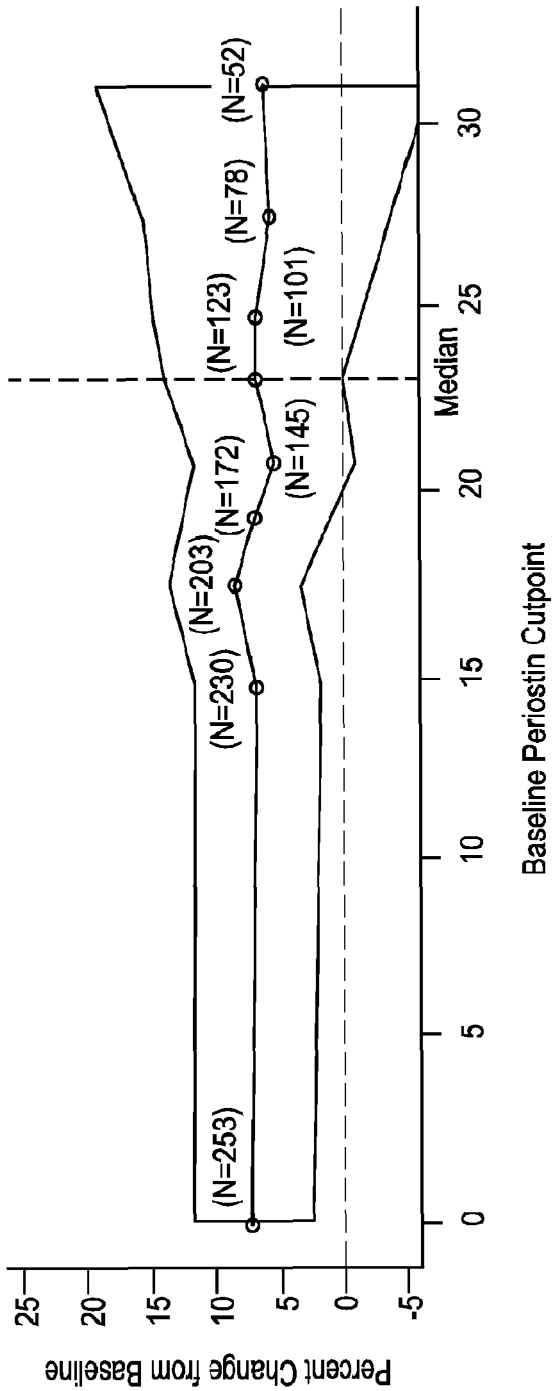

# FIG. 14A

Percent Change from Baseline in Pre-Bronchodilator FEV1 Over Time by Treatment Group
Baseline Serum Periostin >=Median ITT Population

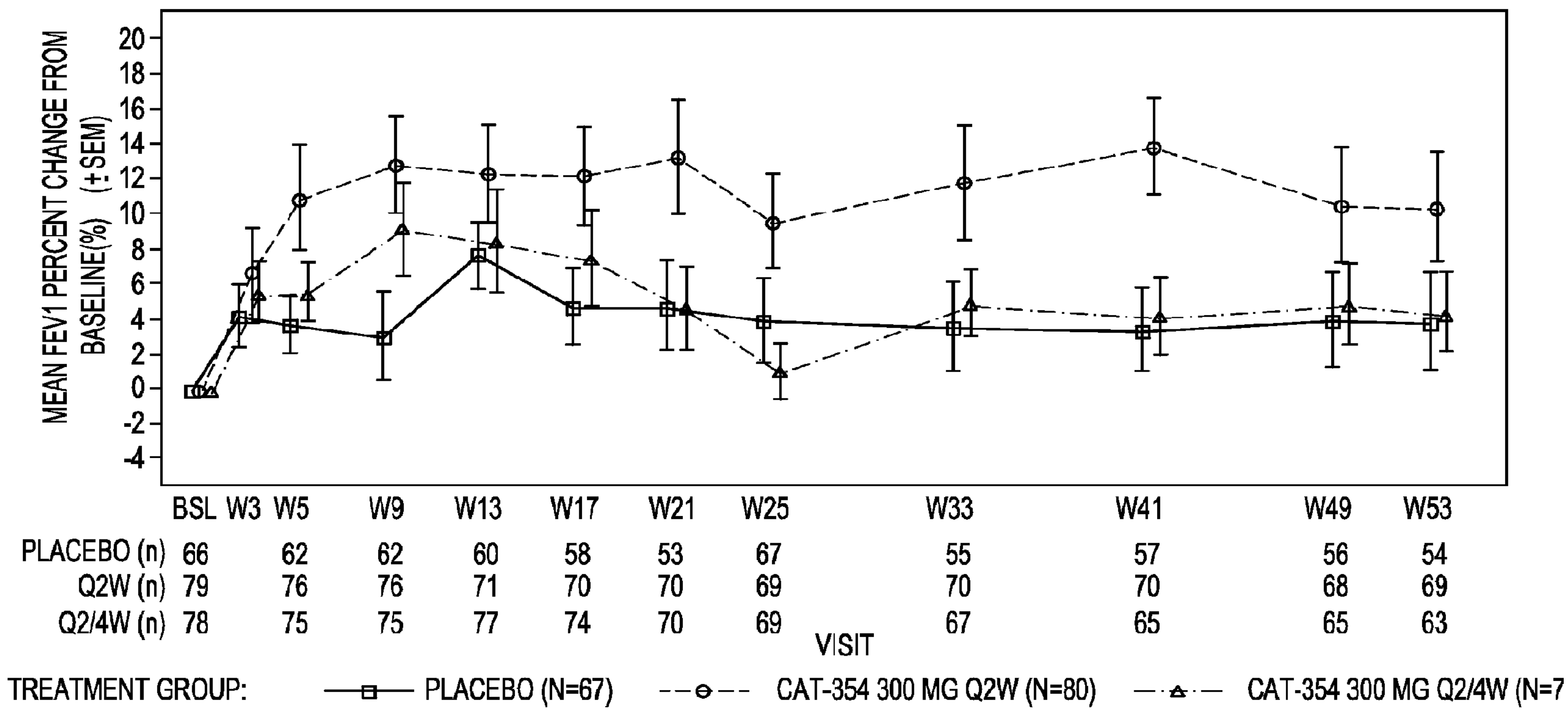

# FIG. 14B

Percent Change from Baseline in Pre-Bronchodilator FEV1 Over Time by Treatment Group
Baseline Serum Periostin <Median ITT Population

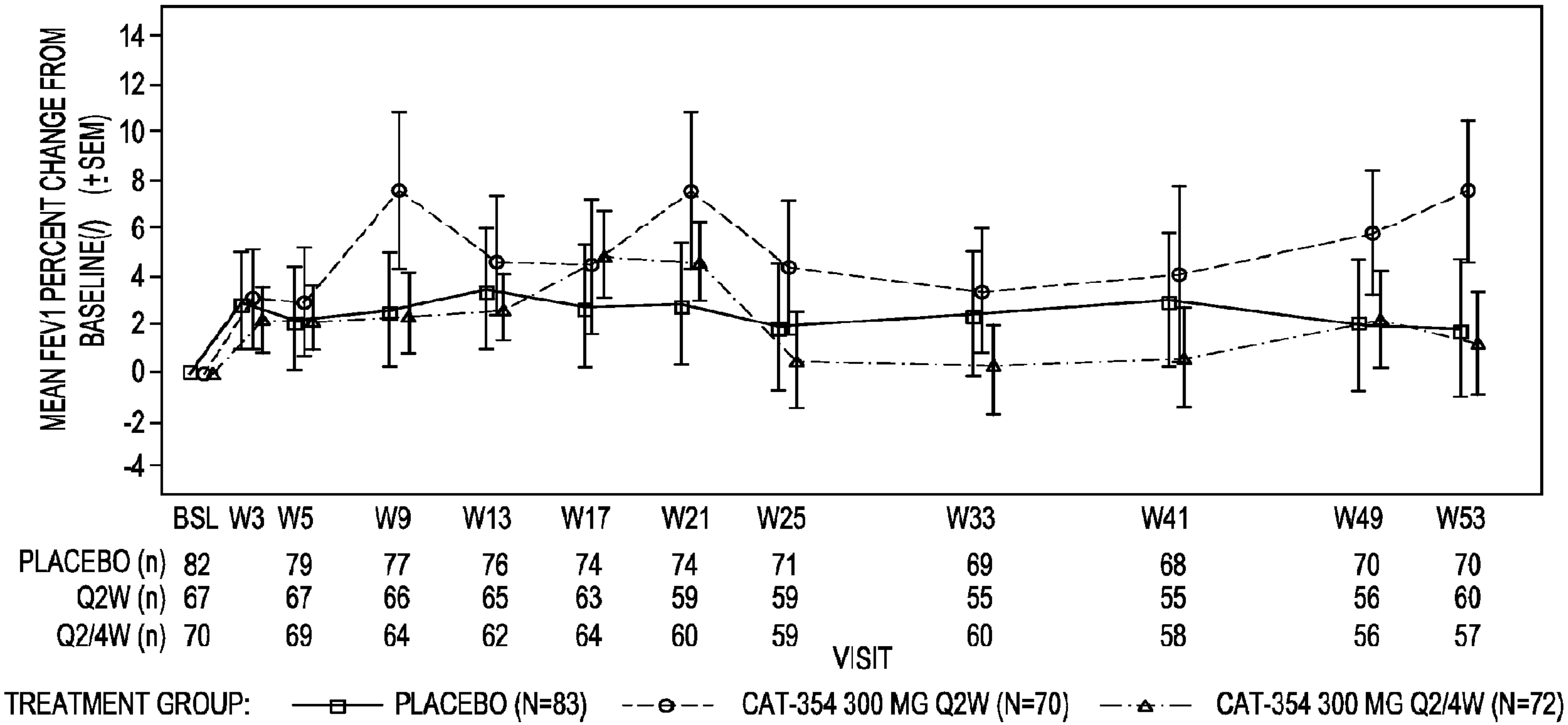

# FIG. 15

DPP4 Outperforms Periostin in the Intention-To-Treat Population – Q2W vs Placebo

| Endpoint | All-comers (N* = 150) | Periostin >= Median (N* = 80) | DPP4 >=Median (N* = 77) |
|---|---|---|---|
| Primary<br><br>Acute Exacerbation Rate Reduction[a] (95% CI) | 7% (-30%, 33%)<br>P = 0.669 | 25% (-19%, 53%)<br>P = 0.219 | 34% (-6%, 59%)<br>P=0.083 |
| Secondary (Difference from placebo)<br>Percent change from baseline in FEV1 (95% CI) | 7.1 (2.35, 11.84)<br>P = 0.003 | 6.75 (-0.31, 13.82)<br>P = 0.061 | 10.8 (3.27, 18.23)<br>P=0.005 |
| ACQ-6, change from baseline (95% CI) | -0.18 (-0.43, 0.06)<br>P = 0.137 | -0.23 (-0.56, 0.09)<br>P = 0.163 | -0.50 (-0.86, -0.14<br>P=0.007 |
| AQLQ, change from baseline (95% CI) | 0.21 (-0.05, 0.46)<br>P = 0.114 | 0.22 (-0.15, 0.59)<br>P = 0.245 | 0.69 (0.30, 1.08)<br>P<0.001 |

* N for tralokinumab 300 mg Q2W

Abbreviations: CI, confidence interval; $FEV_1$, forced expiratory volume at 1 second; ITT, intent-to-treat. ACQ-6, asthma control questionnaire, AQLQ, asthma quality of life questionnaire

[a]Asthma exacerbation rate reductions were calculated using Poisson regression model adjusted for over dispersion with treatment group, age, gender, number of asthma exacerbations in the past year, atopic asthma status, presence or absence of chronic OCS use and geographical region as covariates and the log of number of days in the study as offset

# FIG. 16

Q2W vs Placebo for Above and Below

| Endpoint | Periostin >= Median (N* = 80) | Periostin < Median (N* = 80) | DPP4 >=Median (N* = 77) | DPP4 < Median (N* = 68) |
|---|---|---|---|---|
| Primary<br><br>Acute Exacerbation Rate Reduction (95% CI) | 25% (-19%, 53%)<br>P = 0.219 | -8% (-73%, 32%)<br>P = 0.742 | 34% (-6%, 59%)<br>P=0.083 | -25% (-102%, 23%)<br>P = 0.359 |
| Secondary (Difference from placebo)<br><br>Percent change from baseline in FEV1 (95% CI) | 6.75 (-0.31, 13.82)<br>P = 0.061 | 7.06 (0.51, 13.60)<br>P = 0.035 | 10.8 (3.27, 18.23)<br>P=0.005 | 3.79 (-2.33, 9.92)<br>P = 0.330 |
| ACQ-6, change from baseline (95% CI) | -0.23 (-0.56, 0.09)<br>P = 0.163 | -0.02 (-0.38, 0.34)<br>P = 0.919 | -0.50 (-0.86, -0.14)<br>P=0.007 | 0.05 (-0.29, 0.38)<br>P = 0.424 |
| AQLQ, change from baseline (95% CI) | 0.22 (-0.15, 0.59)<br>P = 0.245 | 0.21 (-0.16, 0.58)<br>P = 0.272 | 0.69 (0.30, 1.08)<br>P<0.001 | -0.20 (-0.54, 0.15)<br>P =0.143 |

* N for tralokinumab 300 mg Q2W

FIG. 17A

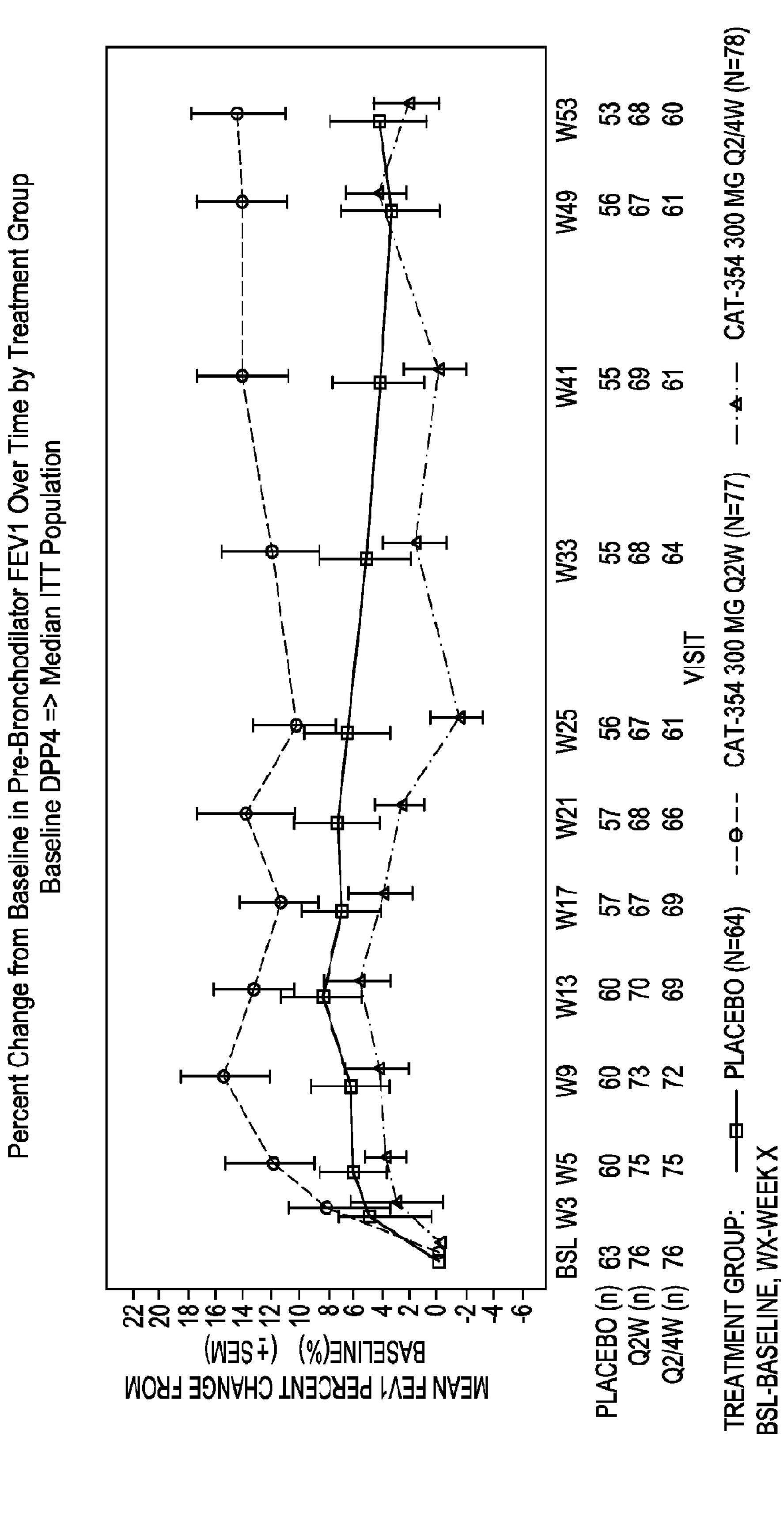
Percent Change from Baseline in Pre-Bronchodilator FEV1 Over Time by Treatment Group
Baseline DPP4 => Median ITT Population
MEAN FEV1 PERCENT CHANGE FROM BASELINE(%) (+ SEM)
22 20 18 16 14 12 10 8 6 4 2 0 -2 -4 -6
BSL W3 W5 W9 W13 W17 W21 W25 W33 W41 W49 W53
VISIT
PLACEBO (n) 63 60 60 60 57 57 56 55 55 56 53
Q2W (n) 76 75 73 70 67 68 67 68 69 67 68
Q2/4W (n) 76 75 72 69 69 66 61 64 61 61 60
TREATMENT GROUP: PLACEBO (N=64) CAT-354 300 MG Q2W (N=77) CAT-354 300 MG Q2/4W (N=78)
BSL-BASELINE, WX-WEEK X

FIG. 17B

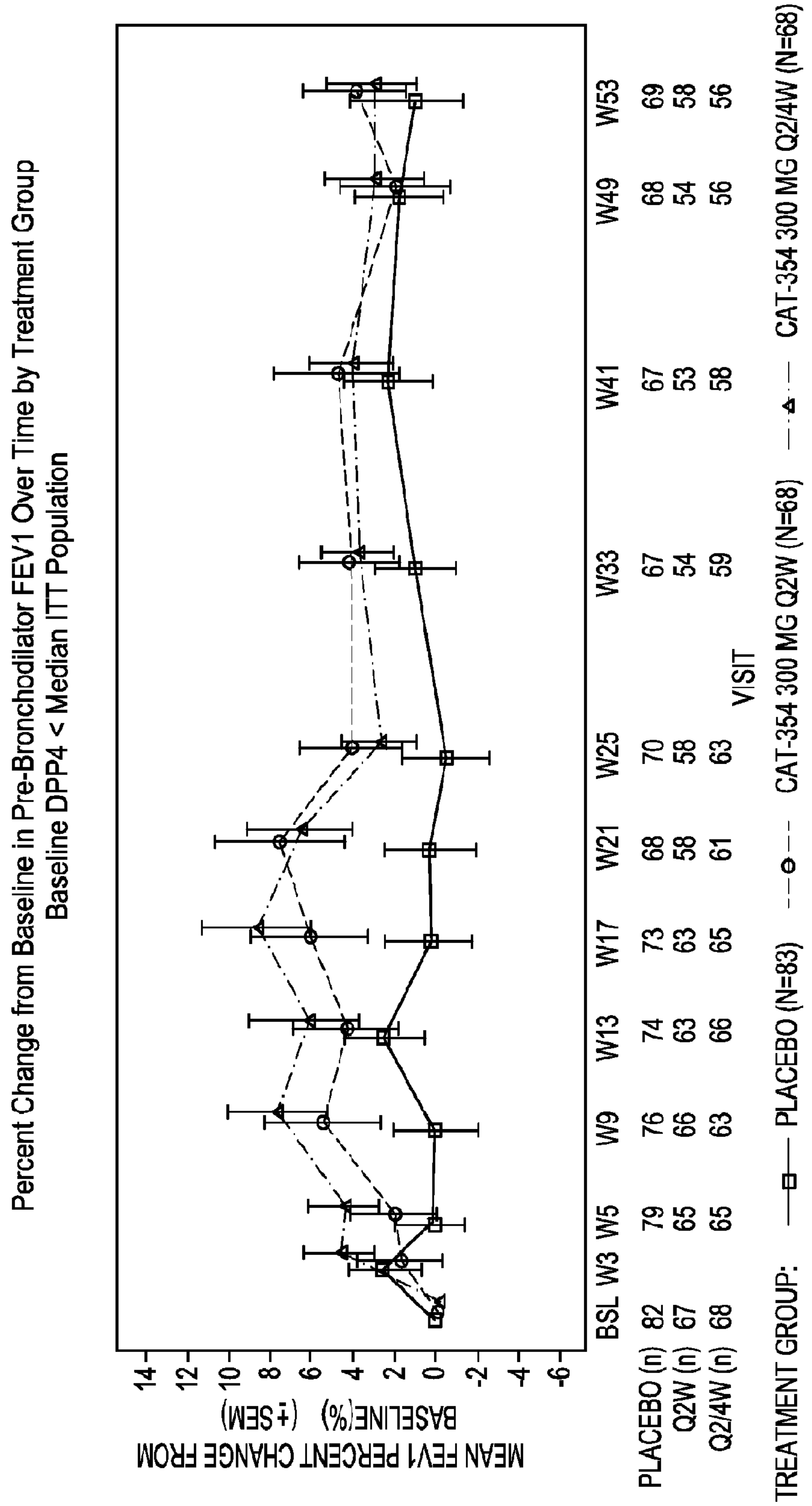
Percent Change from Baseline in Pre-Bronchodilator FEV1 Over Time by Treatment Group
Baseline DPP4 < Median ITT Population
MEAN FEV1 PERCENT CHANGE FROM BASELINE(%) (±SEM)
14 12 10 8 6 4 2 0 -2 -4 -6
BSL W3 W5 W9 W13 W17 W21 W25 W33 W41 W49 W53
VISIT
PLACEBO (n) 82 79 76 74 73 68 70 67 67 68 69
Q2W (n) 67 65 66 63 63 58 58 54 53 54 58
Q2/4W (n) 68 65 63 66 65 61 63 59 58 56 56
TREATMENT GROUP: PLACEBO (N=83) CAT-354 300 MG Q2W (N=68) CAT-354 300 MG Q2/4W (N=68)
BSL-BASELINE, WX-WEEK X

FIG. 17C

Change from Baseline in Mean ACQ-6 Score Over Time by Treatment Group
Baseline DPP4 >= Median ITT Population

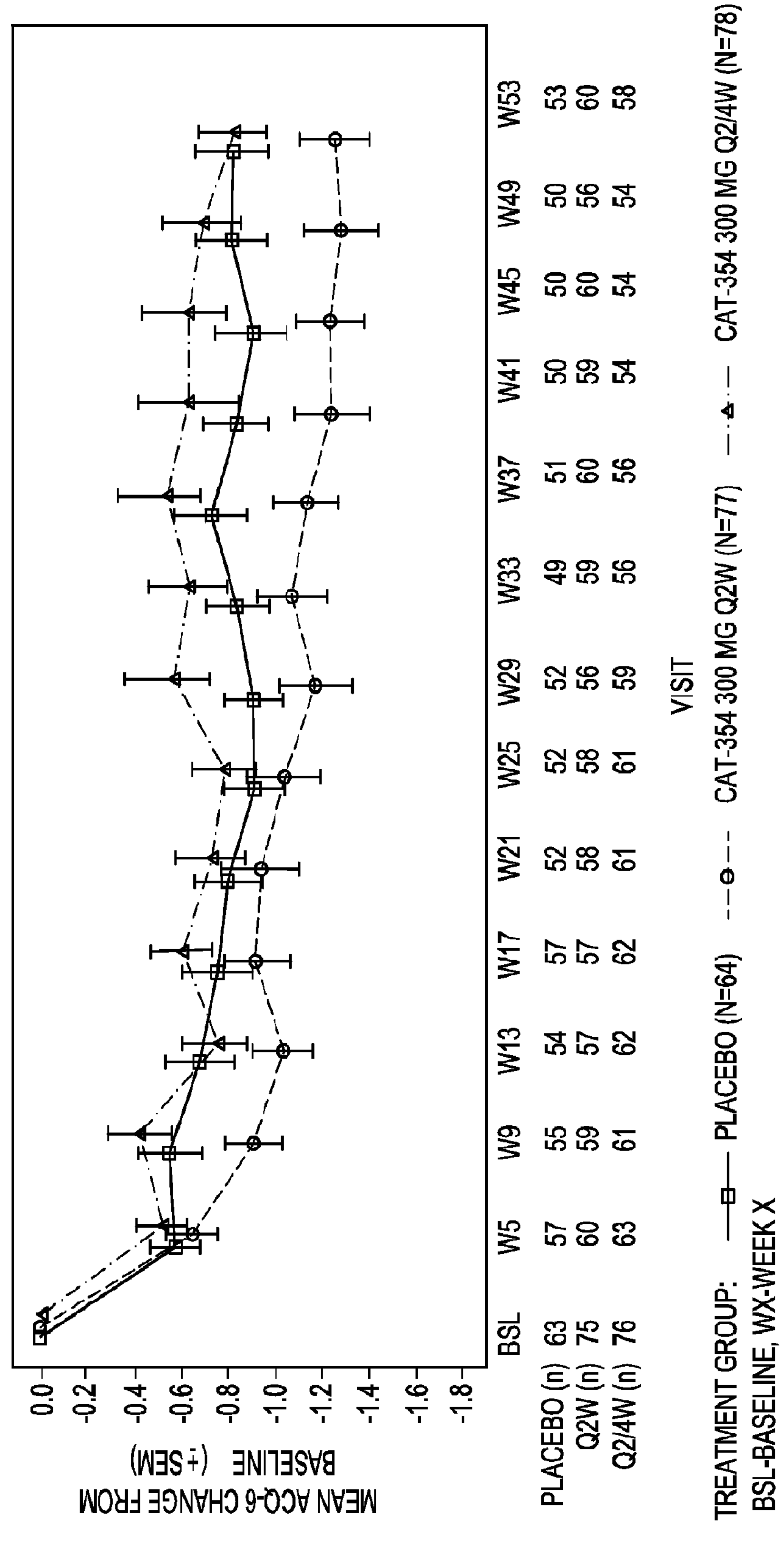

# FIG. 17D

Change from Baseline in Mean ACQ-6 Score Over Time by Treatment Group
Baseline DPP4 < Median ITT Population

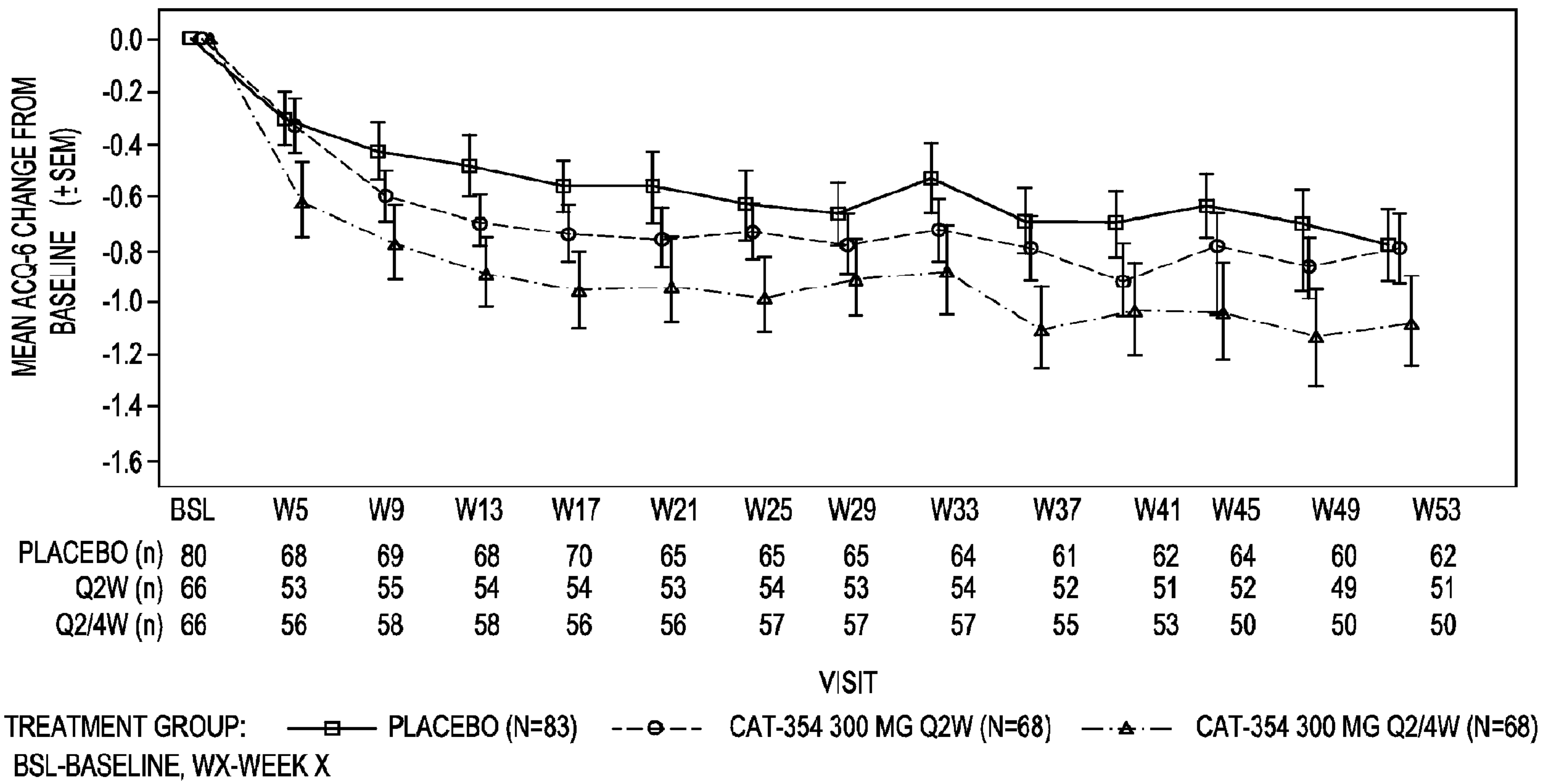

FIG. 17E

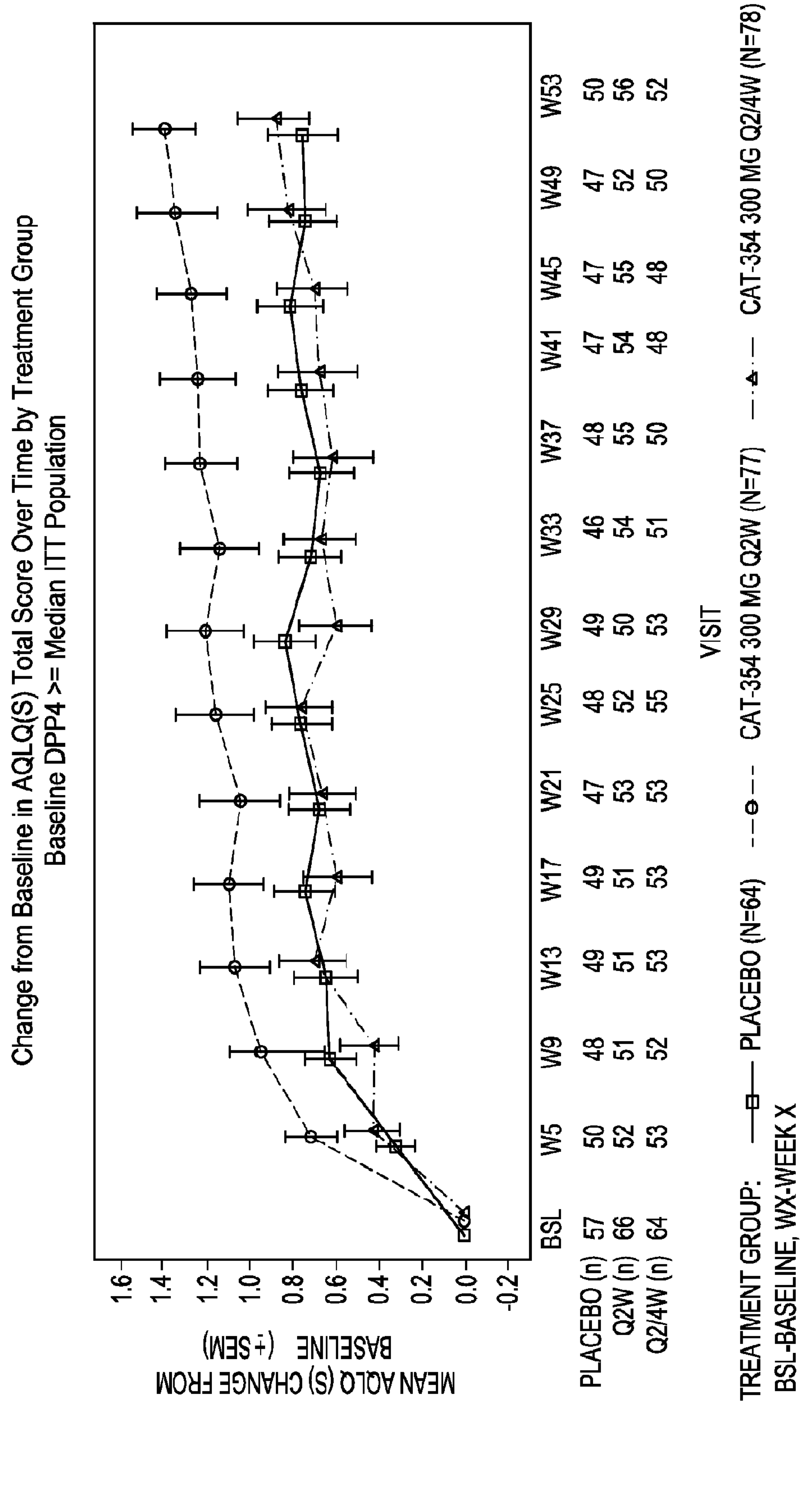
Change from Baseline in AQLQ(S) Total Score Over Time by Treatment Group
Baseline DPP4 >= Median ITT Population
MEAN AQLQ (S) CHANGE FROM BASELINE (+ SEM)
1.6
1.4
1.2
1.0
0.8
0.6
0.4
0.2
0.0
-0.2
BSL W5 W9 W13 W17 W21 W25 W29 W33 W37 W41 W45 W49 W53
PLACEBO (n) 57 50 48 49 49 47 48 49 46 48 47 47 47 50
Q2W (n) 66 52 51 51 51 53 52 50 54 55 54 55 52 56
Q2/4W (n) 64 53 52 53 53 53 55 53 51 50 48 48 50 52
VISIT
TREATMENT GROUP: PLACEBO (N=64) CAT-354 300 MG Q2W (N=77) CAT-354 300 MG Q2/4W (N=78)
BSL-BASELINE, WX-WEEK X

FIG. 17F

Change from Baseline in AQLQ(S) Total Score Over Time by Treatment Group
Baseline DPP4 < Median ITT Population

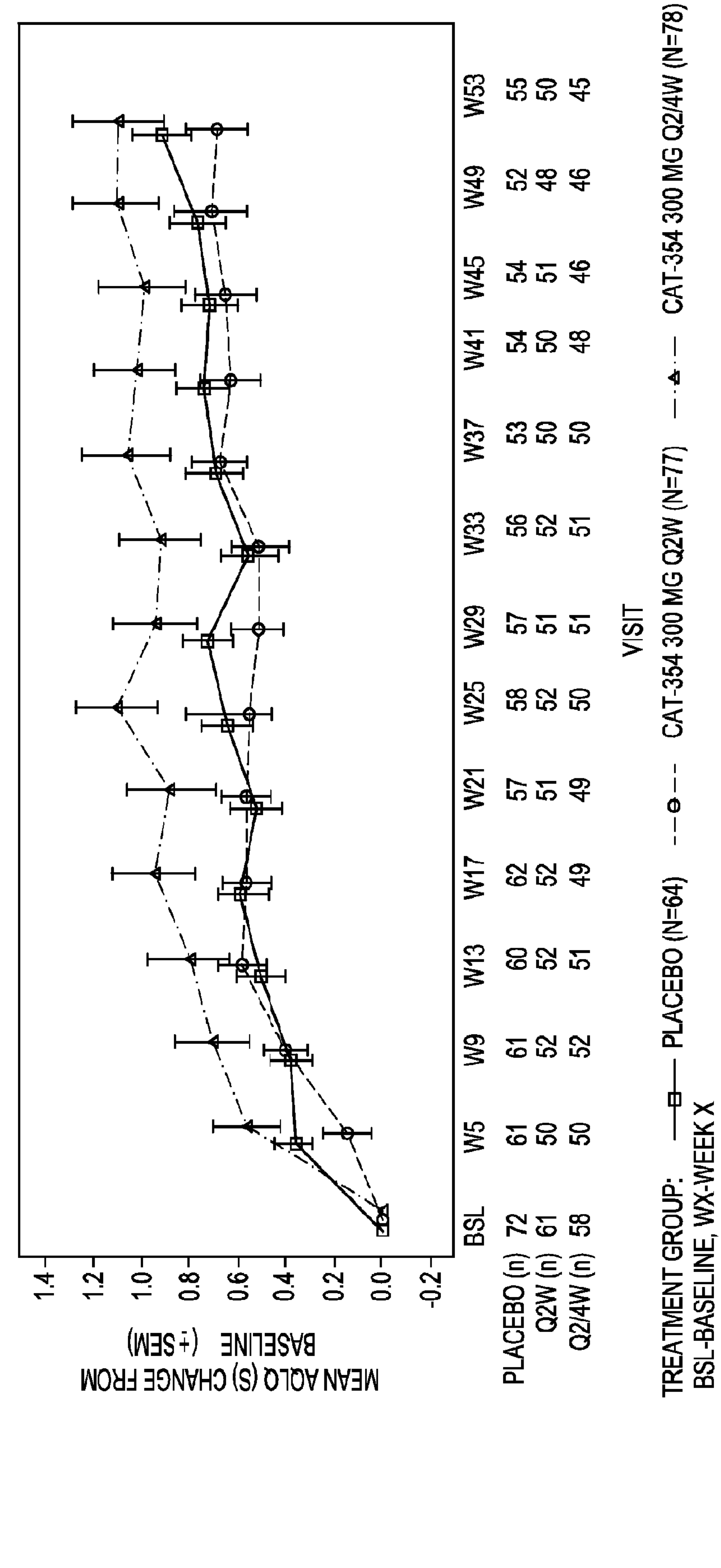

FIG. 17G

Percent Change from Baseline in Pre-Bronchodilator FEV1 Over Time By Treatment Group
Baseline FEV1 Reversibility >= 12% + Baseline DPP4 >= Median ITT Population

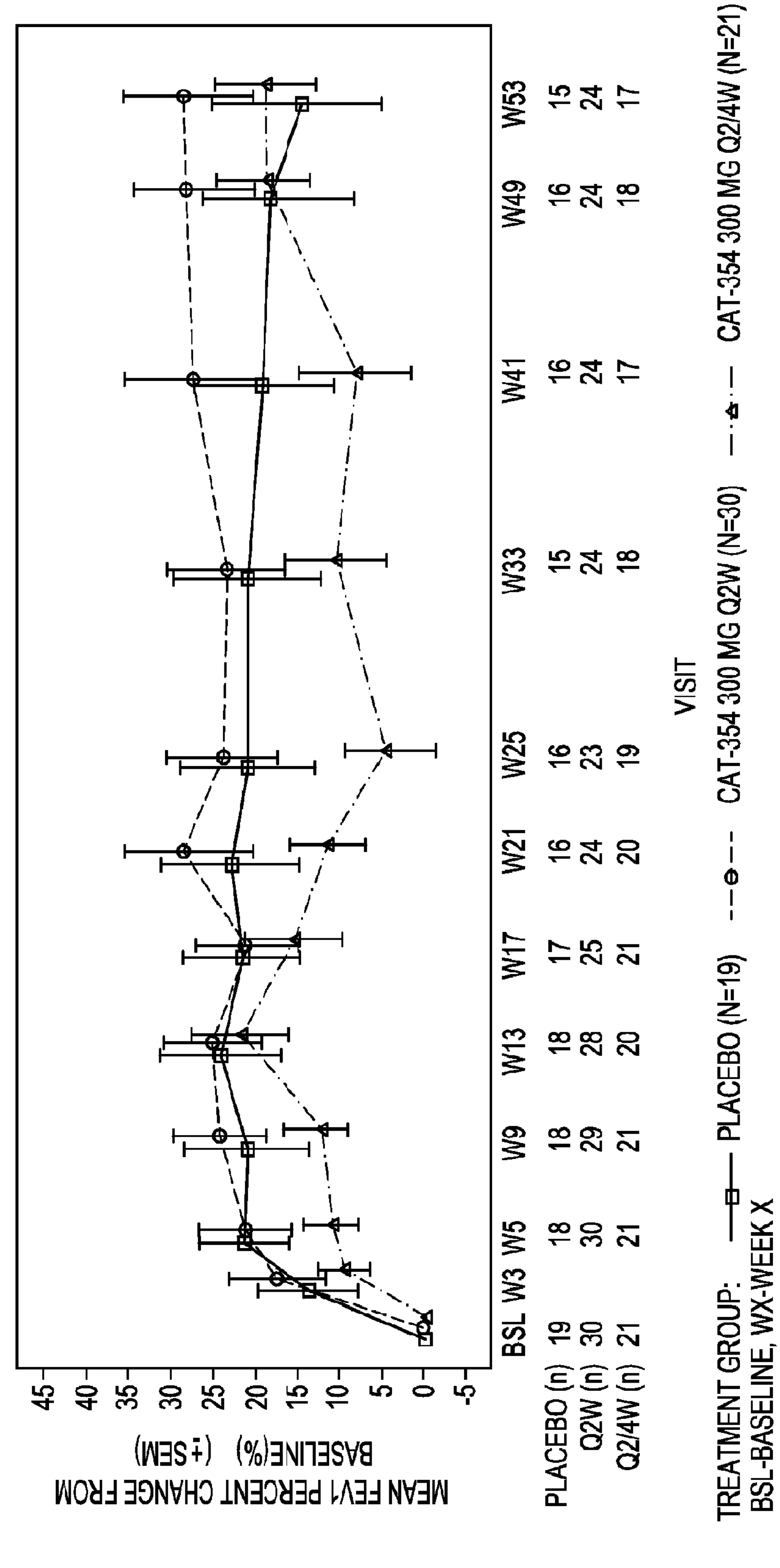

FIG. 17H

Change from Baselin in Mean ACQ-6 Score Over Time By Treatment Group
Baseline FEV1 Reversibility >= 12% + Baseline DPP4 >= Median ITT Population

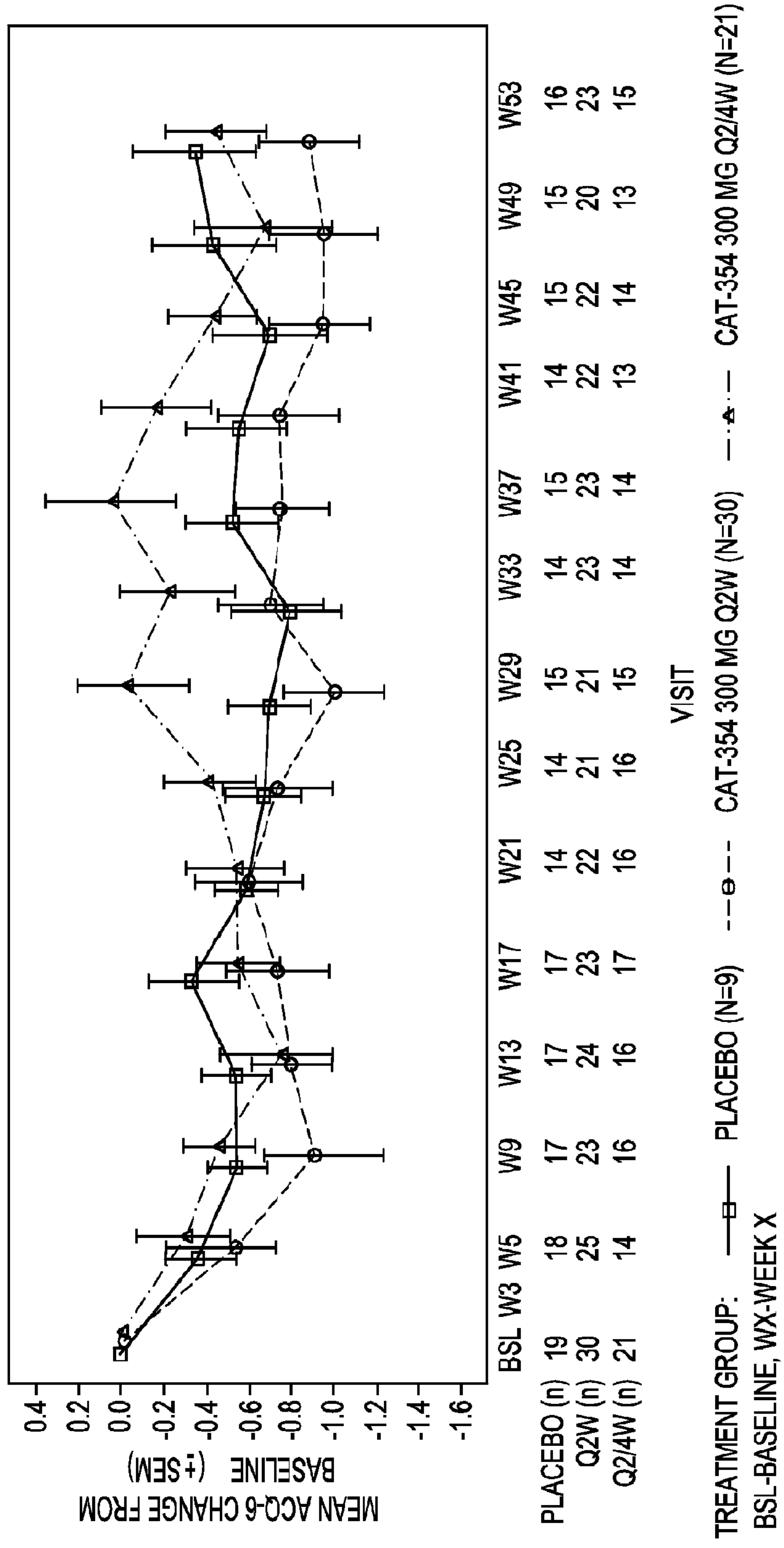

FIG. 17I

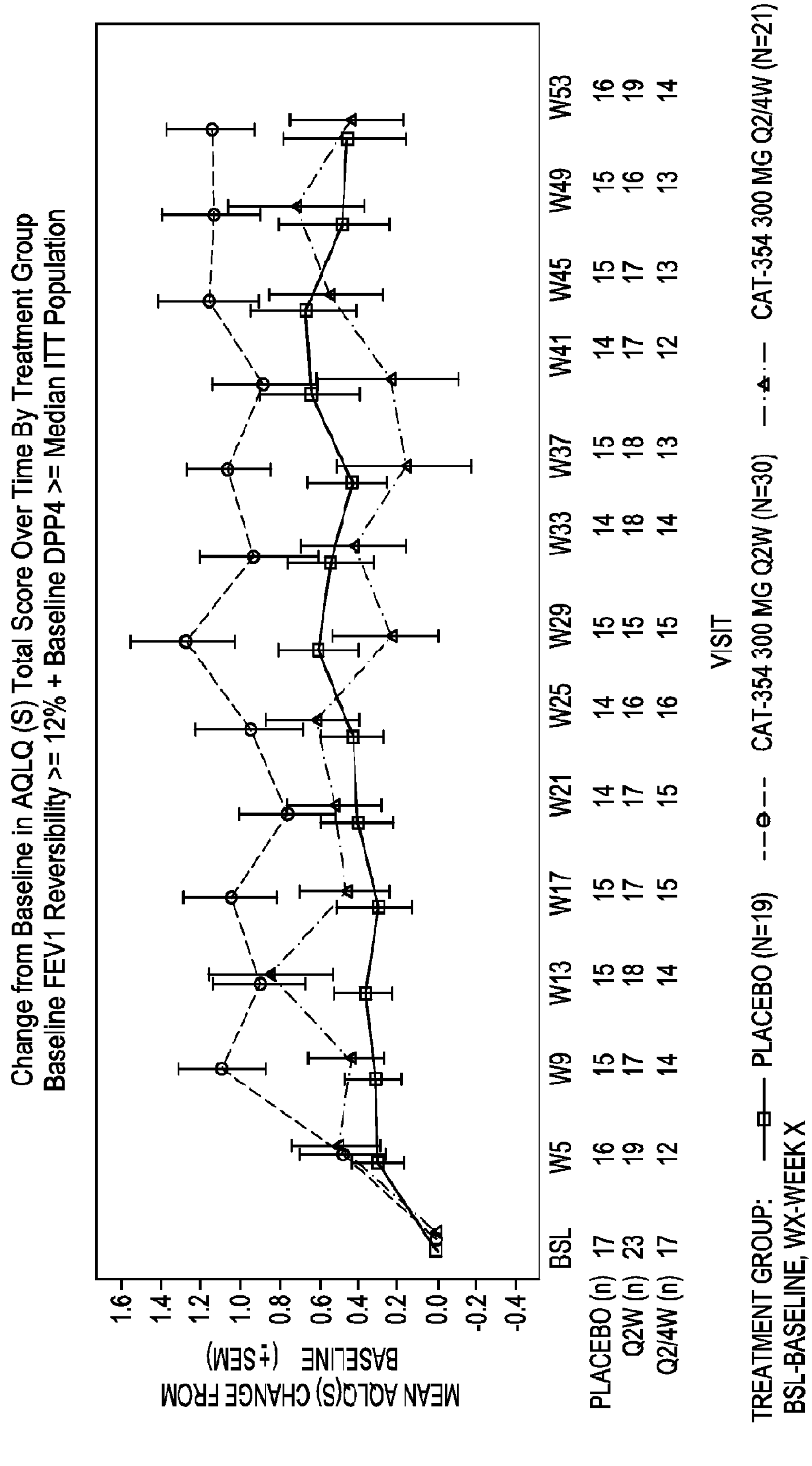
Change from Baseline in AQLQ (S) Total Score Over Time By Treatment Group
Baseline FEV1 Reversibility >= 12% + Baseline DPP4 >= Median ITT Population
MEAN AQLQ(S) CHANGE FROM BASELINE (± SEM)
1.6
1.4
1.2
1.0
0.8
0.6
0.4
0.2
0.0
-0.2
-0.4
BSL W5 W9 W13 W17 W21 W25 W29 W33 W37 W41 W45 W49 W53
PLACEBO (n) 17 16 15 15 15 14 14 15 14 15 14 15 15 16
Q2W (n) 23 19 17 18 17 17 16 15 18 18 17 17 16 19
Q2/4W (n) 17 12 14 14 15 15 16 15 14 13 12 13 13 14
VISIT
TREATMENT GROUP: PLACEBO (N=19) CAT-354 300 MG Q2W (N=30) CAT-354 300 MG Q2/4W (N=21)
BSL-BASELINE, WX-WEEK X

FIG. 18

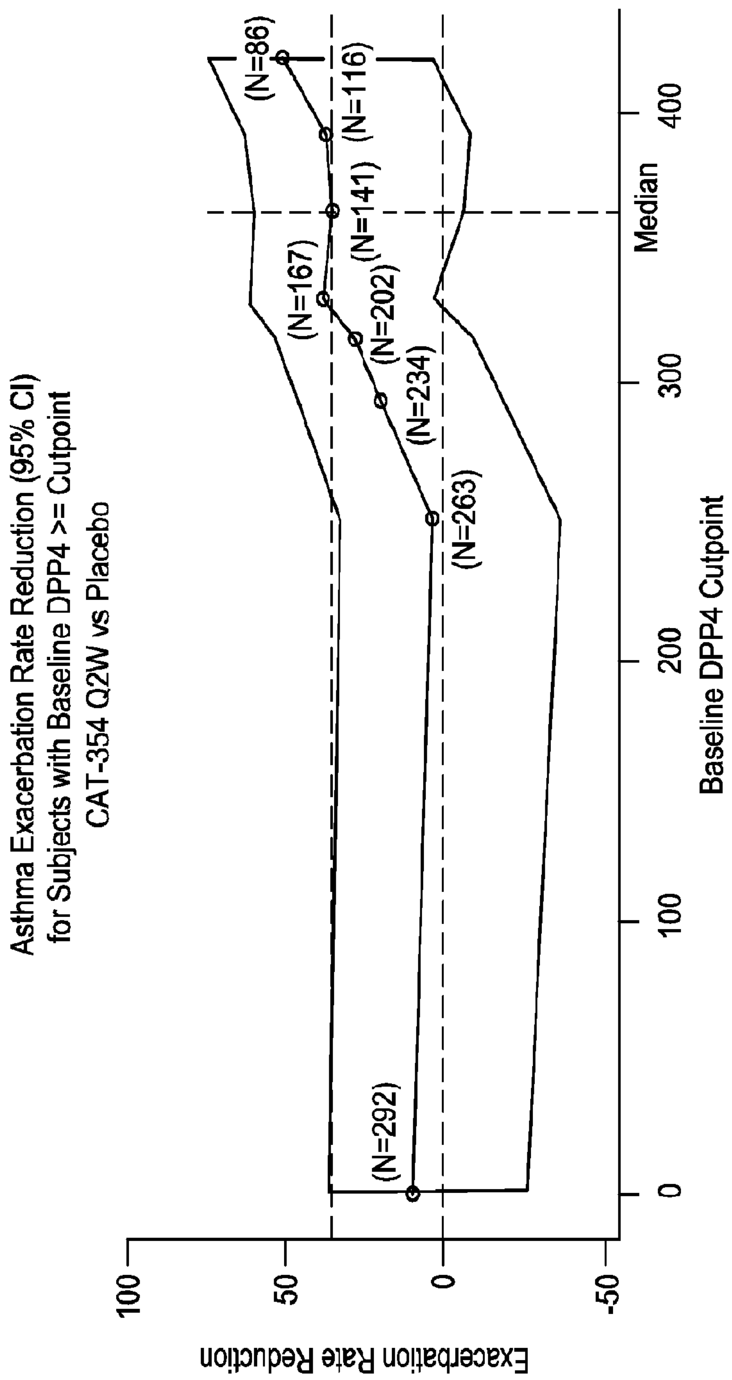
Asthma Exacerbation Rate Reduction (95% CI)
for Subjects with Baseline DPP4 >= Cutpoint
CAT-354 Q2W vs Placebo
Exacerbation Rate Reduction
100
50
0
-50
Baseline DPP4 Cutpoint
0
100
200
300
400
Median
(N=292)
(N=263)
(N=234)
(N=202)
(N=167)
(N=141)
(N=116)
(N=86)

FIG. 19

Mean Percent Change from Baseline in Pre-BD FEV1 at Week 53
(95% CI) for subjects with Baseline DPP4 >= Cutpoint
CAT-354 Q2W vs Placebo

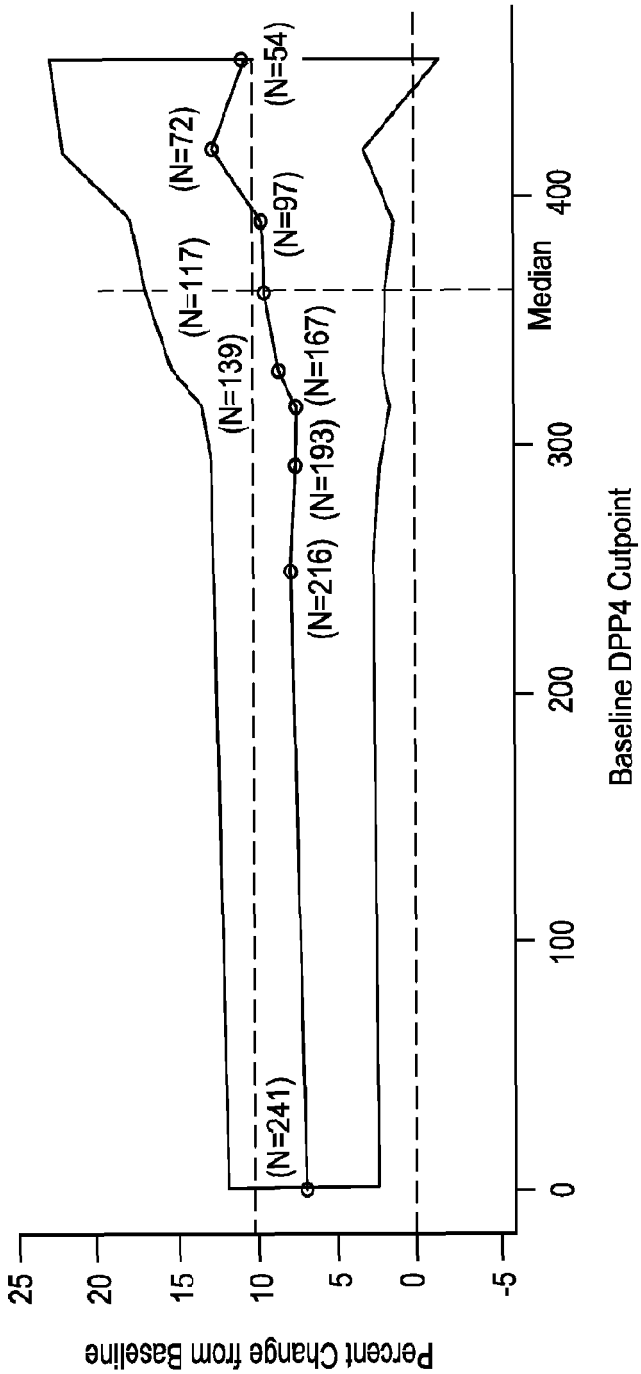

FIG. 20

Mean Change from Baseline in Mean ACQ-6 Score at Week 53
(95% CI) for Subjects with Baseline DPP4 >= Cutpoint
CAT-354 Q2W vs Placebo

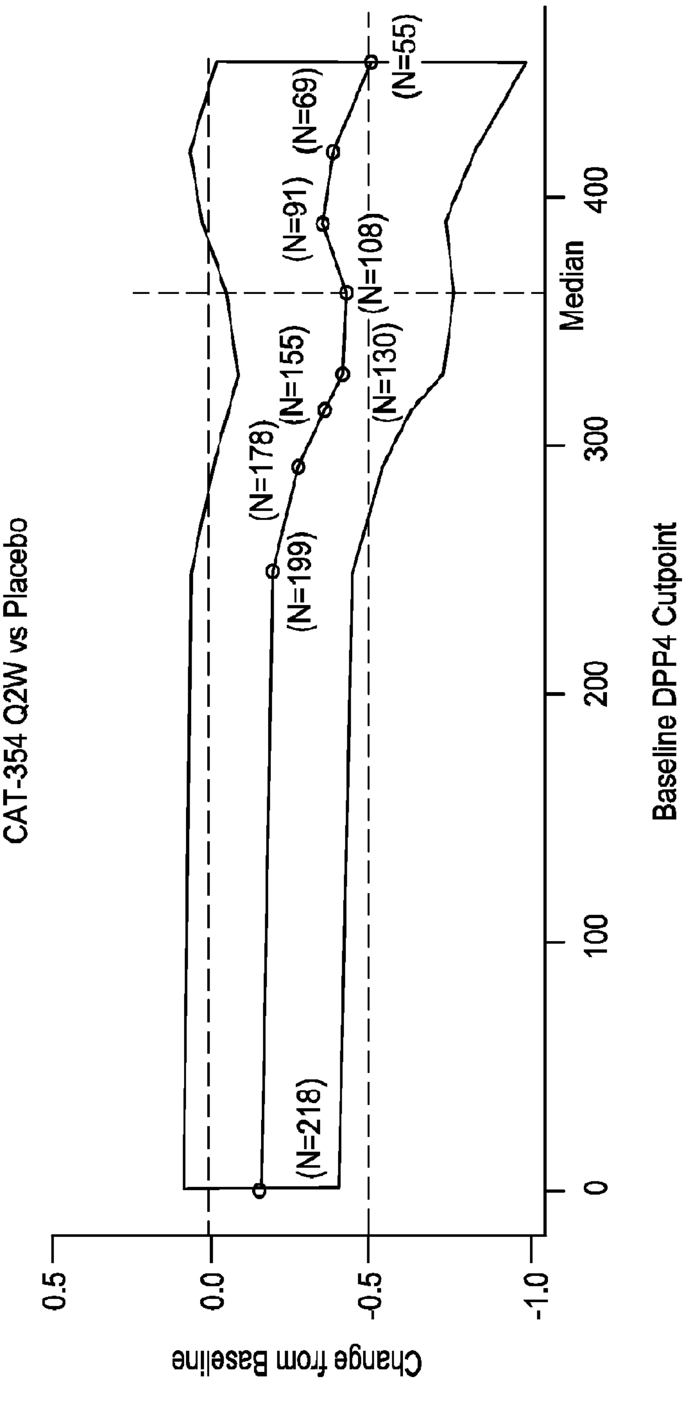

# FIG. 21

Partial Overlap between Periostin-High and DPP4-High

| | | Frequency Percent | DPP4 Low 1 | DPP4 High 2 | Total |
|---|---|---|---|---|---|
| Periostin | Low | 1 | 126<br>28.06 | 99<br>22.05 | 225<br>50.11 |
| | High | 2 | 99<br>22.05 | 125<br>27.84 | 224<br>49.89 |
| | | Total | 225<br>50.11 | 224<br>49.89 | 449<br>100.00 |

Frequency Missing = 3

# FIG. 22

Partial Overlap between Th2-High and DPP4-High

| | | DPP4 Low | DPP4 High | |
|---|---|---|---|---|
| | Frequency<br>Percent | 1 | 2 | Total |
| Th2 Low | 1 | 112<br>27.18 | 91<br>22.09 | 203<br>49.27 |
| Th2 High | 2 | 95<br>23.06 | 114<br>27.67 | 209<br>50.73 |
| | Total | 207<br>50.24 | 205<br>49.76 | 412<br>100.00 |

Frequency Missing = 40

# FIG. 23

Partial Overlap between DDP- 4 High and Eos-High

DPP4

| | | Frequency<br>Percent | Low<br>1 | High<br>2 | Total |
|---|---|---|---|---|---|
| Eos | < 300 | 1 | 134<br>31.68 | 127<br>30.02 | 261<br>61.70 |
| | >= 300 | 2 | 78<br>18.44 | 84<br>19.86 | 162<br>38.30 |
| | | Total | 212<br>50.12 | 211<br>49.88 | 423<br>100.00 |

Frequency Missing = 29

# FIG. 24

DPP4 Levels Reduced in OCS-treated Subjects

| CHRONIC OCS USE | N Obs | Mean | Median | Minimum | Maximum | Std Dev |
|---|---|---|---|---|---|---|
| NEGATIVE | 373 | 380.9463807 | 371.0000000 | 134.0000000 | 905.0000000 | 105.6377830 |
| POSITIVE | 77 | 335.7922078 | 321.0000000 | 169.0000000 | 540.0000000 | 92.9282884 |

# FIG. 25

DPP4 <u>and</u> $FEV_1$ reversibility <u>and</u> No Chronic OCS Use

| BL $FEV_1$ reversibility | BL DPP4 | N for AERR (placebo/ CAT-354) | Exacerbation rate (placebo/ CAT-354) | AERR Adjusted for Covariates | Δ % change from BL $FEV_1$ vs. Pbo | Δ mean change from BL ACQ-6 vs. pbo | Δ mean change from BL AQLQ vs. pbo |
|---|---|---|---|---|---|---|---|
| -- | -- | (124/124) | 0.80/0.67 | 21% (-19, 47) | 6.59% (1.7, 11.4) | -0.11 (-0.37, 0.16) | 0.18 (-0.11, 0.46) |
| ≥ 12% at BL | -- | (48/33) | 0.87/0.56 | 46% (-18, 75) | 10.72% (-0.0, 21.4) | -0.50 (-1.02, 0.03) | 0.66 (0.07, 1.25) |
| | ≥ median | (15/24) | 1.25/0.63 | 57% (-30, 86) | 20.3% (-1.2, 39.5) | -0.89 (-1.63, -0.14) | 1.26 (0.48, 2.04) |
| | < median | (32/8) | 0.72/0.41 | -7% (-886, 88) | -0.9% (-15.4, 13.5) | -0.43 (-1.41, 0.56) | 0.24 (-0.87, 1.35) |
| < 12% at BL | -- | (73/85) | 0.76/0.69 | 21% (-38, 55) | 6.97% (2.0, 11.9) | 0.06 (-0.27, 0.39) | -0.05 (-0.40, 0.31) |
| | ≥ median | (36/42) | 0.80/0.49 | 41% (-115, 70) | 7.01% (0.1, 13.9) | -0.36 (-0.85, 0.13) | 0.37 (-0.18, 0.92) |
| | < median | (34/41) | 0.75/0.93 | -14% (-256, 50) | 8.49 (1.4, 15.6) | 0.39 (-0.07, 0.82) | -0.44 (-0.88, 0.01) |
| -- | ≥ median | (52/68) | 0.94/0.54 | 35% (-7, 61) | 11.06% (3.6, 18.6) | -0.48 (-0.87, -0.09) | 0.68 (0.26, 1.09) |
| -- | < median | (68/51) | 0.72/0.81 | -18% (-120, 37) | 4.79% (-3.67, 8.4) | 0.21 (-0.16, 0.58) | -0.29 (-0.68, 0.11) |

95% CI in parentheses

# FIG. 26

Periostin and $FEV_1$ reversibility and No Chronic OCS Use

| BL $FEV_1$ reversibility | BL Periostin | N for AERR (placebo/ CAT-354) | Exacerbation rate (placebo/ CAT-354) | AERR Adjusted for Covariates | AERR Unadjusted | Δ % change from BL $FEV_1$ vs. pbo | Δ mean change from BL ACQ-6 vs. pbo | Δ mean change from BL AQLQ vs. pbo |
|---|---|---|---|---|---|---|---|---|
| -- | -- | (124/124) | 0.80/0.67 | 21% (-19, 47) | 16% | 6.59% (1.7, 11.4) | -0.11 (-0.37, 0.16) | 0.18 (-0.11, 0.46) |
| ≥ 12% at BL | -- | (48/33) | 0.87/0.56 | 46% (-18, 75) | 37% | 10.72% (-0.0, 21.4) | -0.50 (-1.02, 0.03) | 0.66 (0.07, 1.25) |
| | ≥ median | (22/18) | 1.22/0.59 | 67% (2, 89) | 52% | 14.7% (-0.2, 29.5) | -0.68 (-1.31, -0.06) | 0.64 (-0.11, 1.39) |
| | < median | (26/15) | 0.58/0.52 | -32% (-273, 53) | 10% | 8.00% (-7.0, 23.0) | -0.23 (-1.10, 0.64) | 0.71 (-0.23, 1.65) |
| < 12% at BL | -- | (73/85) | 0.76/0.69 | 21% (-38, 55) | 9% | 6.97% (2.0, 11.9) | 0.06 (-0.27, 0.39) | -0.05 (-0.40, 0.31) |
| | ≥ median | (34/50) | 0.96/0.72 | 49% (-8, 76) | 25% | 4.10% (-3.2, 11.4) | -0.14 (-0.59, 0.31) | -0.04 (-0.53, 0.46) |
| | < median | (38/35) | 0.59/0.64 | -1% (-142, 59) | -8% | 8.61 (1.8, 15.4) | 0.42 (-0.07, 0.91) | -0.13 (-0.64, 0.39) |
| -- | ≥ median | (58/69) | 1.04/0.69 | 41% (-2, 66) | 34% | 6.39% (-0.6, 13.4) | -0.26 (-0.61, 0.09) | 0.19 (-0.21, 0.58) |
| -- | < median | (65/55) | 0.59/0.64 | -7% (-97, 42) | -8% | 6.46% (-0.4, 13.3) | 0.24 (-0.17, 0.65) | 0.12 (-0.31, 0.56) |

95% CI in parentheses

FIG. 27A

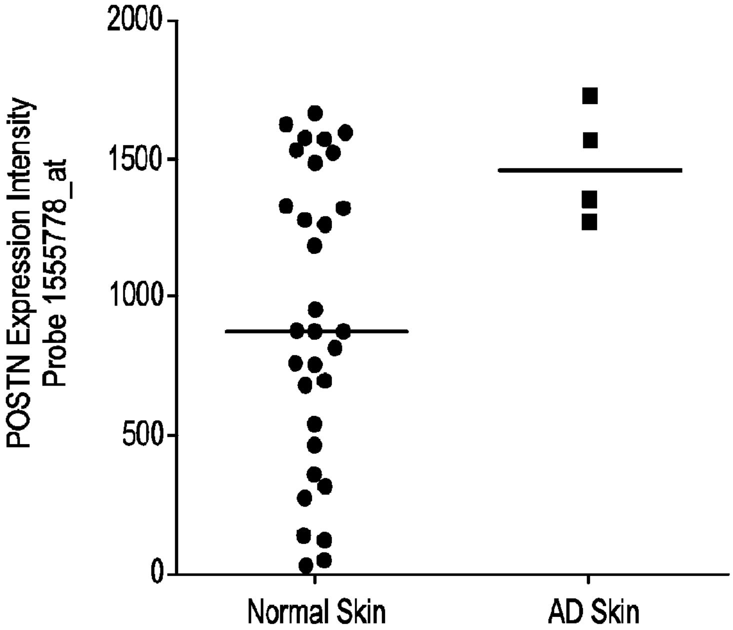


FIG. 27B

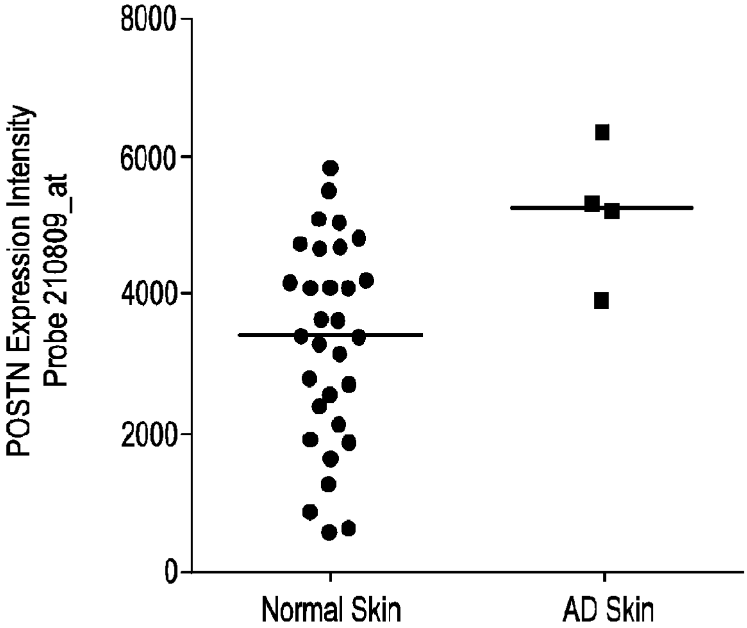

FIG. 28A

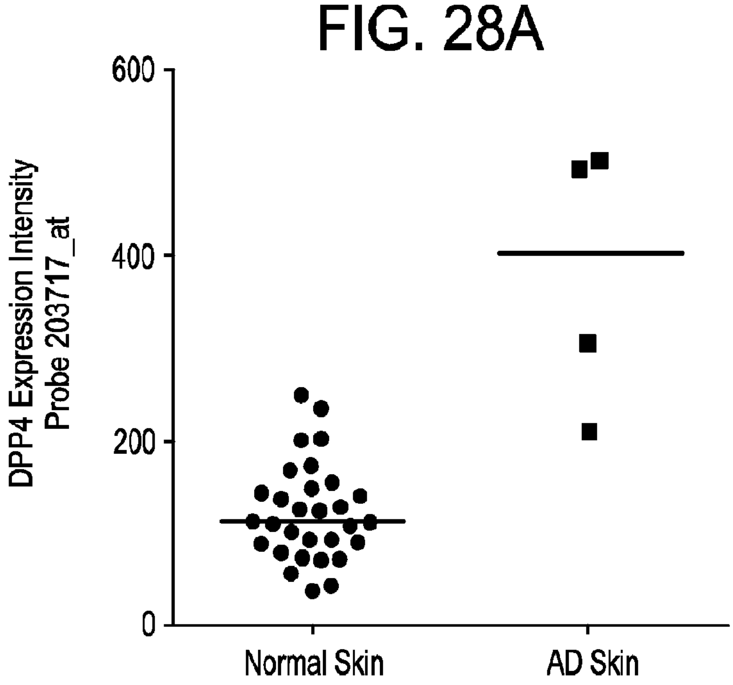


FIG. 28B

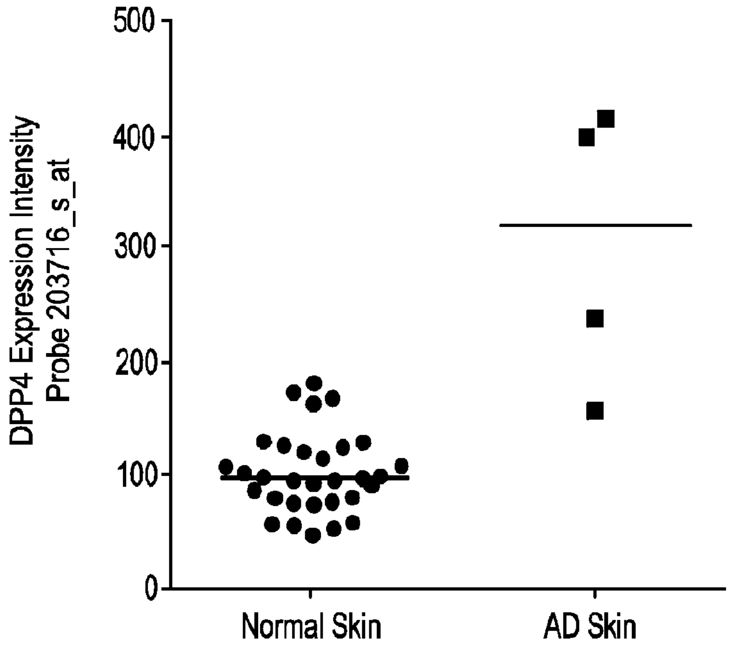

FIG. 29A

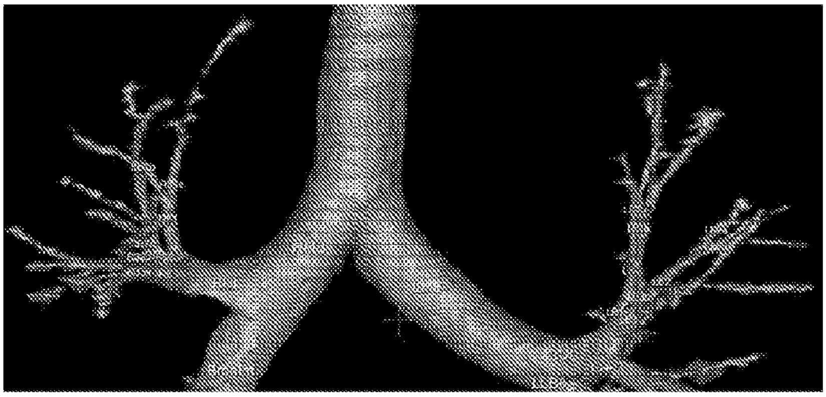

E13024010332 Visit 4

FIG. 29B

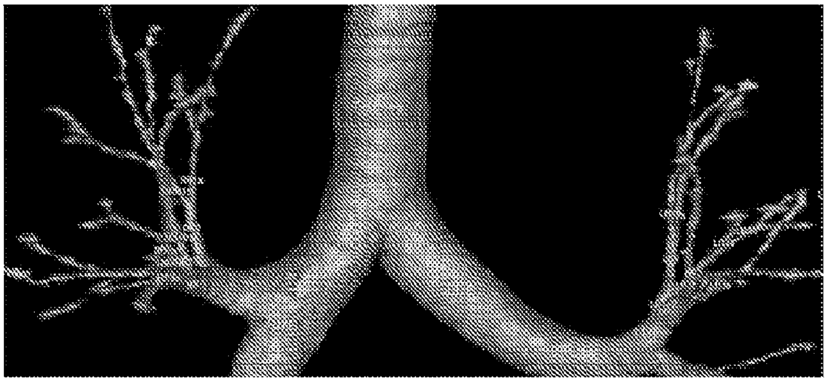

E13024010332 Visit 30

FIG. 30A

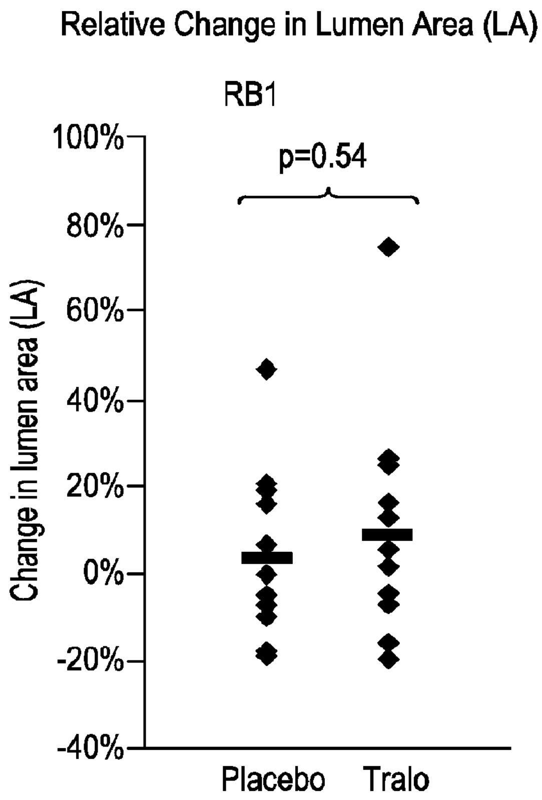


FIG. 30B

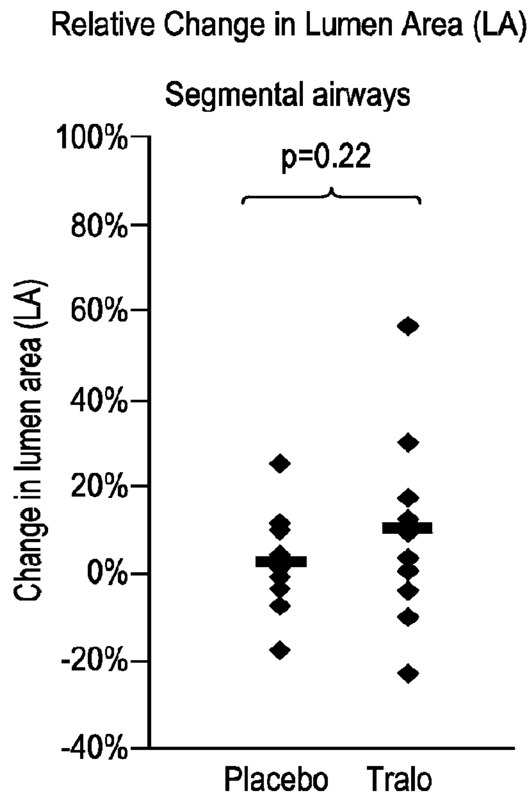


FIG. 30C

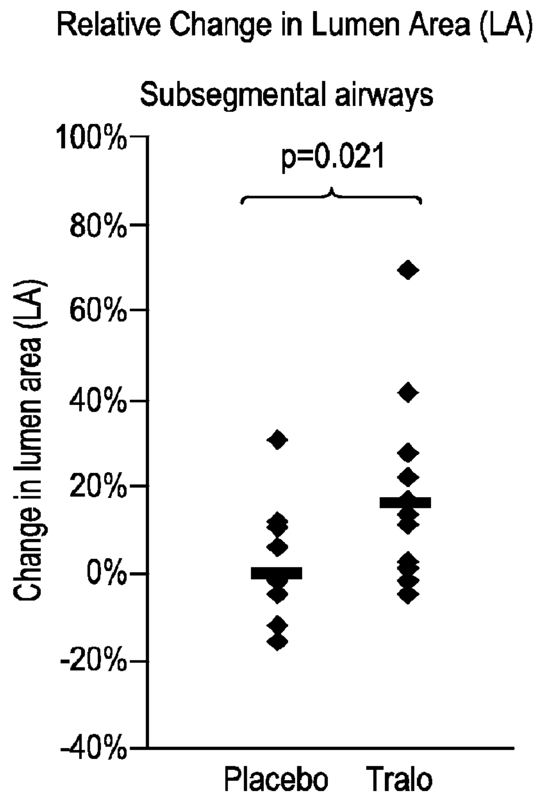

FIG. 31A

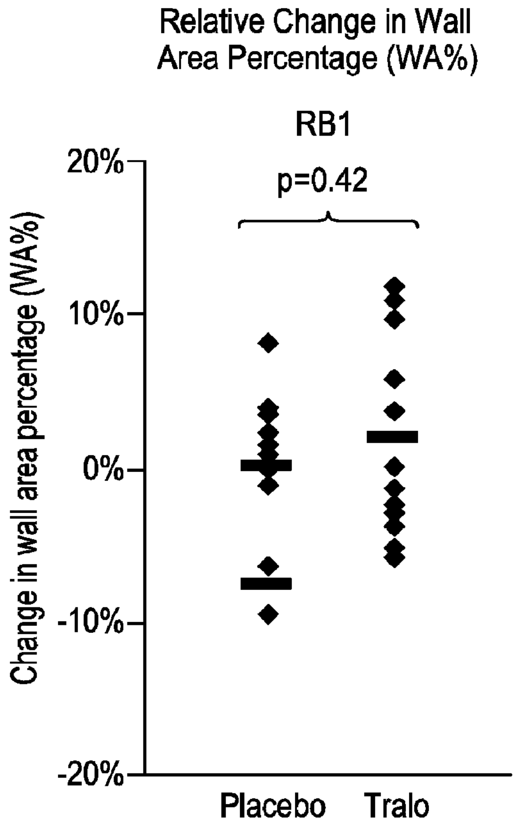


FIG. 31B

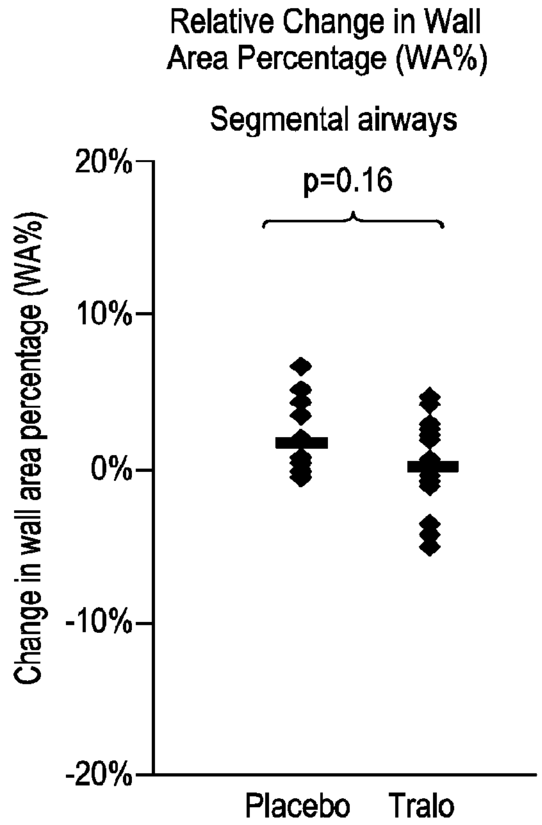


FIG. 31C

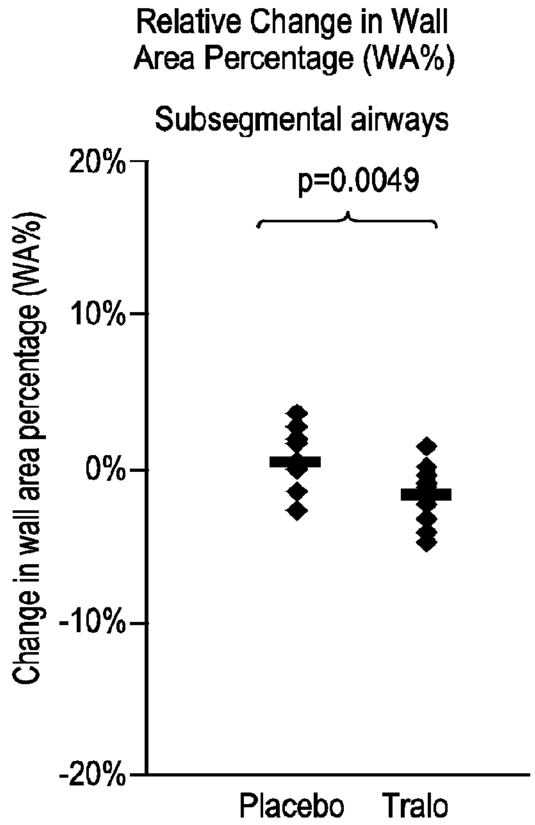

FIG. 32A

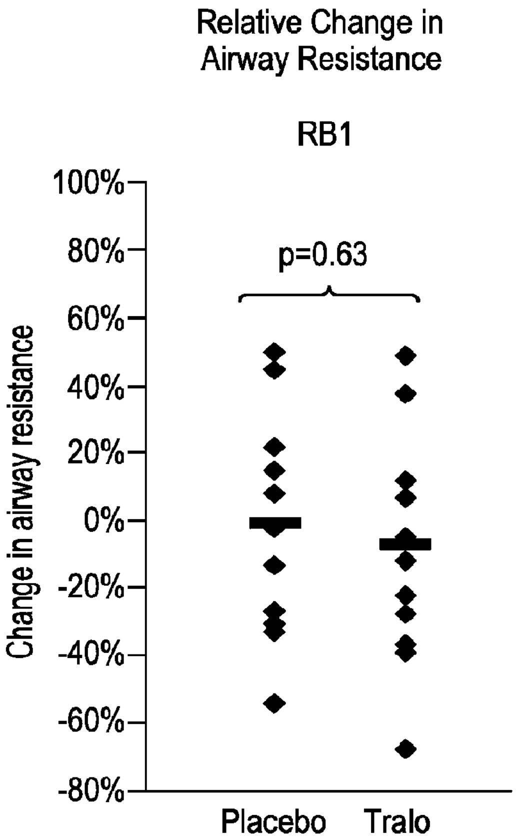


FIG. 32B

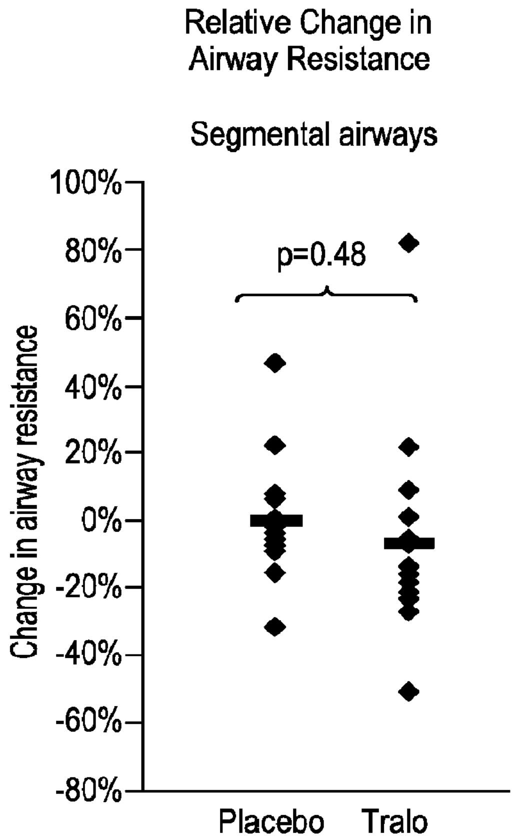


FIG. 32C

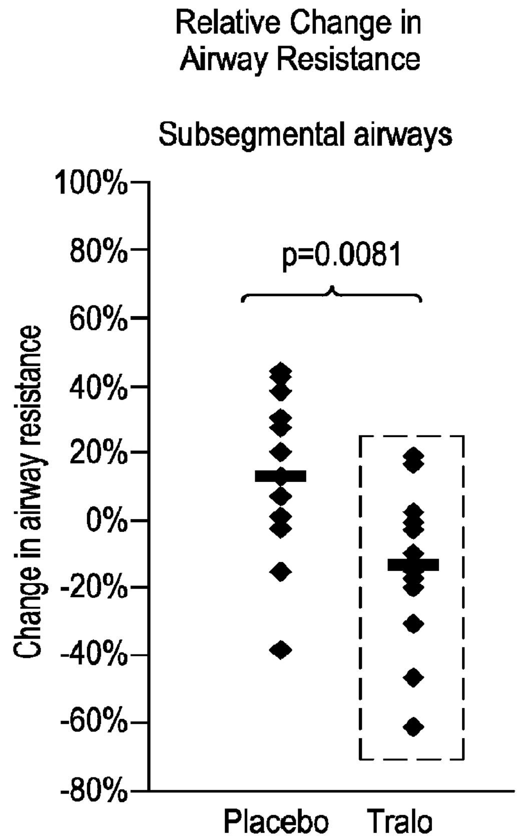

# FIG. 33A

Relative Change in Airway Resistance (Calculated)
and FEV1: Effect of Tralokinumab in different sub-groups
*WA% at subsegmental level

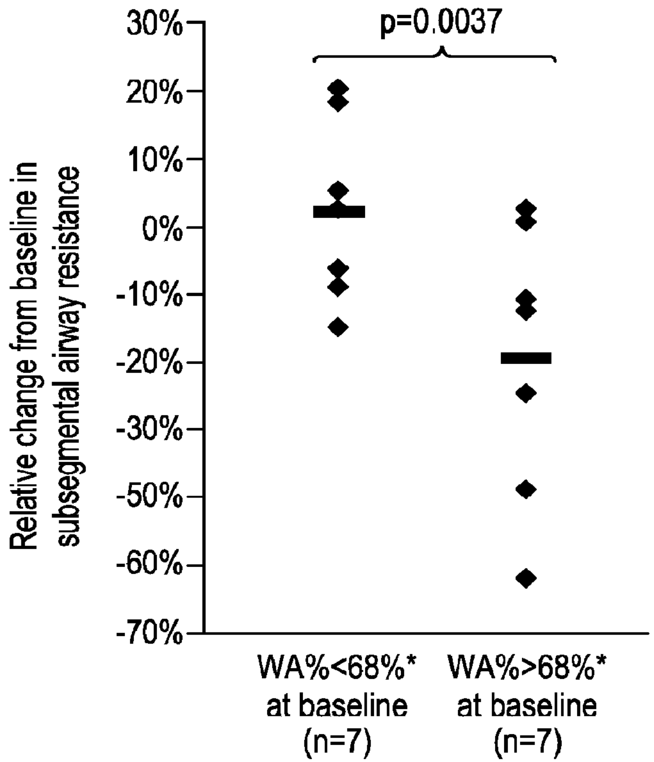


# FIG. 33B

Relative Change in Airway Resistance (Calculated)
and FEV1: Effect of Tralokinumab in different sub-groups
*WA% at subsegmental level

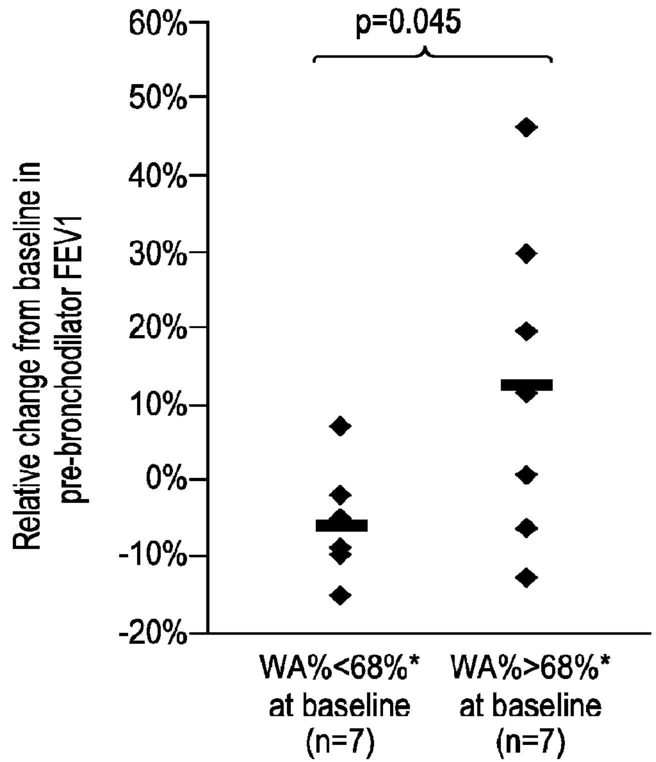

FIG. 34A

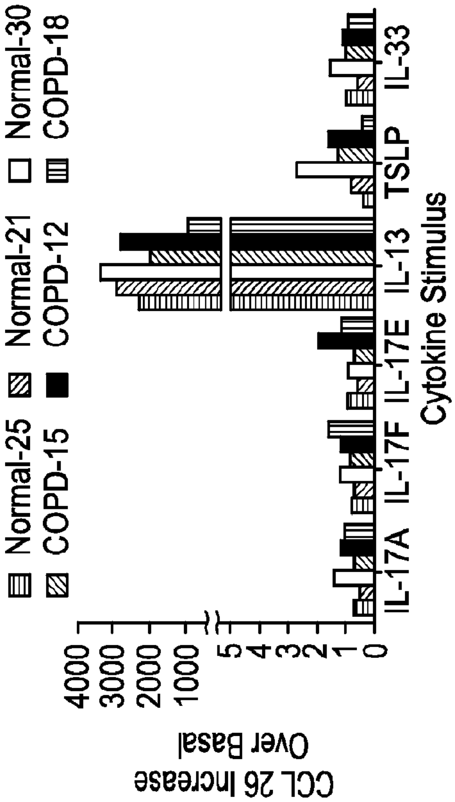


FIG. 34B

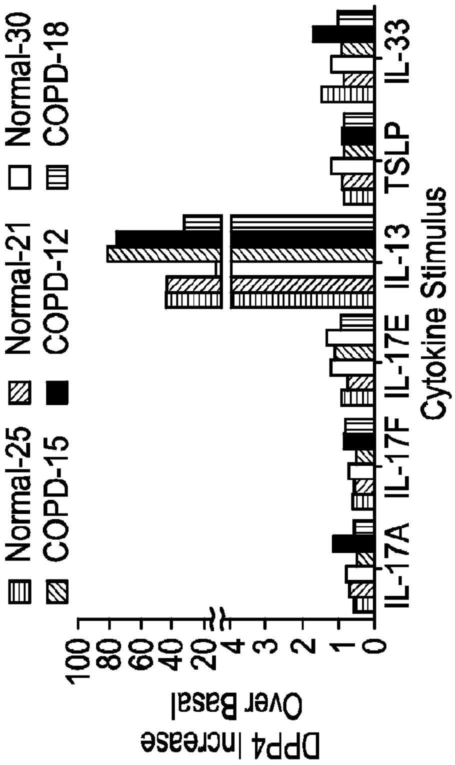


FIG. 34C

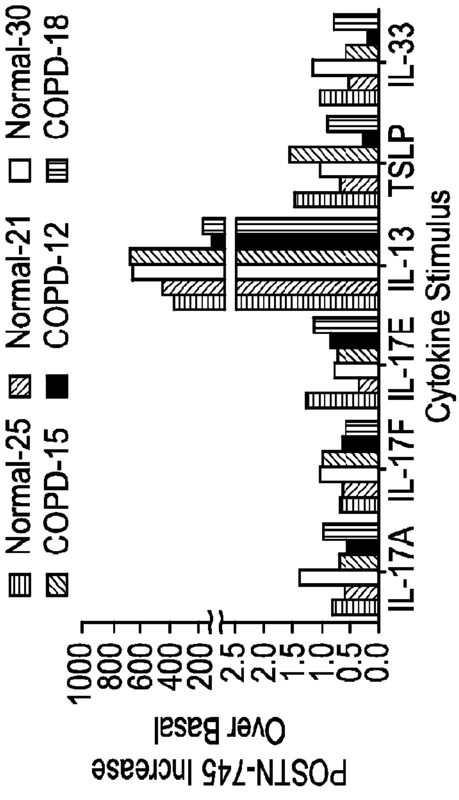


FIG. 34D

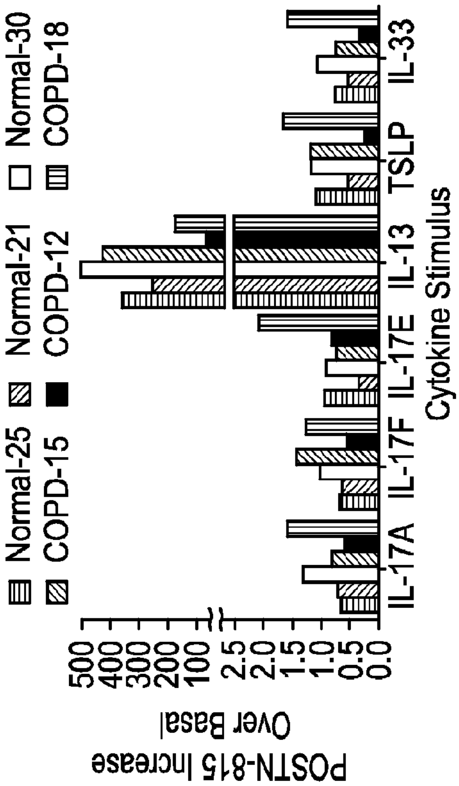

FIG. 35A

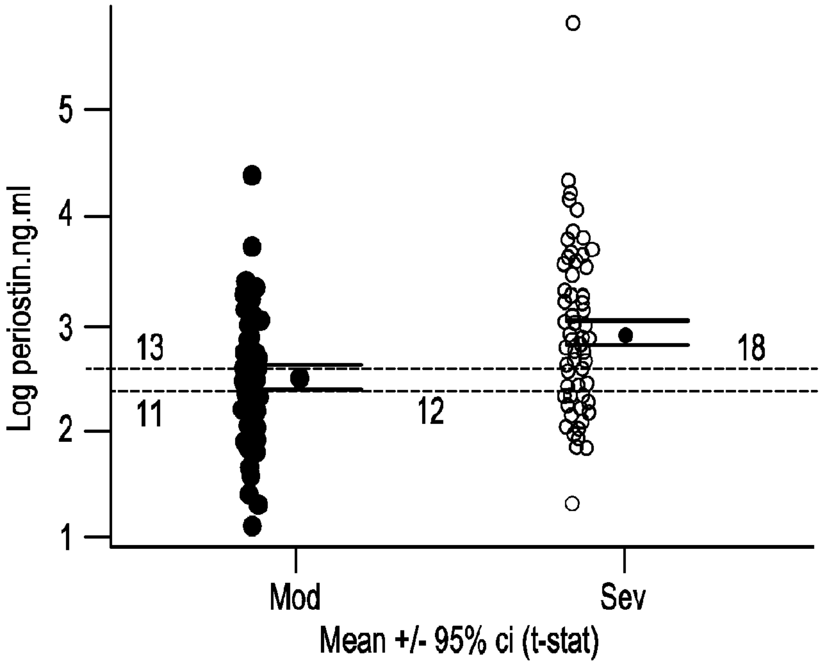


FIG. 35B

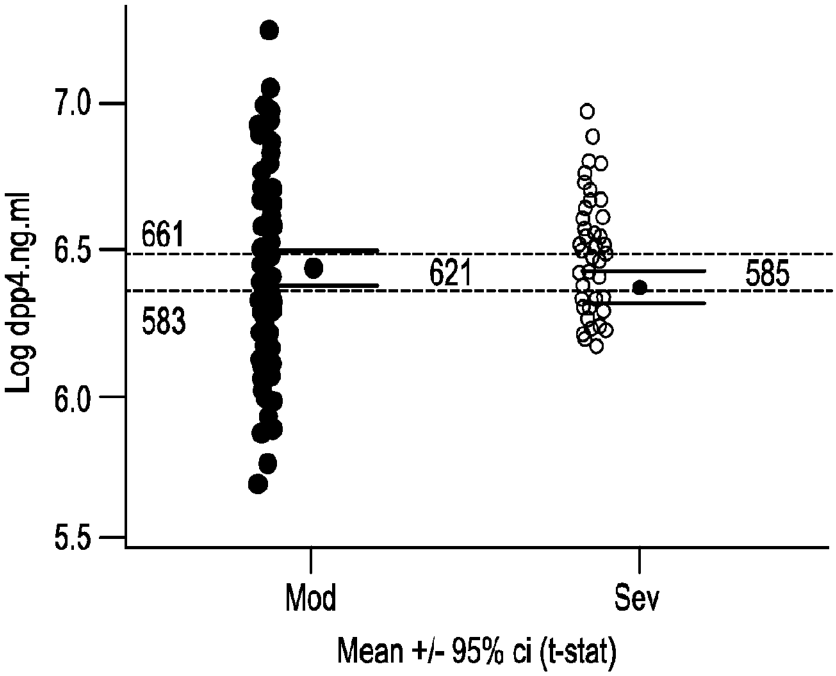

FIG. 36

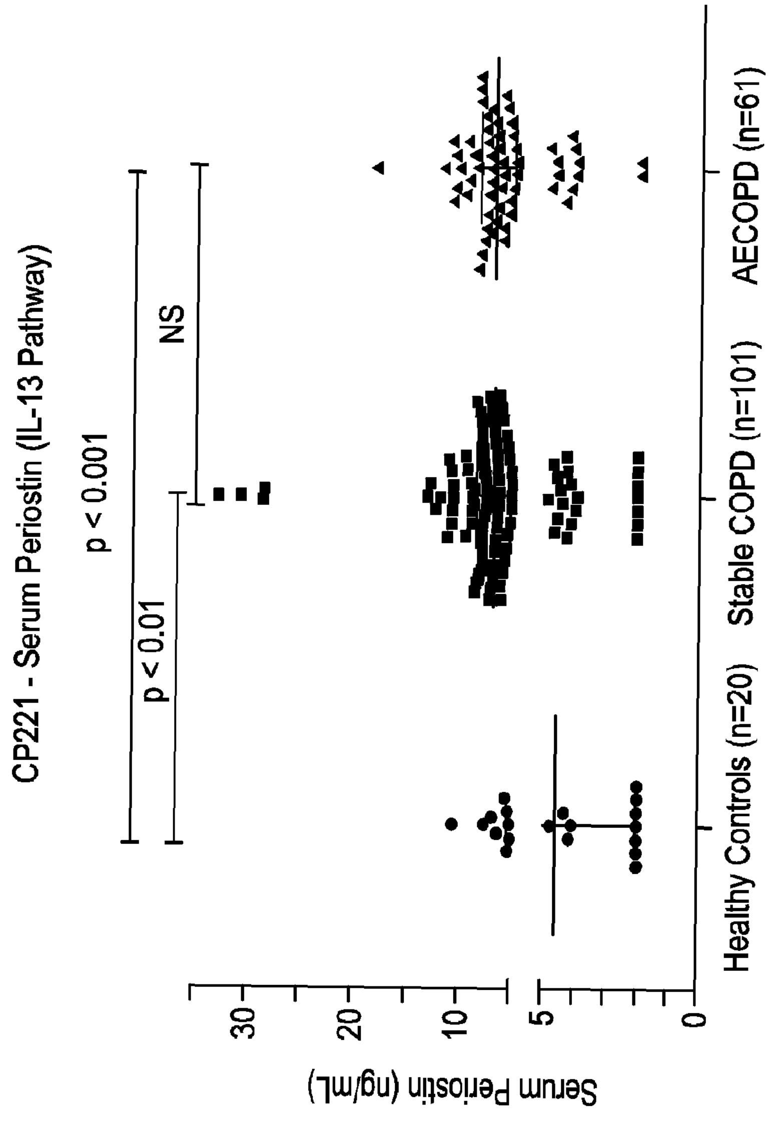

CP221 - Serum Periostin (IL-13 Pathway)
p < 0.001
p < 0.01
NS
Serum Periostin (ng/mL)
30
20
10
5
0
Healthy Controls (n=20)
Stable COPD (n=101)
AECOPD (n=61)

# FIG. 37

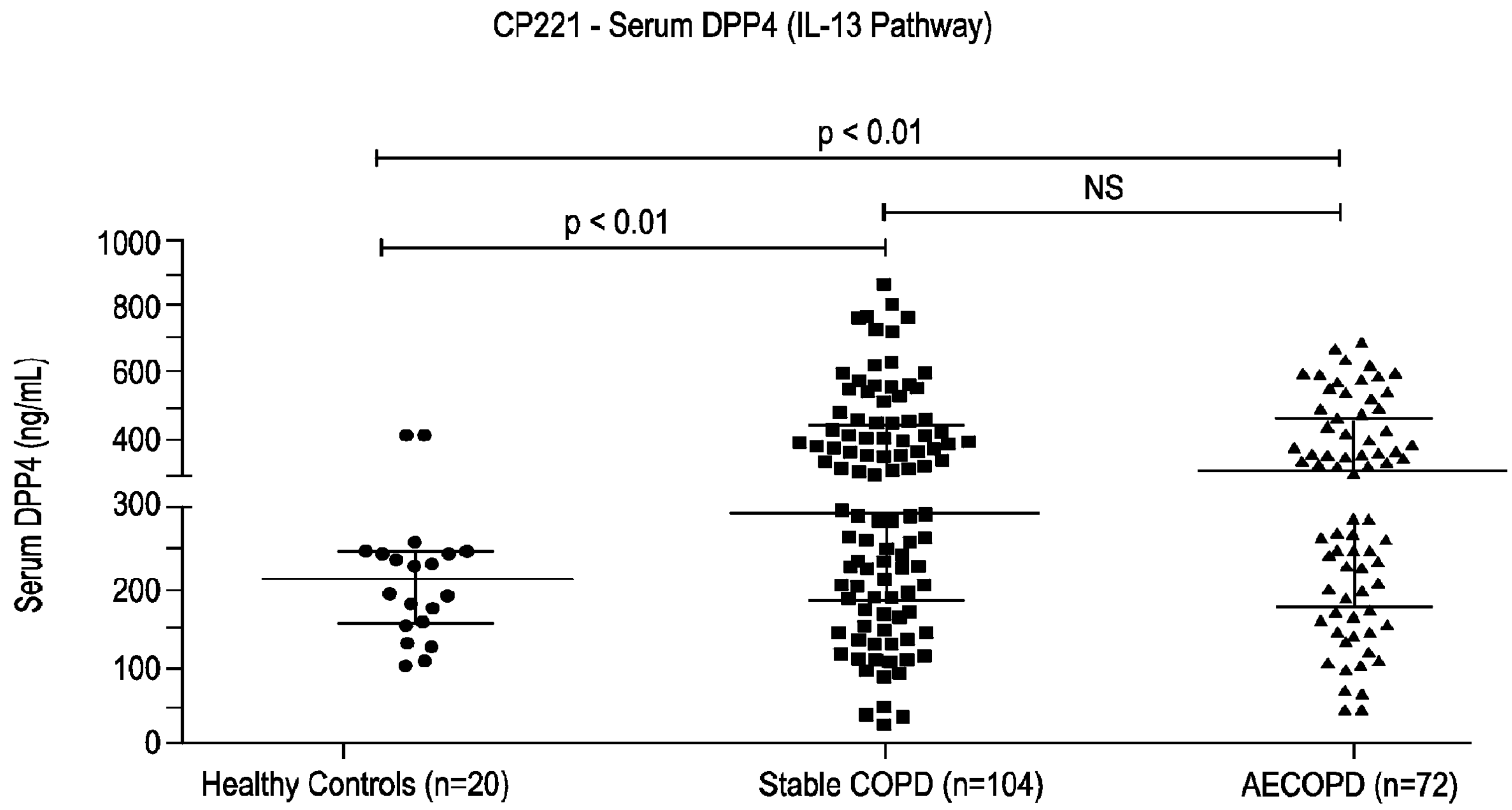
CP221 - Serum DPP4 (IL-13 Pathway)
p < 0.01
NS
p < 0.01
Serum DPP4 (ng/mL)
1000
800
600
400
300
200
100
0
Healthy Controls (n=20)
Stable COPD (n=104)
AECOPD (n=72)

Europäisches Patentamt
European Patent Office
Office européen des brevets

# EUROPEAN SEARCH REPORT

Application Number
EP 20 15 8832

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | US 2012/052060 A1 (MONK PHILLIP DAVID [GB] ET AL) 1 March 2012 (2012-03-01)<br>* the whole document *<br>----- | 1-7 | INV.<br>A61K39/395<br>G01N33/573<br>G01N33/53 |
| Y | WOOD S H ET AL: "Gene expression in canine atopic dermatitis and correlation with clinical severity scores", JOURNAL OF DERMATOLOGICAL SCIENCE, ELSEVIER, AMSTERDAM, NL, vol. 55, no. 1, 1 July 2009 (2009-07-01), pages 27-33, XP027195141, ISSN: 0923-1811 [retrieved on 2009-04-24]<br>* the whole document *<br>----- | 1-7 | |
| A,D | LISA GIOVANNINI-CHAMI ET AL: "Distinct epithelial gene expression phenotypes in childhood respiratory allergy", EUROPEAN RESPIRATORY JOURNAL., vol. 39, no. 5, 17 October 2011 (2011-10-17), pages 1197-1205, XP055400761, DK ISSN: 0903-1936, DOI: 10.1183/09031936.00070511<br>* abstract *<br>* page 1198; figures 1,2; table 2 *<br>* page 1201 *<br>-----<br>-/-- | 1-7 | |

TECHNICAL FIELDS SEARCHED (IPC)

C07K
A61K
G01N

The present search report has been drawn up for all claims

1

| Place of search | Date of completion of the search | Examiner |
|---|---|---|
| The Hague | 27 May 2020 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 15 8832

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | TAKAYAMA ET AL: "Periostin: A novel component of subepithelial fibrosis of bronchial asthma downstream of IL-4 and IL-13 signals", JOURNAL OF ALLERGY AND CLINICAL IMMUNO, ELSEVIER, AMSTERDAM, NL, vol. 118, no. 1, 1 July 2006 (2006-07-01), pages 98-104, XP005610109, ISSN: 0091-6749, DOI: 10.1016/J.JACI.2006.02.046 * the whole document * ----- | 1 | |
| | | | TECHNICAL FIELDS SEARCHED (IPC) |

1 The present search report has been drawn up for all claims

| Place of search | Date of completion of the search | Examiner |
|---|---|---|
| The Hague | 27 May 2020 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 15 8832

This annex lists the patent family members relating to the patent documents cited in the above-mentioned European search report. The members are as contained in the European Patent Office EDP file on
The European Patent Office is in no way liable for these particulars which are merely given for the purpose of information.

27-05-2020

| Patent document cited in search report | Publication date | Patent family member(s) | Publication date |
|---|---|---|---|
| US 2012052060 A1 | 01-03-2012 | AT 500274 T | 15-03-2011 |
| | | AU 2004256976 A1 | 27-01-2005 |
| | | BR PI0412651 A | 26-09-2006 |
| | | CA 2532799 A1 | 27-01-2005 |
| | | CA 2804901 A1 | 27-01-2005 |
| | | CA 2805464 A1 | 27-01-2005 |
| | | CN 1852923 A | 25-10-2006 |
| | | CN 104987419 A | 21-10-2015 |
| | | DK 1646656 T3 | 30-05-2011 |
| | | EP 1646656 A2 | 19-04-2006 |
| | | EP 2292660 A2 | 09-03-2011 |
| | | EP 2314624 A2 | 27-04-2011 |
| | | EP 3064510 A1 | 07-09-2016 |
| | | ES 2361079 T3 | 13-06-2011 |
| | | GB 2403952 A | 19-01-2005 |
| | | IL 172869 A | 31-10-2013 |
| | | JP 4891074 B2 | 07-03-2012 |
| | | JP 2007537702 A | 27-12-2007 |
| | | KR 20110023914 A | 08-03-2011 |
| | | NZ 544662 A | 25-09-2009 |
| | | PL 1646656 T3 | 31-08-2011 |
| | | PT 1646656 E | 07-06-2011 |
| | | SI 1646656 T1 | 29-07-2011 |
| | | US 2005065327 A1 | 24-03-2005 |
| | | US 2007128192 A1 | 07-06-2007 |
| | | US 2009123478 A1 | 14-05-2009 |
| | | US 2012052060 A1 | 01-03-2012 |
| | | US 2016280779 A1 | 29-09-2016 |
| | | US 2018230209 A1 | 16-08-2018 |
| | | WO 2005007699 A2 | 27-01-2005 |
| | | ZA 200601340 B | 31-12-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120052060 A
- US 5892019 A
- US 7829090 B
- US 201000221752 A
- WO 2012158954 A
- US 20120328606
- WO 2012083132 PCT
- WO 2009124090 A
- WO 2009124090 A1
- US 61936967


### Non-patent literature cited in the description


- Thorax. GINA, Global Strategy for Asthma Management and Prevention, 2002, vol. 1, 1-94
- **MILGROM, H. et al.** *Ann Allergy Asthma Immunol,* 2002, vol. 88, 429-31
- **FISH, L. ; C. L. LUNG.** *Ann Allergy Asthma Immunol,* 2001, vol. 86, 24-30
- **BENDER, B. G.** *J. Allergy Clin. Immunol,* 2002, vol. 109, 554-9
- **BIEBER.** *New England Journal of Medicine,* 2008, vol. 358, 1483-1494
- **TAZAWA et al.** *Arch. Dermatol. Res.,* 2004, vol. 295, 459-464
- **PURWAR et al.** *J. Invest. Derm.,* 2006, vol. 126, 1043-1051
- **OH et al.** *J Immunol.,* 2011, vol. 186, 7232-42
- **ZHENG et al.** *J Clin Invest,* 2000, vol. 106, 1081-93
- **TASHKIN et al.** *Am J Respir Crit Care Med,* 1996, vol. 153 (1), 1802-1 1
- **VAN DER POUW KRAAN et al.** *Genes Immun.,* 2002, vol. 3, 436-9
- **CHEN et al.** *PLoS One,* 2013, vol. 8, e68222
- **DENTE et al.** *Respiration,* 2012, vol. 84, 98-100
- **GRUBEK-JAWORSKA et al.** *Respiration,* 2012, vol. 84, 101-107
- **WALSH.** *Curr. Opin. Investig. Drugs,* 2010, vol. 11, 1305-12
- **MCKENZIE, A. N. et al.** *J Immunol,* 1993, vol. 150, 5436-44
- **MINTY, A. et al.** *Nature,* 1993, vol. 362, 248-50
- **JUO, PEI-SHOW.** the Concise Dictionary of Biomedicine and Molecular Biology. CRC Press
- The Dictionary of Cell and Molecular Biology. Academic Press
- the Oxford Dictionary Of Biochemistry And Molecular Biology. Oxford University Press, 2000
- **HARLOW et al.** Antibodies: A Laboratory Manual. Cold Spring Harbor Laboratory Press, 1988
- the Expert Panel Report 3: Guidelines for the Diagnosis and Management of Asthma. National Asthma Education and Prevention Program, 2007
- *Am. J. Respir. Crit. Care Med.,* 2000, vol. 161, 646-664
- **OH et al.** *J. Immunol.,* 2011, vol. 186, 7232-42
- **METWALLY et al.** *Egypt J. Immunol.,* 2004, vol. 11, 171-7
- **ZHONG et al.** *Atherosclerosis,* 2013, vol. 226, 305-314
- **GORRELL, M.** *Clin. Sci.,* 2005, vol. 108, 277-292
- **MCINTOSH, C. H. S. et al.** *Int. J. Biochem. Cell Biol.,* 2006, vol. 38, 860-872
- **GIOVANNINI-CHAMI et al.** *European Respiratory Journal,* May 2012, vol. 39 (5), 1197-205
- **R. SCOPES.** Polypeptide Purification. Springer-Verlag, 1982
- Guide to Polypeptide Purification. **DEUTSCHER.** Methods in Enzymology. Academic Press, Inc, 1990, vol. 182
- Antibodies in Cell Biology. Methods in Cell Biology. Academic Press, Inc, 1993, vol. 37
- Basic and Clinical Immunology. 1991
- **KIOI M et al.** *Cell Immunol.,* 2004, vol. 229, 41-51
- **ANTONIU SA.** *Curr Opin Investig Drugs.,* 2010, vol. 11, 1286-94
- **GUPTA et al.** *J Allergy Clin Immunol.,* 2014, vol. 133 (3), 729-738
- **GUPTA et al.** *Thorax,* 2010, vol. 65 (9), 775-81
- **NAIDICH et al.** *Imaging of the Airways - Functional and Radiologic Correlations,* 2005
- **JIA et al.** *J Allergy Clin. Immunol,* 2012, vol. 130, 647-654
- **TAKAYAMA et al.** *J Allergy Clin Immunol,* 2006, vol. 118, 98-104
- **TAKESHITA et al.** *Biochem J.,* 1993, vol. 294, 271-278
- **SASAKI et al.** *Cancer,* 2001, vol. 92, 843-848

- **BLANCHARD et al.** *Mucosal Immunol.,* 2008, vol. 1, 289-296
- **BLANCHARD ; ROTHENBERG.** *Immunol. Allergy Clin. North. Am.,* 2009, vol. 29, 141-148
- **SIDHU et al.** *Proc. Natl. Acad. Sci. USA,* 2010, vol. 107, 14170-14175
- **KANEMITSU et al.** *J. Allergy Clin. Immunol.,* 2013, vol. 132, 305-12
- **LUN et al.** *J. Clin. Immunol.,* 2007, vol. 27, 430-437
- **CHOI et al.** *J. Immunol.,* 2011, vol. 186 (3), 1861-9
- **LUN et al.** Increased expression of plasma and CD4+ T lymphocyte co-stimulatory molecule CD26 in adult patients with allergic asthma. *J Clin Immunol,* 2007, vol. 27, 430-437
- Report 3: Guidelines for the Diagnosis and Management of Asthma Full Report. Global Strategy for Asthma Management and Prevention, 2007
- **WOODRUFF et al.** *Am. J. Respir. Crit. Care Med.,* 2009, vol. 180, 388-395
- **MILLER et al.** *Eur. Respir. J.,* 2005, vol. 26, 153-61
- **JUNIPER et al.** *Eur. Respir. J.,* 1999, vol. 14, 902-7
- **JUNIPER et al.** *Respir. Med.,* 2006, vol. 100, 616-21
- **JUNIPER et al.** *Respir. Med.,* 2005, vol. 99, 553-8
- **JUNIPER et al.** *Chest.,* May 1999, vol. 115 (5), 1265-70
- **JUNIPER et al.** *J. Clin. Epidemiol.,* 1994, vol. 47, 81-7
- **WOODRUFF.** *Am J Respir Crit Care Med.,* 2009, vol. 180, 388-395
- **JUNIPER et al.** *Respir Med.,* 2006, vol. 100, 616-21
- **JUNIPER et al.** *Chest.,* 1999, vol. 115, 1265-70
- **LUN et al.** *J Clin Immunol.,* 2007, 430-37

SEQUENCE LISTING

<110> MEDIMMUNE LLC

<120> DIPEPTIDYL PEPTIDASE-4 (DPP4/CD26) AS A PERIPHERAL BIOMARKER OF IL-13 ACTIVATION IN ASTHMATIC LUNG

<130> 007617376

<140> EP
<141> 2015-01-26

<150> EP 15740660.4
<151> 2015-01-26

<150> PCT/US2015/012885
<151> 2015-01-26

<150> US 61/931878
<151> 2014-01-27

<150> US 61/990932
<151> 2014-05-09

<160> 78

<170> PatentIn version 3.5

<210> 1
<211> 445
<212> PRT
<213> Homo sapiens

<220>
<221> misc_feature
<222> (1)..(445)
<223> Lebrikizumab Heavy chain

<400> 1

```
Gln Val Thr Leu Arg Glu Ser Gly Pro Ala Leu Val Lys Pro Thr Gln
1               5                   10                  15

Thr Leu Thr Leu Thr Cys Thr Val Ser Gly Phe Ser Leu Ser Ala Tyr
            20                  25                  30

Ser Val Asn Trp Ile Arg Gln Pro Pro Gly Lys Ala Leu Glu Trp Leu
        35                  40                  45

Ala Met Ile Trp Gly Asp Gly Lys Ile Val Tyr Asn Ser Ala Leu Lys
    50                  55                  60

Ser Arg Leu Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95
```

```
Gly Asp Gly Tyr Tyr Pro Tyr Ala Met Asp Asn Trp Gly Gln Gly Ser
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
```

340 345 350

Gln Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Arg Leu Thr Val Asp Lys Ser Arg Trp Gln
405 410 415

Glu Gly Asn Val Phe Ser Cys Ser Val Met His Glu Ala Leu His Asn
420 425 430

His Tyr Thr Gln Lys Ser Leu Ser Leu Ser Leu Gly Lys
435 440 445

<210> 2
<211> 218
<212> PRT
<213> Homo sapiens

<220>
<221> misc_feature
<222> (1)..(218)
<223> Lebrikizumab Light chain

<400> 2

Asp Ile Val Met Thr Gln Ser Pro Asp Ser Leu Ser Val Ser Leu Gly
1 5 10 15

Glu Arg Ala Thr Ile Asn Cys Arg Ala Ser Lys Ser Val Asp Ser Tyr
20 25 30

Gly Asn Ser Phe Met His Trp Tyr Gln Gln Lys Pro Gly Gln Pro Pro
35 40 45

Lys Leu Leu Ile Tyr Leu Ala Ser Asn Leu Glu Ser Gly Val Pro Asp
50 55 60

Arg Phe Ser Gly Ser Gly Ser Gly Thr Asp Phe Thr Leu Thr Ile Ser
65 70 75 80

Ser Leu Gln Ala Glu Asp Val Ala Val Tyr Tyr Cys Gln Gln Asn Asn
85 90 95

Glu Asp Pro Arg Thr Phe Gly Gly Gly Thr Lys Val Glu Ile Lys Arg
100 105 110

Thr Val Ala Ala Pro Ser Val Phe Ile Phe Pro Pro Ser Asp Glu Gln
115 120 125

Leu Lys Ser Gly Thr Ala Ser Val Val Cys Leu Leu Asn Asn Phe Tyr
130 135 140

Pro Arg Glu Ala Lys Val Gln Trp Lys Val Asp Asn Ala Leu Gln Ser
145 150 155 160

Gly Asn Ser Gln Glu Ser Val Thr Glu Gln Asp Ser Lys Asp Ser Thr
165 170 175

Tyr Ser Leu Ser Ser Thr Leu Thr Leu Ser Lys Ala Asp Tyr Glu Lys
180 185 190

His Lys Val Tyr Ala Cys Glu Val Thr His Gln Gly Leu Ser Ser Pro
195 200 205

Val Thr Lys Ser Phe Asn Arg Gly Glu Cys
210 215

<210> 3
<211> 122
<212> PRT
<213> Homo sapiens

<220>
<221> misc_feature
<222> (1)..(122)
<223> Tralokinumab Heavy chain

<400> 3

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asn Tyr
20 25 30

Gly Leu Ser Trp Val Arg Gln Ala Pro Gly Gln Gly Leu Glu Trp Met
35 40 45

Gly Trp Ile Ser Ala Asn Asn Gly Asp Thr Asn Tyr Gly Gln Glu Phe
50 55 60

Gln Gly Arg Val Thr Met Thr Thr Asp Thr Ser Thr Ser Thr Ala Tyr

65 70 75 80

Met Glu Leu Arg Ser Leu Arg Ser Asp Asp Thr Ala Val Tyr Tyr Cys
85 90 95

Ala Arg Asp Ser Ser Ser Ser Trp Ala Arg Trp Phe Phe Asp Leu Trp
100 105 110

Gly Arg Gly Thr Leu Val Thr Val Ser Ser
115 120

<210> 4
<211> 108
<212> PRT
<213> Homo sapiens

<220>
<221> misc_feature
<222> (1)..(108)
<223> Tralokinumab Light chain

<400> 4

Ser Tyr Val Leu Thr Gln Pro Pro Ser Val Ser Val Ala Pro Gly Lys
1 5 10 15

Thr Ala Arg Ile Thr Cys Gly Gly Asn Ile Ile Gly Ser Lys Leu Val
20 25 30

His Trp Tyr Gln Gln Lys Pro Gly Gln Ala Pro Val Leu Val Ile Tyr
35 40 45

Asp Asp Gly Asp Arg Pro Ser Gly Ile Pro Glu Arg Phe Ser Gly Ser
50 55 60

Asn Ser Gly Asn Thr Ala Thr Leu Thr Ile Ser Arg Val Glu Ala Gly
65 70 75 80

Asp Glu Ala Asp Tyr Tyr Cys Gln Val Trp Asp Thr Gly Ser Asp Pro
85 90 95

Val Val Phe Gly Gly Gly Thr Lys Leu Thr Val Leu
100 105

<210> 5
<211> 766
<212> PRT
<213> Homo sapiens

<220>

<221> misc_feature
<222> (1)..(766)
<223> DPP4 membrane bound form

<400> 5

Met Lys Thr Pro Trp Lys Val Leu Leu Gly Leu Leu Gly Ala Ala Ala
1 5 10 15

Leu Val Thr Ile Ile Thr Val Pro Val Val Leu Leu Asn Lys Gly Thr
20 25 30

Asp Asp Ala Thr Ala Asp Ser Arg Lys Thr Tyr Thr Leu Thr Asp Tyr
35 40 45

Leu Lys Asn Thr Tyr Arg Leu Lys Leu Tyr Ser Leu Arg Trp Ile Ser
50 55 60

Asp His Glu Tyr Leu Tyr Lys Gln Glu Asn Asn Ile Leu Val Phe Asn
65 70 75 80

Ala Glu Tyr Gly Asn Ser Ser Val Phe Leu Glu Asn Ser Thr Phe Asp
85 90 95

Glu Phe Gly His Ser Ile Asn Asp Tyr Ser Ile Ser Pro Asp Gly Gln
100 105 110

Phe Ile Leu Leu Glu Tyr Asn Tyr Val Lys Gln Trp Arg His Ser Tyr
115 120 125

Thr Ala Ser Tyr Asp Ile Tyr Asp Leu Asn Lys Arg Gln Leu Ile Thr
130 135 140

Glu Glu Arg Ile Pro Asn Asn Thr Gln Trp Val Thr Trp Ser Pro Val
145 150 155 160

Gly His Lys Leu Ala Tyr Val Trp Asn Asn Asp Ile Tyr Val Lys Ile
165 170 175

Glu Pro Asn Leu Pro Ser Tyr Arg Ile Thr Trp Thr Gly Lys Glu Asp
180 185 190

Ile Ile Tyr Asn Gly Ile Thr Asp Trp Val Tyr Glu Glu Glu Val Phe
195 200 205

Ser Ala Tyr Ser Ala Leu Trp Trp Ser Pro Asn Gly Thr Phe Leu Ala
210 215 220

Tyr Ala Gln Phe Asn Asp Thr Glu Val Pro Leu Ile Glu Tyr Ser Phe

```
225                 230                 235                 240

Tyr Ser Asp Glu Ser Leu Gln Tyr Pro Lys Thr Val Arg Val Pro Tyr
                245                 250                 255

Pro Lys Ala Gly Ala Val Asn Pro Thr Val Lys Phe Phe Val Val Asn
            260                 265                 270

Thr Asp Ser Leu Ser Ser Val Thr Asn Ala Thr Ser Ile Gln Ile Thr
        275                 280                 285

Ala Pro Ala Ser Met Leu Ile Gly Asp His Tyr Leu Cys Asp Val Thr
    290                 295                 300

Trp Ala Thr Gln Glu Arg Ile Ser Leu Gln Trp Leu Arg Arg Ile Gln
305                 310                 315                 320

Asn Tyr Ser Val Met Asp Ile Cys Asp Tyr Asp Glu Ser Ser Gly Arg
                325                 330                 335

Trp Asn Cys Leu Val Ala Arg Gln His Ile Glu Met Ser Thr Thr Gly
            340                 345                 350

Trp Val Gly Arg Phe Arg Pro Ser Glu Pro His Phe Thr Leu Asp Gly
        355                 360                 365

Asn Ser Phe Tyr Lys Ile Ile Ser Asn Glu Glu Gly Tyr Arg His Ile
    370                 375                 380

Cys Tyr Phe Gln Ile Asp Lys Lys Asp Cys Thr Phe Ile Thr Lys Gly
385                 390                 395                 400

Thr Trp Glu Val Ile Gly Ile Glu Ala Leu Thr Ser Asp Tyr Leu Tyr
                405                 410                 415

Tyr Ile Ser Asn Glu Tyr Lys Gly Met Pro Gly Gly Arg Asn Leu Tyr
            420                 425                 430

Lys Ile Gln Leu Ser Asp Tyr Thr Lys Val Thr Cys Leu Ser Cys Glu
        435                 440                 445

Leu Asn Pro Glu Arg Cys Gln Tyr Tyr Ser Val Ser Phe Ser Lys Glu
    450                 455                 460

Ala Lys Tyr Tyr Gln Leu Arg Cys Ser Gly Pro Gly Leu Pro Leu Tyr
465                 470                 475                 480
```

```
Thr Leu His Ser Ser Val Asn Asp Lys Gly Leu Arg Val Leu Glu Asp
                485                 490                 495

Asn Ser Ala Leu Asp Lys Met Leu Gln Asn Val Gln Met Pro Ser Lys
            500                 505                 510

Lys Leu Asp Phe Ile Ile Leu Asn Glu Thr Lys Phe Trp Tyr Gln Met
        515                 520                 525

Ile Leu Pro Pro His Phe Asp Lys Ser Lys Lys Tyr Pro Leu Leu Leu
    530                 535                 540

Asp Val Tyr Ala Gly Pro Cys Ser Gln Lys Ala Asp Thr Val Phe Arg
545                 550                 555                 560

Leu Asn Trp Ala Thr Tyr Leu Ala Ser Thr Glu Asn Ile Ile Val Ala
                565                 570                 575

Ser Phe Asp Gly Arg Gly Ser Gly Tyr Gln Gly Asp Lys Ile Met His
            580                 585                 590

Ala Ile Asn Arg Arg Leu Gly Thr Phe Glu Val Glu Asp Gln Ile Glu
        595                 600                 605

Ala Ala Arg Gln Phe Ser Lys Met Gly Phe Val Asp Asn Lys Arg Ile
    610                 615                 620

Ala Ile Trp Gly Trp Ser Tyr Gly Gly Tyr Val Thr Ser Met Val Leu
625                 630                 635                 640

Gly Ser Gly Ser Gly Val Phe Lys Cys Gly Ile Ala Val Ala Pro Val
                645                 650                 655

Ser Arg Trp Glu Tyr Tyr Asp Ser Val Tyr Thr Glu Arg Tyr Met Gly
            660                 665                 670

Leu Pro Thr Pro Glu Asp Asn Leu Asp His Tyr Arg Asn Ser Thr Val
        675                 680                 685

Met Ser Arg Ala Glu Asn Phe Lys Gln Val Glu Tyr Leu Leu Ile His
    690                 695                 700

Gly Thr Ala Asp Asp Asn Val His Phe Gln Gln Ser Ala Gln Ile Ser
705                 710                 715                 720

Lys Ala Leu Val Asp Val Gly Val Asp Phe Gln Ala Met Trp Tyr Thr
                725                 730                 735
```

Asp Glu Asp His Gly Ile Ala Ser Ser Thr Ala His Gln His Ile Tyr
740 745 750

Thr His Met Ser His Phe Ile Lys Gln Cys Phe Ser Leu Pro
755 760 765

<210> 6
<211> 728
<212> PRT
<213> Homo sapiens

<220>
<221> misc_feature
<222> (1)..(728)
<223> DPP4 soluble form

<400> 6

Ser Arg Lys Thr Tyr Thr Leu Thr Asp Tyr Leu Lys Asn Thr Tyr Arg
1 5 10 15

Leu Lys Leu Tyr Ser Leu Arg Trp Ile Ser Asp His Glu Tyr Leu Tyr
20 25 30

Lys Gln Glu Asn Asn Ile Leu Val Phe Asn Ala Glu Tyr Gly Asn Ser
35 40 45

Ser Val Phe Leu Glu Asn Ser Thr Phe Asp Glu Phe Gly His Ser Ile
50 55 60

Asn Asp Tyr Ser Ile Ser Pro Asp Gly Gln Phe Ile Leu Leu Glu Tyr
65 70 75 80

Asn Tyr Val Lys Gln Trp Arg His Ser Tyr Thr Ala Ser Tyr Asp Ile
85 90 95

Tyr Asp Leu Asn Lys Arg Gln Leu Ile Thr Glu Glu Arg Ile Pro Asn
100 105 110

Asn Thr Gln Trp Val Thr Trp Ser Pro Val Gly His Lys Leu Ala Tyr
115 120 125

Val Trp Asn Asn Asp Ile Tyr Val Lys Ile Glu Pro Asn Leu Pro Ser
130 135 140

Tyr Arg Ile Thr Trp Thr Gly Lys Glu Asp Ile Ile Tyr Asn Gly Ile
145 150 155 160

Thr Asp Trp Val Tyr Glu Glu Glu Val Phe Ser Ala Tyr Ser Ala Leu

165 170 175

Trp Trp Ser Pro Asn Gly Thr Phe Leu Ala Tyr Ala Gln Phe Asn Asp
180 185 190

Thr Glu Val Pro Leu Ile Glu Tyr Ser Phe Tyr Ser Asp Glu Ser Leu
195 200 205

Gln Tyr Pro Lys Thr Val Arg Val Pro Tyr Pro Lys Ala Gly Ala Val
210 215 220

Asn Pro Thr Val Lys Phe Phe Val Val Asn Thr Asp Ser Leu Ser Ser
225 230 235 240

Val Thr Asn Ala Thr Ser Ile Gln Ile Thr Ala Pro Ala Ser Met Leu
245 250 255

Ile Gly Asp His Tyr Leu Cys Asp Val Thr Trp Ala Thr Gln Glu Arg
260 265 270

Ile Ser Leu Gln Trp Leu Arg Arg Ile Gln Asn Tyr Ser Val Met Asp
275 280 285

Ile Cys Asp Tyr Asp Glu Ser Ser Gly Arg Trp Asn Cys Leu Val Ala
290 295 300

Arg Gln His Ile Glu Met Ser Thr Thr Gly Trp Val Gly Arg Phe Arg
305 310 315 320

Pro Ser Glu Pro His Phe Thr Leu Asp Gly Asn Ser Phe Tyr Lys Ile
325 330 335

Ile Ser Asn Glu Glu Gly Tyr Arg His Ile Cys Tyr Phe Gln Ile Asp
340 345 350

Lys Lys Asp Cys Thr Phe Ile Thr Lys Gly Thr Trp Glu Val Ile Gly
355 360 365

Ile Glu Ala Leu Thr Ser Asp Tyr Leu Tyr Tyr Ile Ser Asn Glu Tyr
370 375 380

Lys Gly Met Pro Gly Gly Arg Asn Leu Tyr Lys Ile Gln Leu Ser Asp
385 390 395 400

Tyr Thr Lys Val Thr Cys Leu Ser Cys Glu Leu Asn Pro Glu Arg Cys
405 410 415

Gln Tyr Tyr Ser Val Ser Phe Ser Lys Glu Ala Lys Tyr Tyr Gln Leu
420 425 430

Arg Cys Ser Gly Pro Gly Leu Pro Leu Tyr Thr Leu His Ser Ser Val
435 440 445

Asn Asp Lys Gly Leu Arg Val Leu Glu Asp Asn Ser Ala Leu Asp Lys
450 455 460

Met Leu Gln Asn Val Gln Met Pro Ser Lys Lys Leu Asp Phe Ile Ile
465 470 475 480

Leu Asn Glu Thr Lys Phe Trp Tyr Gln Met Ile Leu Pro Pro His Phe
485 490 495

Asp Lys Ser Lys Lys Tyr Pro Leu Leu Leu Asp Val Tyr Ala Gly Pro
500 505 510

Cys Ser Gln Lys Ala Asp Thr Val Phe Arg Leu Asn Trp Ala Thr Tyr
515 520 525

Leu Ala Ser Thr Glu Asn Ile Ile Val Ala Ser Phe Asp Gly Arg Gly
530 535 540

Ser Gly Tyr Gln Gly Asp Lys Ile Met His Ala Ile Asn Arg Arg Leu
545 550 555 560

Gly Thr Phe Glu Val Glu Asp Gln Ile Glu Ala Ala Arg Gln Phe Ser
565 570 575

Lys Met Gly Phe Val Asp Asn Lys Arg Ile Ala Ile Trp Gly Trp Ser
580 585 590

Tyr Gly Gly Tyr Val Thr Ser Met Val Leu Gly Ser Gly Ser Gly Val
595 600 605

Phe Lys Cys Gly Ile Ala Val Ala Pro Val Ser Arg Trp Glu Tyr Tyr
610 615 620

Asp Ser Val Tyr Thr Glu Arg Tyr Met Gly Leu Pro Thr Pro Glu Asp
625 630 635 640

Asn Leu Asp His Tyr Arg Asn Ser Thr Val Met Ser Arg Ala Glu Asn
645 650 655

Phe Lys Gln Val Glu Tyr Leu Leu Ile His Gly Thr Ala Asp Asp Asn
660 665 670

Val His Phe Gln Gln Ser Ala Gln Ile Ser Lys Ala Leu Val Asp Val
675 680 685

Gly Val Asp Phe Gln Ala Met Trp Tyr Thr Asp Glu Asp His Gly Ile
690 695 700

Ala Ser Ser Thr Ala His Gln His Ile Tyr Thr His Met Ser His Phe
705 710 715 720

Ile Lys Gln Cys Phe Ser Leu Pro
725

<210> 7
<211> 3913
<212> DNA
<213> Homo sapiens

<220>
<221> misc_feature
<222> (1)..(766)
<223> DPP4 membrane bound form (full sequence), cDNA

<400> 7

ctttcactgg caagagacgg agtcctgggt ttcagttcca gttgcctgcg gtgggctgtg 60

tgagtttgcc aaagtcccct gccctctctg ggtctcggtt ccctcgcctg tccacgtgag 120

gttggaggag ctgaacgccg acgtcatttt tagctaagag ggagcagggt ccccgagtcg 180

ccggcccagg gtctgcgcat ccgaggccgc gcgccctttc ccctccccca cggctcctcc 240

gggccccgca ctctgcgccc cggctgccgc ccagcgccct acaccgccct caggggggccc 300

tcgcgggctc cccccggccg ggatgccagt gccccgcgcc acgcgcgcct gctcccgcgc 360

cgcctgccct gcagcctgcc cgcggcgcct ttatacccag cgggctcggc gctcactaat 420

gtttaactcg ggccgaaac ttgccagcgg cgagtgactc caccgcccgg agcagcggtg 480

caggacgcgc gtctccgccg cccgcggtga cttctgcctg cgctccttct ctgaacgctc 540

acttccgagg agacgccgac gatgaagaca ccgtggaagg ttcttctggg actgctgggt 600

gctgctgcgc ttgtcaccat catcaccgtg cccgtggttc tgctgaacaa aggcacagat 660

gatgctacag ctgacagtcg caaaacttac actctaactg attacttaaa aaatacttat 720

agactgaagt tatactcctt aagatggatt tcagatcatg aatatctcta caaacaagaa 780

aataatatct tggtattcaa tgctgaatat ggaaacagct cagttttctt ggagaacagt 840

acatttgatg agtttggaca ttctatcaat gattattcaa tatctcctga tgggcagttt 900

attctcttag aatacaacta cgtgaagcaa tggaggcatt cctacacagc ttcatatgac 960

atttatgatt taaataaaag gcagctgatt acagaagaga ggattccaaa caacacacag 1020

tgggtcacat ggtcaccagt gggtcataaa ttggcatatg tttggaacaa tgacatttat 1080
gttaaaattg aaccaaattt accaagttac agaatcacat ggacggggaa agaagatata 1140
atatataatg gaataactga ctgggtttat gaagaggaag tcttcagtgc ctactctgct 1200
ctgtggtggt ctccaaacgg cactttttta gcatatgccc aatttaacga cacagaagtc 1260
ccacttattg aatactcctt ctactctgat gagtcactgc agtacccaaa gactgtacgg 1320
gttccatatc caaaggcagg agctgtgaat ccaactgtaa agttctttgt tgtaaataca 1380
gactctctca gctcagtcac caatgcaact tccatacaaa tcactgctcc tgcttctatg 1440
ttgatagggg atcactactt gtgtgatgtg acatgggcaa cacaagaaag aatttctttg 1500
cagtggctca ggaggattca gaactattcg gtcatggata tttgtgacta tgatgaatcc 1560
agtggaagat ggaactgctt agtggcacgg caacacattg aaatgagtac tactggctgg 1620
gttggaagat ttaggccttc agaacctcat tttacccttg atggtaatag cttctacaag 1680
atcatcagca atgaagaagg ttacagacac atttgctatt tccaaataga taaaaaagac 1740
tgcacattta ttacaaaagg cacctgggaa gtcatcggga tagaagctct aaccagtgat 1800
tatctatact acattagtaa tgaatataaa ggaatgccag gaggaaggaa tctttataaa 1860
atccaactta gtgactatac aaaagtgaca tgcctcagtt gtgagctgaa tccggaaagg 1920
tgtcagtact attctgtgtc attcagtaaa gaggcgaagt attatcagct gagatgttcc 1980
ggtcctggtc tgcccctcta tactctacac agcagcgtga atgataaagg gctgagagtc 2040
ctggaagaca attcagcttt ggataaaatg ctgcagaatg tccagatgcc ctccaaaaaa 2100
ctggacttca ttattttgaa tgaaacaaaa ttttggtatc agatgatctt gcctcctcat 2160
tttgataaat ccaagaaata tcctctacta ttagatgtgt atgcaggccc atgtagtcaa 2220
aaagcagaca ctgtcttcag actgaactgg gccacttacc ttgcaagcac agaaaacatt 2280
atagtagcta gctttgatgg cagaggaagt ggttaccaag gagataagat catgcatgca 2340
atcaacagaa gactgggaac atttgaagtt gaagatcaaa ttgaagcagc cagacaattt 2400
tcaaaaatgg gatttgtgga caacaaacga attgcaattt ggggctggtc atatggaggg 2460
tacgtaacct caatggtcct gggatcggga agtggcgtgt tcaagtgtgg aatagccgtg 2520
gcgcctgtat cccggtggga gtactatgac tcagtgtaca cagaacgtta catgggtctc 2580
ccaactccag aagacaacct tgaccattac agaaattcaa cagtcatgag cagagctgaa 2640
aattttaaac aagttgagta cctccttatt catggaacag cagatgataa cgttcacttt 2700
cagcagtcag ctcagatctc caaagccctg gtcgatgttg gagtggattt ccaggcaatg 2760
tggtatactg atgaagacca tggaatagct agcagcacag cacaccaaca tatatatacc 2820
cacatgagcc acttcataaa acaatgtttc tctttacctt agcacctcaa aataccatgc 2880

catttaaagc ttattaaaac tcatttttgt tttcattatc tcaaaactgc actgtcaaga 2940

tgatgatgat ctttaaaata cacactcaaa tcaagaaact taaggttacc tttgttccca 3000

aatttcatac ctatcatctt aagtagggac ttctgtcttc acaacagatt attaccttac 3060

agaagtttga attatccggt cgggttttat tgtttaaaat catttctgca tcagctgctg 3120

aaacaacaaa taggaattgt ttttatggag gctttgcata gattccctga gcaggatttt 3180

aatctttttc taactggact ggttcaaatg ttgttctctt ctttaaaggg atggcaagat 3240

gtgggcagtg atgtcactag ggcagggaca ggataagagg gattagggag agaagatagc 3300

agggcatggc tgggaaccca agtccaagca taccaacacg agcaggctac tgtcagctcc 3360

cctcggagaa gagctgttca cagccagact ggcacagttt tctgagaaag actattcaaa 3420

cagtctcagg aaatcaaata tgcaaagcac tgacttctaa gtaaaaccac agcagttgaa 3480

aagactccaa agaaatgtaa gggaaactgc cagcaacgca ggcccccagg tgccagttat 3540

ggctataggt gctacaaaaa cacagcaagg gtgatgggaa agcattgtaa atgtgctttt 3600

aaaaaaaaat actgatgttc ctagtgaaag aggcagcttg aaactgagat gtgaacacat 3660

cagcttgccc tgttaaaaga tgaaaatatt tgtatcacaa atcttaactt gaaggagtcc 3720

ttgcatcaat ttttcttatt tcatttcttt gagtgtctta attaaaagaa tattttaact 3780

tccttggact cattttaaaa aatggaacat aaaatacaat gttatgtatt attattccca 3840

ttctacatac tatggaattt ctcccagtca tttaataaat gtgccttcat tttttcagaa 3900

aaaaaaaaaa aaa 3913

<210> 8
<211> 836
<212> PRT
<213> Homo sapiens

<400> 8
Met Ile Pro Phe Leu Pro Met Phe Ser Leu Leu Leu Leu Leu Ile Val
1               5                   10                  15

Asn Pro Ile Asn Ala Asn Asn His Tyr Asp Lys Ile Leu Ala His Ser
            20                  25                  30

Arg Ile Arg Gly Arg Asp Gln Gly Pro Asn Val Cys Ala Leu Gln Gln
        35                  40                  45

Ile Leu Gly Thr Lys Lys Lys Tyr Phe Ser Thr Cys Lys Asn Trp Tyr
    50                  55                  60

Lys Lys Ser Ile Cys Gly Gln Lys Thr Thr Val Leu Tyr Glu Cys Cys
65                  70                  75                  80

Pro Gly Tyr Met Arg Met Glu Gly Met Lys Gly Cys Pro Ala Val Leu
85 90 95

Pro Ile Asp His Val Tyr Gly Thr Leu Gly Ile Val Gly Ala Thr Thr
100 105 110

Thr Gln Arg Tyr Ser Asp Ala Ser Lys Leu Arg Glu Glu Ile Glu Gly
115 120 125

Lys Gly Ser Phe Thr Tyr Phe Ala Pro Ser Asn Glu Ala Trp Asp Asn
130 135 140

Leu Asp Ser Asp Ile Arg Arg Gly Leu Glu Ser Asn Val Asn Val Glu
145 150 155 160

Leu Leu Asn Ala Leu His Ser His Met Ile Asn Lys Arg Met Leu Thr
165 170 175

Lys Asp Leu Lys Asn Gly Met Ile Ile Pro Ser Met Tyr Asn Asn Leu
180 185 190

Gly Leu Phe Ile Asn His Tyr Pro Asn Gly Val Val Thr Val Asn Cys
195 200 205

Ala Arg Ile Ile His Gly Asn Gln Ile Ala Thr Asn Gly Val Val His
210 215 220

Val Ile Asp Arg Val Leu Thr Gln Ile Gly Thr Ser Ile Gln Asp Phe
225 230 235 240

Ile Glu Ala Glu Asp Asp Leu Ser Ser Phe Arg Ala Ala Ala Ile Thr
245 250 255

Ser Asp Ile Leu Glu Ala Leu Gly Arg Asp Gly His Phe Thr Leu Phe
260 265 270

Ala Pro Thr Asn Glu Ala Phe Glu Lys Leu Pro Arg Gly Val Leu Glu
275 280 285

Arg Ile Met Gly Asp Lys Val Ala Ser Glu Ala Leu Met Lys Tyr His
290 295 300

Ile Leu Asn Thr Leu Gln Cys Ser Glu Ser Ile Met Gly Gly Ala Val
305 310 315 320

Phe Glu Thr Leu Glu Gly Asn Thr Ile Glu Ile Gly Cys Asp Gly Asp
325 330 335

Ser Ile Thr Val Asn Gly Ile Lys Met Val Asn Lys Lys Asp Ile Val
340 345 350

Thr Asn Asn Gly Val Ile His Leu Ile Asp Gln Val Leu Ile Pro Asp
355 360 365

Ser Ala Lys Gln Val Ile Glu Leu Ala Gly Lys Gln Gln Thr Thr Phe
370 375 380

Thr Asp Leu Val Ala Gln Leu Gly Leu Ala Ser Ala Leu Arg Pro Asp
385 390 395 400

Gly Glu Tyr Thr Leu Leu Ala Pro Val Asn Asn Ala Phe Ser Asp Asp
405 410 415

Thr Leu Ser Met Asp Gln Arg Leu Leu Lys Leu Ile Leu Gln Asn His
420 425 430

Ile Leu Lys Val Lys Val Gly Leu Asn Glu Leu Tyr Asn Gly Gln Ile
435 440 445

Leu Glu Thr Ile Gly Gly Lys Gln Leu Arg Val Phe Val Tyr Arg Thr
450 455 460

Ala Val Cys Ile Glu Asn Ser Cys Met Glu Lys Gly Ser Lys Gln Gly
465 470 475 480

Arg Asn Gly Ala Ile His Ile Phe Arg Glu Ile Ile Lys Pro Ala Glu
485 490 495

Lys Ser Leu His Glu Lys Leu Lys Gln Asp Lys Arg Phe Ser Thr Phe
500 505 510

Leu Ser Leu Leu Glu Ala Ala Asp Leu Lys Glu Leu Leu Thr Gln Pro
515 520 525

Gly Asp Trp Thr Leu Phe Val Pro Thr Asn Asp Ala Phe Lys Gly Met
530 535 540

Thr Ser Glu Glu Lys Glu Ile Leu Ile Arg Asp Lys Asn Ala Leu Gln
545 550 555 560

Asn Ile Ile Leu Tyr His Leu Thr Pro Gly Val Phe Ile Gly Lys Gly
565 570 575

Phe Glu Pro Gly Val Thr Asn Ile Leu Lys Thr Thr Gln Gly Ser Lys
580 585 590

Ile Phe Leu Lys Glu Val Asn Asp Thr Leu Leu Val Asn Glu Leu Lys
595 600 605

Ser Lys Glu Ser Asp Ile Met Thr Thr Asn Gly Val Ile His Val Val
610 615 620

Asp Lys Leu Leu Tyr Pro Ala Asp Thr Pro Val Gly Asn Asp Gln Leu
625 630 635 640

Leu Glu Ile Leu Asn Lys Leu Ile Lys Tyr Ile Gln Ile Lys Phe Val
645 650 655

Arg Gly Ser Thr Phe Lys Glu Ile Pro Val Thr Val Tyr Thr Thr Lys
660 665 670

Ile Ile Thr Lys Val Val Glu Pro Lys Ile Lys Val Ile Glu Gly Ser
675 680 685

Leu Gln Pro Ile Ile Lys Thr Glu Gly Pro Thr Leu Thr Lys Val Lys
690 695 700

Ile Glu Gly Glu Pro Glu Phe Arg Leu Ile Lys Glu Gly Glu Thr Ile
705 710 715 720

Thr Glu Val Ile His Gly Glu Pro Ile Ile Lys Lys Tyr Thr Lys Ile
725 730 735

Ile Asp Gly Val Pro Val Glu Ile Thr Glu Lys Glu Thr Arg Glu Glu
740 745 750

Arg Ile Ile Thr Gly Pro Glu Ile Lys Tyr Thr Arg Ile Ser Thr Gly
755 760 765

Gly Gly Glu Thr Glu Glu Thr Leu Lys Lys Leu Leu Gln Glu Glu Val
770 775 780

Thr Lys Val Thr Lys Phe Ile Glu Gly Gly Asp Gly His Leu Phe Glu
785 790 795 800

Asp Glu Glu Ile Lys Arg Leu Leu Gln Gly Asp Thr Pro Val Arg Lys
805 810 815

Leu Gln Ala Asn Lys Lys Val Gln Gly Ser Arg Arg Arg Leu Arg Glu
820 825 830

Gly Arg Ser Gln

835

<210> 9
<211> 141
<212> PRT
<213> Homo sapiens

<400> 9
```
Met Ala Gln His Leu Ser Thr Leu Leu Leu Leu Leu Ala Thr Leu Ala
1               5                   10                  15

Val Ala Leu Ala Trp Ser Pro Lys Glu Glu Asp Arg Ile Ile Pro Gly
            20                  25                  30

Gly Ile Tyr Asn Ala Asp Leu Asn Asp Glu Trp Val Gln Arg Ala Leu
        35                  40                  45

His Phe Ala Ile Ser Glu Tyr Asn Lys Ala Thr Lys Asp Asp Tyr Tyr
    50                  55                  60

Arg Arg Pro Leu Arg Val Leu Arg Ala Arg Gln Gln Thr Val Gly Gly
65                  70                  75                  80

Val Asn Tyr Phe Phe Asp Val Glu Val Gly Arg Thr Ile Cys Thr Lys
                85                  90                  95

Ser Gln Pro Asn Leu Asp Thr Cys Ala Phe His Glu Gln Pro Glu Leu
            100                 105                 110

Gln Lys Lys Gln Leu Cys Ser Phe Glu Ile Tyr Glu Val Pro Trp Glu
        115                 120                 125

Asn Arg Arg Ser Leu Val Lys Ser Arg Cys Gln Glu Ser
    130                 135                 140
```

<210> 10
<211> 94
<212> PRT
<213> Homo sapiens

<400> 10
```
Met Met Gly Leu Ser Leu Ala Ser Ala Val Leu Leu Ala Ser Leu Leu
1               5                   10                  15

Ser Leu His Leu Gly Thr Ala Thr Arg Gly Ser Asp Ile Ser Lys Thr
            20                  25                  30

Cys Cys Phe Gln Tyr Ser His Lys Pro Leu Pro Trp Thr Trp Val Arg
        35                  40                  45
```

Ser Tyr Glu Phe Thr Ser Asn Ser Cys Ser Gln Arg Ala Val Ile Phe
    50                  55                  60

Thr Thr Lys Arg Gly Lys Lys Val Cys Thr His Pro Arg Lys Lys Trp
65                  70                  75                  80

Val Gln Lys Tyr Ile Ser Leu Leu Lys Thr Pro Lys Gln Leu
                85                  90

<210> 11
<211> 914
<212> PRT
<213> Homo sapiens

<400> 11
Met Gly Pro Phe Lys Ser Ser Val Phe Ile Leu Ile Leu His Leu Leu
1               5                   10                  15

Glu Gly Ala Leu Ser Asn Ser Leu Ile Gln Leu Asn Asn Asn Gly Tyr
            20                  25                  30

Glu Gly Ile Val Val Ala Ile Asp Pro Asn Val Pro Glu Asp Glu Thr
        35                  40                  45

Leu Ile Gln Gln Ile Lys Asp Met Val Thr Gln Ala Ser Leu Tyr Leu
    50                  55                  60

Phe Glu Ala Thr Gly Lys Arg Phe Tyr Phe Lys Asn Val Ala Ile Leu
65                  70                  75                  80

Ile Pro Glu Thr Trp Lys Thr Lys Ala Asp Tyr Val Arg Pro Lys Leu
                85                  90                  95

Glu Thr Tyr Lys Asn Ala Asp Val Leu Val Ala Glu Ser Thr Pro Pro
            100                 105                 110

Gly Asn Asp Glu Pro Tyr Thr Glu Gln Met Gly Asn Cys Gly Glu Lys
        115                 120                 125

Gly Glu Arg Ile His Leu Thr Pro Asp Phe Ile Ala Gly Lys Lys Leu
    130                 135                 140

Ala Glu Tyr Gly Pro Gln Gly Lys Ala Phe Val His Glu Trp Ala His
145                 150                 155                 160

Leu Arg Trp Gly Val Phe Asp Glu Tyr Asn Asn Asp Glu Lys Phe Tyr
                165                 170                 175

Leu Ser Asn Gly Arg Ile Gln Ala Val Arg Cys Ser Ala Gly Ile Thr

180 185 190

Gly Thr Asn Val Val Lys Lys Cys Gln Gly Gly Ser Cys Tyr Thr Lys
195 200 205

Arg Cys Thr Phe Asn Lys Val Thr Gly Leu Tyr Glu Lys Gly Cys Glu
210 215 220

Phe Val Leu Gln Ser Arg Gln Thr Glu Lys Ala Ser Ile Met Phe Ala
225 230 235 240

Gln His Val Asp Ser Ile Val Glu Phe Cys Thr Glu Gln Asn His Asn
245 250 255

Lys Glu Ala Pro Asn Lys Gln Asn Gln Lys Cys Asn Leu Arg Ser Thr
260 265 270

Trp Glu Val Ile Arg Asp Ser Glu Asp Phe Lys Lys Thr Thr Pro Met
275 280 285

Thr Thr Gln Pro Pro Asn Pro Thr Phe Ser Leu Leu Gln Ile Gly Gln
290 295 300

Arg Ile Val Cys Leu Val Leu Asp Lys Ser Gly Ser Met Ala Thr Gly
305 310 315 320

Asn Arg Leu Asn Arg Leu Asn Gln Ala Gly Gln Leu Phe Leu Leu Gln
325 330 335

Thr Val Glu Leu Gly Ser Trp Val Gly Met Val Thr Phe Asp Ser Ala
340 345 350

Ala His Val Gln Ser Glu Leu Ile Gln Ile Asn Ser Gly Ser Asp Arg
355 360 365

Asp Thr Leu Ala Lys Arg Leu Pro Ala Ala Ala Ser Gly Gly Thr Ser
370 375 380

Ile Cys Ser Gly Leu Arg Ser Ala Phe Thr Val Ile Arg Lys Lys Tyr
385 390 395 400

Pro Thr Asp Gly Ser Glu Ile Val Leu Leu Thr Asp Gly Glu Asp Asn
405 410 415

Thr Ile Ser Gly Cys Phe Asn Glu Val Lys Gln Ser Gly Ala Ile Ile
420 425 430

```
His Thr Val Ala Leu Gly Pro Ser Ala Ala Gln Glu Leu Glu Glu Leu
        435                 440                 445

Ser Lys Met Thr Gly Gly Leu Gln Thr Tyr Ala Ser Asp Gln Val Gln
    450                 455                 460

Asn Asn Gly Leu Ile Asp Ala Phe Gly Ala Leu Ser Ser Gly Asn Gly
465                 470                 475                 480

Ala Val Ser Gln Arg Ser Ile Gln Leu Glu Ser Lys Gly Leu Thr Leu
                485                 490                 495

Gln Asn Ser Gln Trp Met Asn Gly Thr Val Ile Val Asp Ser Thr Val
            500                 505                 510

Gly Lys Asp Thr Leu Phe Leu Ile Thr Trp Thr Thr Gln Pro Pro Gln
        515                 520                 525

Ile Leu Leu Trp Asp Pro Ser Gly Gln Lys Gln Gly Gly Phe Val Val
    530                 535                 540

Asp Lys Asn Thr Lys Met Ala Tyr Leu Gln Ile Pro Gly Ile Ala Lys
545                 550                 555                 560

Val Gly Thr Trp Lys Tyr Ser Leu Gln Ala Ser Ser Gln Thr Leu Thr
                565                 570                 575

Leu Thr Val Thr Ser Arg Ala Ser Asn Ala Thr Leu Pro Pro Ile Thr
            580                 585                 590

Val Thr Ser Lys Thr Asn Lys Asp Thr Ser Lys Phe Pro Ser Pro Leu
        595                 600                 605

Val Val Tyr Ala Asn Ile Arg Gln Gly Ala Ser Pro Ile Leu Arg Ala
    610                 615                 620

Ser Val Thr Ala Leu Ile Glu Ser Val Asn Gly Lys Thr Val Thr Leu
625                 630                 635                 640

Glu Leu Leu Asp Asn Gly Ala Gly Ala Asp Ala Thr Lys Asp Asp Gly
                645                 650                 655

Val Tyr Ser Arg Tyr Phe Thr Thr Tyr Asp Thr Asn Gly Arg Tyr Ser
            660                 665                 670

Val Lys Val Arg Ala Leu Gly Gly Val Asn Ala Ala Arg Arg Arg Val
        675                 680                 685
```

```
Ile Pro Gln Gln Ser Gly Ala Leu Tyr Ile Pro Gly Trp Ile Glu Asn
    690                 695                 700

Asp Glu Ile Gln Trp Asn Pro Pro Arg Pro Glu Ile Asn Lys Asp Asp
705                 710                 715                 720

Val Gln His Lys Gln Val Cys Phe Ser Arg Thr Ser Ser Gly Gly Ser
                725                 730                 735

Phe Val Ala Ser Asp Val Pro Asn Ala Pro Ile Pro Asp Leu Phe Pro
            740                 745                 750

Pro Gly Gln Ile Thr Asp Leu Lys Ala Glu Ile His Gly Gly Ser Leu
        755                 760                 765

Ile Asn Leu Thr Trp Thr Ala Pro Gly Asp Asp Tyr Asp His Gly Thr
    770                 775                 780

Ala His Lys Tyr Ile Ile Arg Ile Ser Thr Ser Ile Leu Asp Leu Arg
785                 790                 795                 800

Asp Lys Phe Asn Glu Ser Leu Gln Val Asn Thr Thr Ala Leu Ile Pro
                805                 810                 815

Lys Glu Ala Asn Ser Glu Glu Val Phe Leu Phe Lys Pro Glu Asn Ile
            820                 825                 830

Thr Phe Glu Asn Gly Thr Asp Leu Phe Ile Ala Ile Gln Ala Val Asp
        835                 840                 845

Lys Val Asp Leu Lys Ser Glu Ile Ser Asn Ile Ala Arg Val Ser Leu
    850                 855                 860

Phe Ile Pro Pro Gln Thr Pro Pro Glu Thr Pro Ser Pro Asp Glu Thr
865                 870                 875                 880

Ser Ala Pro Cys Pro Asn Ile His Ile Asn Ser Thr Ile Pro Gly Ile
                885                 890                 895

His Ile Leu Lys Ile Met Trp Lys Trp Ile Gly Glu Leu Gln Leu Ser
            900                 905                 910

Ile Ala
```

<210> 12
<211> 141

<212> PRT
<213> Homo sapiens

<400> 12
Met Ala Trp Pro Leu Cys Thr Leu Leu Leu Leu Leu Ala Thr Gln Ala
1 5 10 15

Val Ala Leu Ala Trp Ser Pro Gln Glu Glu Asp Arg Ile Ile Glu Gly
20 25 30

Gly Ile Tyr Asp Ala Asp Leu Asn Asp Glu Arg Val Gln Arg Ala Leu
35 40 45

His Phe Val Ile Ser Glu Tyr Asn Lys Ala Thr Glu Asp Glu Tyr Tyr
50 55 60

Arg Arg Leu Leu Arg Val Leu Arg Ala Arg Glu Gln Ile Val Gly Gly
65 70 75 80

Val Asn Tyr Phe Phe Asp Ile Glu Val Gly Arg Thr Ile Cys Thr Lys
85 90 95

Ser Gln Pro Asn Leu Asp Thr Cys Ala Phe His Glu Gln Pro Glu Leu
100 105 110

Gln Lys Lys Gln Leu Cys Ser Phe Gln Ile Tyr Glu Val Pro Trp Glu
115 120 125

Asp Arg Met Ser Leu Val Asn Ser Arg Cys Gln Glu Ala
130 135 140

<210> 13
<211> 134
<212> PRT
<213> Homo sapiens

<400> 13
Met Leu Leu Val Leu Leu Ser Val Val Leu Leu Ala Leu Ser Ser Ala
1 5 10 15

Gln Ser Thr Asp Asn Asp Val Asn Tyr Glu Asp Phe Thr Phe Thr Ile
20 25 30

Pro Asp Val Glu Asp Ser Ser Gln Arg Pro Asp Gln Gly Pro Gln Arg
35 40 45

Pro Pro Pro Glu Gly Leu Leu Pro Arg Pro Pro Gly Asp Ser Gly Asn
50 55 60

Gln Asp Asp Gly Pro Gln Gln Arg Pro Pro Lys Pro Gly Gly His His

65 70 75 80

Arg His Pro Pro Pro Pro Pro Phe Gln Asn Gln Gln Arg Pro Pro Arg
85 90 95

Arg Gly His Arg Gln Leu Ser Leu Pro Arg Phe Pro Ser Val Ser Leu
100 105 110

Gln Glu Ala Ser Ser Phe Phe Arg Arg Asp Arg Pro Ala Arg His Pro
115 120 125

Gln Glu Gln Pro Leu Trp
130

<210> 14
<211> 415
<212> PRT
<213> Homo sapiens

<400> 14
Met Glu Asp Leu Cys Val Ala Asn Thr Leu Phe Ala Leu Asn Leu Phe
1 5 10 15

Lys His Leu Ala Lys Ala Ser Pro Thr Gln Asn Leu Phe Leu Ser Pro
20 25 30

Trp Ser Ile Ser Ser Thr Met Ala Met Val Tyr Met Gly Ser Arg Gly
35 40 45

Ser Thr Glu Asp Gln Met Ala Lys Val Leu Gln Phe Asn Glu Val Gly
50 55 60

Ala Asn Ala Val Thr Pro Met Thr Pro Glu Asn Phe Thr Ser Cys Gly
65 70 75 80

Phe Met Gln Gln Ile Gln Lys Gly Ser Tyr Pro Asp Ala Ile Leu Gln
85 90 95

Ala Gln Ala Ala Asp Lys Ile His Ser Ser Phe Arg Ser Leu Ser Ser
100 105 110

Ala Ile Asn Ala Ser Thr Gly Asn Tyr Leu Leu Glu Ser Val Asn Lys
115 120 125

Leu Phe Gly Glu Lys Ser Ala Ser Phe Arg Glu Glu Tyr Ile Arg Leu
130 135 140

Cys Gln Lys Tyr Tyr Ser Ser Glu Pro Gln Ala Val Asp Phe Leu Glu
145 150 155 160

Cys Ala Glu Glu Ala Arg Lys Lys Ile Asn Ser Trp Val Lys Thr Gln
165 170 175

Thr Lys Gly Lys Ile Pro Asn Leu Leu Pro Glu Gly Ser Val Asp Gly
180 185 190

Asp Thr Arg Met Val Leu Val Asn Ala Val Tyr Phe Lys Gly Lys Trp
195 200 205

Lys Thr Pro Phe Glu Lys Lys Leu Asn Gly Leu Tyr Pro Phe Arg Val
210 215 220

Asn Ser Ala Gln Arg Thr Pro Val Gln Met Met Tyr Leu Arg Glu Lys
225 230 235 240

Leu Asn Ile Gly Tyr Ile Glu Asp Leu Lys Ala Gln Ile Leu Glu Leu
245 250 255

Pro Tyr Ala Gly Asp Val Ser Met Phe Leu Leu Leu Pro Asp Glu Ile
260 265 270

Ala Asp Val Ser Thr Gly Leu Glu Leu Leu Glu Ser Glu Ile Thr Tyr
275 280 285

Asp Lys Leu Asn Lys Trp Thr Ser Lys Asp Lys Met Ala Glu Asp Glu
290 295 300

Val Glu Val Tyr Ile Pro Gln Phe Lys Leu Glu Glu His Tyr Glu Leu
305 310 315 320

Arg Ser Ile Leu Arg Ser Met Gly Met Glu Asp Ala Phe Asn Lys Gly
325 330 335

Arg Ala Asn Phe Ser Gly Met Ser Glu Arg Asn Asp Leu Phe Leu Ser
340 345 350

Glu Val Phe His Gln Ala Met Val Asp Val Asn Glu Glu Gly Thr Glu
355 360 365

Ala Ala Ala Gly Thr Gly Gly Val Met Thr Gly Arg Thr Gly His Gly
370 375 380

Gly Pro Gln Phe Val Ala Asp His Pro Phe Leu Phe Leu Ile Met His
385 390 395 400

Lys Ile Thr Asn Cys Ile Leu Phe Phe Gly Arg Phe Ser Ser Pro

405 410 415

<210> 15
<211> 702
<212> PRT
<213> Homo sapiens

<400> 15
Met Glu Ser Pro Ser Ala Pro Pro His Arg Trp Cys Ile Pro Trp Gln
1 5 10 15

Arg Leu Leu Leu Thr Ala Ser Leu Leu Thr Phe Trp Asn Pro Pro Thr
20 25 30

Thr Ala Lys Leu Thr Ile Glu Ser Thr Pro Phe Asn Val Ala Glu Gly
35 40 45

Lys Glu Val Leu Leu Leu Val His Asn Leu Pro Gln His Leu Phe Gly
50 55 60

Tyr Ser Trp Tyr Lys Gly Glu Arg Val Asp Gly Asn Arg Gln Ile Ile
65 70 75 80

Gly Tyr Val Ile Gly Thr Gln Gln Ala Thr Pro Gly Pro Ala Tyr Ser
85 90 95

Gly Arg Glu Ile Ile Tyr Pro Asn Ala Ser Leu Leu Ile Gln Asn Ile
100 105 110

Ile Gln Asn Asp Thr Gly Phe Tyr Thr Leu His Val Ile Lys Ser Asp
115 120 125

Leu Val Asn Glu Glu Ala Thr Gly Gln Phe Arg Val Tyr Pro Glu Leu
130 135 140

Pro Lys Pro Ser Ile Ser Ser Asn Asn Ser Lys Pro Val Glu Asp Lys
145 150 155 160

Asp Ala Val Ala Phe Thr Cys Glu Pro Glu Thr Gln Asp Ala Thr Tyr
165 170 175

Leu Trp Trp Val Asn Asn Gln Ser Leu Pro Val Ser Pro Arg Leu Gln
180 185 190

Leu Ser Asn Gly Asn Arg Thr Leu Thr Leu Phe Asn Val Thr Arg Asn
195 200 205

Asp Thr Ala Ser Tyr Lys Cys Glu Thr Gln Asn Pro Val Ser Ala Arg
210 215 220

Arg Ser Asp Ser Val Ile Leu Asn Val Leu Tyr Gly Pro Asp Ala Pro
225 230 235 240

Thr Ile Ser Pro Leu Asn Thr Ser Tyr Arg Ser Gly Glu Asn Leu Asn
245 250 255

Leu Ser Cys His Ala Ala Ser Asn Pro Pro Ala Gln Tyr Ser Trp Phe
260 265 270

Val Asn Gly Thr Phe Gln Gln Ser Thr Gln Glu Leu Phe Ile Pro Asn
275 280 285

Ile Thr Val Asn Asn Ser Gly Ser Tyr Thr Cys Gln Ala His Asn Ser
290 295 300

Asp Thr Gly Leu Asn Arg Thr Thr Val Thr Thr Ile Thr Val Tyr Ala
305 310 315 320

Glu Pro Pro Lys Pro Phe Ile Thr Ser Asn Asn Ser Asn Pro Val Glu
325 330 335

Asp Glu Asp Ala Val Ala Leu Thr Cys Glu Pro Glu Ile Gln Asn Thr
340 345 350

Thr Tyr Leu Trp Trp Val Asn Asn Gln Ser Leu Pro Val Ser Pro Arg
355 360 365

Leu Gln Leu Ser Asn Asp Asn Arg Thr Leu Thr Leu Leu Ser Val Thr
370 375 380

Arg Asn Asp Val Gly Pro Tyr Glu Cys Gly Ile Gln Asn Glu Leu Ser
385 390 395 400

Val Asp His Ser Asp Pro Val Ile Leu Asn Val Leu Tyr Gly Pro Asp
405 410 415

Asp Pro Thr Ile Ser Pro Ser Tyr Thr Tyr Tyr Arg Pro Gly Val Asn
420 425 430

Leu Ser Leu Ser Cys His Ala Ala Ser Asn Pro Pro Ala Gln Tyr Ser
435 440 445

Trp Leu Ile Asp Gly Asn Ile Gln Gln His Thr Gln Glu Leu Phe Ile
450 455 460

Ser Asn Ile Thr Glu Lys Asn Ser Gly Leu Tyr Thr Cys Gln Ala Asn

465 470 475 480

Asn Ser Ala Ser Gly His Ser Arg Thr Thr Val Lys Thr Ile Thr Val
485 490 495

Ser Ala Glu Leu Pro Lys Pro Ser Ile Ser Ser Asn Asn Ser Lys Pro
500 505 510

Val Glu Asp Lys Asp Ala Val Ala Phe Thr Cys Glu Pro Glu Ala Gln
515 520 525

Asn Thr Thr Tyr Leu Trp Trp Val Asn Gly Gln Ser Leu Pro Val Ser
530 535 540

Pro Arg Leu Gln Leu Ser Asn Gly Asn Arg Thr Leu Thr Leu Phe Asn
545 550 555 560

Val Thr Arg Asn Asp Ala Arg Ala Tyr Val Cys Gly Ile Gln Asn Ser
565 570 575

Val Ser Ala Asn Arg Ser Asp Pro Val Thr Leu Asp Val Leu Tyr Gly
580 585 590

Pro Asp Thr Pro Ile Ile Ser Pro Pro Asp Ser Ser Tyr Leu Ser Gly
595 600 605

Ala Asn Leu Asn Leu Ser Cys His Ser Ala Ser Asn Pro Ser Pro Gln
610 615 620

Tyr Ser Trp Arg Ile Asn Gly Ile Pro Gln Gln His Thr Gln Val Leu
625 630 635 640

Phe Ile Ala Lys Ile Thr Pro Asn Asn Asn Gly Thr Tyr Ala Cys Phe
645 650 655

Val Ser Asn Leu Ala Thr Gly Arg Asn Asn Ser Ile Val Lys Ser Ile
660 665 670

Thr Val Ser Ala Ser Gly Thr Ser Pro Gly Leu Ser Ala Gly Ala Thr
675 680 685

Val Gly Ile Met Ile Gly Val Leu Val Gly Val Ala Leu Ile
690 695 700

<210> 16
<211> 1153
<212> PRT
<213> Homo sapiens

<400> 16

```
Met Ala Cys Pro Trp Lys Phe Leu Phe Lys Thr Lys Phe His Gln Tyr
1               5                   10                  15

Ala Met Asn Gly Glu Lys Asp Ile Asn Asn Asn Val Glu Lys Ala Pro
            20                  25                  30

Cys Ala Thr Ser Ser Pro Val Thr Gln Asp Asp Leu Gln Tyr His Asn
        35                  40                  45

Leu Ser Lys Gln Gln Asn Glu Ser Pro Gln Pro Leu Val Glu Thr Gly
    50                  55                  60

Lys Lys Ser Pro Glu Ser Leu Val Lys Leu Asp Ala Thr Pro Leu Ser
65                  70                  75                  80

Ser Pro Arg His Val Arg Ile Lys Asn Trp Gly Ser Gly Met Thr Phe
                85                  90                  95

Gln Asp Thr Leu His His Lys Ala Lys Gly Ile Leu Thr Cys Arg Ser
            100                 105                 110

Lys Ser Cys Leu Gly Ser Ile Met Thr Pro Lys Ser Leu Thr Arg Gly
        115                 120                 125

Pro Arg Asp Lys Pro Thr Pro Pro Asp Glu Leu Leu Pro Gln Ala Ile
    130                 135                 140

Glu Phe Val Asn Gln Tyr Tyr Gly Ser Phe Lys Glu Ala Lys Ile Glu
145                 150                 155                 160

Glu His Leu Ala Arg Val Glu Ala Val Thr Lys Glu Ile Glu Thr Thr
                165                 170                 175

Gly Thr Tyr Gln Leu Thr Gly Asp Glu Leu Ile Phe Ala Thr Lys Gln
            180                 185                 190

Ala Trp Arg Asn Ala Pro Arg Cys Ile Gly Arg Ile Gln Trp Ser Asn
        195                 200                 205

Leu Gln Val Phe Asp Ala Arg Ser Cys Ser Thr Ala Arg Glu Met Phe
    210                 215                 220

Glu His Ile Cys Arg His Val Arg Tyr Ser Thr Asn Asn Gly Asn Ile
225                 230                 235                 240

Arg Ser Ala Ile Thr Val Phe Pro Gln Arg Ser Asp Gly Lys His Asp
```

245 250 255

Phe Arg Val Trp Asn Ala Gln Leu Ile Arg Tyr Ala Gly Tyr Gln Met
260 265 270

Pro Asp Gly Ser Ile Arg Gly Asp Pro Ala Asn Val Glu Phe Thr Gln
275 280 285

Leu Cys Ile Asp Leu Gly Trp Lys Pro Lys Tyr Gly Arg Phe Asp Val
290 295 300

Val Pro Leu Val Leu Gln Ala Asn Gly Arg Asp Pro Glu Leu Phe Glu
305 310 315 320

Ile Pro Pro Asp Leu Val Leu Glu Val Ala Met Glu His Pro Lys Tyr
325 330 335

Glu Trp Phe Arg Glu Leu Glu Leu Lys Trp Tyr Ala Leu Pro Ala Val
340 345 350

Ala Asn Met Leu Leu Glu Val Gly Gly Leu Glu Phe Pro Gly Cys Pro
355 360 365

Phe Asn Gly Trp Tyr Met Gly Thr Glu Ile Gly Val Arg Asp Phe Cys
370 375 380

Asp Val Gln Arg Tyr Asn Ile Leu Glu Glu Val Gly Arg Arg Met Gly
385 390 395 400

Leu Glu Thr His Lys Leu Ala Ser Leu Trp Lys Asp Gln Ala Val Val
405 410 415

Glu Ile Asn Ile Ala Val Leu His Ser Phe Gln Lys Gln Asn Val Thr
420 425 430

Ile Met Asp His His Ser Ala Ala Glu Ser Phe Met Lys Tyr Met Gln
435 440 445

Asn Glu Tyr Arg Ser Arg Gly Gly Cys Pro Ala Asp Trp Ile Trp Leu
450 455 460

Val Pro Pro Met Ser Gly Ser Ile Thr Pro Val Phe His Gln Glu Met
465 470 475 480

Leu Asn Tyr Val Leu Ser Pro Phe Tyr Tyr Tyr Gln Val Glu Ala Trp
485 490 495

Lys Thr His Val Trp Gln Asp Glu Lys Arg Arg Pro Lys Arg Arg Glu
500 505 510

Ile Pro Leu Lys Val Leu Val Lys Ala Val Leu Phe Ala Cys Met Leu
515 520 525

Met Arg Lys Thr Met Ala Ser Arg Val Arg Val Thr Ile Leu Phe Ala
530 535 540

Thr Glu Thr Gly Lys Ser Glu Ala Leu Ala Trp Asp Leu Gly Ala Leu
545 550 555 560

Phe Ser Cys Ala Phe Asn Pro Lys Val Val Cys Met Asp Lys Tyr Arg
565 570 575

Leu Ser Cys Leu Glu Glu Glu Arg Leu Leu Leu Val Val Thr Ser Thr
580 585 590

Phe Gly Asn Gly Asp Cys Pro Gly Asn Gly Glu Lys Leu Lys Lys Ser
595 600 605

Leu Phe Met Leu Lys Glu Leu Asn Asn Lys Phe Arg Tyr Ala Val Phe
610 615 620

Gly Leu Gly Ser Ser Met Tyr Pro Arg Phe Cys Ala Phe Ala His Asp
625 630 635 640

Ile Asp Gln Lys Leu Ser His Leu Gly Ala Ser Gln Leu Thr Pro Met
645 650 655

Gly Glu Gly Asp Glu Leu Ser Gly Gln Glu Asp Ala Phe Arg Ser Trp
660 665 670

Ala Val Gln Thr Phe Lys Ala Ala Cys Glu Thr Phe Asp Val Arg Gly
675 680 685

Lys Gln His Ile Gln Ile Pro Lys Leu Tyr Thr Ser Asn Val Thr Trp
690 695 700

Asp Pro His His Tyr Arg Leu Val Gln Asp Ser Gln Pro Leu Asp Leu
705 710 715 720

Ser Lys Ala Leu Ser Ser Met His Ala Lys Asn Val Phe Thr Met Arg
725 730 735

Leu Lys Ser Arg Gln Asn Leu Gln Ser Pro Thr Ser Ser Arg Ala Thr
740 745 750

Ile Leu Val Glu Leu Ser Cys Glu Asp Gly Gln Gly Leu Asn Tyr Leu
755 760 765

Pro Gly Glu His Leu Gly Val Cys Pro Gly Asn Gln Pro Ala Leu Val
770 775 780

Gln Gly Ile Leu Glu Arg Val Val Asp Gly Pro Thr Pro His Gln Thr
785 790 795 800

Val Arg Leu Glu Ala Leu Asp Glu Ser Gly Ser Tyr Trp Val Ser Asp
805 810 815

Lys Arg Leu Pro Pro Cys Ser Leu Ser Gln Ala Leu Thr Tyr Phe Leu
820 825 830

Asp Ile Thr Thr Pro Pro Thr Gln Leu Leu Leu Gln Lys Leu Ala Gln
835 840 845

Val Ala Thr Glu Glu Pro Glu Arg Gln Arg Leu Glu Ala Leu Cys Gln
850 855 860

Pro Ser Glu Tyr Ser Lys Trp Lys Phe Thr Asn Ser Pro Thr Phe Leu
865 870 875 880

Glu Val Leu Glu Glu Phe Pro Ser Leu Arg Val Ser Ala Gly Phe Leu
885 890 895

Leu Ser Gln Leu Pro Ile Leu Lys Pro Arg Phe Tyr Ser Ile Ser Ser
900 905 910

Ser Arg Asp His Thr Pro Thr Glu Ile His Leu Thr Val Ala Val Val
915 920 925

Thr Tyr His Thr Arg Asp Gly Gln Gly Pro Leu His His Gly Val Cys
930 935 940

Ser Thr Trp Leu Asn Ser Leu Lys Pro Gln Asp Pro Val Pro Cys Phe
945 950 955 960

Val Arg Asn Ala Ser Gly Phe His Leu Pro Glu Asp Pro Ser His Pro
965 970 975

Cys Ile Leu Ile Gly Pro Gly Thr Gly Ile Ala Pro Phe Arg Ser Phe
980 985 990

Trp Gln Gln Arg Leu His Asp Ser Gln His Lys Gly Val Arg Gly Gly
995 1000 1005

Arg Met Thr Leu Val Phe Gly Cys Arg Arg Pro Asp Glu Asp His
1010 1015 1020

Ile Tyr Gln Glu Glu Met Leu Glu Met Ala Gln Lys Gly Val Leu
1025 1030 1035

His Ala Val His Thr Ala Tyr Ser Arg Leu Pro Gly Lys Pro Lys
1040 1045 1050

Val Tyr Val Gln Asp Ile Leu Arg Gln Gln Leu Ala Ser Glu Val
1055 1060 1065

Leu Arg Val Leu His Lys Glu Pro Gly His Leu Tyr Val Cys Gly
1070 1075 1080

Asp Val Arg Met Ala Arg Asp Val Ala His Thr Leu Lys Gln Leu
1085 1090 1095

Val Ala Ala Lys Leu Lys Leu Asn Glu Glu Gln Val Glu Asp Tyr
1100 1105 1110

Phe Phe Gln Leu Lys Ser Gln Lys Arg Tyr His Glu Asp Ile Phe
1115 1120 1125

Gly Ala Val Phe Pro Tyr Glu Ala Lys Lys Asp Arg Val Ala Val
1130 1135 1140

Gln Pro Ser Ser Leu Glu Met Ser Ala Leu
1145 1150

<210> 17
<211> 390
<212> PRT
<213> Homo sapiens

<400> 17
Met Asn Ser Leu Ser Glu Ala Asn Thr Lys Phe Met Phe Asp Leu Phe
1 5 10 15

Gln Gln Phe Arg Lys Ser Lys Glu Asn Asn Ile Phe Tyr Ser Pro Ile
20 25 30

Ser Ile Thr Ser Ala Leu Gly Met Val Leu Leu Gly Ala Lys Asp Asn
35 40 45

Thr Ala Gln Gln Ile Ser Lys Val Leu His Phe Asp Gln Val Thr Glu
50 55 60

Asn Thr Thr Glu Lys Ala Ala Thr Tyr His Val Asp Arg Ser Gly Asn
65 70 75 80

Val His His Gln Phe Gln Lys Leu Leu Thr Glu Phe Asn Lys Ser Thr
85 90 95

Asp Ala Tyr Glu Leu Lys Ile Ala Asn Lys Leu Phe Gly Glu Lys Thr
100 105 110

Tyr Gln Phe Leu Gln Glu Tyr Leu Asp Ala Ile Lys Lys Phe Tyr Gln
115 120 125

Thr Ser Val Glu Ser Thr Asp Phe Ala Asn Ala Pro Glu Glu Ser Arg
130 135 140

Lys Lys Ile Asn Ser Trp Val Glu Ser Gln Thr Asn Glu Lys Ile Lys
145 150 155 160

Asn Leu Phe Pro Asp Gly Thr Ile Gly Asn Asp Thr Thr Leu Val Leu
165 170 175

Val Asn Ala Ile Tyr Phe Lys Gly Gln Trp Glu Asn Lys Phe Lys Lys
180 185 190

Glu Asn Thr Lys Glu Glu Lys Phe Trp Pro Asn Lys Asn Thr Tyr Lys
195 200 205

Ser Val Gln Met Met Arg Gln Tyr Asn Ser Phe Asn Phe Ala Leu Leu
210 215 220

Glu Asp Val Gln Ala Lys Val Leu Glu Ile Pro Tyr Lys Gly Lys Asp
225 230 235 240

Leu Ser Met Ile Val Leu Leu Pro Asn Glu Ile Asp Gly Leu Gln Lys
245 250 255

Leu Glu Glu Lys Leu Thr Ala Glu Lys Leu Met Glu Trp Thr Ser Leu
260 265 270

Gln Asn Met Arg Glu Thr Cys Val Asp Leu His Leu Pro Arg Phe Lys
275 280 285

Met Glu Glu Ser Tyr Asp Leu Lys Asp Thr Leu Arg Thr Met Gly Met
290 295 300

Val Asn Ile Phe Asn Gly Asp Ala Asp Leu Ser Gly Met Thr Trp Ser
305 310 315 320

His Gly Leu Ser Val Ser Lys Val Leu His Lys Ala Phe Val Glu Val
325 330 335

Thr Glu Glu Gly Val Glu Ala Ala Ala Ala Thr Ala Val Val Val Val
340 345 350

Glu Leu Ser Ser Pro Ser Thr Asn Glu Glu Phe Cys Cys Asn His Pro
355 360 365

Phe Leu Phe Phe Ile Arg Gln Asn Lys Thr Asn Ser Ile Leu Phe Tyr
370 375 380

Gly Arg Phe Ser Ser Pro
385 390

<210> 18
<211> 141
<212> PRT
<213> Homo sapiens

<400> 18
Met Ala Arg Pro Leu Cys Thr Leu Leu Leu Leu Met Ala Thr Leu Ala
1 5 10 15

Gly Ala Leu Ala Ser Ser Ser Lys Glu Glu Asn Arg Ile Ile Pro Gly
20 25 30

Gly Ile Tyr Asp Ala Asp Leu Asn Asp Glu Trp Val Gln Arg Ala Leu
35 40 45

His Phe Ala Ile Ser Glu Tyr Asn Lys Ala Thr Glu Asp Glu Tyr Tyr
50 55 60

Arg Arg Pro Leu Gln Val Leu Arg Ala Arg Glu Gln Thr Phe Gly Gly
65 70 75 80

Val Asn Tyr Phe Phe Asp Val Glu Val Gly Arg Thr Ile Cys Thr Lys
85 90 95

Ser Gln Pro Asn Leu Asp Thr Cys Ala Phe His Glu Gln Pro Glu Leu
100 105 110

Gln Lys Lys Gln Leu Cys Ser Phe Glu Ile Tyr Glu Val Pro Trp Glu
115 120 125

Asp Arg Met Ser Leu Val Asn Ser Arg Cys Gln Glu Ala
130 135 140

```
<210> 19
<211> 393
<212> PRT
<213> Homo sapiens

<400> 19
Met Leu Leu Ile Leu Leu Ser Val Ala Leu Leu Ala Leu Ser Ser Ala
1               5                   10                  15

Gln Asn Leu Asn Glu Asp Val Ser Gln Glu Glu Ser Pro Ser Leu Ile
            20                  25                  30

Ala Gly Lys Pro Gln Gly Pro Pro Pro Gln Gly Gly Asn Gln Pro Gln
        35                  40                  45

Gly Pro Pro Pro Pro Pro Gly Lys Pro Gln Gly Pro Pro Pro Gln Gly
    50                  55                  60

Gly Asn Lys Pro Gln Gly Pro Pro Pro Pro Gly Lys Pro Gln Gly Pro
65                  70                  75                  80

Pro Pro Gln Gly Asp Lys Ser Arg Ser Pro Arg Ser Pro Pro Gly Lys
                85                  90                  95

Pro Gln Gly Pro Pro Pro Gln Gly Gly Asn Gln Pro Gln Gly Pro Pro
            100                 105                 110

Pro Pro Pro Gly Lys Pro Gln Gly Pro Pro Pro Gln Gly Gly Asn Lys
        115                 120                 125

Pro Gln Gly Pro Pro Pro Pro Gly Lys Pro Gln Gly Pro Pro Pro Gln
    130                 135                 140

Gly Asp Asn Lys Ser Arg Ser Ser Arg Ser Pro Pro Gly Lys Pro Gln
145                 150                 155                 160

Gly Pro Pro Pro Gln Gly Gly Asn Gln Pro Gln Gly Pro Pro Pro Pro
                165                 170                 175

Pro Gly Lys Pro Gln Gly Pro Pro Pro Gln Gly Gly Asn Lys Pro Gln
            180                 185                 190

Gly Pro Pro Pro Pro Gly Lys Pro Gln Gly Pro Pro Pro Gln Gly Asp
        195                 200                 205

Lys Ser Arg Ser Pro Arg Ser Pro Pro Gly Lys Pro Gln Gly Pro Pro
    210                 215                 220
```

Pro Gln Gly Gly Asn Gln Pro Gln Gly Pro Pro Pro Pro Pro Gly Lys
225 230 235 240

Pro Gln Gly Pro Pro Pro Gln Gly Gly Asn Arg Pro Gln Gly Pro Pro
245 250 255

Pro Pro Gly Lys Pro Gln Gly Pro Pro Pro Gln Gly Asp Lys Ser Arg
260 265 270

Ser Ser Gln Ser Pro Pro Gly Lys Pro Gln Gly Pro Pro Pro Gln Gly
275 280 285

Gly Asn Gln Pro Gln Gly Pro Pro Pro Pro Pro Gly Lys Pro Gln Gly
290 295 300

Pro Pro Pro Gln Gly Gly Asn Lys Pro Gln Gly Pro Pro Pro Pro Gly
305 310 315 320

Lys Pro Gln Gly Pro Pro Ala Gln Gly Gly Ser Lys Ser Gln Ser Ala
325 330 335

Arg Ser Pro Pro Gly Lys Pro Gln Gly Pro Pro Gln Gln Glu Gly Asn
340 345 350

Asn Pro Gln Gly Pro Pro Pro Pro Ala Gly Gly Asn Pro Gln Gln Pro
355 360 365

Gln Ala Pro Pro Ala Gly Gln Pro Gln Gly Pro Pro Arg Pro Pro Gln
370 375 380

Gly Gly Arg Pro Ser Arg Pro Pro Gln
385 390

<210> 20
<211> 235
<212> PRT
<213> Homo sapiens

<400> 20
Met Leu Ser Leu Leu Leu Leu Ala Leu Pro Val Leu Ala Ser Pro Ala
1 5 10 15

Tyr Val Ala Pro Ala Pro Gly Gln Ala Leu Gln Gln Thr Gly Ile Val
20 25 30

Gly Gly Gln Glu Ala Pro Arg Ser Lys Trp Pro Trp Gln Val Ser Leu
35 40 45

Arg Val Arg Gly Pro Tyr Trp Met His Phe Cys Gly Gly Ser Leu Ile

50 55 60

His Pro Gln Trp Val Leu Thr Ala Ala His Cys Val Glu Pro Asp Ile
65 70 75 80

Lys Asp Leu Ala Ala Leu Arg Val Gln Leu Arg Glu Gln His Leu Tyr
85 90 95

Tyr Gln Asp Gln Leu Leu Pro Val Ser Arg Ile Ile Val His Pro Gln
100 105 110

Phe Tyr Ile Ile Gln Thr Gly Ala Asp Ile Ala Leu Leu Glu Leu Glu
115 120 125

Glu Pro Val Asn Ile Ser Ser His Ile His Thr Val Thr Leu Pro Pro
130 135 140

Ala Ser Glu Thr Phe Pro Pro Gly Met Pro Cys Trp Val Thr Gly Trp
145 150 155 160

Gly Asp Val Asp Asn Asn Val His Leu Pro Pro Pro Tyr Pro Leu Lys
165 170 175

Glu Val Glu Val Pro Val Val Glu Asn His Leu Cys Asn Ala Glu Tyr
180 185 190

His Thr Gly Leu His Thr Gly His Ser Phe Gln Ile Val Arg Asp Asp
195 200 205

Met Leu Cys Ala Gly Ser Glu Asn His Asp Ser Cys Gln Gly Asp Ser
210 215 220

Gly Gly Pro Leu Val Cys Lys Val Asn Gly Thr
225 230 235

<210> 21
<211> 321
<212> PRT
<213> Homo sapiens

<400> 21
Met Ala Leu Gly Ala Cys Gly Leu Leu Leu Leu Leu Ala Val Pro Gly
1 5 10 15

Val Ser Leu Arg Thr Leu Gln Pro Gly Cys Gly Arg Pro Gln Val Ser
20 25 30

Asp Ala Gly Gly Arg Ile Val Gly Gly His Ala Ala Pro Ala Gly Ala
35 40 45

Trp Pro Trp Gln Ala Ser Leu Arg Leu Arg Arg Met His Val Cys Gly
50 55 60

Gly Ser Leu Leu Ser Pro Gln Trp Val Leu Thr Ala Ala His Cys Phe
65 70 75 80

Ser Gly Ser Leu Asn Ser Ser Asp Tyr Gln Val His Leu Gly Glu Leu
85 90 95

Glu Ile Thr Leu Ser Pro His Phe Ser Thr Val Arg Gln Ile Ile Leu
100 105 110

His Ser Ser Pro Ser Gly Gln Pro Gly Thr Ser Gly Asp Ile Ala Leu
115 120 125

Val Glu Leu Ser Val Pro Val Thr Leu Ser Ser Arg Ile Leu Pro Val
130 135 140

Cys Leu Pro Glu Ala Ser Asp Asp Phe Cys Pro Gly Ile Arg Cys Trp
145 150 155 160

Val Thr Gly Trp Gly Tyr Thr Arg Glu Gly Glu Pro Leu Pro Pro Pro
165 170 175

Tyr Ser Leu Arg Glu Val Lys Val Ser Val Val Asp Thr Glu Thr Cys
180 185 190

Arg Arg Asp Tyr Pro Gly Pro Gly Gly Ser Ile Leu Gln Pro Asp Met
195 200 205

Leu Cys Ala Arg Gly Pro Gly Asp Ala Cys Gln Asp Asp Ser Gly Gly
210 215 220

Pro Leu Val Cys Gln Val Asn Gly Ala Trp Val Gln Ala Gly Ile Val
225 230 235 240

Ser Trp Gly Glu Gly Cys Gly Arg Pro Asn Arg Pro Gly Val Tyr Thr
245 250 255

Arg Val Pro Ala Tyr Val Asn Trp Ile Arg Arg His Ile Thr Ala Ser
260 265 270

Gly Gly Ser Glu Ser Gly Tyr Pro Arg Leu Pro Leu Leu Ala Gly Leu
275 280 285

Phe Leu Pro Gly Leu Phe Leu Leu Leu Val Ser Cys Val Leu Leu Ala

290 295 300

Lys Cys Leu Leu His Pro Ser Ala Asp Gly Thr Pro Phe Pro Ala Pro
305 310 315 320

Asp

<210> 22
<211> 540
<212> PRT
<213> Homo sapiens

<400> 22
Met Ala Lys Gly Glu Gly Ala Glu Ser Gly Ser Ala Ala Gly Leu Leu
1 5 10 15

Pro Thr Ser Ile Leu Gln Ser Thr Glu Arg Pro Ala Gln Val Lys Lys
20 25 30

Glu Pro Lys Lys Lys Lys Gln Gln Leu Ser Val Cys Asn Lys Leu Cys
35 40 45

Tyr Ala Leu Gly Gly Ala Pro Tyr Gln Val Thr Gly Cys Ala Leu Gly
50 55 60

Phe Phe Leu Gln Ile Tyr Leu Leu Asp Val Ala Gln Lys Asp Glu Glu
65 70 75 80

Val Val Phe Cys Phe Ser Ser Phe Gln Val Gly Pro Phe Ser Ala Ser
85 90 95

Ile Ile Leu Phe Val Gly Arg Ala Trp Asp Ala Ile Thr Asp Pro Leu
100 105 110

Val Gly Leu Cys Ile Ser Lys Ser Pro Trp Thr Cys Leu Gly Arg Leu
115 120 125

Met Pro Trp Ile Ile Phe Ser Thr Pro Leu Ala Val Ile Ala Tyr Phe
130 135 140

Leu Ile Trp Phe Val Pro Asp Phe Pro His Gly Gln Thr Tyr Trp Tyr
145 150 155 160

Leu Leu Phe Tyr Cys Leu Phe Glu Thr Met Val Thr Cys Phe His Val
165 170 175

Pro Tyr Ser Ala Leu Thr Met Phe Ile Ser Thr Glu Gln Thr Glu Arg
180 185 190

Asp Ser Ala Thr Ala Tyr Arg Met Thr Val Glu Val Leu Gly Thr Val
195 200 205

Leu Gly Thr Ala Ile Gln Gly Gln Ile Val Gly Gln Ala Asp Thr Pro
210 215 220

Cys Phe Gln Asp Leu Asn Ser Ser Thr Val Ala Ser Gln Ser Ala Asn
225 230 235 240

His Thr His Gly Thr Thr Ser His Arg Glu Thr Gln Lys Ala Tyr Leu
245 250 255

Leu Ala Ala Gly Val Ile Val Cys Ile Tyr Ile Ile Cys Ala Val Ile
260 265 270

Leu Ile Leu Gly Val Arg Glu Gln Arg Glu Pro Tyr Glu Ala Gln Gln
275 280 285

Ser Glu Pro Ile Ala Tyr Phe Arg Gly Leu Arg Leu Val Met Ser His
290 295 300

Gly Pro Tyr Ile Lys Leu Ile Thr Gly Phe Leu Phe Thr Ser Leu Ala
305 310 315 320

Phe Met Leu Val Glu Gly Asn Phe Val Leu Phe Cys Thr Tyr Thr Leu
325 330 335

Gly Phe Arg Asn Glu Phe Gln Asn Leu Leu Leu Ala Ile Met Leu Ser
340 345 350

Ala Thr Leu Thr Ile Pro Ile Trp Gln Trp Phe Leu Thr Arg Phe Gly
355 360 365

Lys Lys Thr Ala Val Tyr Val Gly Ile Ser Ser Ala Val Pro Phe Leu
370 375 380

Ile Leu Val Ala Leu Met Glu Ser Asn Leu Ile Ile Thr Tyr Ala Val
385 390 395 400

Ala Val Ala Ala Gly Ile Ser Val Ala Ala Ala Phe Leu Leu Pro Trp
405 410 415

Ser Met Leu Pro Asp Val Ile Asp Asp Phe His Leu Lys Gln Pro His
420 425 430

Phe His Gly Thr Glu Pro Ile Phe Phe Ser Phe Tyr Val Phe Phe Thr

435 440 445

Lys Phe Ala Ser Gly Val Ser Leu Gly Ile Ser Thr Leu Ser Leu Asp
450 455 460

Phe Ala Gly Tyr Gln Thr Arg Gly Cys Ser Gln Pro Glu Arg Val Lys
465 470 475 480

Phe Thr Leu Asn Met Leu Val Thr Met Ala Pro Ile Val Leu Ile Leu
485 490 495

Leu Gly Leu Leu Leu Phe Lys Met Tyr Pro Ile Asp Glu Glu Arg Arg
500 505 510

Arg Gln Asn Lys Lys Ala Leu Gln Ala Leu Arg Asp Glu Ala Ser Ser
515 520 525

Ser Gly Cys Ser Glu Thr Asp Ser Thr Glu Leu Ala
530 535 540

<210> 23
<211> 417
<212> PRT
<213> Homo sapiens

<400> 23
Met Arg Leu Ile Leu Pro Val Gly Leu Ile Ala Thr Thr Leu Ala Ile
1 5 10 15

Ala Pro Val Arg Phe Asp Arg Glu Lys Val Phe Arg Val Lys Pro Gln
20 25 30

Asp Glu Lys Gln Ala Asp Ile Ile Lys Asp Leu Ala Lys Thr Asn Glu
35 40 45

Leu Asp Phe Trp Tyr Pro Gly Ala Thr His His Val Ala Ala Asn Met
50 55 60

Met Val Asp Phe Arg Val Ser Glu Lys Glu Ser Gln Ala Ile Gln Ser
65 70 75 80

Ala Leu Asp Gln Asn Lys Met His Tyr Glu Ile Leu Ile His Asp Leu
85 90 95

Gln Glu Glu Ile Glu Lys Gln Phe Asp Val Lys Glu Asp Ile Pro Gly
100 105 110

Arg His Ser Tyr Ala Lys Tyr Asn Asn Trp Glu Lys Ile Val Ala Trp
115 120 125

```
Thr Glu Lys Met Met Asp Lys Tyr Pro Glu Met Val Ser Arg Ile Lys
    130                 135                 140

Ile Gly Ser Thr Val Glu Asp Asn Pro Leu Tyr Val Leu Lys Ile Gly
145                 150                 155                 160

Glu Lys Asn Glu Arg Arg Lys Ala Ile Phe Met Asp Cys Gly Ile His
                165                 170                 175

Ala Arg Glu Trp Val Ser Pro Ala Phe Cys Gln Trp Phe Val Tyr Gln
            180                 185                 190

Ala Thr Lys Thr Tyr Gly Arg Asn Lys Ile Met Thr Lys Leu Leu Asp
        195                 200                 205

Arg Met Asn Phe Tyr Ile Leu Pro Val Phe Asn Val Asp Gly Tyr Ile
    210                 215                 220

Trp Ser Trp Thr Lys Asn Arg Met Trp Arg Lys Asn Arg Ser Lys Asn
225                 230                 235                 240

Gln Asn Ser Lys Cys Ile Gly Thr Asp Leu Asn Arg Asn Phe Asn Ala
                245                 250                 255

Ser Trp Asn Ser Ile Pro Asn Thr Asn Asp Pro Cys Ala Asp Asn Tyr
            260                 265                 270

Arg Gly Ser Ala Pro Glu Ser Glu Lys Glu Thr Lys Ala Val Thr Asn
        275                 280                 285

Phe Ile Arg Ser His Leu Asn Glu Ile Lys Val Tyr Ile Thr Phe His
    290                 295                 300

Ser Tyr Ser Gln Met Leu Leu Phe Pro Tyr Gly Tyr Thr Ser Lys Leu
305                 310                 315                 320

Pro Pro Asn His Glu Asp Leu Ala Lys Val Ala Lys Ile Gly Thr Asp
                325                 330                 335

Val Leu Ser Thr Arg Tyr Glu Thr Arg Tyr Ile Tyr Gly Pro Ile Glu
            340                 345                 350

Ser Thr Ile Tyr Pro Ile Ser Gly Ser Ser Leu Asp Trp Ala Tyr Asp
        355                 360                 365

Leu Gly Ile Lys His Thr Phe Ala Phe Glu Leu Arg Asp Lys Gly Lys
```

370 375 380

Phe Gly Phe Leu Leu Pro Glu Ser Arg Ile Lys Pro Thr Cys Arg Glu
385 390 395 400

Thr Met Leu Ala Val Lys Phe Ile Ala Lys Tyr Ile Leu Lys His Thr
405 410 415

Ser

<210> 24
<211> 338
<212> PRT
<213> Homo sapiens

<400> 24
Met Ile Asn Ser Thr Ser Thr Gln Pro Pro Asp Glu Ser Cys Ser Gln
1 5 10 15

Asn Leu Leu Ile Thr Gln Gln Ile Ile Pro Val Leu Tyr Cys Met Val
20 25 30

Phe Ile Ala Gly Ile Leu Leu Asn Gly Val Ser Gly Trp Ile Phe Phe
35 40 45

Tyr Val Pro Ser Ser Lys Ser Phe Ile Ile Tyr Leu Lys Asn Ile Val
50 55 60

Ile Ala Asp Phe Val Met Ser Leu Thr Phe Pro Phe Lys Ile Leu Gly
65 70 75 80

Asp Ser Gly Leu Gly Pro Trp Gln Leu Asn Val Phe Val Cys Arg Val
85 90 95

Ser Ala Val Leu Phe Tyr Val Asn Met Tyr Val Ser Ile Val Phe Phe
100 105 110

Gly Leu Ile Ser Phe Asp Arg Tyr Tyr Lys Ile Val Lys Pro Leu Trp
115 120 125

Thr Ser Phe Ile Gln Ser Val Ser Tyr Ser Lys Leu Leu Ser Val Ile
130 135 140

Val Trp Met Leu Met Leu Leu Leu Ala Val Pro Asn Ile Ile Leu Thr
145 150 155 160

Asn Gln Ser Val Arg Glu Val Thr Gln Ile Lys Cys Ile Glu Leu Lys
165 170 175

Ser Glu Leu Gly Arg Lys Trp His Lys Ala Ser Asn Tyr Ile Phe Val
180 185 190

Ala Ile Phe Trp Ile Val Phe Leu Leu Leu Ile Val Phe Tyr Thr Ala
195 200 205

Ile Thr Lys Lys Ile Phe Lys Ser His Leu Lys Ser Ser Arg Asn Ser
210 215 220

Thr Ser Val Lys Lys Lys Ser Ser Arg Asn Ile Phe Ser Ile Val Phe
225 230 235 240

Val Phe Phe Val Cys Phe Val Pro Tyr His Ile Ala Arg Ile Pro Tyr
245 250 255

Thr Lys Ser Gln Thr Glu Ala His Tyr Ser Cys Gln Ser Lys Glu Ile
260 265 270

Leu Arg Tyr Met Lys Glu Phe Thr Leu Leu Leu Ser Ala Ala Asn Val
275 280 285

Cys Leu Asp Pro Ile Ile Tyr Phe Phe Leu Cys Gln Pro Phe Arg Glu
290 295 300

Ile Leu Cys Lys Lys Leu His Ile Pro Leu Lys Ala Gln Asn Asp Leu
305 310 315 320

Asp Ile Ser Arg Ile Lys Arg Gly Asn Thr Thr Leu Glu Ser Thr Asp
325 330 335

Thr Leu

<210> 25
<211> 852
<212> PRT
<213> Homo sapiens

<400> 25
Met Ala Met Arg Ser Gly Arg His Pro Ser Leu Leu Leu Leu Leu Val
1 5 10 15

Leu Leu Leu Trp Leu Leu Gln Val Ser Ile Ile Asp Ser Val Gln Gln
20 25 30

Glu Thr Asp Asp Leu Thr Lys Gln Thr Lys Glu Lys Ile Tyr Gln Pro
35 40 45

Leu Arg Arg Ser Lys Arg Arg Trp Val Ile Thr Thr Leu Glu Leu Glu
50 55 60

Glu Glu Asp Pro Gly Pro Phe Pro Lys Leu Ile Gly Glu Leu Phe Asn
65 70 75 80

Asn Met Ser Tyr Asn Met Ser Leu Met Tyr Leu Ile Ser Gly Pro Gly
85 90 95

Val Asp Glu Tyr Pro Glu Ile Gly Leu Phe Ser Leu Glu Asp His Glu
100 105 110

Asn Gly Arg Ile Tyr Val His Arg Pro Val Asp Arg Glu Met Thr Pro
115 120 125

Ser Phe Thr Val Tyr Phe Asp Val Val Glu Arg Ser Thr Gly Lys Ile
130 135 140

Val Asp Thr Ser Leu Ile Phe Asn Ile Arg Ile Ser Asp Val Asn Asp
145 150 155 160

His Ala Pro Gln Phe Pro Glu Lys Glu Phe Asn Ile Thr Val Gln Glu
165 170 175

Asn Gln Ser Ala Gly Gln Pro Ile Phe Gln Met Leu Ala Val Asp Leu
180 185 190

Asp Glu Glu Asn Thr Pro Asn Ser Gln Val Leu Tyr Phe Leu Ile Ser
195 200 205

Gln Thr Pro Leu Leu Lys Glu Ser Gly Phe Arg Val Asp Arg Leu Ser
210 215 220

Gly Glu Ile Arg Leu Ser Gly Cys Leu Asp Tyr Glu Thr Ala Pro Gln
225 230 235 240

Phe Thr Leu Leu Ile Arg Ala Arg Asp Cys Gly Glu Pro Ser Leu Ser
245 250 255

Ser Thr Thr Thr Val His Val Asp Val Gln Glu Gly Asn Asn His Arg
260 265 270

Pro Ala Phe Thr Gln Glu Asn Tyr Lys Val Gln Ile Pro Glu Gly Arg
275 280 285

Ala Ser Gln Gly Val Leu Arg Leu Leu Val Gln Asp Arg Asp Ser Pro
290 295 300

```
Phe Thr Ser Ala Trp Arg Ala Lys Phe Asn Ile Leu His Gly Asn Glu
305                 310                 315                 320

Glu Gly His Phe Asp Ile Ser Thr Asp Pro Glu Thr Asn Glu Gly Ile
                325                 330                 335

Leu Asn Val Ile Lys Pro Leu Asp Tyr Glu Thr Arg Pro Ala Gln Ser
            340                 345                 350

Leu Ile Ile Val Val Glu Asn Glu Glu Arg Leu Val Phe Cys Glu Arg
        355                 360                 365

Gly Lys Leu Gln Pro Pro Arg Lys Ala Ala Ala Ser Ala Thr Val Ser
    370                 375                 380

Val Gln Val Thr Asp Ala Asn Asp Pro Pro Ala Phe His Pro Gln Ser
385                 390                 395                 400

Phe Ile Val Asn Lys Glu Glu Gly Ala Arg Pro Gly Thr Leu Leu Gly
                405                 410                 415

Thr Phe Asn Ala Met Asp Pro Asp Ser Gln Ile Arg Tyr Glu Leu Val
            420                 425                 430

His Asp Pro Ala Asn Trp Val Ser Val Asp Lys Asn Ser Gly Val Val
        435                 440                 445

Ile Thr Val Glu Pro Ile Asp Arg Glu Ser Pro His Val Asn Asn Ser
    450                 455                 460

Phe Tyr Val Ile Ile Ile His Ala Val Asp Asp Gly Phe Pro Pro Gln
465                 470                 475                 480

Thr Ala Thr Gly Thr Leu Met Leu Phe Leu Ser Asp Ile Asn Asp Asn
                485                 490                 495

Val Pro Thr Leu Arg Pro Arg Ser Arg Tyr Met Glu Val Cys Glu Ser
            500                 505                 510

Ala Val His Glu Pro Leu His Ile Glu Ala Glu Asp Pro Asp Leu Glu
        515                 520                 525

Pro Phe Ser Asp Pro Phe Thr Phe Glu Leu Asp Asn Thr Trp Gly Asn
    530                 535                 540

Ala Glu Asp Thr Trp Lys Leu Gly Arg Asn Trp Gly Gln Ser Val Glu
```

```
545                 550                 555                 560

Leu Leu Thr Leu Arg Ser Leu Pro Arg Gly Asn Tyr Leu Val Pro Leu
                565                 570                 575

Phe Ile Gly Asp Lys Gln Gly Leu Ser Gln Lys Gln Thr Val His Val
            580                 585                 590

Arg Ile Cys Pro Cys Ala Ser Gly Leu Thr Cys Val Glu Leu Ala Asp
        595                 600                 605

Ala Glu Val Gly Leu His Val Gly Ala Leu Phe Pro Val Cys Ala Ala
    610                 615                 620

Phe Val Ala Leu Ala Val Ala Leu Leu Phe Leu Leu Arg Cys Tyr Phe
625                 630                 635                 640

Val Leu Glu Pro Lys Arg His Gly Cys Ser Val Ser Asn Asp Glu Gly
                645                 650                 655

His Gln Thr Leu Val Met Tyr Asn Ala Glu Ser Lys Gly Thr Ser Ala
            660                 665                 670

Gln Thr Trp Ser Asp Val Glu Gly Gln Arg Pro Ala Leu Leu Ile Cys
        675                 680                 685

Thr Ala Ala Ala Gly Pro Thr Gln Gly Val Lys Ala Tyr Pro Asp Ala
    690                 695                 700

Thr Met His Arg Gln Leu Leu Ala Pro Val Glu Gly Arg Met Ala Glu
705                 710                 715                 720

Thr Leu Asn Gln Ser Lys Glu Arg Asn Arg Phe Ser Leu Ser Arg Gly
                725                 730                 735

Cys Ile Ile Pro Gln Gly Arg Ala Thr Ala Gly Arg Gly Leu Pro Gln
            740                 745                 750

Asp Ile Tyr Lys Glu Met Met Pro Arg Arg Leu Thr Gln Thr Gly Lys
        755                 760                 765

Arg Lys His Gly Ala Leu Ala Arg Thr Pro Ser Phe Lys Lys Val Val
    770                 775                 780

Tyr Asp His Lys Glu Asp Glu Glu Asn Lys Ala Gly Arg Lys Gln Arg
785                 790                 795                 800
```

```
Ser His Leu Phe Lys Val Met Gln Leu Arg Asn Glu Gln Gly Gly Val
                805                 810                 815

Arg Val Gln Ser Ala His Ser Pro Ser Pro Leu Asn Lys Lys Ala Cys
            820                 825                 830

Phe Pro Gly Asp Tyr Arg Gly Glu Ser Ala Gly Gly His Asn Cys Arg
        835                 840                 845

Ala Val Ser Gly
    850


<210> 26
<211> 782
<212> PRT
<213> Homo sapiens

<400> 26
Met Ala Pro His Arg Pro Ala Pro Ala Leu Leu Cys Ala Leu Ser Leu
1               5                   10                  15

Ala Leu Cys Ala Leu Ser Leu Pro Val Arg Ala Ala Thr Ala Ser Arg
            20                  25                  30

Gly Ala Ser Gln Ala Gly Ala Pro Gln Gly Arg Val Pro Glu Ala Arg
        35                  40                  45

Pro Asn Ser Met Val Val Glu His Pro Glu Phe Leu Lys Ala Gly Lys
    50                  55                  60

Glu Pro Gly Leu Gln Ile Trp Arg Val Glu Lys Phe Asp Leu Val Pro
65                  70                  75                  80

Val Pro Thr Asn Leu Tyr Gly Asp Phe Phe Thr Gly Asp Ala Tyr Val
                85                  90                  95

Ile Leu Lys Thr Val Gln Leu Arg Asn Gly Asn Leu Gln Tyr Asp Leu
            100                 105                 110

His Tyr Trp Leu Gly Asn Glu Cys Ser Gln Asp Glu Ser Gly Ala Ala
        115                 120                 125

Ala Ile Phe Thr Val Gln Leu Asp Asp Tyr Leu Asn Gly Arg Ala Val
    130                 135                 140

Gln His Arg Glu Val Gln Gly Phe Glu Ser Ala Thr Phe Leu Gly Tyr
145                 150                 155                 160

Phe Lys Ser Gly Leu Lys Tyr Lys Lys Gly Gly Val Ala Ser Gly Phe
```

165 170 175

Lys His Val Val Pro Asn Glu Val Val Val Gln Arg Leu Phe Gln Val
180 185 190

Lys Gly Arg Arg Val Val Arg Ala Thr Glu Val Pro Val Ser Trp Glu
195 200 205

Ser Phe Asn Asn Gly Asp Cys Phe Ile Leu Asp Leu Gly Asn Asn Ile
210 215 220

His Gln Trp Cys Gly Ser Asn Ser Asn Arg Tyr Glu Arg Leu Lys Ala
225 230 235 240

Thr Gln Val Ser Lys Gly Ile Arg Asp Asn Glu Arg Ser Gly Arg Ala
245 250 255

Arg Val His Val Ser Glu Glu Gly Thr Glu Pro Glu Ala Met Leu Gln
260 265 270

Val Leu Gly Pro Lys Pro Ala Leu Pro Ala Gly Thr Glu Asp Thr Ala
275 280 285

Lys Glu Asp Ala Ala Asn Arg Lys Leu Ala Lys Leu Tyr Lys Val Ser
290 295 300

Asn Gly Ala Gly Thr Met Ser Val Ser Leu Val Ala Asp Glu Asn Pro
305 310 315 320

Phe Ala Gln Gly Ala Leu Lys Ser Glu Asp Cys Phe Ile Leu Asp His
325 330 335

Gly Lys Asp Gly Lys Ile Phe Val Trp Lys Gly Lys Gln Ala Asn Thr
340 345 350

Glu Glu Arg Lys Ala Ala Leu Lys Thr Ala Ser Asp Phe Ile Thr Lys
355 360 365

Met Asp Tyr Pro Lys Gln Thr Gln Val Ser Val Leu Pro Glu Gly Gly
370 375 380

Glu Thr Pro Leu Phe Lys Gln Phe Phe Lys Asn Trp Arg Asp Pro Asp
385 390 395 400

Gln Thr Asp Gly Leu Gly Leu Ser Tyr Leu Ser Ser His Ile Ala Asn
405 410 415

Val Glu Arg Val Pro Phe Asp Ala Ala Thr Leu His Thr Ser Thr Ala
420 425 430

Met Ala Ala Gln His Gly Met Asp Asp Asp Gly Thr Gly Gln Lys Gln
435 440 445

Ile Trp Arg Ile Glu Gly Ser Asn Lys Val Pro Val Asp Pro Ala Thr
450 455 460

Tyr Gly Gln Phe Tyr Gly Gly Asp Ser Tyr Ile Ile Leu Tyr Asn Tyr
465 470 475 480

Arg His Gly Gly Arg Gln Gly Gln Ile Ile Tyr Asn Trp Gln Gly Ala
485 490 495

Gln Ser Thr Gln Asp Glu Val Ala Ala Ser Ala Ile Leu Thr Ala Gln
500 505 510

Leu Asp Glu Glu Leu Gly Gly Thr Pro Val Gln Ser Arg Val Val Gln
515 520 525

Gly Lys Glu Pro Ala His Leu Met Ser Leu Phe Gly Gly Lys Pro Met
530 535 540

Ile Ile Tyr Lys Gly Gly Thr Ser Arg Glu Gly Gly Gln Thr Ala Pro
545 550 555 560

Ala Ser Thr Arg Leu Phe Gln Val Arg Ala Asn Ser Ala Gly Ala Thr
565 570 575

Arg Ala Val Glu Val Leu Pro Lys Ala Gly Ala Leu Asn Ser Asn Asp
580 585 590

Ala Phe Val Leu Lys Thr Pro Ser Ala Ala Tyr Leu Trp Val Gly Thr
595 600 605

Gly Ala Ser Glu Ala Glu Lys Thr Gly Ala Gln Glu Leu Leu Arg Val
610 615 620

Leu Arg Ala Gln Pro Val Gln Val Ala Glu Gly Ser Glu Pro Asp Gly
625 630 635 640

Phe Trp Glu Ala Leu Gly Gly Lys Ala Ala Tyr Arg Thr Ser Pro Arg
645 650 655

Leu Lys Asp Lys Lys Met Asp Ala His Pro Pro Arg Leu Phe Ala Cys
660 665 670

Ser Asn Lys Ile Gly Arg Phe Val Ile Glu Glu Val Pro Gly Glu Leu
675 680 685

Met Gln Glu Asp Leu Ala Thr Asp Asp Val Met Leu Leu Asp Thr Trp
690 695 700

Asp Gln Val Phe Val Trp Val Gly Lys Asp Ser Gln Glu Glu Glu Lys
705 710 715 720

Thr Glu Ala Leu Thr Ser Ala Lys Arg Tyr Ile Glu Thr Asp Pro Ala
725 730 735

Asn Arg Asp Arg Arg Thr Pro Ile Thr Val Val Lys Gln Gly Phe Glu
740 745 750

Pro Pro Ser Phe Val Gly Trp Phe Leu Gly Trp Asp Asp Asp Tyr Trp
755 760 765

Ser Val Asp Pro Leu Asp Arg Ala Met Ala Glu Leu Ala Ala
770 775 780

<210> 27
<211> 164
<212> PRT
<213> Homo sapiens

<400> 27
Met Gly Asp Leu Pro Gly Leu Val Arg Leu Ser Ile Ala Leu Arg Ile
1 5 10 15

Gln Pro Asn Asp Gly Pro Val Phe Tyr Lys Val Asp Gly Gln Arg Phe
20 25 30

Gly Gln Asn Arg Thr Ile Lys Leu Leu Thr Gly Ser Ser Tyr Lys Val
35 40 45

Glu Val Lys Ile Lys Pro Ser Thr Leu Gln Val Glu Asn Ile Ser Ile
50 55 60

Gly Gly Val Leu Val Pro Leu Glu Leu Lys Ser Lys Glu Pro Asp Gly
65 70 75 80

Asp Arg Val Val Tyr Thr Gly Thr Tyr Asp Thr Glu Gly Val Thr Pro
85 90 95

Thr Lys Ser Gly Glu Arg Gln Pro Ile Gln Ile Thr Met Pro Phe Thr
100 105 110

Asp Ile Gly Thr Phe Glu Thr Val Trp Gln Val Lys Phe Tyr Asn Tyr
115 120 125

His Lys Arg Asp His Cys Gln Trp Gly Ser Pro Phe Ser Val Ile Glu
130 135 140

Tyr Glu Cys Lys Pro Asn Glu Thr Arg Ser Leu Met Trp Val Asn Lys
145 150 155 160

Glu Ser Phe Leu

<210> 28
<211> 295
<212> PRT
<213> Homo sapiens

<400> 28
Met Tyr Arg Ile Ser Gln Leu Met Ser Thr Pro Val Ala Ser Ser Ser
1 5 10 15

Arg Leu Glu Arg Glu Tyr Ala Gly Glu Leu Ser Pro Thr Cys Ile Phe
20 25 30

Pro Ser Phe Thr Cys Asp Ser Leu Asp Gly Tyr His Ser Phe Glu Cys
35 40 45

Gly Ser Ile Asp Pro Leu Thr Gly Ser His Tyr Thr Cys Arg Arg Ser
50 55 60

Pro Arg Leu Leu Thr Asn Gly Tyr Tyr Ile Trp Thr Glu Asp Ser Phe
65 70 75 80

Leu Cys Asp Lys Asp Gly Asn Ile Thr Leu Asn Pro Ser Gln Thr Ser
85 90 95

Val Met Tyr Lys Glu Asn Leu Val Arg Ile Phe Arg Lys Lys Lys Arg
100 105 110

Ile Cys His Ser Phe Ser Ser Leu Phe Asn Leu Ser Thr Ser Lys Ser
115 120 125

Trp Leu His Gly Ser Ile Phe Gly Asp Ile Asn Ser Ser Pro Ser Glu
130 135 140

Asp Asn Trp Leu Lys Gly Thr Arg Arg Leu Asp Thr Asp His Cys Asn
145 150 155 160

Gly Asn Ala Asp Asp Leu Asp Cys Ser Ser Leu Thr Asp Asp Trp Glu

```
                165                 170                 175

Ser Gly Lys Met Asn Ala Glu Ser Val Ile Thr Ser Ser Ser Ser His
            180                 185                 190

Ile Ile Ser Gln Pro Pro Gly Gly Asn Ser His Ser Leu Ser Leu Gln
        195                 200                 205

Ser Gln Leu Thr Ala Ser Glu Arg Phe Gln Glu Asn Ser Ser Asp His
    210                 215                 220

Ser Glu Thr Arg Leu Leu Gln Glu Val Phe Phe Gln Ala Ile Leu Leu
225                 230                 235                 240

Ala Val Cys Leu Ile Ile Ser Ala Cys Ala Arg Trp Phe Met Gly Glu
                245                 250                 255

Ile Leu Ala Ser Val Phe Thr Cys Ser Leu Met Ile Thr Val Ala Tyr
            260                 265                 270

Val Lys Ser Leu Phe Leu Ser Leu Ala Ser Tyr Phe Lys Thr Thr Ala
        275                 280                 285

Cys Ala Arg Phe Val Lys Ile
    290                 295


<210> 29
<211> 198
<212> PRT
<213> Homo sapiens

<400> 29
Met Asp Ala Ile Leu Asn Tyr Arg Ser Glu Asp Thr Glu Asp Tyr Tyr
1               5                   10                  15

Thr Leu Leu Gly Cys Asp Glu Leu Ser Ser Val Glu Gln Ile Leu Ala
            20                  25                  30

Glu Phe Lys Val Arg Ala Leu Glu Cys His Pro Asp Lys His Pro Glu
        35                  40                  45

Asn Pro Lys Ala Val Glu Thr Phe Gln Lys Leu Gln Lys Ala Lys Glu
    50                  55                  60

Ile Leu Thr Asn Glu Glu Ser Arg Ala Arg Tyr Asp His Trp Arg Arg
65                  70                  75                  80

Ser Gln Met Ser Met Pro Phe Gln Gln Trp Glu Ala Leu Asn Asp Ser
                85                  90                  95
```

Val Lys Thr Ser Met His Trp Val Val Arg Gly Lys Lys Asp Leu Met
100 105 110

Leu Glu Glu Ser Asp Lys Thr His Thr Thr Lys Met Glu Asn Glu Glu
115 120 125

Cys Asn Glu Gln Arg Glu Arg Lys Lys Glu Glu Leu Ala Ser Thr Ala
130 135 140

Glu Lys Thr Glu Gln Lys Glu Pro Lys Pro Leu Glu Lys Ser Val Ser
145 150 155 160

Pro Gln Asn Ser Asp Ser Ser Gly Phe Ala Asp Val Asn Gly Trp His
165 170 175

Leu Arg Phe Arg Trp Ser Lys Asp Ala Pro Ser Glu Leu Leu Arg Lys
180 185 190

Phe Arg Asn Tyr Glu Ile
195

<210> 30
<211> 159
<212> PRT
<213> Homo sapiens

<400> 30
Met Ser Arg Arg Asn Cys Trp Ile Cys Lys Met Cys Arg Asp Glu Ser
1 5 10 15

Lys Arg Pro Pro Ser Asn Leu Thr Leu Glu Glu Val Leu Gln Trp Ala
20 25 30

Gln Ser Phe Glu Asn Leu Met Ala Thr Lys Tyr Gly Pro Val Val Tyr
35 40 45

Ala Ala Tyr Leu Lys Met Glu His Ser Asp Glu Asn Ile Gln Phe Trp
50 55 60

Met Ala Cys Glu Thr Tyr Lys Lys Ile Ala Ser Arg Trp Ser Arg Ile
65 70 75 80

Ser Arg Ala Lys Lys Leu Tyr Lys Ile Tyr Ile Gln Pro Gln Ser Pro
85 90 95

Arg Glu Ile Asn Ile Asp Ser Ser Thr Arg Glu Thr Ile Ile Arg Asn
100 105 110

```
Ile Gln Glu Pro Thr Glu Thr Cys Phe Glu Glu Ala Gln Lys Ile Val
        115                 120                 125

Tyr Met His Met Glu Arg Asp Ser Tyr Pro Arg Phe Leu Lys Ser Glu
    130                 135                 140

Met Tyr Gln Lys Leu Leu Lys Thr Met Gln Ser Asn Asn Ser Phe
145                 150                 155


<210> 31
<211> 514
<212> PRT
<213> Homo sapiens

<400> 31
Met Ala Leu Ser Glu Leu Ala Leu Val Arg Trp Leu Gln Glu Ser Arg
1               5                   10                  15

Arg Ser Arg Lys Leu Ile Leu Phe Ile Val Phe Leu Ala Leu Leu Leu
            20                  25                  30

Asp Asn Met Leu Leu Thr Val Val Val Pro Ile Ile Pro Ser Tyr Leu
        35                  40                  45

Tyr Ser Ile Lys His Glu Lys Asn Ala Thr Glu Ile Gln Thr Ala Arg
    50                  55                  60

Pro Val His Thr Ala Ser Ile Ser Asp Ser Phe Gln Ser Ile Phe Ser
65                  70                  75                  80

Tyr Tyr Asp Asn Ser Thr Met Val Thr Gly Asn Ala Thr Arg Asp Leu
                85                  90                  95

Thr Leu His Gln Thr Ala Thr Gln His Met Val Thr Asn Ala Ser Ala
            100                 105                 110

Val Pro Ser Asp Cys Pro Ser Glu Asp Lys Asp Leu Leu Asn Glu Asn
        115                 120                 125

Val Gln Val Gly Leu Leu Phe Ala Ser Lys Ala Thr Val Gln Leu Ile
    130                 135                 140

Thr Asn Pro Phe Ile Gly Leu Leu Thr Asn Arg Ile Gly Tyr Pro Ile
145                 150                 155                 160

Pro Ile Phe Ala Gly Phe Cys Ile Met Phe Val Ser Thr Ile Met Phe
                165                 170                 175
```

Ala Phe Ser Ser Ser Tyr Ala Phe Leu Leu Ile Ala Arg Ser Leu Gln
180 185 190

Gly Ile Gly Ser Ser Cys Ser Ser Val Ala Gly Met Gly Met Leu Ala
195 200 205

Ser Val Tyr Thr Asp Asp Glu Glu Arg Gly Asn Val Met Gly Ile Ala
210 215 220

Leu Gly Gly Leu Ala Met Gly Val Leu Val Gly Pro Pro Phe Gly Ser
225 230 235 240

Val Leu Tyr Glu Phe Val Gly Lys Thr Ala Pro Phe Leu Val Leu Ala
245 250 255

Ala Leu Val Leu Leu Asp Gly Ala Ile Gln Leu Phe Val Leu Gln Pro
260 265 270

Ser Arg Val Gln Pro Glu Ser Gln Lys Gly Thr Pro Leu Thr Thr Leu
275 280 285

Leu Lys Asp Pro Tyr Ile Leu Ile Ala Ala Gly Ser Ile Cys Phe Ala
290 295 300

Asn Met Gly Ile Ala Met Leu Glu Pro Ala Leu Pro Ile Trp Met Met
305 310 315 320

Glu Thr Met Cys Ser Arg Lys Trp Gln Leu Gly Val Ala Phe Leu Pro
325 330 335

Ala Ser Ile Ser Tyr Leu Ile Gly Thr Asn Ile Phe Gly Ile Leu Ala
340 345 350

His Lys Met Gly Arg Trp Leu Cys Ala Leu Leu Gly Met Ile Ile Val
355 360 365

Gly Val Ser Ile Leu Cys Ile Pro Phe Ala Lys Asn Ile Tyr Gly Leu
370 375 380

Ile Ala Pro Asn Phe Gly Val Gly Phe Ala Ile Gly Met Val Asp Ser
385 390 395 400

Ser Met Met Pro Ile Met Gly Tyr Leu Val Asp Leu Arg His Val Ser
405 410 415

Val Tyr Gly Ser Val Tyr Ala Ile Ala Asp Val Ala Phe Cys Met Gly
420 425 430

Tyr Ala Ile Gly Pro Ser Ala Gly Gly Ala Ile Ala Lys Ala Ile Gly
435 440 445

Phe Pro Trp Leu Met Thr Ile Ile Gly Ile Ile Asp Ile Leu Phe Ala
450 455 460

Pro Leu Cys Phe Phe Leu Arg Ser Pro Pro Ala Lys Glu Glu Lys Met
465 470 475 480

Ala Ile Leu Met Asp His Asn Cys Pro Ile Lys Thr Lys Met Tyr Thr
485 490 495

Gln Asn Asn Ile Gln Ser Tyr Pro Ile Gly Glu Asp Glu Glu Ser Glu
500 505 510

Ser Asp

<210> 32
<211> 397
<212> PRT
<213> Homo sapiens

<400> 32
Met Asp Ser Leu Ala Thr Ser Ile Asn Gln Phe Ala Leu Glu Leu Ser
1 5 10 15

Lys Lys Leu Ala Glu Ser Ala Gln Gly Lys Asn Ile Phe Phe Ser Ser
20 25 30

Trp Ser Ile Ser Thr Ser Leu Thr Ile Val Tyr Leu Gly Ala Lys Gly
35 40 45

Thr Thr Ala Ala Gln Met Ala Gln Val Leu Gln Phe Asn Arg Asp Gln
50 55 60

Gly Val Lys Cys Asp Pro Glu Ser Glu Lys Lys Arg Lys Met Glu Phe
65 70 75 80

Asn Leu Ser Asn Ser Glu Glu Ile His Ser Asp Phe Gln Thr Leu Ile
85 90 95

Ser Glu Ile Leu Lys Pro Asn Asp Asp Tyr Leu Leu Lys Thr Ala Asn
100 105 110

Ala Ile Tyr Gly Glu Lys Thr Tyr Ala Phe His Asn Lys Tyr Leu Glu
115 120 125

Asp Met Lys Thr Tyr Phe Gly Ala Glu Pro Gln Pro Val Asn Phe Val
130 135 140

Glu Ala Ser Asp Gln Ile Arg Lys Asp Ile Asn Ser Trp Val Glu Arg
145 150 155 160

Gln Thr Glu Gly Lys Ile Gln Asn Leu Leu Pro Asp Asp Ser Val Asp
165 170 175

Ser Thr Thr Arg Met Ile Leu Val Asn Ala Leu Tyr Phe Lys Gly Ile
180 185 190

Trp Glu His Gln Phe Leu Val Gln Asn Thr Thr Glu Lys Pro Phe Arg
195 200 205

Ile Asn Glu Thr Thr Ser Lys Pro Val Gln Met Met Phe Met Lys Lys
210 215 220

Lys Leu His Ile Phe His Ile Glu Lys Pro Lys Ala Val Gly Leu Gln
225 230 235 240

Leu Tyr Tyr Lys Ser Arg Asp Leu Ser Leu Leu Ile Leu Leu Pro Glu
245 250 255

Asp Ile Asn Gly Leu Glu Gln Leu Glu Lys Ala Ile Thr Tyr Glu Lys
260 265 270

Leu Asn Glu Trp Thr Ser Ala Asp Met Met Glu Leu Tyr Glu Val Gln
275 280 285

Leu His Leu Pro Lys Phe Lys Leu Glu Asp Ser Tyr Asp Leu Lys Ser
290 295 300

Thr Leu Ser Ser Met Gly Met Ser Asp Ala Phe Ser Gln Ser Lys Ala
305 310 315 320

Asp Phe Ser Gly Met Ser Ser Ala Arg Asn Leu Phe Leu Ser Asn Val
325 330 335

Phe His Lys Ala Phe Val Glu Ile Asn Glu Gln Gly Thr Glu Ala Ala
340 345 350

Ala Gly Ser Gly Ser Glu Ile Asp Ile Arg Ile Arg Val Pro Ser Ile
355 360 365

Glu Phe Asn Ala Asn His Pro Phe Leu Phe Phe Ile Arg His Asn Lys
370 375 380

Thr Asn Thr Ile Leu Phe Tyr Gly Arg Leu Cys Ser Pro
385 390 395

<210> 33
<211> 729
<212> PRT
<213> Homo sapiens

<400> 33
Met Asp Asp Leu Thr Leu Leu Asp Leu Leu Glu Cys Pro Val Cys Phe
1 5 10 15

Glu Lys Leu Asp Val Thr Ala Lys Val Leu Pro Cys Gln His Thr Phe
20 25 30

Cys Lys Pro Cys Leu Gln Arg Val Phe Lys Ala His Lys Glu Leu Arg
35 40 45

Cys Pro Glu Cys Arg Thr Pro Val Phe Ser Asn Ile Glu Ala Leu Pro
50 55 60

Ala Asn Leu Leu Leu Val Arg Leu Leu Asp Gly Val Arg Ser Gly Gln
65 70 75 80

Ser Ser Gly Arg Gly Gly Ser Phe Arg Arg Pro Gly Thr Met Thr Leu
85 90 95

Gln Asp Gly Arg Lys Ser Arg Thr Asn Pro Arg Arg Leu Gln Ala Ser
100 105 110

Pro Phe Arg Leu Val Pro Asn Val Arg Ile His Met Asp Gly Val Pro
115 120 125

Arg Ala Lys Ala Leu Cys Asn Tyr Arg Gly Gln Asn Pro Gly Asp Leu
130 135 140

Arg Phe Asn Lys Gly Asp Ile Ile Leu Leu Arg Arg Gln Leu Asp Glu
145 150 155 160

Asn Trp Tyr Gln Gly Glu Ile Asn Gly Ile Ser Gly Asn Phe Pro Ala
165 170 175

Ser Ser Val Glu Val Ile Lys Gln Leu Pro Gln Pro Pro Pro Leu Cys
180 185 190

Arg Ala Leu Tyr Asn Phe Asp Leu Arg Gly Lys Asp Lys Ser Glu Asn
195 200 205

Gln Asp Cys Leu Thr Phe Leu Lys Asp Asp Ile Ile Thr Val Ile Ser
210 215 220

Arg Val Asp Glu Asn Trp Ala Glu Gly Lys Leu Gly Asp Lys Val Gly
225 230 235 240

Ile Phe Pro Ile Leu Phe Val Glu Pro Asn Leu Thr Ala Arg His Leu
245 250 255

Leu Glu Lys Asn Lys Gly Arg Gln Ser Ser Cys Thr Lys Asn Leu Ser
260 265 270

Leu Val Ser Ser Ser Ser Arg Gly Asn Thr Ser Thr Leu Arg Arg Gly
275 280 285

Pro Gly Ser Arg Arg Lys Val Pro Gly Gln Phe Ser Ile Thr Thr Ala
290 295 300

Leu Asn Thr Leu Asn Arg Met Val His Ser Pro Ser Gly Arg His Met
305 310 315 320

Val Glu Ile Ser Thr Pro Val Leu Ile Ser Ser Ser Asn Pro Ser Val
325 330 335

Ile Thr Gln Pro Met Glu Lys Ala Asp Val Pro Ser Ser Cys Val Gly
340 345 350

Gln Val Ser Thr Tyr His Pro Ala Pro Val Ser Pro Gly His Ser Thr
355 360 365

Ala Val Val Ser Leu Pro Gly Ser Gln Gln His Leu Ser Ala Asn Met
370 375 380

Phe Val Ala Leu His Ser Tyr Ser Ala His Gly Pro Asp Glu Leu Asp
385 390 395 400

Leu Gln Lys Gly Glu Gly Val Arg Val Leu Gly Lys Cys Gln Asp Gly
405 410 415

Trp Leu Arg Gly Val Ser Leu Val Thr Gly Arg Val Gly Ile Phe Pro
420 425 430

Asn Asn Tyr Val Ile Pro Ile Phe Arg Lys Thr Ser Ser Phe Pro Asp
435 440 445

Ser Arg Ser Pro Gly Leu Tyr Thr Thr Trp Thr Leu Ser Thr Ser Ser
450 455 460

Val Ser Ser Gln Gly Ser Ile Ser Glu Gly Asp Pro Arg Gln Ser Arg
465 470 475 480

Pro Phe Lys Ser Val Phe Val Pro Thr Ala Ile Val Asn Pro Val Arg
485 490 495

Ser Thr Ala Gly Pro Gly Thr Leu Gly Gln Gly Ser Leu Arg Lys Gly
500 505 510

Arg Ser Ser Met Arg Lys Asn Gly Ser Leu Gln Arg Pro Leu Gln Ser
515 520 525

Gly Ile Pro Thr Leu Val Val Gly Ser Leu Arg Arg Ser Pro Thr Met
530 535 540

Val Leu Arg Pro Gln Gln Phe Gln Phe Tyr Gln Pro Gln Gly Ile Pro
545 550 555 560

Ser Ser Pro Ser Ala Val Val Val Glu Met Gly Ser Lys Pro Ala Leu
565 570 575

Thr Gly Glu Pro Ala Leu Thr Cys Ile Ser Arg Gly Ser Glu Ala Arg
580 585 590

Ile His Ser Ala Ala Ser Ser Leu Ile Met Glu Asp Lys Glu Ile Pro
595 600 605

Ile Lys Ser Glu Pro Leu Pro Lys Pro Pro Ala Ser Ala Pro Pro Ser
610 615 620

Ile Leu Val Lys Pro Glu Asn Ser Arg Asn Gly Ile Glu Lys Gln Val
625 630 635 640

Lys Thr Val Arg Phe Gln Asn Tyr Ser Pro Pro Pro Thr Lys His Tyr
645 650 655

Thr Ser His Pro Thr Ser Gly Lys Pro Glu Gln Pro Ala Thr Leu Lys
660 665 670

Ala Ser Gln Pro Glu Ala Ala Ser Leu Gly Pro Glu Met Thr Val Leu
675 680 685

Phe Ala His Arg Ser Gly Cys His Ser Gly Gln Gln Thr Asp Leu Arg
690 695 700

Arg Lys Ser Ala Leu Ala Lys Ala Thr Thr Leu Val Ser Thr Ala Ser
705 710 715 720

Gly Thr Gln Thr Val Phe Pro Ser Lys
725

<210> 34
<211> 240
<212> PRT
<213> Homo sapiens

<400> 34
Met Asp Thr Glu Ser Asn Arg Arg Ala Asn Leu Ala Leu Pro Gln Glu
1 5 10 15

Pro Ser Ser Val Pro Ala Phe Glu Val Leu Glu Ile Ser Pro Gln Glu
20 25 30

Val Ser Ser Gly Arg Leu Leu Lys Ser Ala Ser Ser Pro Pro Leu His
35 40 45

Thr Trp Leu Thr Val Leu Lys Lys Glu Gln Glu Phe Leu Gly Val Thr
50 55 60

Gln Ile Leu Thr Ala Met Ile Cys Leu Cys Phe Gly Thr Val Val Cys
65 70 75 80

Ser Val Leu Asp Ile Ser His Ile Glu Gly Asp Ile Phe Ser Ser Phe
85 90 95

Lys Ala Gly Tyr Pro Phe Trp Gly Ala Ile Phe Phe Ser Ile Ser Gly
100 105 110

Met Leu Ser Ile Ile Ser Glu Arg Arg Asn Ala Thr Tyr Leu Val Arg
115 120 125

Gly Ser Leu Gly Ala Asn Thr Ala Ser Ser Ile Ala Gly Gly Thr Gly
130 135 140

Ile Thr Ile Leu Ile Ile Asn Leu Lys Lys Ser Leu Ala Tyr Ile His
145 150 155 160

Ile His Ser Cys Gln Lys Phe Phe Glu Thr Lys Cys Phe Met Ala Ser
165 170 175

Phe Ser Thr Glu Ile Val Val Met Met Leu Phe Leu Thr Ile Leu Gly
180 185 190

Leu Gly Ser Ala Val Ser Leu Thr Ile Cys Gly Ala Gly Glu Glu Leu
195 200 205

Lys Gly Asn Lys Val Pro Glu Asp Arg Val Tyr Glu Glu Leu Asn Ile
210 215 220

Tyr Ser Ala Thr Tyr Ser Glu Leu Glu Asp Pro Gly Glu Met Ser Pro
225 230 235 240

<210> 35
<211> 521
<212> PRT
<213> Homo sapiens

<400> 35
Met Ala Ser Asn Ser Leu Phe Ser Thr Val Thr Pro Cys Gln Gln Asn
1 5 10 15

Phe Phe Trp Asp Pro Ser Thr Ser Arg Arg Phe Ser Pro Pro Ser Ser
20 25 30

Ser Leu Gln Pro Gly Lys Met Ser Asp Val Ser Pro Val Val Ala Ala
35 40 45

Gln Gln Gln Gln Gln Gln Gln Gln Gln Gln Gln Gln Gln Gln Gln Gln
50 55 60

Gln Gln Gln Gln Gln Gln Gln Glu Ala Ala Ala Ala Ala Ala Ala Ala
65 70 75 80

Ala Ala Ala Ala Ala Ala Ala Ala Ala Val Pro Arg Leu Arg Pro Pro
85 90 95

His Asp Asn Arg Thr Met Val Glu Ile Ile Ala Asp His Pro Ala Glu
100 105 110

Leu Val Arg Thr Asp Ser Pro Asn Phe Leu Cys Ser Val Leu Pro Ser
115 120 125

His Trp Arg Cys Asn Lys Thr Leu Pro Val Ala Phe Lys Val Val Ala
130 135 140

Leu Gly Glu Val Pro Asp Gly Thr Val Val Thr Val Met Ala Gly Asn
145 150 155 160

Asp Glu Asn Tyr Ser Ala Glu Leu Arg Asn Ala Ser Ala Val Met Lys
165 170 175

Asn Gln Val Ala Arg Phe Asn Asp Leu Arg Phe Val Gly Arg Ser Gly
180 185 190

```
Arg Gly Lys Ser Phe Thr Leu Thr Ile Thr Val Phe Thr Asn Pro Pro
        195                 200                 205

Gln Val Ala Thr Tyr His Arg Ala Ile Lys Val Thr Val Asp Gly Pro
    210                 215                 220

Arg Glu Pro Arg Arg His Arg Gln Lys Leu Asp Asp Ser Lys Pro Ser
225                 230                 235                 240

Leu Phe Ser Asp Arg Leu Ser Asp Leu Gly Arg Ile Pro His Pro Ser
                245                 250                 255

Met Arg Val Gly Val Pro Pro Gln Asn Pro Arg Pro Ser Leu Asn Ser
            260                 265                 270

Ala Pro Ser Pro Phe Asn Pro Gln Gly Gln Ser Gln Ile Thr Asp Pro
        275                 280                 285

Arg Gln Ala Gln Ser Ser Pro Pro Trp Ser Tyr Asp Gln Ser Tyr Pro
    290                 295                 300

Ser Tyr Leu Ser Gln Met Thr Ser Pro Ser Ile His Ser Thr Thr Pro
305                 310                 315                 320

Leu Ser Ser Thr Arg Gly Thr Gly Leu Pro Ala Ile Thr Asp Val Pro
                325                 330                 335

Arg Arg Ile Ser Asp Asp Asp Thr Ala Thr Ser Asp Phe Cys Leu Trp
            340                 345                 350

Pro Ser Thr Leu Ser Lys Lys Ser Gln Ala Gly Ala Ser Glu Leu Gly
        355                 360                 365

Pro Phe Ser Asp Pro Arg Gln Phe Pro Ser Ile Ser Ser Leu Thr Glu
    370                 375                 380

Ser Arg Phe Ser Asn Pro Arg Met His Tyr Pro Ala Thr Phe Thr Tyr
385                 390                 395                 400

Thr Pro Pro Val Thr Ser Gly Met Ser Leu Gly Met Ser Ala Thr Thr
                405                 410                 415

His Tyr His Thr Tyr Leu Pro Pro Pro Tyr Pro Gly Ser Ser Gln Ser
            420                 425                 430

Gln Ser Gly Pro Phe Gln Thr Ser Ser Thr Pro Tyr Leu Tyr Tyr Gly
        435                 440                 445
```

```
Thr Ser Ser Gly Ser Tyr Gln Phe Pro Met Val Pro Gly Gly Asp Arg
    450                 455                 460

Ser Pro Ser Arg Met Leu Pro Pro Cys Thr Thr Thr Ser Asn Gly Ser
465                 470                 475                 480

Thr Leu Leu Asn Pro Asn Leu Pro Asn Gln Asn Asp Gly Val Asp Ala
                485                 490                 495

Asp Gly Ser His Ser Ser Ser Pro Thr Val Leu Asn Ser Ser Gly Arg
            500                 505                 510

Met Asp Glu Ser Val Trp Arg Pro Tyr
        515                 520


<210> 36
<211> 599
<212> PRT
<213> Homo sapiens

<400> 36
Met Ser Arg Ser Leu Leu Leu Arg Phe Leu Leu Phe Leu Leu Leu Leu
1               5                   10                  15

Pro Pro Leu Pro Val Leu Leu Ala Asp Pro Gly Ala Pro Thr Pro Val
            20                  25                  30

Asn Pro Cys Cys Tyr Tyr Pro Cys Gln His Gln Gly Ile Cys Val Arg
        35                  40                  45

Phe Gly Leu Asp Arg Tyr Gln Cys Asp Cys Thr Arg Thr Gly Tyr Ser
    50                  55                  60

Gly Pro Asn Cys Thr Ile Pro Gly Leu Trp Thr Trp Leu Arg Asn Ser
65                  70                  75                  80

Leu Arg Pro Ser Pro Ser Phe Thr His Phe Leu Leu Thr His Gly Arg
                85                  90                  95

Trp Phe Trp Glu Phe Val Asn Ala Thr Phe Ile Arg Glu Met Leu Met
            100                 105                 110

Arg Leu Val Leu Thr Val Arg Ser Asn Leu Ile Pro Ser Pro Pro Thr
        115                 120                 125

Tyr Asn Ser Ala His Asp Tyr Ile Ser Trp Glu Ser Phe Ser Asn Val
    130                 135                 140
```

Ser Tyr Tyr Thr Arg Ile Leu Pro Ser Val Pro Lys Asp Cys Pro Thr
145 150 155 160

Pro Met Gly Thr Lys Gly Lys Lys Gln Leu Pro Asp Ala Gln Leu Leu
165 170 175

Ala Arg Arg Phe Leu Leu Arg Arg Lys Phe Ile Pro Asp Pro Gln Gly
180 185 190

Thr Asn Leu Met Phe Ala Phe Phe Ala Gln His Phe Thr His Gln Phe
195 200 205

Phe Lys Thr Ser Gly Lys Met Gly Pro Gly Phe Thr Lys Ala Leu Gly
210 215 220

His Gly Val Asp Leu Gly His Ile Tyr Gly Asp Asn Leu Glu Arg Gln
225 230 235 240

Tyr Gln Leu Arg Leu Phe Lys Asp Gly Lys Leu Lys Tyr Gln Val Leu
245 250 255

Asp Gly Glu Met Tyr Pro Pro Ser Val Glu Glu Ala Pro Val Leu Met
260 265 270

His Tyr Pro Arg Gly Ile Pro Pro Gln Ser Gln Met Ala Val Gly Gln
275 280 285

Glu Val Phe Gly Leu Leu Pro Gly Leu Met Leu Tyr Ala Thr Leu Trp
290 295 300

Leu Arg Glu His Asn Arg Val Cys Asp Leu Leu Lys Ala Glu His Pro
305 310 315 320

Thr Trp Gly Asp Glu Gln Leu Phe Gln Thr Thr Arg Leu Ile Leu Ile
325 330 335

Gly Glu Thr Ile Lys Ile Val Ile Glu Glu Tyr Val Gln Gln Leu Ser
340 345 350

Gly Tyr Phe Leu Gln Leu Lys Phe Asp Pro Glu Leu Leu Phe Gly Val
355 360 365

Gln Phe Gln Tyr Arg Asn Arg Ile Ala Met Glu Phe Asn His Leu Tyr
370 375 380

His Trp His Pro Leu Met Pro Asp Ser Phe Lys Val Gly Ser Gln Glu
385 390 395 400

Tyr Ser Tyr Glu Gln Phe Leu Phe Asn Thr Ser Met Leu Val Asp Tyr
405 410 415

Gly Val Glu Ala Leu Val Asp Ala Phe Ser Arg Gln Ile Ala Gly Arg
420 425 430

Ile Gly Gly Gly Arg Asn Met Asp His His Ile Leu His Val Ala Val
435 440 445

Asp Val Ile Arg Glu Ser Arg Glu Met Arg Leu Gln Pro Phe Asn Glu
450 455 460

Tyr Arg Lys Arg Phe Gly Met Lys Pro Tyr Thr Ser Phe Gln Glu Leu
465 470 475 480

Val Gly Glu Lys Glu Met Ala Ala Glu Leu Glu Glu Leu Tyr Gly Asp
485 490 495

Ile Asp Ala Leu Glu Phe Tyr Pro Gly Leu Leu Leu Glu Lys Cys His
500 505 510

Pro Asn Ser Ile Phe Gly Glu Ser Met Ile Glu Ile Gly Ala Pro Phe
515 520 525

Ser Leu Lys Gly Leu Leu Gly Asn Pro Ile Cys Ser Pro Glu Tyr Trp
530 535 540

Lys Pro Ser Thr Phe Gly Gly Glu Val Gly Phe Asn Ile Val Lys Thr
545 550 555 560

Ala Thr Leu Lys Lys Leu Val Cys Leu Asn Thr Lys Thr Cys Pro Tyr
565 570 575

Val Ser Phe Arg Val Pro Asp Ala Ser Gln Asp Asp Gly Pro Ala Val
580 585 590

Glu Arg Pro Ser Thr Glu Leu
595

<210> 37
<211> 662
<212> PRT
<213> Homo sapiens

<400> 37
Met Gly Leu Tyr Arg Ile Arg Val Ser Thr Gly Ala Ser Leu Tyr Ala
1 5 10 15

```
Gly Ser Asn Asn Gln Val Gln Leu Trp Leu Val Gly Gln His Gly Glu
            20                  25                  30

Ala Ala Leu Gly Lys Arg Leu Trp Pro Ala Arg Gly Lys Glu Thr Glu
        35                  40                  45

Leu Lys Val Glu Val Pro Glu Tyr Leu Gly Pro Leu Leu Phe Val Lys
    50                  55                  60

Leu Arg Lys Arg His Leu Leu Lys Asp Asp Ala Trp Phe Cys Asn Trp
65                  70                  75                  80

Ile Ser Val Gln Gly Pro Gly Ala Gly Asp Glu Val Arg Phe Pro Cys
                85                  90                  95

Tyr Arg Trp Val Glu Gly Asn Gly Val Leu Ser Leu Pro Glu Gly Thr
            100                 105                 110

Gly Arg Thr Val Gly Glu Asp Pro Gln Gly Leu Phe Gln Lys His Arg
        115                 120                 125

Glu Glu Glu Leu Glu Glu Arg Arg Lys Leu Tyr Arg Trp Gly Asn Trp
    130                 135                 140

Lys Asp Gly Leu Ile Leu Asn Met Ala Gly Ala Lys Leu Tyr Asp Leu
145                 150                 155                 160

Pro Val Asp Glu Arg Phe Leu Glu Asp Lys Arg Val Asp Phe Glu Val
                165                 170                 175

Ser Leu Ala Lys Gly Leu Ala Asp Leu Ala Ile Lys Asp Ser Leu Asn
            180                 185                 190

Val Leu Thr Cys Trp Lys Asp Leu Asp Asp Phe Asn Arg Ile Phe Trp
        195                 200                 205

Cys Gly Gln Ser Lys Leu Ala Glu Arg Val Arg Asp Ser Trp Lys Glu
    210                 215                 220

Asp Ala Leu Phe Gly Tyr Gln Phe Leu Asn Gly Ala Asn Pro Val Val
225                 230                 235                 240

Leu Arg Arg Ser Ala His Leu Pro Ala Arg Leu Val Phe Pro Pro Gly
                245                 250                 255

Met Glu Glu Leu Gln Ala Gln Leu Glu Lys Glu Leu Glu Gly Gly Thr
            260                 265                 270
```

```
Leu Phe Glu Ala Asp Phe Ser Leu Leu Asp Gly Ile Lys Ala Asn Val
        275                 280                 285

Ile Leu Cys Ser Gln Gln His Leu Ala Ala Pro Leu Val Met Leu Lys
    290                 295                 300

Leu Gln Pro Asp Gly Lys Leu Leu Pro Met Val Ile Gln Leu Gln Leu
305                 310                 315                 320

Pro Arg Thr Gly Ser Pro Pro Pro Pro Leu Phe Leu Pro Thr Asp Pro
                325                 330                 335

Pro Met Ala Trp Leu Leu Ala Lys Cys Trp Val Arg Ser Ser Asp Phe
            340                 345                 350

Gln Leu His Glu Leu Gln Ser His Leu Leu Arg Gly His Leu Met Ala
        355                 360                 365

Glu Val Ile Val Val Ala Thr Met Arg Cys Leu Pro Ser Ile His Pro
    370                 375                 380

Ile Phe Lys Leu Ile Ile Pro His Leu Arg Tyr Thr Leu Glu Ile Asn
385                 390                 395                 400

Val Arg Ala Arg Thr Gly Leu Val Ser Asp Met Gly Ile Phe Asp Gln
                405                 410                 415

Ile Met Ser Thr Gly Gly Gly Gly His Val Gln Leu Leu Lys Gln Ala
            420                 425                 430

Gly Ala Phe Leu Thr Tyr Ser Ser Phe Cys Pro Pro Asp Asp Leu Ala
        435                 440                 445

Asp Arg Gly Leu Leu Gly Val Lys Ser Ser Phe Tyr Ala Gln Asp Ala
    450                 455                 460

Leu Arg Leu Trp Glu Ile Ile Tyr Arg Tyr Val Glu Gly Ile Val Ser
465                 470                 475                 480

Leu His Tyr Lys Thr Asp Val Ala Val Lys Asp Asp Pro Glu Leu Gln
                485                 490                 495

Thr Trp Cys Arg Glu Ile Thr Glu Ile Gly Leu Gln Gly Ala Gln Asp
            500                 505                 510

Arg Gly Phe Pro Val Ser Leu Gln Ala Arg Asp Gln Val Cys His Phe
        515                 520                 525
```

Val Thr Met Cys Ile Phe Thr Cys Thr Gly Gln His Ala Ser Val His
    530                 535                 540

Leu Gly Gln Leu Asp Trp Tyr Ser Trp Val Pro Asn Ala Pro Cys Thr
545                 550                 555                 560

Met Arg Leu Pro Pro Pro Thr Thr Lys Asp Ala Thr Leu Glu Thr Val
                565                 570                 575

Met Ala Thr Leu Pro Asn Phe His Gln Ala Ser Leu Gln Met Ser Ile
            580                 585                 590

Thr Trp Gln Leu Gly Arg Arg Gln Pro Val Met Val Ala Val Gly Gln
        595                 600                 605

His Glu Glu Glu Tyr Phe Ser Gly Pro Glu Pro Lys Ala Val Leu Lys
    610                 615                 620

Lys Phe Arg Glu Glu Leu Ala Ala Leu Asp Lys Glu Ile Glu Ile Arg
625                 630                 635                 640

Asn Ala Lys Leu Asp Met Pro Tyr Glu Tyr Leu Arg Pro Ser Val Val
                645                 650                 655

Glu Asn Ser Val Ala Ile
            660

<210> 38
<211> 530
<212> DNA
<213> Homo sapiens

<220>
<221> modified_base
<222> (484)..(484)
<223> a, c, g, t, unknown or other

<400> 38
aaagaatctg acatcatgac aacaaatggt gtaattcatg ttgtagataa actcctctat 60

ccagcagaca cacctgttgg aaatgatcaa ctgctggaaa tacttaataa attaatcaaa 120

tacatccaaa ttaagtttgt tcgtggtagc accttcaaag aaatccccgt gactgtctat 180

agacccacac taacaaaagt caaaattgaa ggtgaacctg aattcagact gattaaagaa 240

ggtgaaacaa taactgaagt gatccatgga gagccaatta ttaaaaaata caccaaaatc 300

attgatggag tgcctgtgga aataactgaa aaagagacac gagaagaacg aatcattaca 360

ggtcctgaaa taaaatacac taggatttct actggaggtg gagaaacaga agaaactctg 420

aagaaattgt tacaagaaga agacacaccc gtgaggaagt tgcaagccaa caaaaaagtt 480

caanggatct agaagacgat taagggaagg tcgttctcag tgaaaatcca 530

<210> 39
<211> 413
<212> DNA
<213> Homo sapiens

<400> 39
aaattgtgga gttagcctcc tgtggagtta gcctcctgtg gtaaaggaat tgaagaaaat 60

ataacacctt acaccctttt tcatcttgac attaaaagtt ctggctaact ttggaatcca 120

ttagagaaaa atccttgtca ccagattcat tacaattcaa atcgaagagt tgtgaactgt 180

tatcccattg aaaagaccga gccttgtatg tatgttatgg atacataaaa tgcacgcaag 240

ccattatctc tccatgggaa gctaagttat aaaaataggt gcttggtgta caaaactttt 300

tatatcaaaa ggctttgcac atttctatat gagtgggttt actggtaaat tatgttattt 360

tttacaacta attttgtact ctcagaatgt ttgtcatatg cttcttgcaa tgc 413

<210> 40
<211> 493
<212> DNA
<213> Homo sapiens

<400> 40
gcgagtacaa caaggccacc gaagatgagt actacagacg cccgctgcag gtgctgcgag 60

ccagggagca gacctttggg ggggtgaatt acttcttcga cgtagaggtg ggccgcacca 120

tatgtaccaa gtcccagccc aacttggaca cctgtgcctt ccatgaacag ccagaactgc 180

agaagaaaca gttatgctct ttcgagatct acgaagttcc ctgggaggac agaatgtccc 240

tggtgaattc caggtgtcaa gaagcctagg ggtctgtgcc aggccagtca caccgaccac 300

cacccactcc caccccctgt agtgctccca cccctggact ggtggccccc accctgcggg 360

aggcctcccc atgtgcctgt gccaagagac agacagagaa ggctgcagga gtcctttgtt 420

gctcagcagg gcgctctgcc ctccctcctt ccttcttgct tctaatagac ctggtacatg 480

gtacacacac ccc 493

<210> 41
<211> 365
<212> DNA
<213> Homo sapiens

<400> 41
ggaggatagg ataatcccgg gtggcatcta taacgcagac ctcaatgatg agtgggtaca 60

gcgtgccctt cacttcgcca tcagcgagta taacaaggcc accaaagatg actactacag 120

acgtccgctg cgggtactaa gagccaggca acagaccgtt ggggggtga attacttctt 180

cgacgtagag gtgggccgaa ccatatgtac caagtcccag cccaacttgg acacctgtgc 240

cttccatgaa cagccagaac tgcagaagaa acagttgtgc tctttcgaga tctacgaagt 300

tccctgggag aacagaaggt ccctggtgaa atccaggtgt caagaatcct agggatctgt 360

gccag 365

<210> 42
<211> 410
<212> DNA
<213> Homo sapiens

<400> 42
gagaagggcc tgatttgcag catcatgatg ggcctctcct tggcctctgc tgtgctcctg 60

gcctccctcc tgagtctcca ccttggaact gccacacgtg ggagtgacat atccaagacc 120

tgctgcttcc aatacagcca caagcccctt ccctggacct gggtgcgaag ctatgaattc 180

accagtaaca gctgctccca gcgggctgtg atattcacta ccaaaagagg caagaaagtc 240

tgtacccatc caaggaaaaa atgggtgcaa aaatacattt ctttactgaa aactccgaaa 300

caattgtgac tcagctgaat tttcatccga ggacgcttgg accccgctct tggctctgca 360

gccctctggg gagcctgcgg aatcttttct gaaggctaca tggacccgct 410

<210> 43
<211> 489
<212> DNA
<213> Homo sapiens

<400> 43
ggccaaatca ccgacctgaa ggcggaaatt cacgggggca gtctcattaa tctgacttgg 60

acagctcctg ggatgatta tgaccatgga acagctcaca agtatatcat tcgaataagt 120

acaagtattc ttgatctcag agacaagttc aatgaatctc ttcaagtgaa tactactgct 180

ctcatcccaa aggaagccaa ctctgaggaa gtctttttgt ttaaaccaga aaacattact 240

tttgaaaatg gcacagatct tttcattgct attcaggctg ttgataaggt cgatctgaaa 300

tcagaaatat ccaacattgc acgagtatct ttgtttattc ctccacagac tccgccagag 360

acacctagtc ctgatgaaac gtctgctcct tgtcctaata ttcatatcaa cagcaccatt 420

cctggcattc acattttaaa aattatgtgg aagtggatag gagaactgca gctgtcaata 480

gcctagggc 489

<210> 44
<211> 324
<212> DNA
<213> Homo sapiens

<400> 44
gagcccccag gaggaggaca ggataatcga gggtggcatc tatgatgcag acctcaatga 60

tgagcgggta cagcgtgccc ttcactttgt catcagcgag tataacaagg ccactgaaga 120

tgagtactac agacgcctgc tgcgggtgct acgagccagg gagcagatcg tgggcggggt 180

gaattacttc ttcgacatag aggtgggccg aaccatatgt accaagtccc agcccaactt 240

ggacacctgt gccttccatg aacagccaga actgcagaag aaacagttgt gctctttcca 300

gatctacgaa gttccctggg agga 324

<210> 45
<211> 310
<212> DNA
<213> Homo sapiens

<400> 45
aagactttac tttcaccata ccagatgtag aggactcaag tcagagacca gatcagggac 60

cccagagacc tcctcctgaa ggactcctac ctagaccccc tggtgatagt ggtaaccaag 120

atgatggtcc tcagcagaga ccaccaaaac caggaggcca tcaccgccat cctcccccac 180

ctccttttca aaatcagcaa cgaccacccc aacgaggaca ccgtcaactc tctctacccc 240

gatttccttc tgtcagcctg caggaagcat catcattctt ccggagggac agaccagcaa 300

gacatcccca 310

<210> 46
<211> 465
<212> DNA
<213> Homo sapiens

<400> 46
ttcctcaccc taaaactaag cgtgctgctt ctgcaaaaga tttttgtaga tgagctgtgt 60

gcctcagaat tgctatttca aattgccaaa aatttagaga tgttttctac atatttctgc 120

tcttctgaac aacttctgct acccactaaa taaaaacaca gaaataatta gacaattgtc 180

tattataaca tgacaaccct attaatcatt tggtcttcta aaatgggatc atgcccattt 240

agattttcct tactatcagt ttatttttat aacattaact tttactttgt tatttattat 300

tttatataat ggtgagtttt taaattattg ctcactgcct atttaatgta gctaataaag 360

ttatagaagc agatgatctg ttaatttcct atctaataaa tgcctttaat tgttctcata 420

atgaagaata agtaggtacc ctccatgccc ttctgtaata aatat 465

<210> 47
<211> 323
<212> DNA
<213> Homo sapiens

<400> 47
agaagactct gacctgtact cttgaataca agtttctgat accactgcac tgtctgagaa 60

tttccaaaac tttaatgaac taactgacag cttcatgaaa ctgtccacca agatcaagca 120

gagaaaataa ttaatttcat gggactaaat gaactaatga ggattgctga ttctttaaat 180

gtcttgtttc ccagatttca ggaaactttt tttcttttaa gctatccact cttacagcaa 240

tttgataaaa tatacttttg tgaacaaaaa ttgagacatt tacattttct ccctatgtgg 300

tcgctccaga cttgggaaac tat 323

<210> 48
<211> 500
<212> DNA
<213> Homo sapiens

<400> 48
tcatcgggcc tggcacaggc atcgcgccct tccgcagttt ctggcagcaa cggctccatg 60

actcccagca caagggagtg cggggaggcc gcatgacctt ggtgtttggg tgccgccgcc 120

cagatgagga ccacatctac caggaggaga tgctggagat ggcccagaag gggggtgctgc 180

atgcggtgca cacagcctat tcccgcctgc ctggcaagcc caaggtctat gttcaggaca 240

tcctgcggca gcagctggcc agcgaggtgc tccgtgtgct ccacaaggag ccaggccacc 300

tctatgtttg cggggatgtg cgcatggccc gggacgtggc ccacaccctg aagcagctgg 360

tggctgccaa gctgaaattg aatgaggagc aggtcgagga ctatttcttt cagctcaaga 420

gccagaagcg ctatcacgaa gatatctttg gtgctgtatt tccttacgag gcgaagaagg 480

acagggtggc ggtgcagccc 500

<210> 49
<211> 363
<212> DNA
<213> Homo sapiens

<400> 49
gtcgatttac acttacctcg gttcaaaatg gaagagagct atgacctcaa ggacacgttg 60

agaaccatgg gaatggtgaa tatcttcaat ggggatgcag acctctcagg catgacctgg 120

agccacggtc tctcagtatc taaagtccta cacaaggcct ttgtggaggt cactgaggag 180

ggagtggaag ctgcagctgc caccgctgta gtagtagtcg aattatcatc tccttcaact 240

aatgaagagt tctgttgtaa tcaccctttc ctattcttca taaggcaaaa taagaccaac 300

agcatcctct tctatggcag attctcatcc ccatagatgc aattagtctg tcactccatt 360

tag 363

<210> 50
<211> 508
<212> DNA
<213> Homo sapiens

<400> 50
gatacgacac tggttcttgt gaacgcaatc tatttcaaag ggcagtggga gaataaattt 60

aaaaaagaaa acactaaaga ggaaaaattt tggccaaaca aggatgtaca ggccaaggtc 120

ctggaaatac catacaaagg caaagatcta agcatgattg tgctgctgcc aaatgaaatc 180

gatggtctgc agaagcttga agagaaactc actgctgaga aattgatgga atggacaagt 240

ttgcagaata tgagagagac atgtgtcgat ttacacttac ctcggttcaa aatggaagag 300

agctatgacc tcaaggacac gttgagaacc atgggaatgg tgaatatctt caatggggat 360

gcagacctct caggcatgac ctggagccac ggtctctcag tatctaaagt cctacacaag 420

gcctttgtgg aggtcactga ggagggagtg gaagctgcag ctgccaccgc tgtagtagta 480

gtcgaattat catctccttc aactaatg 508

<210> 51
<211> 493
<212> DNA
<213> Homo sapiens

<400> 51
gcgagtacaa caaggccacc gaagatgagt actacagacg cccgctgcag gtgctgcgag 60

ccagggagca gacctttggg ggggtgaatt acttcttcga cgtagaggtg ggccgcacca 120

tatgtaccaa gtcccagccc aacttggaca cctgtgcctt ccatgaacag ccagaactgc 180

agaagaaaca gttatgctct ttcgagatct acgaagttcc ctgggaggac agaatgtccc 240

tggtgaattc caggtgtcaa gaagcctagg ggtctgtgcc aggccagtca caccgaccac 300

cacccactcc cacccctgt agtgctccca cccctggact ggtggccccc accctgcggg 360

aggcctcccc atgtgcctgt gccaagagac agacagagaa ggctgcagga gtcctttgtt 420

gctcagcagg gcgctctgcc ctccctcctt ccttcttgct tctaatagac ctggtacatg 480

gtacacacac ccc 493

<210> 52
<211> 447
<212> DNA
<213> Homo sapiens

<400> 52
ccacctcctc caggaaagcc agaaagacca cccccacaag gaggtaacca gtcccaaggt 60

cccccacctc atccaggaaa gccagaagga ccaccccccac aggaaggaaa caagtcccga 120

agtgcccgat ctcctccagg aaagccacaa ggaccacccc aacaagaagg caacaagcct 180

caaggtcccc cacctcctgg aaagccacaa ggcccacccc cagcaggagg caatccccag 240

cagcctcagg cacctcctgc tggaaagccc caggggccac ctccacctcc tcaagggggc 300

aggccaccca gacctgccca gggacaacag cctccccagt aatctaggat tcaatgacag 360

gaagtgaata agaagatatc agtgaattca aataattcaa ttgctacaaa tgccgtgaca 420

ttggaacaag gtcatcatag ctctaac 447

<210> 53
<211> 304
<212> DNA
<213> Homo sapiens

<400> 53
tgacgcaaaa taccaccttg gcgcctacac gggagacgac gtccgcatca tccgtgacga 60

catgctgtgt gccgggaaca gccagaggga ctcctgcaag ggcgactctg gagggcccct 120

ggtgtgcaag gtgaatggca cctggctaca ggcgggcgtg gtcagctggg acgagggctg 180

tgcccagccc aaccggcctg gcatctacac ccgtgtcacc tactacttgg actggatcca 240

ccactatgtc cccaaaaagc cgtgagtcag gcctgggtgt gccacctggg tcactggagg 300

acca 304

<210> 54
<211> 358
<212> DNA
<213> Homo sapiens

<400> 54
ccgccatttc ctctgaagca ggtgaaggtc cccataatgg aaaaccacat ttgtgacgca 60

aaataccacc ttggcgccta cacgggagac gacgtccgca tcgtccgtga cgacatgctg 120

tgtgccggga acacccggag ggactcatgc caggcgact ccggagggcc cctggtgtgc 180

aaggtgaatg gcacctggct gcaggcgggc gtggtcagct ggggcgaggg ctgtgcccag 240

cccaaccggc ctggcatcta cacccgtgtc acctactact tggactggat ccaccactat 300

gtccccaaaa agccgtgagt caggcctggg ttggccacct gggtcactgg aggaccaa 358

<210> 55
<211> 542
<212> DNA
<213> Homo sapiens

<400> 55
tgacgcaaaa taccaccttg gcgcctacac gggagacgac gtccgcatcg tccgtgacga 60

catgctgtgt gccgggaaca cccggaggga ctcatgccag ggcgactccg gagggcccct 120

ggtgtgcaag gtgaatggca cctggctgca ggcgggcgtg gtcagctggg gcgagggctg 180

tgcccagccc aaccggcctg gcatctacac ccgtgtcacc tactacttgg actggatcca 240

ccactatgtc cccaaaaagc cgtgagtcag gcctgggttg gccacctggg tcactggagg 300

accaacccct gctgtccaaa acaccactgc ttcctaccca ggtggcgact gccccccaca 360

ccttccctgc cccgtcctga gtgccccttc ctgtcctaag ccccctgctc tcttctgagc 420

cccttcccct gtcctgagga cccttcccta tcctgagccc ccttccctgt cctaagcctg 480

acgcctgcac cgggccctcc agccctcccc tgcccagata gctggtggtg ggcgctaatc 540

ct 542

<210> 56
<211> 536
<212> DNA
<213> Homo sapiens

<400> 56
tgacgcaaaa taccaccttg gcgcctacac gggagacgac gtccgcatcg tccgtgacga 60

catgctgtgt gccgggaaca cccggaggga ctcatgccag ggcgactccg gagggcccct 120

ggtgtgcaag gtgaatggca cctggctgca ggcgggcgtg gtcagctggg gcgagggctg 180

tgcccagccc aaccggcctg gcatctacac ccgtgtcacc tactacttgg actggatcca 240

ccactatgtc cccaaaaagc cgtgagtcag gcctgggttg gccacctggg tcactggagg 300

accaacccct gctgtccaaa acaccactgc ttcctaccca ggtggcgact gcccccccaca 360

ccttccctgc cccgtcctga gtgccccttc ctgtcctaag ccccctgctc tcttctgagc 420

cccttcccct gtcctgagga cccttcccca tcctgagccc ccttccctgt cctaagcctg 480

acgcctgcac cgggccctcc ggccctcccc tgcccaggca gctggtggtg ggcgct 536

<210> 57
<211> 538
<212> DNA
<213> Homo sapiens

<400> 57
ccggtcagca ggatcatcgt gcacccacag ttctacatca tccagactgg agcggatatc 60

gccctgctgg agctggagga gcccgtgaac atctccagcc gcgtccacac ggtcatgctg 120

ccccctgcct cggagacctt cccccggggg atgccgtgct gggtcactgg ctggggcgat 180

gtggacaatg atgagcccct cccaccgcca tttcccctga agcaggtgaa ggtccccata 240

atggaaaacc acatttgtga cgcaaaatac caccttggcg cctacacggg agacgacgtc 300

cgcatcatcc gtgacgacat gctgtgtgcc gggaacaccc ggagggactc atgccagggc 360

gactctggag ggcccctggt gtgcaaggtg aatggcacct ggctacaggc gggcgtggtc 420

agctgggacg agggctgtgc ccagcccaac cggcctggca tctacacccg tgtcacctac 480

tacttggact ggatccacca ctatgtcccc aaaaagccgt gagtcaggcc tggggtgt 538

<210> 58
<211> 366
<212> DNA
<213> Homo sapiens

<400> 58
ccggtcagca ggatcatcgt gcacccacag ttctacaccg cccagatcgg agcggacatc 60

gccctgctgg agctggagga gccggtgaac gtctccagcc acgtccacac ggtcaccctg 120

ccccctgcct cagagacctt ccccccgggg atgccgtgct gggtcactgg ctggggcgat 180

gtggacaatg atgagcgcct cccaccgcca tttcctctga agcaggtgaa ggtccccata 240

atggaaaacc acatttgtga cgcaaaatac caccttggcg cctacacggg agacgacgtc 300

cgcatcgtcc gtgacgacat gctgtgtgcc gggaacaccc ggagggactc atgccaggtg 360

gcgact 366

<210> 59
<211> 496
<212> DNA
<213> Homo sapiens

<220>
<221> modified_base
<222> (150)..(151)
<223> a, c, g, t, unknown or other

<220>
<221> modified_base
<222> (345)..(345)
<223> a, c, g, t, unknown or other

<220>
<221> modified_base
<222> (347)..(348)
<223> a, c, g, t, unknown or other

<220>
<221> modified_base
<222> (366)..(366)
<223> a, c, g, t, unknown or other

<220>
<221> modified_base
<222> (405)..(405)
<223> a, c, g, t, unknown or other

<220>
<221> modified_base
<222> (428)..(428)
<223> a, c, g, t, unknown or other

<400> 59
ccggtcagca ggatcatcgt gcacccacag ttctacatca tccagactgg agcggatatc 60

gccctgctgg agctggagga gcccgtgaac atctccagcc gcgtccacac ggtcatgctg 120

ccccctgcct cggagacctt ccccccgggn ntgccgtgct gggtcactgg ctggggcgat 180

gtggacaatg atgagcccct cccaccgcca tttcccctga agcaggtgaa ggtccccata 240

atggaaaacc acatttgtga cgcaaaatac caccttggcg cctacacggg agacgacgtc 300

cgcatcatcc gtgacgacat gctgtgtgcc gggaacaccc ggagngnntc atgccagggc 360

gactcnggag ggcccctggt gtgcaaggtg aatggcacct ggctncaggc gggcgtggtc 420

agctgggncg agggctgtgc ccagcccaac cggcctggca tctacacccg tgtcacctac 480

tacttggact ggatcc 496

<210> 60
<211> 408
<212> DNA
<213> Homo sapiens

<400> 60
gtcgtcacgg acgatgcgga cgtcgtctcc cgtgtaggcg ccaaggtggt attttgcgtc 60

acaaatgtgg ttttccatta tggggacctt cacctgcttc agaggaaatg gcggtgggag 120

gcgctcatca ttgtccacat cgccccagcc agtgacccag cacggcatcc ccggggggaa 180

ggtctctgag gcaggggca gggtgaccgt gtggacgtgg ctggagacgt tcaccggctc 240

ctccagctcc agcagggcga tgtccgctcc gatctgggcg gtgtagaact gtgggtgcac 300

gatgatcctg ctgaccggca gcagctggtc ctggtagtag aggtgctgct cccgcagttg 360

caccggtccc acgcagtgcg ctgcggtcag cacccactgg gggtggat 408

<210> 61
<211> 520
<212> DNA
<213> Homo sapiens

<400> 61
ggtgaaagtc tccgtggtgg acacagagac ctgccgccgg gactatcccg gccccggggg 60

cagcatcctt cagcccgaca tgctgtgtgc ccggggcccc ggggatgcct gccaggacga 120

ctccgggggg cctctggtct gccaggtgaa cggtgcctgg gtgcaggctg gcattgtgag 180

ctggggtgag ggctgcggcc gccccaacag gccgggagtc tacactcgtg tccctgccta 240

cgtgaactgg atccgccgcc acatcacagc atcagggggc tcagagtctg ggtaccccag 300

gctcccccctc ctggctggct tattcctccc cggcctcttc cttctgctag tctcctgtgt 360

cctgctggcc aagtgcctgc tgcacccatc tgcggatggt actcccttcc ccgcccctga 420

ctgatggcag gaatccaagt gcatttctta aataagttac tatttattcc gctccgcccc 480

ctccctctcc cttgagaagc tgagtcttct gcatcagatt 520

<210> 62
<211> 508
<212> DNA
<213> Homo sapiens

<400> 62
tgccacaagg tcgctgctta tgagggcgca aacttcttgg cttgtgctcg gaccctttc 60

tcgtactcca ctctgttttg gcagtaaatc gtgtaggcct ctgcttgagc tgggtcttgg 120

atatttggtt catttagaag ttcctgtatt cctaatagga tctgtttgat tgtgatggct 180

ggcctccagt ccttgtcctc ctctaagatg gacaggcaca ctgtccccga agggtacaca 240

ttcgggtgaa ataatggtgg ttcgaattta cattttggtg gcgaagatgg ataatcatct 300

ttgaaaagca tccgtagttt aaacaagcct ccttcccacg gagtcccttt ctttcctgga 360

atggcgcact cccagttcat gaggttcatc gtgccatcgg gatttttgt tgggacagcc 420

acgaaaccaa atgggtggtc tttcctccat gctttcctct cctgggcgag tctgctgagg 480

gcgatccccg acatgttcaa agtccctc 508

<210> 63
<211> 510
<212> DNA
<213> Homo sapiens

<400> 63
agagactttc agggcatacg tgggggcctt ggccttcctc actcgctcga tggcctcagt 60

gtgctcctca aggctggtgc caaacacctg ctggagatag ctgagcaggg cctcctcgtc 120

gtccacctgg tcagggccca tggtacccgc gcggtaaagc accgtgtaca gggcctcctc 180

gtagagcatc tccacctcct ctggggccag ggctctcagg ccgaggctgg gatccacagg 240

ctccgggggt gctggcgagc cactgcgcag ggggacctcg aggcacggca agccctgtct 300

gccttccccc ttcttcagca tgaggcgcat gtgggcaaag aactccacgc catccccggg 360

tttccaggcc cccgtggcag gctcctgcgg gtcggcgctg gcactccctg ggtcctgctc 420

agtcctgcgg cggaaggacg ggcacacctg cacctgcctg agcacgctgc tcttaatgtc 480

cagcaaggtc gacatggcgg gtgaccgtgg 510

<210> 64
<211> 407
<212> DNA
<213> Homo sapiens

<400> 64
tgctgctctt caaaatgtac cccattgatg aggagaggcg gcggcagaat aagaaggccc 60

tgcaggcact gagggacgag gccagcagct ctggctgctc agaaacagac tccacagagc 120

tggctagcat cctctagggc ccgccacgtt gcccgaagcc accatgcaga aggccacaga 180

agggatcagg acctgtctgc cggcttgctg agcagctgga ctgcaggtgc taggaaggga 240

actgaagact caaggaggtg gcccaggaca cttgctgtgc tcactgtggg gccggctgct 300

ctgtggcctc ctgcctcccc tctgcctgcc tgtggggcca agccctgggg ctgccactgt 360

gaatatgcca aggactgatc gggcctagcc cggaacacta atgtaga 407

<210> 65
<211> 565
<212> DNA

<213> Homo sapiens

<400> 65
tatgaaaccc gctacatcta tggcccaata gaatcaacaa tttacccgat atcaggttct 60

tctttagact gggcttatga cctgggcatc aaacacacat ttgcctttga gctccgagat 120

aaaggcaaat ttggttttct ccttccagaa tcccggataa agccaacgtg cagagagacc 180

atgctagctg tcaaatttat tgccaagtat atcctcaagc atacttccta aagaactgcc 240

ctctgtttgg aataagccaa ttaatccttt tttgtgcctt tcatcagaaa gtcaatcttc 300

agttatcccc aaatgcagct tctatttcac ctgaatcctt ctcttgctca tttaagtccc 360

atgttactgc tgtttgcttt tacttacttt cagtagcacc ataacgaagt agctttaagt 420

gaaacctttt aactaccttt ctttgctcca agtgaagttt ggacccagca gaaagcatta 480

ttttgaaagg tgatatacag tggggcacag aaaacaaatg aaaaccctca gtttctcaca 540

gattttcacc atgtggcttc atcaa 565

<210> 66
<211> 459
<212> DNA
<213> Homo sapiens

<400> 66
tgagcctggg gttctggtgt tagaatattt ttaagtaggc tttactgaga gaaactaaat 60

attggcatac gttatcagca acttcccctg ttcaatagta tgggaaaaat aagatgactg 120

ggaaaaagac acacccacac cgtagaacat atattaatct actggcgaat gggaaaggag 180

accattttct tagaaagcaa ataaacttga ttttttaaa tctaaaattt acattaatga 240

gtgcaaaata acacataaaa tgaaaattca cacatcacat ttttctggaa aacagacgga 300

ttttacttct ggagacatgg catacggtta ctgacttatg agctaccaaa actaaattct 360

ttctctgcta ttaactggct agaagacatt catctatttt tcaaatgttc tttcaaaaca 420

tttttataag taatgtttgt atctatttca tgctttact 459

<210> 67
<211> 430
<212> DNA
<213> Homo sapiens

<220>
<221> modified_base
<222> (343)..(345)
<223> a, c, g, t, unknown or other

<220>
<221> modified_base
<222> (347)..(347)
<223> a, c, g, t, unknown or other

<220>
<221> modified_base
<222> (349)..(352)
<223> a, c, g, t, unknown or other

<400> 67
gggaatcact attcagggat ttttcccctt tgctcttctt ttccctcctt aaaagaaaaa 60

ttaccttcta gtcctaggat gaggacacac tattagtttg aattaaatgc tttgatattc 120

tcagatcagc catcttgaac caaagcaaaa ccacaagtta cactttctta aaatttgatt 180

tgtcatattt tctagagaaa cttgaattta attgtgttat tcttagcttc cactggcagc 240

ctagctttga gggtaaatga aaatataacc catagattac ccagccactt gggaacagca 300

ggtaatactg aagaaaaata aaaatagatt ttgaaaacgt tannnanann nntatgatta 360

tgattctgtt ccatttaagg gaaaacttag gtaaatagag aaatttttttc tataacattg 420

tgtagtcagt 430

<210> 68
<211> 358
<212> DNA
<213> Homo sapiens

<400> 68
tgcttctgga cacctgggac caggtctttg tctgggttgg aaaggattct caagaagaag 60

aaaagacaga agccttgact tctgctaagc ggtacatcga gacggaccca gccaatcggg 120

atcggcggac gcccatcacc gtggtgaagc aaggctttga gcctccctcc tttgtgggct 180

ggttccttgg ctgggatgat gattactggt ctgtggaccc cttggacagg gccatggctg 240

agctggctgc ctgaggaggg gcagggccca cccatgtcac cggtcagtgc cttttggaac 300

tgtccttccc tcaaagaggc cttagagcga gcagagcagc tctgctatga gtgtgtgt 358

<210> 69
<211> 506
<212> DNA
<213> Homo sapiens

<220>
<221> modified_base
<222> (86)..(86)
<223> a, c, g, t, unknown or other

<220>
<221> modified_base
<222> (118)..(118)
<223> a, c, g, t, unknown or other

<400> 69
acttttgacc acttgtgact ggagttcagt ggccctggca ggcttgtcct gctcttgacc 60

attccactga ctaactttgg tgtttngttt ccaagttaag tgattcctcc tttttttngt 120

tcaatgttaa atttaaaaat aacaatgtgt atgggtcctc ccatgtgtaa tatggtaaca 180

tgtaacttgc agtgtttgcc agctttcaaa gcaggctttg tgaaaatgta atacaaacag 240

cagtgaatgg gactcaaatg ttgtgcttcc tataaacagc tccgctcttt cagggaagga 300

tggtaacaaa ctagaaggac aaatatgtac gtatttataa cgtattaaaa ctcttttaag 360

tagcttaagg tattgtgcaa tggcctagcc tagtagaaat gggggaaaag cattgctgtg 420

gaccattgtt aaagtgacag gagttgtagg gttacccctt tgacaagctt ccatagtctt 480

cagacacgca cattgatggc atccct 506

<210> 70
<211> 496
<212> DNA
<213> Homo sapiens

<220>
<221> modified_base
<222> (60)..(60)
<223> a, c, g, t, unknown or other

<220>
<221> modified_base
<222> (75)..(75)
<223> a, c, g, t, unknown or other

<220>
<221> modified_base
<222> (114)..(114)
<223> a, c, g, t, unknown or other

<220>
<221> modified_base
<222> (131)..(131)
<223> a, c, g, t, unknown or other

<220>
<221> modified_base
<222> (138)..(138)
<223> a, c, g, t, unknown or other

<220>
<221> modified_base
<222> (141)..(141)
<223> a, c, g, t, unknown or other

<220>
<221> modified_base
<222> (159)..(159)
<223> a, c, g, t, unknown or other

<220>
<221> modified_base
<222> (175)..(175)
<223> a, c, g, t, unknown or other

<220>
<221> modified_base

<222> (203)..(203)
<223> a, c, g, t, unknown or other

<220>
<221> modified_base
<222> (207)..(207)
<223> a, c, g, t, unknown or other

<400> 70
ctaactaata ccaacctgac aacttgaata acaataaatg caatttgtac ataaaatatn 60

atgctgcaaa agttngtcat tcacctcagt ggagtgactt gatattaggt ggtnaccgta 120

gatgatggtt natatganaa ntggacagga aagaagcant ttctgaaagt tatantcttt 180

tgaaccacgt tctaaaccaa gtntttnatc ttcttggggc tcgtaattac ctttcacttt 240

aatgtcactt aaagatataa cacagaaaaa tgccttgagg gcaaaatata ggcaaaacac 300

caatgcgctt tcaaatgcat gaaaatggtg cagttgtacc cttgagcctt gactcaaggg 360

ctgtagatgt tccctttcca ccccccacac ttggtgcgtg ttcacaaagc aaatatggcc 420

tgtaattcaa atttgttcta tgtgatactc tctgagtaaa aactcataca tgcagaaaat 480

tgtctttgct cgaaat 496

<210> 71
<211> 560
<212> DNA
<213> Homo sapiens

<400> 71
cccaagcccc tagagaagtc agtctccccg caaaattcag attcttcagg ttttgcagat 60

gtgaatggtt ggcaccttcg tttccgctgg tccaaggatg ctccctcaga actcctgagg 120

aagttcagaa actatgaaat atgaaatatc tctgcttcaa aaaatgagga agagcaagac 180

tgtcccctat gctgccaaca tgcagtcttt gtttatgtct taaaaatgtc atgtttatgt 240

catgtctgtg aattgctgag tactaattga ttcctccatc cttgaatcag ttctcataat 300

gctttttaaa taagaaaaat tcagaagatg aatttcttcc aatatttgaa taaattaaag 360

ctcttagata cagagtagat tgtattatat gctttttcct attaatacta cttatagaaa 420

tccattaaaa agcaatctct gtacagtgta tttaaatatt tcattgacat actgtgatct 480

ctattagtga tggatgtaca aaaaatgttt tcttaccctt gacttacaat gaaatgtgaa 540

attacttgtc tgaaccccgt 560

<210> 72
<211> 512
<212> DNA
<213> Homo sapiens

<220>
<221> modified_base

<222> (443)..(443)
<223> a, c, g, t, unknown or other

<400> 72
acagcaagcc tatgtagttc aattaatata taaggaaaag gaaggtcttt cttcatgata 60

caagcattat aaagtttttta ctgtagtagt caattaatgg atatttcctt gttaataaaa 120

ttttgtgtca taatttacaa attagttctt taaaaattgt tgttatatga attgtgtttc 180

tagcatgaat gttctataga gtactctaaa taacttgaat ttatagacaa atgctactca 240

cagtacaatc aattgtatta taccatgaga aaatcaaaaa ggtgttcttc agagacattt 300

tatctataaa attttcctac tattatgttc attaacaaac ttctttatca catgtatctt 360

ctacgtgtaa aacatttctg atgatttttt aacaaaaaat atatgaattt cttcatttgc 420

tcttgcatct acattgctat aanggatata aaatgtggtt tctatatttt gagatgtttt 480

ttccttacaa tgtgaactca tcgtgatctt gg 512

<210> 73
<211> 551
<212> DNA
<213> Homo sapiens

<220>
<221> modified_base
<222> (114)..(114)
<223> a, c, g, t, unknown or other

<220>
<221> modified_base
<222> (119)..(120)
<223> a, c, g, t, unknown or other

<220>
<221> modified_base
<222> (127)..(127)
<223> a, c, g, t, unknown or other

<220>
<221> modified_base
<222> (129)..(129)
<223> a, c, g, t, unknown or other

<220>
<221> modified_base
<222> (149)..(149)
<223> a, c, g, t, unknown or other

<220>
<221> modified_base
<222> (152)..(152)
<223> a, c, g, t, unknown or other

<220>
<221> modified_base
<222> (163)..(163)
<223> a, c, g, t, unknown or other

<220>
<221> modified_base
<222> (167)..(168)
<223> a, c, g, t, unknown or other

<220>
<221> modified_base
<222> (189)..(189)
<223> a, c, g, t, unknown or other

<220>
<221> modified_base
<222> (243)..(243)
<223> a, c, g, t, unknown or other

<400> 73
ctgctacttt ggaagatggc tctggaggaa actctcatat ggctaaaaag gcaggctagt 60

ttcttacttc tacaggggta gagccttaaa aaagaacgtg ctacaaattg gttntcttnn 120

agggttncng gttctccctg cccccaatnc cnatatactt tantgcnntt ttatttttgc 180

ctttacggnc tctgtgtctt tctgcaagaa ggcctggcaa aggtatgcct gctgttggtc 240

ccntcgggat aagataaaat ataaataaaa ccttcagaac tgttttggag caaaagatag 300

cttgtacttg gggaaaaaaa ttctaagttc ttttatatga ctaatattct tggttagcaa 360

gactggaaag aggtgttttt ttaaaatgta cataccagaa caaagaacat acagctctct 420

gaacatttat tttttgaaca gaggtggttt ttatgtttgg acctggtaat acagatacaa 480

aaactttaat gaggtagcaa tgaatattca actgtttgac tgctaagtgt atctgtccat 540

attttagcaa g 551

<210> 74
<211> 474
<212> DNA
<213> Homo sapiens

<220>
<221> modified_base
<222> (365)..(365)
<223> a, c, g, t, unknown or other

<220>
<221> modified_base
<222> (429)..(429)
<223> a, c, g, t, unknown or other

<400> 74
tactacaaaa gccgtgacct cagcctgctt atactactgc cagaagacat taatgggctg 60

gaacagctgg aaaaggccat cacctatgag aagctgaatg agtggaccag tgcagacatg 120

atggagttgt atgaagtgca gctacacctt cccaagttca agctggaaga cagttatgat 180

ctcaagtcaa ccctgagcag tatggggatg agtgatgcct tcagccaaag caaagctgat 240

ttctcaggaa tgtcttcagc aagaaaccta tttttgtcca atgttttcca taaggctttt 300

gtggaaataa atgaacaagg tactgaagct gcagctggca gtgggagtga gatagatata 360

cgaantagag tcccatccat tgaattcaat gcaaatcacc cattcctctt cttcatcagg 420

cacaataana accaacacca ttcttttta tggaagatta tgctccccct aatc 474

<210> 75
<211> 287
<212> DNA
<213> Homo sapiens

<400> 75
gattctgtgg tagactcagt gctttcagag tccagagctt gacttgggtt agtggcctta 60

atgaagtgct aaatttgctc tttaccgcga gactgatcag aagaagcaaa aggggaaagg 120

gggctagagg tccactcgca ccttttacat cagacaagag gaggactgtg ccagaaatct 180

gtgcatgaaa caccatctgc tcttcatgca gggaggggtc aaccgtgtga acgtgcagag 240

attactcgag ccttctttgc caaaaatatg cattcttccc agctgta 287

<210> 76
<211> 545
<212> DNA
<213> Homo sapiens

<400> 76
taatcacatc acttccatgg catggatgtt cacatacaga ctcttaaccc tggtttacca 60

ggacctctag gagtggatcc aatctatatc tttacagttg tatagtatat gatatctctt 120

ttatttcact caatttatat tttcatcatt gactacatat ttcttataca caacacacaa 180

tttatgaatt ttttctcaag atcattctga gagttgcccc accctacctg ccttttatag 240

tacgcccacc tcaggcagac acagagcaca atgctggggt tctcttcaca ctatcactgc 300

cccaaattgt ctttctaaat ttcaacttca atgtcatctt ctccatgaag accactgaat 360

gaacaccttt tcatccagcc ttaatttctt gctccataac tactctatcc cacgatgcag 420

tattgtatca ttaattatta gtgtgcttgt gacctcctta tgtattctca attacctgta 480

tttgtgcaat aaattggaat aatgtaactt gatttcttat ctgtgtttgt gttggcatgc 540

aagat 545

<210> 77
<211> 420
<212> DNA
<213> Homo sapiens

<400> 77
aagacacttc ttccaaacct tgaatttgtt gtttttagaa aacgaatgca tttaaaaata 60

ttttctatgt gagaattttt tagatgtgtg tttacttcat gtttacaaat aactgtttgc 120

```
tttttaatgc agtactttga aatatatcag ccaaaaccat aacttacaat aatttcttag      180

gtattctgaa taaaattcca tttcttttgg atatgcttta ccattcttag gtttctgtgg      240

aacaaaaata tttgtagcat tttgtgtaaa tacaagcttt catttttatt ttttccaatt      300

gctattgccc aagaattgct ttccatgcac atattgtaaa aattccgctt tgtgccacag      360

gtcatgattg tggatgagtt tactcttaac ttcaaaggga ctatttgtat tgtatgttgc      420


<210> 78
<211> 534
<212> DNA
<213> Homo sapiens


<220>
<221> modified_base
<222> (486)..(493)
<223> a, c, g, t, unknown or other

<400> 78
agtattgaca actgcacatg aaagttttgc aaagggaaac aggctaaatg caccaagaaa       60

gcttcttcag agtgaagaat cttaatgctt gtaatttaaa catttgttcc tggagttttg      120

atttggtgga tgtgatggtt ggttttattt gtcagtttgg ttgggctata gcacacagtt      180

atttaatcaa acagtaatct aggtgtggct gtgaaggtat tttgtagatg tgattaacat      240

ctacaatcag ttgactttaa gtgaaagaga ttacttaaat aatttgggtg agctgcacct      300

gattagttga aaggcctcaa gaacaaacac tgcagtttcc tggaaaagaa gaaactttgc      360

ctcaagacta tagccatcga ctcctgcctg agtttccagc ctgctagtct gccctatgga      420

tttgaagttt gccaacccca acaattgtgt gaattaattt ctaaaaataa agctatatac      480

agccannnnn nnntatttgt gggggatttg tttcaggatc tctacagata ccaa            534
```